# EUROPEAN PATENT APPLICATION

(11) **EP 4 442 691 A1**
(43) Date of publication of application: **09.10.2024**
(21) Application number: 22901745.4
(22) Date of filing: 30.11.2022
(51) Int. Cl.: C07D 498/22, A61K 31/53, A61P 35/00, C07D 498/18, C07D 487/22, C07D 471/22, C07D 487/04

(54) **2,7-SUBSTITUTED PYRROLO[2,1-F][1,2,4]TRIAZINE COMPOUND HAVING PROTEIN KINASE INHIBITORY ACTIVITY**

(30) Priority: 30.11.2021 KR 20210169177; 29.11.2022 KR 20220162449
(71) Applicant: Magicbullettherapeutics Co., Ltd., Seoul 03722 (KR)
(72) Inventor: SIM, Tae Bo, Seoul 06635 (KR); SHIN, In Jae, Seoul 04341 (KR); SANDIP, Sengupta, Seoul 02793 (KR); KIM, Nam Kyoung, Seoul 03782 (KR); KIM, Young Hoon, Seoul 03724 (KR); CHOI, Ha Soon, Seoul 03764 (KR)
(74) Representative: Mewburn Ellis LLP
(86) International application number: PCT/KR2022/019157
(87) International publication number: WO 2023/101387

(57) **Abstract**

The present invention relates to a 2,7-substituted pyrrolo[2,1-f][1,2,4]triazine compound having a protein kinase inhibitory activity, a pharmaceutically acceptable salt thereof, and a pharmaceutical composition for the prevention, alleviation, or treatment of diseases induced by abnormal cell growth containing the compounds as active ingredients. The novel cyclic 2,7-substituted pyrrolo[2,1-f][1,2,4]triazine compounds of the present invention exhibit excellent inhibitory effects on various protein kinases involved in growth factor signaling pathways. Therefore, they are useful as preventives, alleviators, or therapeutics for diseases characterized by abnormal cell growth induced by these protein kinases.

## Description

### Technical Field

The present invention relates to a 2,7-substituted pyrrolo[2,1-f][1,2,4]triazine compound possessing a protein kinase activity, a pharmaceutically acceptable salt, and a pharmaceutical composition for prevention and treatment of diseases caused by abnormal cell growth, including the compound as an effective ingredient.

### Background Art

Protein kinases in normal cells are in a balanced state between active and inactive states and are involved in cellular phenomena (signal transduction, transport, secretion, growth, division, cycle, etc.). When protein kinases are overactivated due to genetic mutation, amplification, or overexpression, the cell signaling system becomes excessively activated. This aberrant signaling cascade promotes the development, growth, and metastasis of cancer cells. Representative examples for kinases that cause the development or growth of cancer cells when abnormally regulated include EGFR, ALK, DDR, BRAF, FGFR, VEGFR, BTK, MET, AKT, HER2, RET, NTRK, mTOR, MEK, ROS1, FLT3, c-kit, ABL1, and PDGFR.

Acute Myeloid Leukemia (AML) is the most common type of leukemia, in which myeloblasts abnormally proliferate in the bone marrow or peripheral blood (accounting for more than 20%). This abnormal proliferation leads to a decrease in hematopoietic cells, resulting in compromised hematopoietic function. It is known that 25-30% of AML patients exhibit mutations in the FLT3 gene, specifically characterized by internal tandem duplication (FLT3-ITD). In addition, point mutations (F691L, D835Y, etc.) occurring within the kinase domain of FLT3 have been continuously reported. It is widely known that thses mutations cause continuous autophosphorylation of FLT3, leading to hyperactivation of signaling pathways related to cancer cell proliferation and division, thereby contributing to the pathogenesis of AML.

Lung cancer refers to a malignant tumor in the lung and is known to be the foremost contributor to cancer-related death worldwide. Depending on histological type, it is divided into small cell lung cancer and non-small cell lung cancer. Non-small cell lung cancer, comprising about 85% of cases, constitutes the predominant subtype among lung cancer patients. While surgery can be a potential therapeutic option for patients with non-small cell lung cancer in its early stages, the absence of discernible symptoms during this critical period complicates early diagnosis and resulting in a lower proportion of patients eligible for surgical intervention. Moreover, despite successful initial surgery in the early stages, non-small cell lung cancer often exhihibits high recurrence rates. Furthermore, the metastatsis to other organs such as the brain, bones, liver, and lymph nodes occurs more frequently compared to other carcinomas. Even when treated with concurrent chemotherapy or radiation therapy, non-small cell lung cancer is notorious for its poor progness relative to other cancer types. The 5-year survival rate remains disamlly low, typically less than 10%.

EGFR (Epidermal Growth Factor Receptor) is a tyrosine kinase receptor known to be a primary factor in the pathogenesis of non-small cell lung cancer. EGFR mutations are prevalent among non-small cell lung cancer patients, with rates ranging from 30-40% in Asia to 10-15% in the US and Europe. Approximately 95% of EGFR mutations result in EGFR activation independent of ligand binding. Notably, mutations in EGFR exon 19 are the most prevalent, with a frequency of 45%, while a point mutation in exon 21 (L858R) accounts for an additional 40% of cases.

Breast cancer refers to a malignant tumor in the breast. Breast cancer is the most common female cancer in the world, accounting for 24.2% of all female cancers, and has the highest mortality rate, affecting approximately 15% of those diagnosed. The incidence of breast cancer is on a steady rise in Korea and worldwide. In 2019, the number of breast cancer cases surged by approximately 41% compared to 2015, with 222,014 individuals diagnosed in 2019 compared to 156,533 in 2015. In particular, triple-negative breast cancer (TNBC), an aggressive breast cancer, accounts for 10-20% of all breast cancers, and the mortality rate for triple-negative breast cancer in the United States is approximately 50%.

Colorectal cancer ranks third among all cancers in terms of public awareness (6.1%) and second in terms of mortality rate (9.25%). The total number of deaths from rectal and colon cancer is expected to increase by 60% and 71.5%, respectively, by 2035. In 2020 alone, there were over 1.9 million new cases of colorectal cancer.

Prostate cancer remains a significant global health concern, with approximately 1.3 million new cases diagnosed annually worldwide. Currently, over 10 million men are 1iving with prostate cancer, among whom around 700,000 are anticipated to have metastatic prostate cancer. This aggressive form of the disease contributes to over 400,000 deaths each year and it is anticipated that the death rate from metastatic prostate cancer will be more than double by 2040.

Gastric cancer ranks as the fourth leading cause of cancer-related deaths globally, with a median survival rate of less than 12 months for advanced stages. While the average age of diagnosis for gastric cancer is 70 years old, it is noteworthy that approximately 10% of cases are detected in individuals under the age of 45.

Liver cancer is predicted to have a prevalence of over 1 million cases worldwide by 2025. Hepatocellular carcinoma (HCC), being the most prevalent type of liver cancer, accounts for approximately 90% of all liver cancers. The main cause of hepatocellular carcinoma is hepatitis B virus and hepatitis C virus infection, but non-alcoholic steatohepatitis and metabolic syndrome related to diabetes are also considered as potential risk factors for liver cancer.

Accordingly, the present inventors conducted extensive research aimed at developing novel compounds capable of overcoming the aforementioned conditions, leading to the completion of the present invention.

### Related Art Document

### Patent Document

WO 2010-071885
WO 2014-193932

### Non-patent Document

Org. Lett. 2011, 13, 4204-4207
J. Med. Chem. 2011, 54, 6328-6341
J. Med. Chem. 2012, 55, 115-125

### Disclosure of the Invention

### Technical Goals

The purpose of the present invention is to provide a novel 2,7-substituted pyrrolo[2,1-f][1,2,4]triazine compound possessing inhibitory activities against protein kinases.

The another purpose of the present invention is to provide a pharmaceutical composition for treatment, prevention, and alleviation of cancer diseases, comprising the novel 2,7-substituted pyrrolo[2,1-f][1,2,4]triazine compound, a pharmaceutically acceptable salt thereof, a hydrate thereof, a solvate thereof, or a stereoisomer thereof as an effective ingredient.

In addition, the present invention is directed to providing cancer treatment, comprising the novel 2,7-substituted pyrrolo[2,1-f][1,2,4]triazine compound, a pharmaceutically acceptable salt thereof, a hydrate thereof, a solvate thereof, or a stereoisomer thereof as an effective ingredient.

In one aspect, the present invention provides a method for the preparation of the novel compound.

In one aspect, the present invention provides a novel intermediate compound synthesized during the process of carrying out the method of preparation.

### Technical Solutions

In one aspect, the present invention provides a 2,7-substituted pyrrolo[2,1-f][1,2,4]triazine compound represented by Chemical Formula 1, a pharmaceutically acceptable salt thereof, a hydrate thereof, or a hydrate thereof, or a solvate thereof: 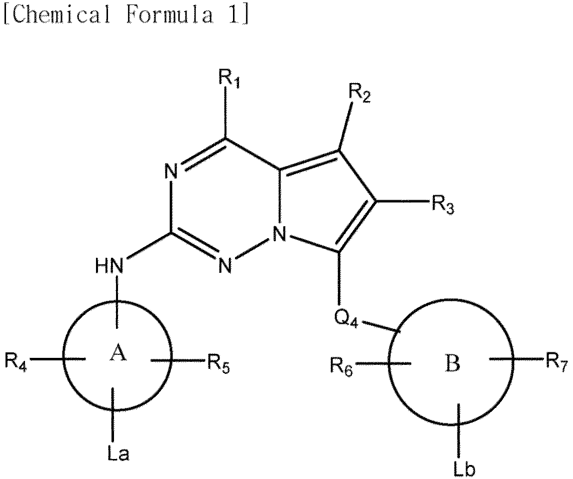 wherein
R₁, R₂, and R₃ each independently represent hydrogen; hydroxy; halogen; C₁-C₁₃ alkyl; C₁-C₆ alkoxy; C₃-C₁₀ cyclyl; C₃-C₁₀ heterocyclyl; -C(O)-(C₁-C₁₃ alkyl); amino(-NR₈R₉); nitro(-N(O)₂); amide(-(C=O)NR₈R₉); ester(-C(O)OR₈); carboxylic acid(-C(0)0H); nitrile(-CN); sulfonamide(-NHS(O)₂R₈); sulfide(-SR₈); sulfonyl(-S(O)₂R₈);or phosphinyl(-P(O)R₈R₉);
A and B each independently represent C₆-C₁₀ aryl; C₃-C₁₀ cyclyl; C₃-C₁₀ heteroaryl; or C₃-C₁₀ heterocyclyl;
R₄, R₅, R₆, and R₇ each independently represent hydrogen; hydroxy; halogen; C₁-C₁₃ alkyl; C₁-C₆ alkoxy; C₃-C₁₀ cyclyl; C₃-C₁₀ heterocyclyl; -C(0)-(C₁-C₁₃ alkyl); amino(-NR₈R₉); nitro(-N(0)₂); amide(-(C=O)NR₈R₉); ester(-C(O)OR₈); carboxylic acid(-C(0)0H); nitrile(-CN); sulfonamide(-NHS(O)₂R₈); sulfide(-SR₈); sulfonyl(-S(O)₂R₈);or phosphinyl(-P(O)R₈R₉);
Lₐ represents hydrogen; hydroxy; halogen; C₁-C₁₃ alkyl; -Zₐ-Q₃-E; or - Zₐ-Q₁-M, wherein in -Zₐ-Q₁-M, when L_{b} is -Z_{b}-Q₂, M is connected to Q₂, therby forming a ring;
L_{b} represents hydrogen; hydroxy; halogen; C₁-C₁₃ alkyl; C₁-C₆ alkoxy; C₃-C₁₀ cyclyl; C₃-C₁₀ heterocyclyl; -C(O)-(C₁-C₁₃ alkyl); amino(-NR_{8R9}); nitro(-N(O)₂); amide(-(C=O)NR₈R₉); ester(-C(O)OR₈); carboxylic acid(-C(0)0H); nitrile(-CN); sulfonamide(-NHS(O)₂R₈); sulfide(-SR₈); sulfonyl(-S(O)₂R₈);or-Z_{b}-Q₂, wherein in -Z_{b}-Q₂, when Lₐ is -Zₐ-Q₁-M, Q₂ is connected to M, therby forming a ring;
Zₐ or Z_{b} may each independently be present or absent, and if Zₐ or Z_{b} is present, Zₐ or Z_{b} each independently represents -0-, -CO-, -C00-, -CₙHₙ+₂-, -O(CₙHₙ₊₂)-, -(CₙHₙ₊₂)O-, -(CₙHₙ₊₂)O(CₘHₘ₊₂)-, -C₃-C₁₀ cyclyl-; or -C₃-C₁₀ heterocyclyl;
Q₁, Q₂, and Q₃ each independently represent -CₙHₙ₊₂-, -O(CₙHₙ₊₂)-, - (OC₂H₄)ₙ-, -(C₂H₄O)ₙ-, -(CₙHₙ₊₂)O-, -(CₙHₙ₊₂)CO-, -NR₈(CₙHₙ+₂)-, -(NR₈C₂H₄)ₙ-, - (C₂H₄NR₈)ₙ-, -(CₙHₙ₊₂)NR₈-, -(CₙHₙ₊₂)O(CₙHₘ₊₂)-, -(CₙHₙ₊₂)O(CₘHₘ₊₂)CO-, -C₃-C₁₀ cyclyl-; or -C₃-C₁₀ heterocyclyl-;
Q₄ may be present or absent, and if present, it represents -CₙHₙ+₂-, - CₙHₙ-, or -CₙHₙ-₂- ;
M represents -0-, -CO-, -C00-, -C₂H₂-, -CₙHₙ+₂-, -O(CₙHₙ+₂)-, -(CₙHₙ₊₂)O-, -(CₙHₙ₊₂)O(CₘHₘ₊₂)-, -NR₈-; -NR₈CH2-; -NHR₈-; -NR₈C(O)-; -NR₈C(O)O-; -C(O)NR₈-; -NR₈C(O)NR₈-; -NC(O)R₈-; -NS(O)₂R₈-; -S(O)₂-; -NR₈S(O)₂-; and -S(O)₂NR₈-;
n and m each independently represent an integer from 0 to 15;
E represents 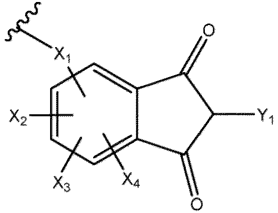 or 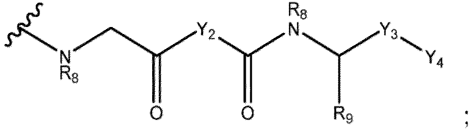
X₂, X₃, and X₄ each independently represent hydrogen; hydroxy; halogen; C₁-C₁₃ alkyl; C₁-C₆ alkoxy; C₃-C₁₀ cyclyl; C₃-C₁₀ heterocyclyl; -C(O)-(C₁-C₁₃ alkyl); amino(-NR_{8R9}); nitro(-N(O)₂); amide(-(C=O)NR₈R₉); ester(-C(O)OR₈); carboxylic acid(-C(O)OH); nitrile(-CN); sulfonamide(-NHS(O)₂R₈); sulfide(-SR₈); sulfonyl(-S(O)₂R₈);or phosphinyl(-P(O)R₈R₉);
X₁ may be present or absent, and if present, X₁ represents -CR₈R₉-, - 0- or -NR₈-;
Y₁, Y₂, Y₃, and Y₄ each independently represent C₆-C₁₀ aryl; C₃-C₁₀ heteroaryl; C₃-C₁₀ cyclyl; or C₃-C₁₀ heterocyclyl;
R₈ and R₉ each independently represent hydrogen; C₁-C₆ alkyl; C₁-C₆ alkenyl; C₁-C₆ alkynyl; C₆-C₁₀ aryl; C₃-C₁₀ heteroaryl; C₃-C₁₀ cyclyl; C₃-C₁₀ heterocyclyl; or R₈ may arbitrarily include at least one of N, 0, S, NH, C=N, C=O, -NHC(O)-, -NHC(O)NH-, -NHS(O)₂-, or SO₂, along with a nitrogen or carbon atom connected to R₉, and form a 3 to 7 membered saturated ring arbitrarily substituted with at least one of hydrogen, C₁-C₁₃ alkyl, C₆-C₁₀ aryl, C₃-C₁₀ heteroaryl, hydroxyl, halide, and cyano groups;
C₁-C₆ alkoxy, C₁-C₁₃ alkyl, or C₃-C₁₀ cyclyl includes 1 or more substituent selected from the group consisting of hydrogen; hydroxy; halogen; C₁-C₁₃ alkyl; C₁-C₆ alkoxy; amino(-NR_{8R9}); nitro(-N(O)₂); amide(-(C=O)NR₈R₉); carboxylic acid(-C(0)0H); ester(-C(O)OR₈); nitrile(-CN); urea(-NR₈(C=O)NR₉-); sulfonamide(-NHS(O)₂-); sulfide(-S-); sulfonyl(-S(O)₂-); phosphinyl(-P(O)R₈R₉); C₆-C₁₀ aryl; C₃-C₁₀ heteroaryl; and C₃-C₁₀ heterocyclyl;
C₆-C₁₀ aryl, C₃-C₁₀ heteroaryl, or C₃-C₁₀ heterocyclyl includes 1 or more substituent selected from the group consisting of hydrogen; hydroxy; halogen; carbonyl(-(C=O)R₈R₉); halogen, or C₁-C₃ alkyl either substituted with C₃-C₁₀ heterocyclyl or unsubstituted; halogen or C₁-C₃ alkoxy either substituted or unsubstituted with C₃-C₁₀ heterocyclyl; C₆-C₁₀ phenoxy; amino(-NR₈R₉); amide(-(C=O)NR₈R₉); C₆-C₁₀ aryl; C₃-C₁₀ heteroaryl and C₃-C₁₀ heterocyclyl;
C₃-C₁₀ heteroaryl and C₃-C₁₀ heterocyclyl include 1 or more heteroatoms selected from the group consisting of N, 0, or S.

### Advantageous Effects

The 2,7-substituted pyrrolo[2,1-f][1,2,4]triazine degrader compound represented by Chemical Formula I, a pharmaceutically acceptable salt thereof, a hydrate thereof, or a solvate thereof as a feature of the present invention has superior capability of degrading and inhibiting the activity of one or more protein kinases selected from ABL1, ABL2, ALK, ARK5, Aurora A, Aurora B, Aurora C, AXL, BLK, BMX, BRK, c-Kit, c-MER, c-Src, CAMK2a, CAMK2d, CDK16, , CDK17, CDK18, CDK2/cyclin A, CDK2/cyclin 0, CDK4/cyclin D1, CDK4/cyclin D2, CDK4/cyclin D3, CDK6/cyclin D1, CDK6/cyclin D3, CDK7/cyclin H, CDK9/cyclin K, CDK9/cyclin T1, CDK9/cyclin T2, CK2a, CK2a2, CLK1, CLK2, CLK4, DAPK1, DAPK2, DDR1, DDR2, DRAK1, DYRK1/DYRK1A, DYRK1B, DYRK3, EPHA1, EPHA2, EPHA4, EPHA5, EPHA6, EPHA7, EPHB1, EPHB2, ERBB4/HER4, ERK7/MAPK15, ERN2/IRE2, FAK/PTK2, FER, FES/FPS, FGFR1, FGFR2, FGFR3, FGR, FLT1/VEGFR1, FLT3, FLT4/VEGFR3, FMS, FRK/PTK5, FYN, GLK/MAP4K3, GSK3a, HIPK2, HIPK3, HIPK4, HPK1/MAP4K1, IGF1R, IKKe/IKBKE, IR, IRAK1, IRR/INSRR, JAK1, JAK2, JAK3, JNK1, JNK2, JNK3, KDR/VEGFR2, KHS/MAP4K5, LCK, LIMK1, LIMK2, LRRK2, LYN, LYN B, MAK, MARK1, MARK2/PAR-1Ba, MARK3, MARK4, MAST3, MEKK2, MELK, MLCK2/MYLK2, MLK1/MAP3K9, MLK2/MAP3K10, MLK3/MAP3K11, MUSK, MYLK4, NEK1, NEK2, NEK5, NEK9, p70S6K/RPS6KB1, PAK4, PAK5, PAK6, PDGFRa, PDGFRb, PHKg1, PHKg2, PKAcb, PKCa, PKCb2, PKCg, PKCmu/PRKD1, PKCnu/PRKD3, PKD2/PRKD2, PKN1/PRK1, PLK4/SAK, PRKX, PYK2, RET, ROS/ROS1, RSK2, RSK3, RSK4, SIK1, SIK2, SLK/STK2, SNARK/NUAK2, STK16, STK22D/TSSK1, STK33, STK38/NDR1, STK38L/NDR2, STK39/STLK3, SYK, TBK1, TEC, TIE2/TEK, TLK2, TNK1, TRKA, TRKB, TRKC, TYK2, TYR03/SKY, ULK1, ULK2, ULK3, WEE1, YES/YES1, YSK4/MAP3K19, and ZIPK/DAPK3, and is effective for preventing, alleviating, treating diseases caused by abnormal cell growth.

The diseases resulting from aberrant cell proliferation, which the compound according to the present invention may prevent or treat, may include various tumors selected from stomach cancer, lung cancer, liver cancer, colorectal cancer, small intestine cancer, pancreatic cancer, brain cancer, bone cancer, melanoma, breast cancer, sclerosing adenoma, uterine cancer, cervical cancer, head and neck cancer, esophageal cancer, thyroid cancer, parathyroid cancer, renal cancer, sarcoma, prostate cancer, urethral cancer, bladder cancer, leukemia, multiple myeloma, hematological malignancy such as myelodysplastic syndrome, lymphoma such as Hodgkin' s disease and non-Hodgkin lymphoma, fibroadenoma, etc.

### Brief Description of the Drawings

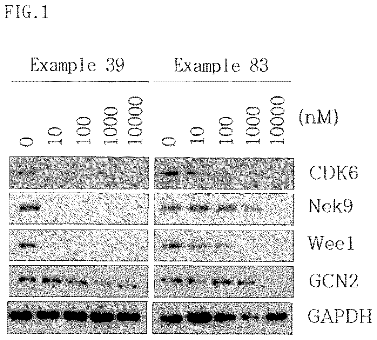

FIG. 1 represents the results of Example 3 of the present invention.

### Best Mode for Carrying out the Invention

### Definition

Unless otherwise specified, all numbers, values, and/or expressions used in this specification to represent ingredients, reaction conditions, and amounts of ingredients are to be understood as approximations reflecting the various uncertainties of measurement inherent in obtaining such values among different entities, and hence are designated by the term "about" in all cases. Additionally, when numerical ranges are provided in this disclosure, such ranges are continuous and include all values from the minimum value stated to the maximum value stated, unless otherwise indicated. Furthermore, when such ranges denote integers, they include all integers from the minimum value stated to the maximum value stated, unless otherwise indicated.

In this specification, when a range is provided for a variable, the variable shall be understood to encompass all values within the stated range, including the specified endpoints of the range. For example, the range "5 to 10" includes values such as 5, 6, 7, 8, 9, and 10, as well as any subranges such as 6 to 10, 7 to 10, 6 to 9, 7 to 9, and so forth. It also includes any arbitrary values between valid integers within the specified range, such as 5.5, 6.5, 7.5, and any integer values between them. Similarly, the range "10% to 30%" includes values such as 10%, 11%, 12%, 13%, and so on up to 30%, as well as any subranges such as 10% to 15%, 12% to 18%, 20% to 30%, and any integer values between them. It also includes any arbitrary values between valid integer values within the specified range, such as 10.5%, 15.5%, 25.5%, and so forth.

In the present specification, the terms "individual(s)", "subject(s)", and "patient(s)" refer to any mammal. In some embodiments, the mammal is a human. In some embodiments, the mammal is not a human. None of the terms require or are limited to situations characterized by supervision by healthcare practitioners (e.g., physicians, registered nurses, licensed practical nurses, nurse practitioners, physician assistants, aides, or hospice workers), either constantly or intermittently.

"Treatment" is an attempt performed with the intent of preventing the progression or alteration of the pathology of a disease. Therefore, "treatment" encompasses both therapeutic treatment and preventive dimensions. Entities needing treatment include not only those already having the disease but also those in a state where the disease should be prevented. In the context of tumor treatment, a therapeutic agent may refer to reducing the pathology of tumor cells directly or making tumor cells more sensitive to other treatments, such as radiation therapy, chemotherapy, and/or immunotherapy. The terms "reduction" or "treated" used in this specification indicate signs of approaching normalized values measured by conventional statistical tests. Signs of approaching normalized values may include values showing less than a 50% difference from normalized values obtained from healthy patients or subjects, preferably less than 25% difference, even more preferably less than 10% difference, or values showing minimal difference from normalized values.

"The treatment of cancer" refers to any one or more of the following effects: 1) i) slowing down or ii) suppression of tumor growth, including complete cessation of growth; 2) reduction in the number of tumor cells; 3) maintenance of tumor size; 4) reduction in tumor size; 5) inhibition of tumor cell infiltration into peripheral organs, including i) reduction, ii) slowing down, or iii) complete prevention; 6) inhibition of metastasis, including i) reduction, ii) slowing down, or iii) complete prevention; 7) enhancement of anti-tumor immune response, which may result in i) maintenance of tumor size, ii) reduction in tumor size, iii) slowing down of tumor growth, or iv) reduction, slowing down, or prevention of invasion, infiltration, or progression, due to the promotion of anti-tumor immune responses.

In this specification, the term "effective dose" or "therapeutically effective dose" refers to a sufficient quantity of the compound disclosed herein that alleviates symptoms to some extent of the disease or condition to be treated (e.g., cancer, inflammatory conditions, periodontal disease, or connective tissue calcification). In some embodiments, the results include 1) reduction and/or alleviation of symptoms, signs, or causes of the disease, or 2) any other desirable changes in biological systems in a clinical setting. In some embodiments, the appropriate "effective" dose for any individual case is determined using techniques such as dose escalation studies.

In some embodiments, the "effective dose" is the amount of the compound disclosed herein, when administered as a single therapy or combination therapy, i.e., when administered in one or more doses, that effectively inhibits EGFR activity in an individual compared to EGFR activity in untreated individuals, or compared to EGFR activity in individuals before or after treatment with the compound, by approximately 20% (20% inhibition), at least about 30% (30% inhibition), at least about 40% (40% inhibition), at least about 50% (50% inhibition), more than about 60% (60% inhibition), more than about 70% (70% inhibition), more than about 80% (80% inhibition), or more than about 90% (90% inhibition).

In some embodiments, the "therapeutically effective dose" is the amount of the compound disclosed herein, when administered as a single therapy or combination therapy, i.e., when administered in one or more doses, that effectively reduces tumor burden in an individual compared to tumor burden in untreated individuals, or compared to tumor burden in individuals before or after treatment with the compound, by approximately 20%, more than about 30%, more than about 40%, more than about 50%, more than about 60%, more than about 70%, more than about 80%, or more than about 90%. The term "tumor burden" as used in this specification refers to the total mass of tumor tissue present in an individual with cancer.

In some embodiments, the "therapeutically effective dose" is the amount of the compound disclosed herein, when administered as a single therapy or combination therapy, i.e., when administered in one or more doses, that effectively reduces tumor size in an individual compared to the amount of radiation therapy required to observe tumor shrinkage in untreated individuals. The effective dose is approximately 20%, more than about 30%, more than about 40%, more than about 50%, more than about 60%, more than about 70%, more than about 80%, or more than about 90% reduction in the amount of radiation therapy required to observe tumor shrinkage in individuals.

### Pharmaceutical Compositions

Pharmaceutically acceptable excipients such as vehicles, adjuvants, carriers, or diluents are readily available to those skilled in the art of pharmacy. Pharmaceutical excipients such as pH adjusters and buffers, appearance modifiers, stabilizers, wetting agents, and other pharmaceutically acceptable auxiliary substances are readily available to those skilled in the art of pharmacy.

In some embodiments, the compounds of the present invention are formulated with aqueous buffers. Suitable aqueous buffers may include but are not limited to various acetate, succinate, citrate, and phosphate buffers with strengths ranging from 5 mM to 1000 mM. In some embodiments, aqueous buffers may include reagents providing isotonic solutions. Such reagents may include sodium chloride, sugars such as mannitol, dextrose, sucrose, among others, but are not limited to these. In some embodiments, aqueous buffers may additionally include non-ionic surfactants such as polysorbate 20 or 80. Arbitrarily, formulations may include preservatives. Suitable preservatives may include but are not limited to benzyl alcohol, phenol, chlorobutanol, benzalkonium chloride, among others. In specific examples, formulations are stored at about 4 °C. Formulations may also be lyophilized, which generally includes freeze-drying agents such as sugars like trehalose, lactose, maltose, mannitol, among others. Lyophilized formulations can be stored for extended periods at room temperature.

In the present disclosure, pharmaceutical compositions may include the compounds disclosed in the present invention, or their pharmaceutically acceptable salts, stereoisomers, or prodrug derivatives thereof, either optionally or necessarily, and additionally, the pharmaceutical compositions may provide pharmaceutical compositions that may include one or more additional active agents of interest or be necessarily composed of pharmaceutical compositions. Any arbitrary convenient active agent may be used in conjunction with the compounds of the present invention in the present method. In one embodiment, in addition to the additional therapeutic agents as disclosed in the present invention for combination therapy, compounds of the present invention and immune checkpoint inhibitors can be administered orally, topically, intramuscularly, intranasally, intradermally, or by other routes. In other specific examples, compounds of the present invention and chemotherapy agents (especially chemotherapy agents that can induce the generation of cGAMP in vivo), as well as additional therapeutic agents as disclosed in the present invention for combination therapy, may be administered orally, topically, intramuscularly, intranasally, intradermally, or by other routes. The compounds of the present invention and the second active agent (if present) may be administered by the same or different routes of administration. Therapeutics may be administered by any suitable means, including but not limited to orally, rectally, intranasally, topically, vaginally, intravenously, intratumorally injection, or any other suitable means to the affected organ.

The compounds of the present invention can be administered in unit dosage form and can be prepared by any method known in the art of the pharmaceutical industry. These methods include the step of combining the compounds of the present invention with one or more pharmaceutically acceptable carriers or diluents, which are well-known in the field of industry. Pharmaceutically acceptable carriers are selected based on the chosen route of administration and standard pharmaceutical practices. Each carrier must be "pharmaceutically acceptable" in that it is compatible with other ingredients of the formulation and does not harm the subject or patient. These carriers can be solid or liquid, and the type is typically selected based on the commonly used route of administration.

Examples of suitable solid carriers include lactose, sucrose, gelatin, starch, and bulk powders. Examples of suitable liquid carriers include water, pharmaceutically acceptable fats and oils, alcohols, or other organic solvents, such as esters, emulsions, syrups, elixirs, suspensions, and solutions and/or suspensions reconstituted from non-effervescent granules. For example, these liquid carriers may contain suitable solvents, preservatives, emulsifiers, suspending agents, diluents, sweeteners, thickening agents, and melting agents.

Hereinafter, the present invention will be described in detail.

In one aspect, the present invention provides a 2,7-substituted pyrrolo[2,1-f][1,2,4]triazine compound represented by Chemical Formula 1, a pharmaceutically acceptable salt thereof, a hydrate thereof, or a solvate thereof: 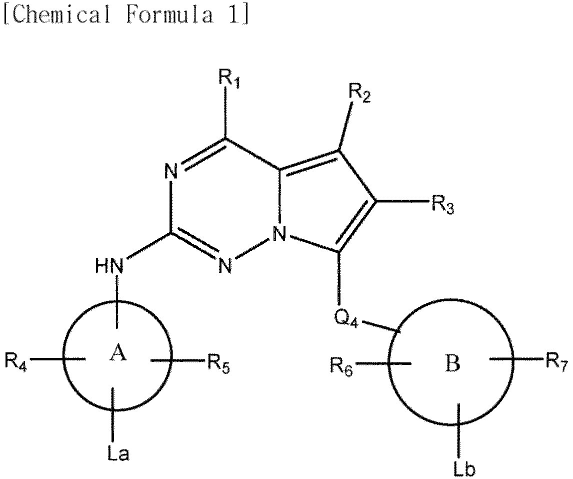 wherein
R₁, R₂, and R₃ each independently represent hydrogen; hydroxy; halogen; C₁-C₁₃ alkyl; C₁-C₆ alkoxy; C₃-C₁₀ cyclyl; C₃-C₁₀ heterocyclyl; -C(O)-(C₁-C₁₃ alkyl); amino(-NR_{8R9}); nitro(-N(O)₂); amide(-(C=O)NR₈R₉); ester(-C(O)OR₈); carboxylic acid(-C(0)0H); nitrile(-CN); sulfonamide(-NHS(O)₂R₈); sulfide(-SR₈); sulfonyl(-S(O)₂R₈);or phosphinyl(-P(O)R₈R₉);
A and B each independently represent C₆-C₁₀ aryl; C₃-C₁₀ cyclyl; C₃-C₁₀ heteroaryl; or C₃-C₁₀ heterocyclyl;
R_{4,} R₅, R₆, and R₇ each independently represent hydrogen; hydroxy; halogen; C₁-C₁₃ alkyl; C₁-C₆ alkoxy; C₃-C₁₀ cyclyl; C₃-C₁₀ heterocyclyl; -C(0)-(C₁-C₁₃ alkyl); amino(-NR_{8R9}); nitro(-N(0)₂); amide(-(C=O)NR₈R₉); ester(-C(O)OR₈); carboxylic acid(-C(0)0H); nitrile(-CN); sulfonamide(-NHS(O)₂R₈); sulfide(-SR₈); sulfonyl(-S(O)₂R₈);or phosphinyl(-P(O)R₈R₉);
Lₐ represents hydrogen; hydroxy; halogen; C₁-C₁₃ alkyl; -Zₐ-Q₃-E; or - Zₐ-Q₁-M, wherein in -Zₐ-Q₁-M, when L_{b} is -Z_{b}-Q₂, M is connected to Q₂, therby forming a ring;
L_{b} represents hydrogen; hydroxy; halogen; C₁-C₁₃ alkyl; C₁-C₆ alkoxy; C₃-C₁₀ cyclyl; C₃-C₁₀ heterocyclyl; -C(O)-(C₁-C₁₃alkyl); amino(-NR₈R₉); nitro(-N(O)₂); amide(-(C=O)NR₈R₉); ester(-C(O)OR₈); carboxylic acid(-C(0)0H); nitrile(-CN); sulfonamide(-NHS(O)₂R₈); sulfide(-SR₈); sulfonyl(-S(O)₂R₈);or-Z_{b}-Q₂, wherein in -Z_{b}-Q₂, when Lₐ is -Zₐ-Q₁-M, Q₂ is connected to M, therby forming a ring;
Zₐ or Z_{b} may each independently be present or absent, and if Zₐ or Z_{b} is present, Zₐ or Z_{b} each independently represents -0-, -CO-, -C00-, -CₙHₙ+₂-, -O(CₙHₙ₊₂)-, -(CₙHₙ₊₂)O-, -(CₙHₙ₊₂)O(CₘHₘ₊₂)-, -C₃-C₁₀ cyclyl-; or -C₃-C₁₀ heterocyclyl;
Q₁, Q₂, and Q₃ each independently represent -CₙHₙ₊₂-, -O(CₙHₙ₊₂)-, - (OC₂H₄)ₙ-, -(C₂H₄O)ₙ-, -(CₙHₙ₊₂)O-,-(CₙHₙ₊₂)CO-, -NR₈(CₙHₙ+₂)-, -(NR₈C₂H₄)ₙ-, - (C₂H₄NR₈)ₙ-, -(CₙHₙ₊₂)NR₈-, -(CₙHₙ₊₂)O(CₙHₘ₊₂)-, -(CₙHₙ₊₂)O(CₘHₘ₊₂)CO-, -C₃-C₁₀ cyclyl-; or -C₃-C₁₀ heterocyclyl-;
Q₄ may be present or absent, and if present, Q₄ represents -CₙHₙ₊₂-, - CₙHₙ-, or -CₙHₙ₋₂- ;
M represents -0-, -CO-, -C00-, -C₂H₂-, -CₙHₙ₊₂-, -O(CₙHₙ₊₂)-, -(CₙHₙ₊₂)O-, -(CₙHₙ₊₂)O(CₘHₘ₊₂)-, -NR₈-; -NR₈CH₂-; -NHR₈-; -NR₈C(O)-; -NR₈C(O)O-; -C(O)NR₈-; -NR₈C(O)NR₈-; -NC(O)R₈-; -NS(O)₂R₈-; -S(O)₂-; -NR₈S(O)₂-; and -S(O)₂NR₈-;
n and m each independently represent an integer from 0 to 15;
E represents 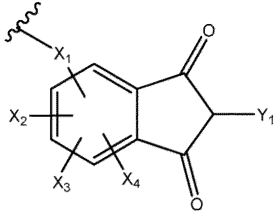 or 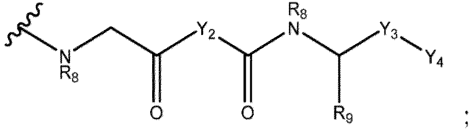
X₂, X₃, and X₄ each independently represent hydrogen; hydroxy; halogen; C₁-C₁₃ alkyl; C₁-C₆ alkoxy; C₃-C₁₀ cyclyl; C₃-C₁₀ heterocyclyl; -C(O)-(C₁-C₁₃ alkyl); amino(-NR_{8R9}); nitro(-N(0)₂); amide(-(C=O)NR₈R₉); ester(-C(O)OR₈); carboxylic acid(-C(0)0H); nitrile(-CN); sulfonamide(-NHS(O)₂R₈); sulfide(-SR₈); sulfonyl(-S(O)₂R₈);or phosphinyl(-P(O)R₈R₉);
X₁ may be present or absent, and if present, X₁ represents -CR₈R₉-, - 0- or -NR₈-;
Y₁, Y₂, Y₃, and Y₄ each independently represent C₆-C₁₀ aryl; C₃-C₁₀ heteroaryl; C₃-C₁₀ cyclyl; or C₃-C₁₀ heterocyclyl;
R₈ and R₉ each independently represent hydrogen; C₁-C₆ alkyl; C₁-C₆ alkenyl; C₁-C₆ alkynyl; C₆-C₁₀ aryl; C₃-C₁₀ heteroaryl; C₃-C₁₀ cyclyl; C₃-C₁₀ heterocyclyl; or R₈ may arbitrarily include at least one of N, 0, S, NH, C=N, C=0, -NHC(0)-, -NHC(0)NH-, -NHS(O)₂-, or SO₂, along with a nitrogen or carbon atom connected to R₉, and form a 3 to 7 membered saturated ring arbitrarily substituted with at least one of hydrogen, C₁-C₁₃ alkyl, C₆-C₁₀ aryl, C₃-C₁₀ heteroaryl, hydroxyl, halide, and cyano groups;
C₁-C₆ alkoxy, C₁-C₁₃ alkyl, or C₃-C₁₀ cyclyl includes 1 or more substituent selected from the group consisting of hydrogen; hydroxy; halogen; C₁-C₁₃ alkyl; C₁-C₆ alkoxy; amino(-NR_{8R9}); nitro(-N(O)₂); amide(-(C=O)NR₈R₉); carboxylic acid(-C(0)0H); ester(-C(O)OR₈); nitrile(-CN); urea(-NR₈(C=O)NR₉-); sulfonamide(-NHS(O)₂-); sulfide(-S-); sulfonyl(-S(O)₂-); phosphinyl(-P(O)R₈R₉); C₆-C₁₀ aryl; C₃-C₁₀ heteroaryl; and C₃-C₁₀ heterocyclyl;
C₆-C₁₀ aryl, C₃-C₁₀ heteroaryl, or C₃-C₁₀ heterocyclyl includes 1 or more substituent selected from the group consisting of hydrogen; hydroxy; halogen; carbonyl(-(C=O)R₈R₉); halogen, or C₁-C₃ alkyl either substituted with C₃-C₁₀ heterocyclyl or unsubstituted; halogen or C₁-C₃ alkoxy either substituted or unsubstituted with C₃-C₁₀ heterocyclyl; C₆-C₁₀ phenoxy; amino(-NR_{8R9}); amide(-(C=O)NR₈R₉); C₆-C₁₀ aryl; C₃-C₁₀ heteroaryl and C₃-C₁₀ heterocyclyl;
C₃-C₁₀ heteroaryl and C₃-C₁₀ heterocyclyl include 1 or more heteroatoms selected from the group consisting of N, 0, or S.

In one aspect, the present invention provides the 2,7-substituted pyrrolo[2,1-f][1,2,4]triazine compound represented by Chemical Formula 1, a pharmaceutically acceptable salt thereof, a hydrate thereof, or a solvate thereof, wherein A and B independently represent furan, benzofuran, pyrrole, indole, thiophene, benzothiophene, imidazole, benzimidazole, purine, pyrazole, indazole, oxazole, benzoxazole, thiazole, benzisoxazole, benzene, naphthalene, anthracene, pyridine, quinoline, pyrazine, quinoxaline, acridine, pyrimidine, quinazoline, pyridazine, quinoline, phthalazine, or triazine.

In one aspect, the present invention provides the 2,7-substituted pyrrolo[2,1-f][1,2,4]triazine compound represented by Chemical Formula 1, a pharmaceutically acceptable salt thereof, a hydrate thereof, or a solvate thereof, wherein A and B each independently represent benzene, pyrazole, pyridine, indazole, pyrimidine, quinoline, or thiazole, and wherein R1, R2, and R3 independently represent hydrogen, halogen, C1-C13 alkyl, or C3-C10 cycloalkyl.

In one aspect, the present invention provides the 2,7-substituted pyrrolo[2,1-f][1,2,4]triazine compound represented by Chemical Formula 1, a pharmaceutically acceptable salt thereof, a hydrate thereof, or a solvate thereof, wherein R4, R5, R6, and R7 independently represent hydrogen; hydroxy; halogen; -NHCH3; -NHMs; -C0(NH)CH3; -OCH3; -0C2H5; or -OC6H12COOCH3.

In one aspect, the present invention provides the 2,7-substituted pyrrolo[2,1-f][1,2,4]triazine compound, wherein the compound of Chemical Formula 1 has a structure represented by Chemical Formula 2, a pharmaceutically acceptable salt thereof, a hydrate thereof, or a solvate thereof: 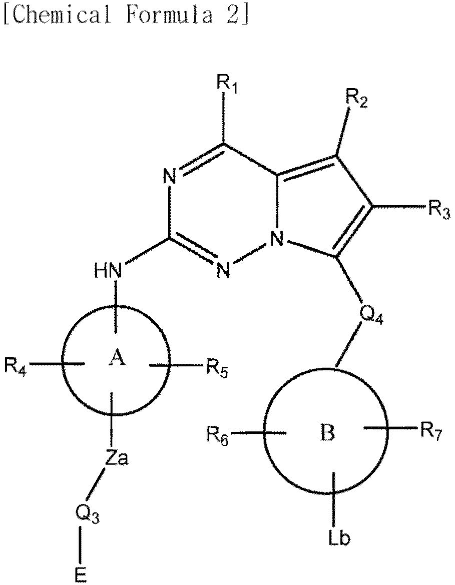 wherein
R₁, R₂, and R₃ each independently represent hydrogen; hydroxy; halogen; C₁-C₁₃ alkyl; C₁-C₆ alkoxy; C₃-C₁₀ cyclyl; C₃-C₁₀ heterocyclyl; -C(O)-(C₁-C₁₃ alkyl); amino(-NR₈R₉); nitro(-N(0)₂); amide(-(C=O)NR₈R₉); ester(-C(O)OR₈); carboxylic acid(-C(0)0H); nitrile(-CN); sulfonamide(-NHS(O)₂R₈); sulfide(-SR₈); sulfonyl(-S(O)₂R₈);or phosphinyl(-P(O)R₈R₉);
A and B each independently represent C₆-C₁₀ aryl; C₃-C₁₀ cyclyl; C₃-C₁₀ heteroaryl; or C₃-C₁₀ heterocyclyl;
R₄, R₅, R₆, and R₇ each independently represent hydrogen; hydroxy; halogen; C₁-C₁₃ alkyl; C₁-C₆ alkoxy; C₃-C₁₀ cyclyl; C₃-C₁₀ heterocyclyl; -C(0)-(C₁-C₁₃ alkyl); amino(-NR₈R₉); nitro(-N(0)₂); amide(-(C=O)NR₈R₉); ester(-C(O)OR₈); carboxylic acid(-C(0)0H); nitrile(-CN); sulfonamide(-NHS(O)₂R₈); sulfide(-SR₈); sulfonyl(-S(O)₂R₈);or phosphinyl(-P(O)R₈R₉);
L_{b} represents hydrogen; hydroxy; halogen; C₁-C₁₃ alkyl; C₁-C₆ alkoxy; C₃-C₁₀ cyclyl; C₃-C₁₀ heterocyclyl; -C(O)-(C₁-C₁₃alkyl); amino(-NR₈R₉); nitro(-N(O)₂); amide(-(C=O)NR₈R₉); carboxylic acid(-C(0)0H); ester(-C(O)OR₈); nitrile(-CN); sulfonamide(-NHS(O)₂R₈); sulfide(-SR₈); or sulfonyl(-S(O)₂R₈);
Zₐ represents -0-, -CO-, -C00-, -CₙHₙ₊₂-, -O(CₙHₙ₊₂)-, -(CₙHₙ₊₂)O-, - (CₙHₙ₊₂O(CₘHₘ₊₂)-, -C₃-C₁₀ cyclyl-; or -C₃-C₁₀ heterocyclyl;
Q₃ represents -CₙHₙ+₂-, -O(CₙHₙ₊₂)-, -(OC₂H₄)ₙ-, -(C₂H₄O)ₙ-, -(CₙHₙ₊₂)O-,-(CₙHₙ+₂)CO-, -NR₈(CₙHₙ₊₂)-, -(NR₈C₂H₄)ₙ-, -(C₂H₄NR₈)ₙ-, -(CₙHₙ₊₂)NR₈-, - (CₙHₙ₊₂)O(CₘHₘ₊₂)-, -(CₙHₙ₊₂)O(CₘHₘ₊₂)CO-, -C₃-C₁₀ cyclyl-; or -C₃-C₁₀ heterocyclyl-;
Q₄ may be present or absent, and if present, Q₄ represents -CₙHₙ₊₂-, - CₙHₙ-, or - CₙHₙ-₂-;
n and m each independently represent an integer from 0 to 15,
E represents 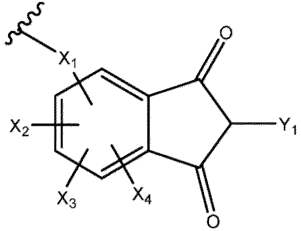 or 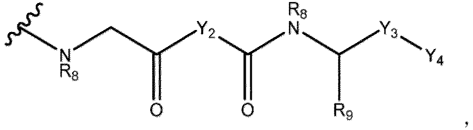
X₂, X₃, and X₄ each independently represent hydrogen; hydroxy; halogen; C₁-C₁₃ alkyl; C₁-C₆ alkoxy; C₃-C₁₀ cyclyl; C₃-C₁₀ heterocyclyl; -C(O)-(C₁-C₁₃ alkyl); amino(-NR_{8R9}); nitro(-N(0)₂); amide(-(C=O)NR₈R₉); ester(-C(O)OR₈); carboxylic acid(-C(0)0H); nitrile(-CN); sulfonamide(-NHS(O)₂R₈); sulfide(-SR₈); sulfonyl(-S(O)₂R₈);or phosphinyl(-P(O)R₈R₉);
X₁ may be present or absent, and if present, X₁ represents -CR₈R₉-, - 0- or -NR₈-;
Y₁, Y₂, Y₃, and Y₄ each independently represent C₆-C₁₀ aryl; C₃-C₁₀ heteroaryl; C₃-C₁₀ cyclyl; or C₃-C₁₀ heterocyclyl;
R₈ and R₉ each independently represent hydrogen; C₁-C₆ alkyl; C₁-C₆ alkenyl; C₁-C₆ alkynyl; C₆-C₁₀ aryl; C₃-C₁₀ heteroaryl; C₃-C₁₀ cyclyl; C₃-C₁₀ heterocyclyl; or R₈ may arbitrarily include at least one of N, 0, S, NH, C=N, C=0, -NHC(0)-, -NHC(0)NH-, -NHS(O)₂-, or SO₂, along with a nitrogen or carbon atom connected to R₉, and form a 3 to 7 membered saturated ring arbitrarily substituted with at least one of hydrogen, C₁-C₁₃ alkyl, C₆-C₁₀ aryl, C₃-C₁₀ heteroaryl, hydroxyl, halide, and cyano groups;
C₁-C₆ alkoxy, C₁-C₁₃ alkyl, or C₃-C₁₀ cyclyl includes 1 or more substituent selected from the group consisting of hydrogen; hydroxy; halogen; C₁-C₁₃ alkyl; C₁-C₆ alkoxy; amino(-NR_{8R9}); nitro(-N(O)₂); amide(-(C=O)NR₈R₉); carboxylic acid(-C(0)0H); ester(-C(O)OR₈); nitrile(-CN); urea(-NR₈(C=O)NR₉-); sulfonamide(-NHS(O)₂-); sulfide(-S-); sulfonyl(-S(O)₂-); phosphinyl(-P(O)R₈R₉); C₆-C₁₀ aryl; C₃-C₁₀ heteroaryl; and C₃-C₁₀ heterocyclyl;
C₆-C₁₀ aryl, C₃-C₁₀ heteroaryl, or C₃-C₁₀ heterocyclyl includes 1 or more substituent selected from the group consisting of hydrogen; hydroxy; halogen; carbonyl(-(C=O)R₈R₉); halogen, or C₁-C₃ alkyl either substituted with C₃-C₁₀ heterocyclyl or unsubstituted; halogen or C₁-C₃ alkoxy either substituted or unsubstituted with C₃-C₁₀ heterocyclyl; C₆-C₁₀ phenoxy; amino(-NR_{8R9}); amide(-(C=O)NR₈R₉); C₆-C₁₀ aryl; C₃-C₁₀ heteroaryl and C₃-C₁₀ heterocyclyl;
C₃-C₁₀ heteroaryl and C₃-C₁₀ heterocyclyl include 1 or more heteroatoms selected from the group consisting of N, 0, or S.

In one exemplary embodiment, the present invention provides the 2,7-substituted pyrrolo[2,1-f][1,2,4]triazine compound of Chemical Formula 1, having a structure represented by Chemical Formula 2, a pharmaceutically acceptable salt thereof, a hydrate thereof, or a solvate thereof, wherein A and B each independently represent benzene, pyrazole, pyridine, indazole, pyrimidine, quinoline, or thiazole, R₁, R₂ , and R₃ each independently represent hydrogen, halogen, C₁-C₁₃ alkyl or C₃-C₁₀ cyclyl, R₄, R₅, R₆, and R₇ each independently hydrogen; hydroxy; halogen; -NHCH₃; -NHMs; -CO(NH)CH₃; - OCH₃; -OC₂H₅; or -OC₆H₁₂COOCH₃;
Y₁ represents 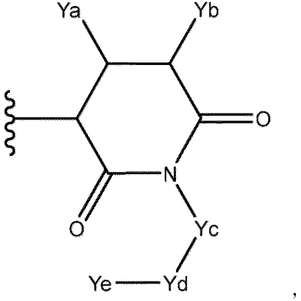 Yₐ and Y_{b} each independently represent hydrogen; halogen; C₁-C₁₃ alkyl; C₁-C₆ haloalkyl; C₁-C₆ alkoxy; C₃-C₁₀ cyclyl; C₃-C₁₀ heterocyclyl; -C(O)-(C₁-C₁₃ alkyl); amino(-NR₈R₉); nitro(-N(O)₂); amide(-(C=O)NR₈R₉); carboxylic acid(-C(0)0H); ester(-C(O)OR₈); nitrile(-CN); sulfonamide(-NHS(O)₂R₈); sulfide(-SR₈); sulfonyl(-S(O)₂R₈); or phosphinyl(-P(O)R₈R₉); Y_{c} and Y_{d} each independently -0-, -CO-, -C00-, -CₙHₙ₊₂-, -O(CₙHₙ₊₂)-, -(CₙHₙ₊₂)O-, -(CₙHₙ₊₂)O(CₘHₘ₊₂)-, -C₃-C₁₀ cyclyl-; or -C₃-C₁₀ heterocyclyl-; Yₑ represents C₆-C₁₀; C₃-C₁₀ cyclyl; C₃-C₁₀ heteroaryl; or C₃-C₁₀ heterocyclyl;

In one exemplary embodiment, the present invention provides the 2,7-substituted pyrrolo[2,1-f][1,2,4]triazine compound of Chemical Formula 1, having a structure represented by Chemical Formula 2, a pharmaceutically acceptable salt thereof, a hydrate thereof, or a solvate thereof, wherein E represents E₁, E₂, E₃, E₄, E₅, E₆, E₇, E₈, E₉, E₁₀, E₁₁, E₁₂, E₁₃, E₁₄ or E₁₅.

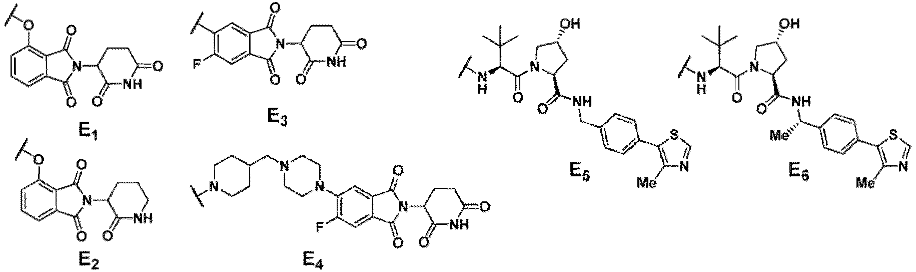

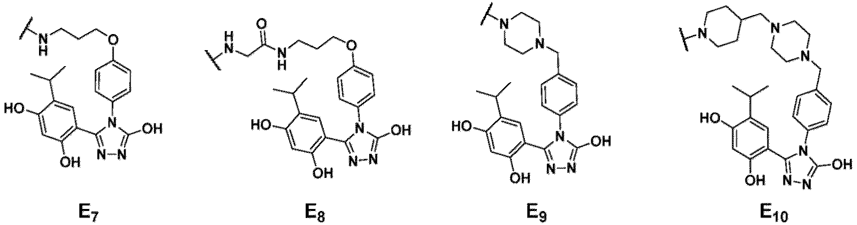

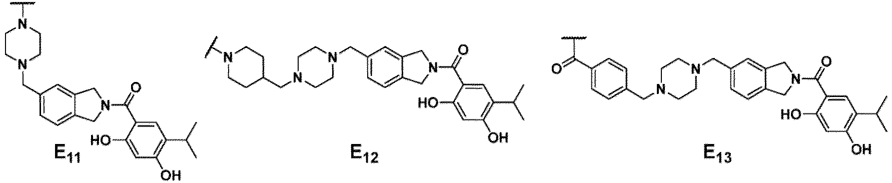

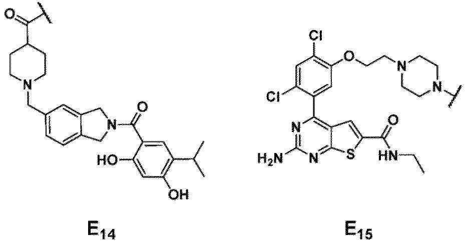

In one exemplary embodiment, the present invention provides the compound represented by Chemical Formula 2, a pharmaceutically acceptable salt thereof, a hydrate thereof, or a solvate thereof, wherein Q3 represents one of -(CH2)n-, -(OC2H4)n-, or -(CH2)nC0-, where n is an integer; A and B each represent one or more of heteroaryl, cyclic hydrocarbon, or alkyl group; wherein n is an integer selected from 0, 1, 2, 3, 4, 5, 6, 7, 8, 9, 10, 11, or 12; heteroaryl is one of phenyl, substituted phenyl, pyrazole, or substituted indole; cyclic hydrocarbon is one or more of piperazine and piperidine.

In one aspect, the present invention provides the 2,7-substituted pyrrolo[2,1-f][1,2,4]triazine compound, wherein the compound of Chemical Formula 1 has a structure represented by Chemical Formula 3, a pharmaceutically acceptable salt thereof, a hydrate thereof, or a solvate thereof: 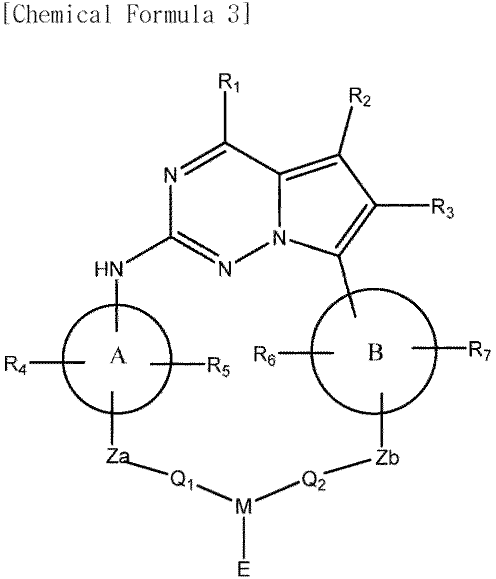 wherein
R₁, R₂, and R₃ each independently represent hydrogen; hydroxy; halogen; C₁-C₁₃ alkyl; C₁-C₆ alkoxy; C₃-C₁₀ cyclyl; C₃-C₁₀ heterocyclyl; -C(O)-(C₁-C₁₃ alkyl); amino(-NR_{8R9}); nitro(-N(O)₂); amide(-(C=O)NR₈R₉); ester(-C(O)OR₈); carboxylic acid(-C(0)0H); nitrile(-CN); sulfonamide(-NHS(O)₂R₈); sulfide(-SR₈); sulfonyl(-S(O)₂R₈); or phosphinyl(-P(O)R₈R₉);
A and B each independently represent C₆-C₁₀ aryl; C₃-C₁₀ cyclyl; C₃-C₁₀ heteroaryl; or C₃-C₁₀ heterocyclyl;
R₄, R₅, R₆, and R₇ each independently represent hydrogen; hydroxy; halogen; C₁-C₁₃ alkyl; C₁-C₆ haloalkyl; C₁-C₆ alkoxy; C₃-C₁₀ cyclyl; C₃-C₁₀ heterocyclyl; -C(O)-(C₁-C₁₃ alkyl); amino(-NR₈R₉); nitro(-N(0)₂); amide(-(C=O)NR₈R₉); carboxylic acid(-C(0)0H); ester(-C(O)OR₈); nitrile(-CN); sulfonamide(-NHS(O)₂R₈); sulfide(-SR₈); sulfonyl(-S(O)₂R₈); or phosphinyl(-P(O)R₈R₉);
Zₐ or Z_{b} each independently represents -0-, -CO-, -C00-, -CₙHₙ₊₂-, - O(CₙHₙ₊₂)-, -(CₙHₙ₊₂)O-, -(CₙHₙ₊₂)O(CₙHₘ₊₂)-, -C₃-C₁₀ cyclyl-; or -C₃-C₁₀ heterocyclyl-;
Q₁ and Q₂ each independently represent -CₙHₙ+₂-, -O(CₙHₙ₊₂)-, -(OC₂H₄)ₙ-, -(C₂H₄O)ₙ-, -(CₙHₙ₊₂)O-,-(CₙHₙ₊₂)CO-, -NR₈(CₙHₙ₊₂)-, -(NR₈C₂H₄)ₙ-, -(C₂H₄NR₈)ₙ-, - (CₙHₙ₊₂)NR₈-, -(CₙHₙ₊₂)O(CₙHₘ₊₂)-, -(CₙHₙ₊₂)O(CₘHₘ₊₂)CO-, -C₃-C₁₀ cyclyl-; or -C₃-C₁₀ heterocyclyl-;
M represents -0-, -CO-, -C00-, -C₂H₂-, -CₙHₙ₊₂-, -O(CₙHₙ₊₂)-, -(CₙHₙ₊₂)O-, -(CₙHₙ₊₂)O(CₘHₘ₊₂)-, -NR₈-; -NR₈CH₂-; -NHR₈-; -NR₈C(O)-; -NR₈C(O)O-; -C(O)NR₈-; -NR₈C(O)NR₈-; -NC(O)R₈-; -NS(O)₂R₈-; -S(O)₂-; -NR₈S(O)₂-; and -S(O)₂NR₈-;
E represents 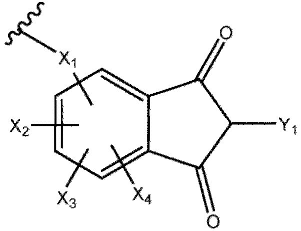 or 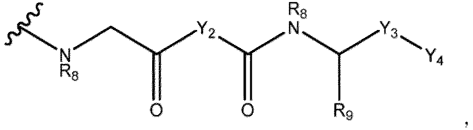
X₂, X₃, and X₄ each independently represents hydrogen; hydroxy; halogen; C₁-C₁₃ alkyl; C₁-C₆ alkoxy; C₃-C₁₀ cyclyl; C₃-C₁₀ heterocyclyl; -C(O)-(C₁-C₁₃ alkyl); amino(-NR₈R₉); nitro(-N(0)₂); amide(-(C=O)NR₈R₉); ester(-C(O)OR₈); carboxylic acid(-C(0)0H); nitrile(-CN); sulfonamide(-NHS(O)₂R₈); sulfide(-SR₈); sulfonyl(-S(O)₂R₈);or phosphinyl(-P(O)R₈R₉);
X₁ may be present or absent, and if present, X₁ represents -CR₈R₉-, - 0- or -NR₈-;
Y₁, Y₂, Y₃, and Y₄ each independently represent C₆-C₁₀ aryl; C₃-C₁₀ heteroaryl; C₃-C₁₀ cyclyl; or C₃-C₁₀ heterocyclyl;
n and m each independently represent an integer from 0 to 15;
R₈ and R₉ each independently represent hydrogen; C₁-C₆ alkyl; C₁-C₆ alkenyl; C₁-C₆ alkynyl; C₆-C₁₀ aryl; C₃-C₁₀ heteroaryl; C₃-C₁₀ cyclyl; C₃-C₁₀ heterocyclyl; or R₈ may arbitrarily include at least one of N, 0, S, NH, C=N, C=0, -NHC(0)-, -NHC(0)NH-, -NHS(O)₂-, or SO₂, along with a nitrogen or carbon atom connected to R₉, and form a 3 to 7 membered saturated ring arbitrarily substituted with at least one of hydrogen, C₁-C₁₃ alkyl, C₆-C₁₀ aryl, C₃-C₁₀ heteroaryl, hydroxyl, halide, and cyano groups;
C₁-C₆ alkoxy, C₁-C₁₃ alkyl, or C₃-C₁₀ cyclyl includes 1 or more substituent selected from the group consisting of hydrogen; hydroxy; halogen; C₁-C₁₃ alkyl; C₁-C₆ alkoxy; amino(-NR_{8R9}); nitro(-N(O)₂); amide(-(C=O)NR₈R₉); carboxylic acid(-C(0)0H); ester(-C(O)OR₈); nitrile(-CN); urea(-NR₈(C=O)NR₉-); sulfonamide(-NHS(O)₂-); sulfide(-S-); sulfonyl(-S(O)₂-); phosphinyl(-P(O)R₈R₉); C₆-C₁₀ aryl; C₃-C₁₀ heteroaryl; and C₃-C₁₀ heterocyclyl;
C₆-C₁₀ aryl, C₃-C₁₀ heteroaryl, or C₃-C₁₀ heterocyclyl includes 1 or more substituent selected from the group consisting of hydrogen; hydroxy; halogen; carbonyl(-(C=O)R₈R₉); halogen, or C₁-C₃ alkyl either substituted with C₃-C₁₀ heterocyclyl or unsubstituted; halogen or C₁-C₃ alkoxy either substituted or unsubstituted with C₃-C₁₀ heterocyclyl; C₆-C₁₀ phenoxy; amino(-NR_{8R9}); amide(-(C=O)NR₈R₉); C₆-C₁₀ aryl; C₃-C₁₀ heteroaryl and C₃-C₁₀ heterocyclyl;
C₃-C₁₀ heteroaryl and C₃-C₁₀ heterocyclyl include 1 or more heteroatoms selected from the group consisting of N, 0, or S.

In one exemplary embodiment, the present invention provides the 2,7-substituted pyrrolo[2,1-f][1,2,4]triazine compound of Chemical Formula 1, having a structure represented by Chemical Formula 3, a pharmaceutically acceptable salt thereof, a hydrate thereof, or a solvate thereof, wherein A and B each independently represent benzene, pyrazole, pyridine, indazole, pyrimidine, quinoline, or thiazole, R₁, R₂ , and R₃ each independently represent hydrogen, halogen, C₁-C₁₃ alkyl or C₃-C₁₀ cyclyl, R₄, R₅, R₆, and R₇ each independently hydrogen; hydroxy; halogen; -NHCH₃; -NHMs; -CO(NH)CH₃; - OCH₃; -OC₂H₅; or -OC₆H₁₂COOCH₃;

In one exemplary embodiment, the present invention provides the 2,7-substituted pyrrolo[2,1-f][1,2,4]triazine compound of Chemical Formula 1, having a structure represented by Chemical Formula 3, a pharmaceutically acceptable salt thereof, a hydrate thereof, or a solvate thereof, wherein E represents E₁, E₂, E₃, E₄, E₅, E₆, E₇, E₈, E₉, E₁₀, E₁₁, E₁₂, E₁₃, E₁₄ or E₁₅.

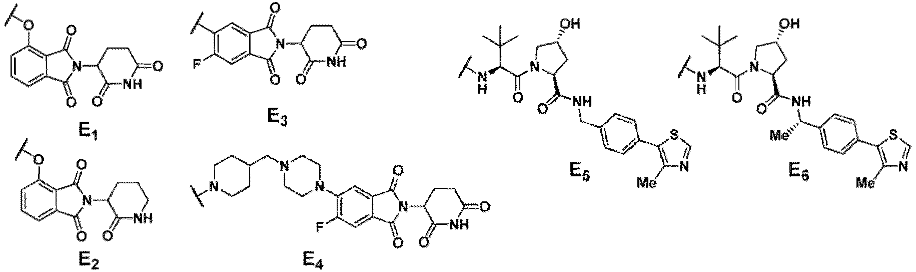

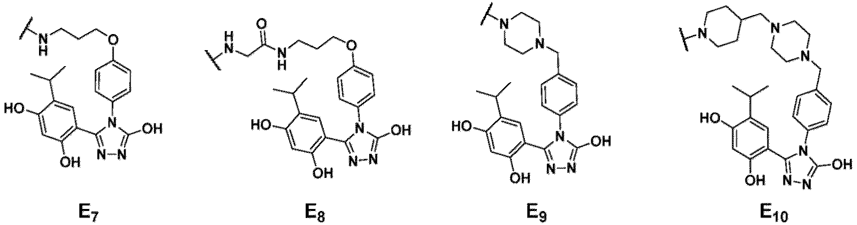

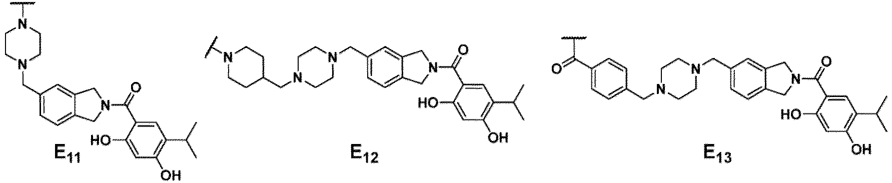

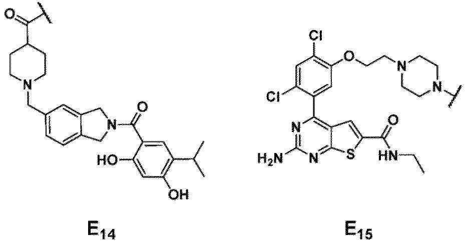

In one exemplary embodiment, the present invention provides the compound represented by Chemical Formula 3, a pharmaceutically acceptable salt thereof, a hydrate thereof, or a solvate thereof, wherein Q₁ and Q₂ each represent one of -(CH2)n-, -0(CH2)n-, -N(CH2)n-, and -NH(CH2)n-, wherein n is an integer; A and B each represent one of heteroaryl, cyclic hydrocarbon, or alkyl group; M represents one of -CHCH-, -CH2CH2-, -NHCO-, -CONH-, -NMe-, -NAc-, -NMs-, -NHCONHR10-, -NHCOR10-, -NHS0NR10-, or -0-, wherein R₁₀ represents one of alkyl group, cyclic hydrocarbon, phenyl group, or substituted phenyl group; wherein n is an integer selected from 0, 1, 2, 3, or 4; heteroaryl is one of phenyl, pyridine, substituted phenyl, or pyrazole; cyclic hydrocarbon is one or more of piperazine and cyclohexane;

In one aspect, the present invention provides the 2,7-substituted pyrrolo[2,1-f][1,2,4]triazine compound represented by Chemical Formula 1, a pharmaceutially acceptable salt thereof, a hydrate thereof, or a solvate thereof, characterized by being selected from the group consisting of compounds numbered 1 to 126:
(Compound No. 1) (2¹Z,4⁴E)-4¹*H-*11-oxa-3,8-diaza-2(7,2)-pyrrolo[2,1-f][1,2,4]triazina- 4(4,1)-pyrazola-1(1,4)-benzenacycloundecaphane;
(Compound No. 2) ((2¹Z,4⁴E)-8-methyl-4¹*H-*11-oxa-3,8-diaza-2(7,2)-pyrrolo[2,1-f][1,2,4]triazina-4(4,1)-pyrazola-1(1,4)-benzenacycloundecaphane;
(Compound No. 3) 1-((2¹Z,4⁴E)-4¹*H-*11-oxa-3,8-diaza-2(7,2)-pyrrolo[2,1-f][1,2,4]triazina-4(4,1)-pyrazola-1(1,4)-benzenacycloundecafphane-8-yl)ethan-1-one;
(Compound No. 4) 1-((2¹Z,4⁴E)-4¹*H-*11-oxa-3,8-diaza-2(7,2)-pyrrolo[2,1-f][1,2,4]triazina-4(4,1)-pyrazola-1(1,4)-benzenacycloundecaphane-8-yl)butan-1-one;
(Compound No. 5) ((2¹Z,4⁴E)-4¹H-11-oxa-3,8-diaza-2(7,2)-pyrrolo[2,1-f][1,2,4]triazina -4(4,1)-pyrazola-1(1,4)-benzenacycloundecaphane-8-yl)(4-chlorophenyl)methanone;
(Compound No. 6) (2¹Z,4⁴E)-*N*-cyclohexyl-4¹*H*-11-oxa-3,8-diaza-2(7,2)-pyrrolo[2,1-f][1,2,4]triazina-4(4,1)-pyrazola-1(1,4)-benzenacycloundecaphane-8-carboxamide;
(Compound No. 7) (2¹Z,4⁴E)-*N*-(3-methoxyphenyl)-4¹*H-*11-oxa-3,8-diaza-2(7,2)-pyrrolo[2,1-f][1,2,4]triazina-4(4,1)-pyrazola-1(1,4)-benzenecycloundecaphane-8-carboxamide;
(Compound No. 8) (2¹Z,4⁴E)-*N*-ethyl-4¹*H*-11-oxa-3,8-diaza-2(7,2)-pyrrolo[2,1-f][1,2,4]triazina-4(4,1)-pyrazola-1(1,4)-benzenacycloundecaphane-8-carboxamide;
(Compound No. 9) (2¹Z,4⁴E)-8-(methylsulfonyl)-4¹*H-*11-oxa-3,8-diaza-2(7,2)-pyrrolo[2,1-f][1,2,4]triazina-4(4,1)-pyrazola-1(1,4)-benzenecycloundecaphane;
(Compound No. 10) (2¹Z,4⁴E)-8-((3,4-difluorophenyl)sulfonyl)-4¹*H-*11-oxa-3,8-diaza-2(7,2)-pyrrolo [2,1-f][1,2,4]triazina-4(4,1)-pyrazola-1(1,4)-benzenecycloundecaphane;
(Compound No. 11) (2¹Z,4⁴E)-8-(phenylsulfonyl)-4¹*H-*11-oxa-3,8-diaza-2(7,2)-pyrrolo[2,1-f][1,2,4]triazina-4(4,1)-pyrazola-1(1,4)-benzenacycloundecaphane;
(Compound No. 12) (2¹Z,4⁴E)-8-(cyclopropylsulfonyl)-4¹*H-*11-oxa-3,8-diaza-2(7,2)-pyrrolo[2,1-f][1,2,4]triazina-4(4,1)-pyrazola-1(1,4)-benzenacycloundecaphane;
(Compound No. 13) 2¹Z,4⁴E)-8-(propylsulfonyl)-4¹*H-*11-oxa-3,8-diaza-2(7,2)-pyrrolo[2,1-f][1,2,4]triazina-4(4,1)-pyrazola-1(1,4)-benzenacycloundecaphane;
(Compound No. 14) (2¹Z,4⁴E)-8-((5-chlorothiophen-2-yl)sulfonyl)-4¹*H-*11-oxa-3,8-diaza-2(7,2)-pyrrolo[2,1-f][1,2,4]triazina-4(4,1)-pyrazola-1(1,4)-benzenacycloundecaphane;
(Compound No. 15) (2¹Z,4⁴E)-8-(cyclohexylsulfonyl)-4¹*H-*11-oxa-3,8-diaza-2(7,2)-pyrrolo[2,1-f][1,2,4]triazina-4(4,1)-pyrazola-1(1,4)-benzenacycloundecaphane;
(Compound No. 16) (Z)-5-oxa-3,9,15-triaza-2(7,2)-pyrrolo[2,1-f][1,2,4]triazina-1, 4(1,4)-dibenzenacyclopentadecaphan-10-one;
(Compound No. 17) (1⁴Z,2¹Z)-1¹H-5-oxa-3,9-diaza-2(7,2)-pyrrolo[2,1-f][1,2,4]triazina- 1(4,1)-pyrazola-4(1,4)-benzenacyclohexadecaphan-10-one;
(Compound No. 18) (1⁴Z,2¹Z,4⁴E)-1¹*H*,4¹*H-*3,8-diaza-2(7,2)-pyrrolo[2,1-f][1,2,4]triazina-1,4(4,1)-dipyrazolacyclopentadecaphan-9-one;
Compound No. 19) (1⁴Z,2¹Z)-1¹*H-*3-aza-2(7,2)-pyrrolo[2,1-f][1,2,4]triazina-5(1,4)-piperazina-1(4,1)-pyrazola-4(1,4)-benzenacyclododecaphane-9-one;
(Compound No. 20) (Z)-13-fluoro-5,17-dioxa-3,9-diaza-2(7,2)-pyrrolo[2,1-f][1,2,4 Itriazina-1,4(1,4)-dibenzenacycloheptadecaphan-10-one;
(Compound No. 21) (2¹Z,4⁴E)-13-fluoro-4¹H-16-oxa-3,8-diaza-2(7,2)-pyrrolo[2,1-f][1,2,4]triazina-4(4,1)-pyrazola-1(1,4)-benzenacyclohexadecaphan-9-one;
(Compound No. 22) (Z)-1³-fluoro-13-oxa-3-aza-2(7,2)-pyrrolo[2,1-f][1,2,4]triazina-5(1,4)-piperazina-1,4(1,4)-dibenzenacyclotridecaphan-6-one;
(Compound No. 23) (Z)-17-oxa-3,9-diaza-2(7,2)-pyrrolo[2,1-f][1,2,4]triazina-5(1,4)-piperazina-1,4(1,4)-dibenzenacycloheptadecaphan-10-one;
(Compound No. 24) (Z)-13-oxa-3-aza-2(7,2)-pyrrolo[2,1-f][1,2,4]triazina-5(1,4)-piperazina-1,4(1,4)-dibenzenacyclotridecaphan-6-one;
(Compound No. 25) (2¹Z,4⁴E)-4¹*H-*11-oxa-3-aza-2(7,2)-pyrrolo[2,1-f][1,2,4]triazina-5(4,1)-piperidina-4(4,1)-pyrazola-1(1,4)-benzenacycloundecaphan-6-one;
(Compound No. 26) (2¹Z,4⁴E)-4¹*H-*15-oxa-3,9-diaza-2(7,2)-pyrrolo[2,1-f][1,2,4]triazina-5(4,1)-piperidina-4(4,1)-pyrazola-1(1,4)-benzenacyclopentadecaphan-10-one;
(Compound No. 27) (2¹Z,4⁴E)-4¹H-17-oxa-3,9-diaza-2(7,2)-pyrrolo[2,1-f][1,2,4]triazina-5(4,1)-piperidina-4(4,1)-pyrazola-1(1,4)-benzenacycloheptadecaphan-10-one;
(Compound No. 28) (Z)-5,12,15-trioxa-3,9-diaza-2(7,2)-pyrrolo[2,1-f][1,2,4]triazina -1,4(1,4)-dibenzenacyclopentadecaphan-10-one;
(Compound No. 29) (Z)-5,17-dioxa-3,9-diaza-2(7,2)-pyrrolo[2,1-f][1,2,4]triazina-1 ,4(1,4)-dibenzenacycloheptadecaphan-10-one;
(Compound No. 30) (Z)-5,12-dioxa-3,9-diaza-2(7,2)-pyrrolo[2,1-f][1,2,4]triazina-1 ,4(1,4)-dibenzenacyclododecaphane;
(Compound No. 31) (Z)-1-(5,12-dioxa-3,9-diaza-2(7,2)-pyrrolo[2,1-f][1,2,4]tria zina-1,4(1,4)-dibenzenacyclododecapan-9-y1)propan-1-one;
(Compound No. 32) (Z)-5,12-dioxa-3,9-diaza-2(7,2)-pyrrolo[2,1-f][1,2,4]triazina-1,4(1,4)-dibenzenacyclododecaphan-9-yl(4-chlorophenyl)methanone;
(Compound No. 33) (2¹Z,7Z)-5,10-dioxa-3-aza-2(7,2)-pyrrolo[2,1-f][1,2,4]tr i az ina-1,4(1,4)-dibenzenacyclodecaphan-7-ene;
(Compound No. 34) (Z)-42-methoxy-5,12,15-trioxa-3,9-diaza-2(7,2)-pyrrolo[2,1-f][1,2 ,4]triazina-1,4(1,4)-dibenzenacyclopentadecaphan-10-one;
(Compound No. 35) (2¹Z,4⁴E)-4¹*H-*11,14-dioxa-3,8-diaza-2(7,2)-pyrrolo[2,1-f][1,2,4]triazina-4(4,1)-pyrazola-1(1,4)-benzenacyclotetradecaphan-9-one;
(Compound No. 36): (2¹Z,4⁴E)-4¹H-14-oxa-3,8-diaza-2(7,2)-pyrrolo[2,1-f][1,2,4]triazina -4(4,1)-pyrazola-1(1,4)-benzenacyclotetradecaphan-9-one;
(Compound No. 37): (2¹Z,4⁴E)-4¹*H-*16-oxa-3,8-diaza-2(7,2)-pyrrolo[2,1-f][1,2,4]triazina-4(4,1)-pyrazola-1(1,4)-benzenacyclohexadecaphan-9-one;
(Compound No. 38): (Z)-5,15-dioxa-3,9-diaza-2(7,2)-pyrrolo[2,1-f][1,2,4]triazina- 1,4(1,4)-dibenzenacyclopentadecaphan-10-one;
(Compound No. 39) *N*-(3-(2-((4-(4-(5-((2-(2,6-dioxopiperidin-3-yl)-1,3-dioxoisoindolin-4-yl)oxy)pentyl)piperidin-1-yl)phenyl)amino)pyrrolo[2,1-f][1,2,4]triazin-7-yl)phenyl)methanesulfonamide;
(Compound No. 40) *N*-(3-(2-((4-(4-(3-((2-(2,6-dioxopiperidin-3-yl)-1,3-dioxoisoindolin-4-yl)oxy)propyl)piperidin-1-yl)phenyl)amino)pyrrolo[2,1-f][1,2,4]triazin-7-yl)phenyl)methanesulfonamide;
(Compound No. 41) *N*-(3-(2-((4-(4-(7-((2-(2,6-dioxopiperidin-3-yl)-1,3-dioxoisoindolin-4-yl)oxy)heptyl)piperidin-1-yl)phenyl)amino)pyrrolo[2,1-f][1,2,4]triazin-7-yl)phenyl)methanesulfonamide;
(Compound No. 42) *N*-(3-(2-((4-(4-(11-((2-(2,6-dioxopiperidin-3-yl)-1,3-dioxoisoindolin-4-yl)oxy)undecyl)piperidin-1-yl)phenyl)amino)pyrrolo [2,1-f][1,2,4]triazin-7-yl)phenyl)methanesulfonamide;
(Compound No. 43) *N*-(3-(2-((4-(4-(2-((2-(2,6-dioxopiperidin-3-yl)-1,3-dioxoisoindolin-4-yl)oxy)ethyl)piperidin-1-yl)phenyl)amino)pyrrolo[2,1-f][1,2,4]triazin-7-yl)phenyl)methanesulfonamide;
(Compound No. 44) *N*-(3-(2-((4-(4-(4-((2-(2,6-dioxopiperidin-3-yl)-1,3-dioxoisoindolin-4-yl)oxy)butyl)piperidin-1-yl)phenyl)amino)pyrrolo[2,1-f][1,2,4]triazin-7-yl)phenyl)methanesulfonamide;
(Compound No. 45) *N*-(3-(2-((4-(4-(6-((2-(2,6-dioxopiperidin-3-yl)-1,3-dioxoisoindolin-4-yl)oxy)hexyl)piperidin-1-yl)phenyl)amino)pyrrolo[2,1-f][1,2,4]triazin-7-yl)phenyl)methanesulfonamide;
(Compound No. 46) *N*-(3-(2-((4-(4-(8-((2-(2,6-dioxopiperidin-3-yl)-1,3-dioxoisoindolin-4-yl)oxy)octyl)piperidin-1-yl)phenyl)amino)pyrrolo[2,1-f][1,2,4]triazin-7-yl)phenyl)methanesulfonamide;
(Compound No. 47) *N*-(3-(2-((4-(4-(8-((2-(2,6-dioxopiperidin-3-yl)-1,3-dioxoisoindolin-4-yl)oxy)nonyl)piperidin-1-yl)phenyl)amino)pyrrolo[2,1-f][1,2,4]triazin-7-yl)phenyl)methanesulfonamide;
(Compound No. 48) *N*-(3-(2-((4-(4-(2-(2-((1,3-dioxo-2-(2-oxopiperidin-3-yl)isoindolin-4-yl)oxy)ethoxy)ethyl)piperazin-1-yl)phenyl)amino)pyrrolo[2,1-f][1,2,4]triazin-7-yl)phenyl) methanesulfonamide;
(Compound No. 49) *N*-(3-(2-((4-(4-(6-((1,3-dioxo-2-(2-oxopiperidin-3-yl)isoindolin-4-yl)oxy)hexyl)piperazin-1-yl)phenyl)amino)pyrrolo[2,1-f][1,2,4]triazin-7-yl)phenyl)methanesulfonamide;
(Compound No. 50) *N*-(3-(2-((4-(4-(8-((1,3-dioxo-2-(2-oxopiperidin-3-yl)isoindolin-4-yl)oxy)octyl)piperazin-1-yl)phenyl)amino)pyrrolo[2,1-f][1,2,4]triazin-7-yl)phenyl)methanesulfonamide;
(Compound No. 51) *N*-(3-(2-((4-(4-((1-(2-(2,6-dioxopiperidin-3-yl)-6-fluoro-1,3-dioxoisoindolin-5-yl)piperidin-4-yl)methyl)piperazin-1-yl)phenyl)amino)pyrrolo[2,1-f][1,2,4]trizine-7-yl)phenyl)methanesulfonamide;
(Compound No. 52) *N*-(3-(2-((1-(1-(9-((2-(2,6-dioxopiperidin-3-yl)-1,3-dioxoindolin-4-yl)oxy)nonyl)piperidin-4-yl)-1*H*pyrazol-4-yl)amino)pyrrolo[2,1-f][1,2,4]triazin-7-yl)phenyl)methanesulfonamide;
(Compound No. 53) 3-(2-((1-(1-(9-((2-(2,6-dioxopiperidin-3-yl)-1,3-dioxoindolin-4-yl)oxy)nonyl)piperidin-4-yl)-1H-pyrazol-4-yl)amino)pyrrolo[2,1-f][1,2,4]triazin-7-yl)-*N*-methylbenzamide;
(Compound No. 54) 2-(2,6-dioxopiperidin-3-yl)-4-((9-(4-(4-((7-phenylpyrrolo[2,1-f][1,2,4]triazin-2-yl)amino)-1*H-*pyrazol-1-yl)piperidin-1-yl)nonyl)oxy)isoindolin-1,3-dione;
(Compound No. 55) 2-(2,6-dioxopiperidin-3-yl)-4-((9-(4-(4-((7-(1-methyl-1*H-*pyrazole-4-1)pyrrolo[2,1-f][1,2,4]triazin-2-yl)amino)-1*H-*pyrazol-1-yl)piperidin-1-yl)nonyl)oxy)isoindolin-1,3-dione;
(Compound No. 56) 4-((9-(4-(4-((7-(1*H-*indol-6-yl)pyrrolo[2,1-f][1,2,4]triazine-2-yl)amino)-1*H-*pyrazol-1-yl)piperidin-1-yl)nonyl)oxy)-2-(2,6-dioxopiperidin-3-yl)isoindolin-1,3-dione;
(Compound No. 57) 2-(2,6-dioxopiperidin-3-yl)-4-((9-(4-(4-((7-(4-hydroxyphenyl)pyrrolo[2,1-f][1,2,4]triazin-2-yl)amino)-1*H-*pyrazol-1-yl)piperidin-1-yl)nonyl)oxy)isoindolin-1,3-dione;
(Compound No. 58) Methyl-7-(4-(2-((1-(1-(9-((2-(2,6-dioxopiperidin-3-yl)-1,3-dioxoisoindolin-4-yl)oxy)nonyl)piperidin-4-yl)-1*H-*pyrazol-4-yl)amino)pyrrolo[2,1-f][1,2,4]triazine -7-day)phenoxy)heptanoate;
(Compound No. 59) *N*-(3-(2-((4-(4-(5-((2-(2,6-dioxopiperidin-3-yl)-1,3-dioxoisoindolin-4-yl)oxy)pentyl)piperazin-1-yl)-2-methoxyphenyl)amino)pyrrolo[2,1-f][1,2,4]triazin-7-yl)phenyl)methanesulfonamide;
(Compound No. 60) *N*-(3-(2-((4-(4-(3-((2-(2,6-dioxopiperidin-3-yl)-1,3-dioxoindolin-4-yl)oxy)propyl)piperazin-1-yl)-2-methoxyphenyl)amino)pyrrolo[2,1-f][1,2,4]triazin-7-yl)phenyl)methanesulfonamide;
(Compound No. 61) *N*-(3-(2-((4-(4-(7-((2-(2,6-dioxopiperidin-3-yl)-1,3-dioxoindolin-4-yl)oxy)heptyl)piperazin-1-yl)-2-methoxyphenyl)amino)pyrrolo[2,1-f][1,2,4]triazin-7-yl)phenyl)methanesulfonamide;
(Compound No. 62) *N*-(3-(2-((4-(4-(11-((2-(2,6-dioxopiperidin-3-yl)-1,3-dioxoindolin-4-yl)oxy)undecyl)piperazin-1-yl)-2-methoxyphenyl)amino)pyrrolo[2,1-f][1,2,4]triazin-7-yl)phenyl)methanesulfonamide;
(Compound No. 63) *N*-(3-(2-((4-(4-(2-((2-(2,6-dioxopiperidin-3-yl)-1,3-dioxoindolin-4-yl)oxy)ethyl)piperazin-1-yl)-2-methoxyphenyl)amino)pyrrolo[2,1-f][1,2,4]triazin-7-yl)phenyl)methanesulfonamide;
(Compound No. 64) *N*-(3-(2-((4-(4-(4-((2-(2,6-dioxopiperidin-3-yl)-1,3-dioxoindolin-4-yl)oxy)butyl)piperazin-1-yl)-2-methoxyphenyl)amino)pyrrolo[2,1-f][1,2,4]triazin-7-yl)phenyl)methanesulfonamide;
(Compound No. 65) *N*-(3-(2-((4-(4-(6-((2-(2,6-dioxopiperidin-3-yl)-1,3-dioxoindolin-4-yl)oxy)hexyl)piperazin-1-yl)-2-methoxyphenyl)amino)pyrrolo[2,1-f][1,2,4]triazin-7-yl)phenyl)methanesulfonamide;
(Compound No. 66) *N*-(3-(2-((4-(4-(8-((2-(2,6-dioxopiperidin-3-yl)-1,3-dioxoindolin-4-yl)oxy)octyl)piperazin-1-yl)-2-methoxyphenyl)amino)pyrrolo[2,1-f][1,2,4]triazin-7-yl)phenyl)methanesulfonamide;
(Compound No. 67) *N*-(3-(2-((4-(4-(9-((2-(2,6-dioxopiperidin-3-yl)-1,3-dioxoindolin-4-yl)oxy)nonyl)piperazin-1-yl)-2-methoxyphenyl)amino)pyrrolo[2,1-f][1,2,4]triazin-7-yl)phenyl)methanesulfonamide;
(Compound No. 68) *N*-(3-(2-((4-(4-(2-(2-((1,3-dioxo-2-(2-oxopiperidin-3-yl)isoindolin-4-yl)oxy)ethoxy)ethyl)piperazin-1-yl)-2-methoxyphenyl)amino)pyrrolo[2,1-f][1,2,4]triazine-7-1)phenyl)methanesulfonamide;
(Compound No. 69) *N*-(3-(2-((4-(4-(6-((1,3-dioxo-2-(2-oxopiperidin-3-yl)isoindolin-4-yl)oxy)hexyl)piperazin-1-yl)-2-methoxyphenyl)amino)pyrrolo[2,1-f][1,2,4]triazin-7-yl)phenyl)methanesulfonamide;
(Compound No. 70) *N*-(3-(2-((4-(4-(8-((1,3-dioxo-2-(2-oxopiperidin-3-yl)isoindoline-4-yl)oxy)octyl)piperazin-1-yl)-2-methoxyphenyl)amino)pyrrolo[2,1-f][1,2,4]triazin-7-yl)phenyl)methanesulfonamide;
(Compound No. 71) *N*-(3-(2-((6-(4-(5-((2-(2,6-dioxopiperidin-3-yl)-1,3-dioxoindolin-4-yl)oxy)pentyl)piperazin-1-yl)pyridin-3-yl)amino)pyrrolo[2,1-f][1,2,4]triazin-7-yl)phenyl)methanesulfonamide;
(Compound No. 72) *N*-(3-(2-((6-(4-(3-((2-(2,6-dioxopiperidin-3-yl)-1,3-dioxoindoline-4-yl)oxy)propyl)piperazin-1-yl)pyridin-3-yl)amino)pyrrolo[2,1-f][1,2,4]triazin-7-yl)phenyl)methanesulfonamide;
(Compound No. 73) *N*-(3-(2-((6-(4-(7-((2-(2,6-dioxopiperidin-3-yl)-1,3-dioxoindolin-4-yl)oxy)heptyl)piperazin-1-yl)pyridin-3-yl)amino)pyrrolo[2,1-f][1,2,4]triazin-7-yl)phenyl)methanesulfonamide;
(Compound No. 74) *N*-(3-(2-((6-(4-(11-((2-(2,6-dioxopiperidin-3-yl)-1,3-dioxoindoline-4-yl)oxy)undecyl)piperazin-1-yl)pyridin-3-yl)amino)pyrrolo[2,1-f][1,2,4]triazin-7-yl)phenyl)methanesulfonamide;
(Compound No. 75) *N*-(3-(2-((6-(4-(2-((2-(2,6-dioxopiperidin-3-yl)-1,3-dioxoindolin-4-yl)oxy)ethyl)piperazin-1-yl)pyridin-3-yl)amino)pyrrolo[2,1-f][1,2,4]triazin-7-yl)phenyl)methanesulfonamide;
(Compound No. 76) *N*-(3-(2-((6-(4-(4-((2-(2,6-dioxopiperidin-3-yl)-1,3-dioxoindoline-4-yl)oxy)butyl)piperazin-1-yl)pyridin-3-yl)amino)pyrrolo[2,1-f][1,2,4]triazin-7-yl)phenyl)methanesulfonamide;
(Compound No. 77) *N*-(3-(2-((6-(4-(6-((2-(2,6-dioxopiperidin-3-yl)-1,3-dioxoindolin-4-yl)oxy)hexyl)piperazin-1-yl)pyridin-3-yl)amino)pyrrolo[2,1-f][1,2,4]triazin-7-yl)phenyl)methanesulfonamide;
(Compound No. 78) *N*-(3-(2-((6-(4-(8-((2-(2,6-dioxopiperidin-3-yl)-1,3-dioxoindolin-4-yl)oxy)octyl)piperazin-1-yl)pyridin-3-yl)amino)pyrrolo[2,1-f][1,2,4]triazin-7-yl)phenyl)methanesulfonamide;
(Compound No. 79) *N*-(3-(2-((6-(4-(9-((2-(2,6-dioxopiperidin-3-yl)-1,3-dioxoindolin-4-yl)oxy)nonyl)piperazin-1-yl)pyridin-3-yl)amino)pyrrolo[2,1-f][1,2,4]triazin-7-yl)phenyl)methanesulfonamide;
(Compound No. 80) *N*-(3-(2-((6-(4-(2-(2-((1,3-dioxo-2-(2-oxopiperidin-3-yl)isoindolin-4-yl)oxy)ethoxy)ethyl)piperazin-1-yl)pyridin-3-yl)amino)pyrrolo[2,1-f][1,2,4]triazin-7-yl)phenyl)methanesulfonamide;
(Compound No. 81) *N*-(3-(2-((6-(4-(6-((1,3-dioxo-2-(2-oxopiperidin-3-yl)isoindolin-4-yl)oxy)hexyl)piperazin-1-yl)pyridin-3-yl)amino)pyrrolo[2,1-f][1,2,4]triazin-7-yl)phenyl)methanesulfonamide;
(Compound No. 82) *N*-(3-(2-((6-(4-(8-((1,3-dioxo-2-(2-oxopiperidin-3-yl)isoindolin-4-yl)oxy)octyl)piperazin-1-yl)pyridin-3-yl)amino)pyrrolo[2,1-f][1,2,4]triazin-7-yl)phenyl)methanesulfonamide;
(Compound No. 83) (2S,4R)-1-((S)-3,3-dimethyl-2-(2-(2-(4-(4-((7-(3-(methanesulfonamido)phenyl)pyrrolo[2,1-f][1,2,4]triazin-2-yl)amino)phenyl) piperazin-1-yl)ethoxy)acetaamino)butanoyl)-4-hydroxy-*N*-(4-(4-methylthiazol-5-yl)benzyl)pyrrolidin-2-carboxamide;
(Compound No. 84) (2S,4R)-1-((S)-3,3-dimethyl-2-(3-(2-(2-(4-(4-((7-(3-(methanesulfonamido)phenyl)pyrrolo[2,1-f][1,2,4]triazin-2-yl)amino) phenyl)piperazin-1-yl)ethoxy)ethoxy)propanamido)butanoyl)-4-hydroxy-*N*-(4-(4-methylthiazol-5-yl)benzyl)pyrrolidin-2-carboxamide;
(Compound No. 85) (2S,4R)-1-((S)-3,3-dimethyl-2-(3-(2-(2-(4-(4-((7-(3-(methylsulfonamido)phenyl)pyrrolo[2,1-f][1,2,4]triazin-2-yl)amino) phenyl)piperazin-1-yl)ethoxy)ethoxy)propanamido)butanoyl)-4-hydroxy-*N*-((S)-1-(4-(4-methylthiazol-5-yl)phenyl)ethyl)pyrrolidin-2-carboxamide;
(Compound No. 86) 2-(2,6-dioxopiperidin-3-yl)-4-((5-(4-(5-((7-(4-(trifluoromethoxy)phenyl)pyrrolo[2,1-f][1,2,4]triazin-2-yl)amino)pyridin-2-yl)piperazin-1-yl)pentyl)oxy)isoindolin-1,3-dione;
(Compound No. 87) 4-((5-(4-(5-((7-(3,5-difluorophenyl)pyrrolo[2,1-f][1,2,4]triazine-2-yl)amino)pyridin-2-yl)piperazin-1-yl)pentyl)oxy)-2-(2,6-dioxopiperidin-3-yl)isoindolin-1,3-dione;
(Compound No. 88) 4-((5-(4-(5-((7-(2-aminopyridin-5-yl)pyrrolo[2,1-f][1,2,4]triazine-2-yl)amino)pyridin-2-yl)piperazin-1-al)pentyl)oxy)-2-(2,6-dioxopiperidin-3-yl)isoindolin-1,3-dione;
(Compound No. 89) 2-(2,6-dioxopiperidin-3-yl)-4-((5-(4-(5-((7-(quinolin-3-yl)pyrrolo[2,1-f][1,2,4]triazin-2-yl)amino)pyridin-2-yl)piperazin-1-yl)pentyl)oxy)isoindoline-1,3-dione;
(Compound No. 90) *tert*-butyl4-(3-(2-((6-(4-(5-((2-(2,6-dioxopiperidin-3-yl)-1,3-dioxoisoindolin-4-yl)oxy)pentyl)piperazin-1-yl)pyridin-3-yl)amino)pyrrolo[2,1-f][1,2,4]triazin-7-yl)benzyl)piperazin-1-carboxylate;
(Compound No. 91) (E)-2-(2,6-dioxopiperidin-3-yl)-4-((5-(4-(5-((7-styrylpyrrolo[2,1-f][1,2,4]triazin-2-yl)amino)pyridin-2-yl)piperazin-1-yl)pentyl)oxy)isoindolin-1,3-dione;
(Compound No. 92) *tert*-butyl4-(4-(2-((6-(4-(5-((2-(2,6-dioxopiperidin-3-yl)-1,3-dioxoisoindolin-4-yl)oxy)pentyl)piperazin-1-yl)pyridin-3-yl)amino)pyrrolo[2,1-f][1,2,4]triazin-7-yl)-1*H-*pyrazol-1-yl)piperidin-1-carboxylate;
(Compound No. 93) 4-((5-(4-(5-((7-(4-(dimethylamino)phenyl)pyrrolo[2,1-f][1,2,4]triazine-2-yl)amino)pyridin-2-yl) piperazin-1-yl)pentyl)oxy)-2-(2,6-dioxopiperidin-3-yl)isoindolin-1,3-dione;
(Compound No. 94) 2-(2,6-dioxopiperidin-3-yl)-4-((5-(4-(5-((7-(pyridin-4-yl)pyrrolo[2,1-f][1,2,4]triazin-2-yl)amino)pyridin-2-yl)piperazin-1-yl)pentyl)oxy)isoindolin-1,3-dione;
(Compound No. 95) 2-(2,6-dioxopiperidin-3-yl)-4-((5-(4-(5-((7-(thiazol-5-yl)pyrrolo[2,1-f][1,2,4]triazin-2-yl)amino)pyridin-2-yl)piperazin-1-yl)pentyl)oxy)isoindolin-1,3-dione;
(Compound No. 96) 2-(2,6-dioxopiperidin-3-yl)-4-((5-(4-(5-((7-(1-(tetrahydro-2*H-*pyran-2 -yl)-1*H-*pyrazol-5-yl)pyrrolo[2,1-f][1,2,4]triazin-2-yl)amino)pyridin-2-yl)piperazin-1-yl)pentyl)oxy)isoindolin-1,3-dione;
(Compound No. 97) 4-((5-(4-(5-((7-((3,5-difluorophenyl)ethynyl)pyrrolo[2,1-f][1,2,4]triazin-2-yl)amino)pyridin-2-yl)piperazin-1-yl)pentyl)oxy)-2-(2,6-dioxopiperidin-3-yl)isoindolin-1,3-dione;
(Compound No. 98) 2-(2,6-dioxopiperidin-3-yl)-4-((5-(4-(5-((7-((3-methoxyphenyl)ethynyl)pyrrolo[2,1-f][1,2,4]triazin-2-yl)amino)aminopyridin-2-yl)piperazin-1-yl)pentyl)oxy)isoindolin-1,3-dione;
(Compound No. 99) *N-*(3-(2-((4-(4-(2-(2-(3-(4-((4-(2-(2,6-dioxopiperidin-3-yl)-6-fluoro-1,3-dioxoisoindolin-5-yl)piperazin-1-yl)methyl)piperidin-1-yl)-3-oxopropoxy)ethoxy)ethyl)piperazin-1-yl)phenyl) amino)pyrrolo[2,1-f][1,2,4]triazin-7-yl)phenyl)methanesulfonamide;
(Compound No. 100) *N-*(3-(4-(3-(2,4-dihydroxy-5-isopropylphenyl)-5-hydroxy-4*H-*1,2,4-triazol-4-yl)phenoxy)propyl)-3-(2-(2-(4-(4-((7-(3-(methylsulfonamido)phenyl)pyrrolo[2,1-f][1,2,4]triazin-2-yl)amino)phenyl)piperazin-1-yl)ethoxy)ethoxy)propanamide;
(Compound No. 101) *N*-(2-((3-(4-(3-(2,4-dihydroxy-5-isopropylphenyl)-5-hydroxy-4*H-*1,2,4-triazol-4-yl)phenoxy)propyl)amino)-2-oxoethyl)-3-(2-(2-(4-(4-((7-(3-(methylsulfonamido)phenyl)pyrrolo[2,1-f][1,2,4]triazin-2-yl)amino)phenyl)piperazin-1-yl)ethoxy)ethoxy)propanamide;
(Compound No. 102) *N*-(3-(2-((4-(4-(2-(2-(3-(4-(4-(3-(2,4-dihydroxy-5-isopropylphenyl)-5-hydroxy-4*H-*1,2,4-triazol-4-yl)benzyl)piperazin-1-yl)-3-oxopropoxy)ethoxy)ethyl)piperazine-1-yl)phenyl)amino)pyrrolo[2,1-f][1,2,4] triazin-7-yl)phenyl)methanesulfonamide;
(Compound No. 103) *N-*(3-(2-((4-(4-(2-(2-(3-(4-((4-(4-(3-(2,4-dihydroxy-5-isopropylphenyl)-5-hydroxy-4*H-*1,2,4-triazol-4-yl)benzyl)piperazin-1-yl)methyl)piperidin-1-yl)-3-oxopropoxy)ethoxy)ethyl) piperazin-1-yl)phenyl)amino)pyrrolo[2,1-f][1,2,4]triazin-7-yl)phenyl) methanesulfonamide;
(Compound No. 104) *N*-(3-(2-((4-(4-(2-(2-(3-(4-((2-(2,4-dihydroxy-5-isopropylbenzoyl)isoindolin-5-yl)methyl)piperazin-1-yl)-3-oxopropoxy) ethoxy)ethyl)piperazin-1-yl)phenyl)amino)pyrrolo[2,1-fl[1,2,4]triazin-7-yl)phenyl)methanesulfonamide;
(Compound No. 105) *N*-(3-(2-((4-(4-(2-(2-(3-(4-((4-((2-(2,4-dihydroxy-5 -isopropylbenzoyl)isoindolin-5-yl)methyl)piperazin-1-yl)methyl)piperidin-1-yl)-3-oxopropoxy)ethoxy)ethyl)piperazin-1-yl)phenyl)amino)pyrrolo[2,1-f][1,2,4]triazin-7-yl)phenyl)methanesulfonamide;
(Compound No. 106) 2-amino-4-(2,4-dichloro-5-(2-(4-(3-(2-(2-(4-(4-((7-(3-(methylsulfonamido)phenyl)pyrrolo[2,1-f][1,2,4]triazin-2-yl)amino) phenyl)piperazin-1-yl)ethoxy)ethoxy)propanoyl)piperazin-1-yl)ethoxy)phenyl)-*N*-ethylthieno[2,3-d]pyrimidin-6-carboxamide;
(Compound No. 107) *N*-(3-(2-((4-(4-(4-((4-((2-(2,4-dihydroxy-5-isopropylbenzoyl)isoindolin-5-yl)methyl)piperazin-1-yl)methyl)benzoic)piperazin-1-yl)phenyl)amino)pyrrolo[2,1-f][1,2,4]triazine-7-1)phenyl)methanesulfonamide;
Compound No. 108) *N*-(3-(2-((4-(4-(1-((2-(2,4-dihydroxy-5-isopropylbenzoic)isoindolin-5-yl)methyl)piperidin-4-carbonyl)piperazin-1-yl) phenyl)amino)pyrrolo[2,1-f][1,2,4]triazin-7-yl)phenyl)methanesulfonamide
(Compound No. 109) 4-(2-((2¹Z,4⁴E)-4¹*H-*11-oxa-3,8-diaza-2(7,2)-pyrrolo[2,1-f][1,2,4]triazin-4(4,1)-pyrazolo-1(1,4)-benzenacycloundecaphan -8-yl)ethoxy)-2-(2,6-dioxopiperidin-3- 1)isoindolin-1,3-dione;
(Compound No. 110) 4-(3-((2¹Z,4⁴E)-4¹*H*-11-oxa-3,8-diaza-2(7,2)-pyrrolo[2,1-f][1,2,4]triazin-4(4,1)-pyrazolo-1(1,4)-benzenacycloundecaphan -8-yl)propoxy)-2-(2,6-dioxopiperidin-3-yl)isoindolin-1,3-dione;
(Compound No. 111) 4-(2-(2-((2¹Z,4⁴E)-4¹*H-*11-oxa-3,8-diaza-2(7,2)-pyrrolo[2,1-f][1,2,4]triazin-4(4,1)-pyrazolo-1(1,4)-benzenacycloundecaphan -8-yl)ethoxy)ethoxy)-2-(2-oxopiperidin-3-yl)isoindolin-1,3-dione;
(Compound No. 112) 4-((6-((2¹Z,4⁴E)-4¹*H-*11-oxa-3,8-diaza-2(7,2)-pyrrolo[2,1-f][1,2,4]triazin-4(4,1)-pyrazol-1(1,4)-benzenacycloundecaphan - 8-yl)hexyl)oxy)-2-(2,6-dioxopiperidin-3-yl)isoindolin-1,3-dione;
(Compound No. 113) 4-((8-((2¹Z,4⁴E)-4¹*H-*11-oxa-3,8-diaza-2(7,2)-pyrrolo[2,1-fl[1,2,4]triazin-4(4,1)-pyrazol-1(1,4)-benzenacycloundecaphan - 8-yl)octyl)oxy)-2-(2,6-dioxopiperidin-3-1)isoindolin-1,3-dione;
(Compound No. 114) 4-((8-((2¹Z,4⁴E)-4¹*H*-11-oxa-3,8-diaza-2(7,2)-pyrrolo[2,1-fl[1,2,4]triazin-4(4,1)-pyrazol-1(1,4)-benzenacycloundecaphan - 8-yl)octyl)oxy)-2-(2-oxopiperidin-3-yl)isoindolin-1,3-dione;
(Compound No. 115) 4-((11-((2¹Z,4⁴E)-4¹*H-*11-oxa-3,8-diaza-2(7,2)-pyrrolo[2,1-fl[1,2,4]triazin-4(4,1)-pyrazol-1(1,4)-benzenacycloundecaphan - 8-yl)undecyl)oxy)-2-(2,6-dioxopiperidin-3-yl)isoindolin-1,3-dione;
(Compound No. 116) 4-((9-((2¹Z,4⁴E)-4¹*H-*11-oxa-3,8-diaza-2(7,2)-pyrrolo[2,1-f][1,2,4]triazin-4(4,1)-pyrazola-1(1,4)-benzenacycloundecaphan -8-yl)nonyl)oxy)-2-(2,6-dioxopiperidin-3-yl)isoindolin-1,3-dione;
(Compound No. 117) 5-(4-((1-(3-(2-(2-((2¹Z,4⁴E)-4¹*H-*11-oxa-3,8-diaza-2(7,2)-pyrrolo[2,1-f][1,2,4]triazin-4(4,1)-pyrazola-1(1,4)-benzenacycloundecaphan-8-yl)ethoxy)ethoxy))propanoyl)piperidin-4-yl)methyl)piperazin-1-yl)-2-(2,6-dioxopiperidin-3-yl)-6-fluoroisoindolin-1, 3-dione;
(Compound No. 118) 3-(2-(2-((2¹Z,4⁴E)-4¹H-11-oxa-3,8-diaza-2(7,2)-pyrrolo[2,1-f][1,2,4]triazin-4(4,1)-pyrazola-1(1,4)-benzenacycloundecaphan -8-yl)ethoxy)ethoxy)-*N-*(3-(4-(3-(2,4-dihydroxy-5-isopropylphenyl)-5-hydroxy-4*H*-1,2,4-triazol-4-yl)phenoxy)propyl)propanamide;
(Compound No. 119) 3-(2-(2-((2¹Z,4⁴E)-4¹*H-*11-oxa-3,8-diaza-2(7,2)-pyrrolo[2,1-f][1,2,4]triazin-4(4,1)-pyrazol-1(1,4)-benzenacycloundecaphan-8-yl)ethoxy)ethoxy)-*N*-(2-((3-(4-(3-(2,4-dihydroxy-5-isopropylphenyl)-5-hydroxy-4*H*-1,2,4-triazol-4-yl)phenoxy)propyl)amino)-2-oxoethyl)propanamide;
(Compound No. 120) 3-(2-(2-((2¹Z,4⁴E)-4¹*H-*11-oxa-3,8-diaza-2(7,2)-pyrrolo[2,1-f][1,2,4]triazin-4(4,1)-pyrazolo-1(1,4)-benzenacycloundecaphan -8-yl)ethoxy)ethoxy)-1-(4-(4-(3-(2,4-dihydroxy-5-isopropylphenyl)-5-hydroxy-4*H-*1,2,4-triazol-4-yl)benzyl)piperazin-1-yl)propan-1-one;
(Compound No. 121) 3-(2-(2-((2¹Z,4⁴E)-4¹*H*-11-oxa-3,8-diaza-2(7,2)-pyrrolo[2,1-f][1,2,4]triazin-4(4,1)-pyrazolo-1(1,4)-benzenacycloundecaphan-8-yl)ethoxy)ethoxy)-1-(4-((4-(4-(3-(2,4-dihydroxy-5-isopropylphenyl)-5-hydroxy-4*H-*1,2,4-triazol-4-yl)benzyl)piperazin-1-yl)methyl)piperidin-1-yl)propan-1-one;
(Compound No. 122) 3-(2-(2-((2¹Z,4⁴E)-4¹*H*-11-oxa-3,8-diaza-2(7,2)-pyrrolo[2,1-f][1,2,4]triazin-4(4,1)-pyrazol-1(1,4)-benzenacycloundecaphan-8-yl)ethoxy)ethoxy)-1-(4-((2-(2,4-dihydroxy-5-isopropylbenzoyl)isoindolin-5-yl)methyl)piperazin-1-yl)propan-1-one;
(Compound No. 123) 3-(2-(2-((2¹Z,4⁴E)-4¹*H-*11-oxa-3,8-diaza-2(7,2)-pyrrolo[2,1-f][1,2,4]triazin-4(4,1)-pyrazola-1(1,4)-benzenacycloundecaphan-8-yl)ethoxy)ethoxy)-1-(4-((4-((2-(2,4-dihydroxy-5-isopropylbenzoyl)isoindolin-5-yl)methyl)piperazin-1-yl)methyl)piperidin-1-yl)propan-1-one;
(Compound No. 124) 4-(5-(2-(4-(3-(2-(2-((2¹Z,4⁴E)-4¹*H*-11-oxa-3,8-diaza-2(7,2)-pyrrolo[2,1-f][1,2,4]triazin-4(4,1)-pyrazola-1(1,4)-benzenacycloundecaphan-8-yl)ethoxy)ethoxy)propanoyl)piperazin-1-yl)ethoxy)-2,4-dichlorophenyl)-2-amino-*N*-ethylthieno[2,3-d]pyrimidin-6-carboxamide;
(Compound No. 125) ((2¹Z,4⁴E)-4¹*H*-11-oxa-3,8-diaza-2(7,2)-pyrrolo[2,1-f][1,2,4]triazin-4(4,1)-pyrazola-1(1,4)-benzenacycloundecaphan-8-yl)(4-((4-((2-(2,4-dihydroxy-5-isopropylbenzoyl)isoindolin-5-yl)methyl)piperazin-1-yl) methyl)phenyl)methanone;
(Compound No. 126) ((2¹Z,4⁴E)-4¹*H*-11-oxa-3,8-diaza-2(7,2)-pyrrolo[2,1-f][1,2,4]triazin-4(4,1)-pyrazola-1(1,4)-benzenacycloundecaphane-8-yl)(1-((2-(2,4-dihydroxy-5-isopropylbenzoyl)isoindolin-5-yl)methyl)piperidin-4-yl)methanone.

In one aspect, the present invention provides the 2,7-substituted pyrrolo[2,1-f][1,2,4]triazine compound represented by Chemical Formula 1, a pharmaceutically acceptable salt thereof, a hydrate thereof, or a solvate thereof, wherein the pharmaceutically acceptable salts are salts of inorganic acids or organic acids selected from the group consisting of hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, hydroxymaleic acid, benzoic acid, hydroxybenzoic acid, phenylacetic acid, salicylic acid, methanesulfonic acid, benzenesulfonic acid, and toluenesulfonic acid.

In another aspect, the present invention provides a pharmaceutical composition for prevention, alleviation, or treatment of cancer, wherein one of the compounds from the present invention is included as an active ingredient.

In another aspect, the present invention provides the pharmaceutical composition for prevention, alleviation, or treatment of cancer, which is a disease induced by abnormal cell growth due to protein kinase activity.

In another aspect, the present invention provides the compound having the pharmaceutical composition for prevention, alleviation, or treatment of cancer with inhibition efficacy of over 80% against protein kinases at 1 uM.

In another aspect, the present invention provides the pharmaceutical composition for use in prevention, alleviation, or treatment of cancer, wherein one or more of the protein kinases selected from the group consisting of ABL1, ABL2, ALK, ARK5, Aurora A, Aurora B, Aurora C, AXL, BLK, BMX, BRK, c-Kit, c-MER, c-Src, CAMK2a, CAMK2d, CDK16, , CDK17, CDK18, CDK2/cyclin A, CDK2/cyclin 0, CDK4/cyclin D1, CDK4/cyclin D2, CDK4/cyclin D3, CDK6/cyclin D1, CDK6/cyclin D3, CDK7/cyclin H, CDK9/cyclin K, CDK9/cyclin T1, CDK9/cyclin T2, CK2a, CK2a2, CLK1, CLK2, CLK4, DAPK1, DAPK2, DDR1, DDR2, DRAK1, DYRK1/DYRK1A, DYRK1B, DYRK3, EPHA1, EPHA2, EPHA4, EPHA5, EPHA6, EPHA7, EPHB1, EPHB2, ERBB4/HER4, ERK7/MAPK15, ERN2/IRE2, FAK/PTK2, FER, FES/FPS, FGFR1, FGFR2, FGFR3, FGR, FLT1/VEGFR1, FLT3, FLT4/VEGFR3, FMS, FRK/PTK5, FYN, GLK/MAP4K3, GSK3a, HIPK2, HIPK3, HIPK4, HPK1/MAP4K1, IGF1R, IKKe/IKBKE, IR, IRAK1, IRR/INSRR, JAK1, JAK2, JAK3, JNK1, JNK2, JNK3, KDR/VEGFR2, KHS/MAP4K5, LCK, LIMK1, LIMK2, LRRK2, LYN, LYN B, MAK, MARK1, MARK2/PAR-1Ba, MARK3, MARK4, MAST3, MEKK2, MELK, MLCK2/MYLK2, MLK1/MAP3K9, MLK2/MAP3K10, MLK3/MAP3K11, MUSK, MYLK4, NEK1, NEK2, NEK5, NEK9, p70S6K/RPS6KB1, PAK4, PAK5, PAK6, PDGFRa, PDGFRb, PHKg1, PHKg2, PKAcb, PKCa, PKCb2, PKCg, PKCmu/PRKD1, PKCnu/PRKD3, PKD2/PRKD2, PKN1/PRK1, PLK4/SAK, PRKX, PYK2, RET, ROS/ROS1, RSK2, RSK3, RSK4, SIK1, SIK2, SLK/STK2, SNARK/NUAK2, STK16, STK22D/TSSK1, STK33, STK38/NDR1, STK38L/NDR2, STK39/STLK3, SYK, TBK1, TEC, TIE2/TEK, TLK2, TNK1, TRKA, TRKB, TRKC, TYK2, TYRO3/SKY, ULK1, ULK2, ULK3, WEE1, YES/YES1, YSK4/MAP3K19 and ZIPK/DAPK3.

In another aspect, the present invention provides the pharmaceutical composition for the prevention, alleviation, or treatment of cancer, wherein the diseases selected from the group consisting of stomach cancer, lung cancer, liver cancer, colon cancer, rectal cancer, pancreatic cancer, brain cancer, bone cancer, melanoma, breast cancer, sclerotic adenosis, uterine cancer, cervical cancer, oral cancer, esophageal cancer, thyroid cancer, parathyroid cancer, renal cancer, sarcoma, prostate cancer, ureter cancer, bladder cancer, leukemia, multiple myeloma, myelodysplastic syndrome, hematologic malignancies such as Hodgkin's disease and non-Hodgkin's lymphoma, and fibrosarcoma

### Hereinafter, the method for the preparation of the present invention is as follows.

The pharmaceutically acceptable salt of the 2,7-substituted pyrrolo[2,1-f][1,2,4]triazine compound represented by Chemical Formula 1 in the present invention can be prepared by conventional methods known in the art. The pharmaceutically acceptable salts should have low toxicity to the human body and should not adversely affect the biological activity and physicochemical properties of the compound. The pharmaceutically acceptable salts include acid addition salts of the alkaline compounds of Chemical Formula 1, alkali metal salts (e.g., sodium salts) and alkaline earth metal salts (e.g., calcium salts), and organic salts and acid addition salts of carboxylic acids of Chemical Formula 1, as well as amino acid addition salts. In the preparation of pharmaceutically acceptable salts, inorganinc acids and organic acids can be used. Inorganic acids may include hydrochloric acid, sulfuric acid, nitric acid, phosphoric acid, hypochlorous acid, and bromic acid. Organic acids may include formic acid, methanesulfonic acid, ethanesulfonic acid, p-toluenesulfonic acid, pimelic acid, malic acid, malonic acid, phthalic acid, succinic acid, lactic acid, gluconic acid, tartaric acid, salicylic acid, maleic acid, aspartic acid, and glutamic acid. Organic bases that can be used in the preparation of organic base addition salts include tris(hydroxymethyl)aminomethane and dicyclohexylamine. Amino acids that can be used in the preparation of amino acid addition salts include natural amino acids such as alanine and glycine.

The 2,7-substituted pyrrolo[2,1-f][1,2,4]triazine compound represented by Chemical Formula 1 in the present invention includes not only the pharmaceutically acceptable salts mentioned above but also all hydrates and solvates. Hydrates and solvates can be obtained by dissolving the 2,7-substituted pyrrolo[2,1-f][1,2,4]triazine compound represented by Chemical Formula 1 in a solvent capable of mixing with water, such as methanol, ethanol, acetone, or 1,4-dioxane, and then adding an acid or a base followed by crystallization or recrystallization. In such cases, solvates (especially hydrates) may be formed. Therefore, as compounds of the present invention, in addition to compounds containing various amounts of water, such as those prepared by methods like freeze-drying, chemically stoichiometric solvates including hydrates are also included.

A more detailed description of the substituents used to define the compounds according to the present invention is as follows:
In the present invention, the term "halogen atom" refers to fluorine, chlorine, bromine, and iodine atoms.

In the present invention, the term "alkyl group" refers to saturated hydrocarbon groups containing 1 to 6 carbon atoms, including methyl, ethyl, n-propyl, i-propyl, cyclopropyl, n-butyl, i-butyl, t-butyl, cyclobutyl, cyclopropylmethyl, n-pentyl, i-pentyl, neopentyl, t-pentyl, cyclopentyl, cyclobutylmethyl, n-hexyl, i-hexyl, cyclohexyl, cyclopentylmethyl, etc.

In the present invention, the term "haloalkyl group" refers to an alkyl group in which one or more hydrogen atoms are replaced by one or more halogen atoms, such as trifluoromethyl group.

In the present invention, the term "alkoxy group" refers to a hydroxy group in which one or more hydrogen atoms are substituted by one of the selected substituents from alkyl groups composed of C-C, including methoxy, ethoxy, n-propoxy, i-propoxy, n-butoxy, i-butoxy, and t-butoxy.

In the present invention, the term "heteroaryl group" refers to a monocyclic, bicyclic, or tricyclic aromatic heterocyclic moiety containing one or more heteroatoms, including but not limited to pyrrolyl, pyridinyl, thiophenyl, pyrazolyl, imidazolyl, oxazolyl, isoxazolyl, thiazolyl, isothiazolyl, triazolyl, oxadiazolyl, thiadiazolyl, tetrazolyl, pyridyl, pyrazinyl, pyrimidinyl, triazinyl, indolyl, isoindolyl, benzofuranyl, benzofurazanyl, dibenzofuranyl, isobenzofuranyl, indazolyl, benzimidazolyl, benzoxazolyl, benzisoxazolyl, benzothiazolyl, dibenzothiophenyl, naphthridinyl, benzisothiazolyl, quinolinyl, isoquinolinyl, quinoxalinyl, phthalazinyl, quinazolinyl, quinazolinyl, etc.

In the present invention, the term "heterocyclic group" refers to a heterocyclic ring system containing one or more heteroatoms, including but not limited to morpholinyl, piperidinyl, piperazinyl, N-protected piperazinyl, etc.

More particularly, the cyclic 2,7-substituted pyrrolo[2,1-f][1,2,4]triazine compound represented by Chemical Formula 1 is exemplified as follows:
(Compound No. 1) (2¹Z,4⁴E)-4¹*H-*11-oxa-3,8-diaza-2(7,2)-pyrrolo[2,1-f][1,2,4]triazina- 4(4,1)-pyrazola-1(1,4)-benzenacycloundecaphane;
(Compound No. 2) ((2¹Z,4⁴E)-8-methyl-4¹*H*-11-oxa-3,8-diaza-2(7,2)-pyrrolo[2,1-f][1,2,4]triazina-4(4,1)-pyrazola-1(1,4)-benzenacycloundecaphane;
(Compound No. 3) 1-((2¹Z,4⁴E)-4¹*H*-11-oxa-3,8-diaza-2(7,2)-pyrrolo[2,1-f][1,2,4]triazina-4(4,1)-pyrazola-1(1,4)-benzenacycloundecafphane-8-yl)ethan-1-one;
(Compound No. 4) 1-((2¹Z,4⁴E)-4¹*H*-11-oxa-3,8-diaza-2(7,2)-pyrrolo[2,1-f][1,2,4]triazina-4(4,1)-pyrazola-1(1,4)-benzenacycloundecaphane-8-yl)butan-1-one;
(Compound No. 5) ((2¹Z,4⁴E)-4¹H-11-oxa-3,8-diaza-2(7,2)-pyrrolo[2,1-f][1,2,4]triazina -4(4,1)-pyrazola-1(1,4)-benzenacycloundecaphane-8-yl)(4-chlorophenyl)methanone;
(Compound No. 6) (2¹Z,4⁴E)-*N*-cyclohexyl-4¹*H*-11-oxa-3,8-diaza-2(7,2)-pyrrolo[2,1-f][1,2,4]triazina-4(4,1)-pyrazola-1(1,4)-benzenacycloundecaphane-8-carboxamide;
(Compound No. 7) (2¹Z,4⁴E)-*N*-(3-methoxyphenyl)-4¹*H*-11-oxa-3,8-diaza-2(7,2)-pyrrolo[2,1-f][1,2,4]triazina-4(4,1)-pyrazola-1(1,4)-benzenecycloundecaphane-8-carboxamide;
(Compound No. 8) (2¹Z,4⁴E)-*N*-ethyl-4¹*H*-11-oxa-3,8-diaza-2(7,2)-pyrrolo[2,1-f][1,2,4]triazina-4(4,1)-pyrazola-1(1,4)-benzenacycloundecaphane-8-carboxamide;
(Compound No. 9) (2¹Z,4⁴E)-8-(methylsulfonyl)-4¹*H*-11-oxa-3,8-diaza-2(7,2)-pyrrolo[2,1-f][1,2,4]triazina-4(4,1)-pyrazola-1(1,4)-benzenecycloundecaphane;
(Compound No. 10) (2¹Z,4⁴E)-8-((3,4-difluorophenyl)sulfonyl)-4¹*H*-11-oxa-3,8-diaza-2(7,2)-pyrrolo [2,1-f][1,2,4]triazina-4(4,1)-pyrazola-1(1,4)-benzenecycloundecaphane;
(Compound No. 11) (2¹Z,4⁴E)-8-(phenylsulfonyl)-4¹*H*-11-oxa-3,8-diaza-2(7,2)-pyrrolo[2,1-f][1,2,4]triazina-4(4,1)-pyrazola-1(1,4)-benzenacycloundecaphane;
(Compound No. 12) (2¹Z,4⁴E)-8-(cyclopropylsulfonyl)-4¹*H*-11-oxa-3,8-diaza-2(7,2)-pyrrolo[2,1-f][1,2,4]triazina-4(4,1)-pyrazola-1(1,4)-benzenacycloundecaphane;
(Compound No. 13) 2¹Z,4⁴E)-8-(propylsulfonyl)-4¹*H*-11-oxa-3,8-diaza-2(7,2)-pyrrolo[2,1-f][1,2,4]triazina-4(4,1)-pyrazola-1(1,4)-benzenacycloundecaphane;
(Compound No. 14) (2¹Z,4⁴E)-8-((5-chlorothiophen-2-yl)sulfonyl)-4¹*H-*11-oxa-3,8-diaza-2(7,2)-pyrrolo[2,1-f][1,2,4]triazina-4(4,1)-pyrazola-1(1,4)-benzenacycloundecaphane;
(Compound No. 15) (2¹Z,4⁴E)-8-(cyclohexylsulfonyl)-4¹*H*-11-oxa-3,8-diaza-2(7,2)-pyrrolo[2,1-f][1,2,4]triazina-4(4,1)-pyrazola-1(1,4)-benzenacycloundecaphane;
(Compound No. 16) (Z)-5-oxa-3,9,15-triaza-2(7,2)-pyrrolo[2,1-f][1,2,4]triazina-1, 4(1,4)-dibenzenacyclopentadecaphan-10-one;
(Compound No. 17) (1⁴Z,2¹Z)-1¹H-5-oxa-3,9-diaza-2(7,2)-pyrrolo[2,1-f][1,2,4]triazina- 1(4,1)-pyrazola-4(1,4)-benzenacyclohexadecaphan-10-one;
(Compound No. 18) (1⁴Z,2¹Z,4⁴E)-1¹*H*,4¹*H*-3,8-diaza-2(7,2)-pyrrolo[2,1-f][1,2,4]triazina-1,4(4,1)-dipyrazolacyclopentadecaphan-9-one;
Compound No. 19) (1⁴Z,2¹Z)-1¹*H*-3-aza-2(7,2)-pyrrolo[2,1-f][1,2,4]triazina-5(1,4)-piperazina-1(4,1)-pyrazola-4(1,4)-benzenacyclododecaphane-9-one;
(Compound No. 20) (Z)-13-fluoro-5,17-dioxa-3,9-diaza-2(7,2)-pyrrolo[2,1-f][1,2,4 Itriazina-1,4(1,4)-dibenzenacycloheptadecaphan-10-one;
(Compound No. 21) (2¹Z,4⁴E)-13-fluoro-4¹H-16-oxa-3,8-diaza-2(7,2)-pyrrolo[2,1-f][1,2,4]triazina-4(4,1)-pyrazola-1(1,4)-benzenacyclohexadecaphan-9-one;
(Compound No. 22) (Z)-1³-fluoro-13-oxa-3-aza-2(7,2)-pyrrolo[2,1-f][1,2,4]triazina-5(1,4)-piperazina-1,4(1,4)-dibenzenacyclotridecaphan-6-one;
(Compound No. 23) (Z)-17-oxa-3,9-diaza-2(7,2)-pyrrolo[2,1-f][1,2,4]triazina-5(1,4)-piperazina-1,4(1,4)-dibenzenacycloheptadecaphan-10-one;
(Compound No. 24) (Z)-13-oxa-3-aza-2(7,2)-pyrrolo[2,1-f][1,2,4]triazina-5(1,4)-piperazina-1,4(1,4)-dibenzenacyclotridecaphan-6-one;
(Compound No. 25) (2¹Z,4⁴E)-4¹*H-*11-oxa-3-aza-2(7,2)-pyrrolo[2,1-f][1,2,4]triazina-5(4,1)-piperidina-4(4,1)-pyrazola-1(1,4)-benzenacycloundecaphan-6-one;
(Compound No. 26) (2¹Z,4⁴E)-4¹*H*-15-oxa-3,9-diaza-2(7,2)-pyrrolo[2,1-f][1,2,4]triazina-5(4,1)-piperidina-4(4,1)-pyrazola-1(1,4)-benzenacyclopentadecaphan-10-one;
(Compound No. 27) (2¹Z,4⁴E)-4¹H-17-oxa-3,9-diaza-2(7,2)-pyrrolo[2,1-f][1,2,4]triazina-5(4,1)-piperidina-4(4,1)-pyrazola-1(1,4)-benzenacycloheptadecaphan-10-one;
(Compound No. 28) (Z)-5,12,15-trioxa-3,9-diaza-2(7,2)-pyrrolo[2,1-f][1,2,4]triazina -1,4(1,4)-dibenzenacyclopentadecaphan-10-one;
(Compound No. 29) (Z)-5,17-dioxa-3,9-diaza-2(7,2)-pyrrolo[2,1-f][1,2,4]triazina-1 ,4(1,4)-dibenzenacycloheptadecaphan-10-one;
(Compound No. 30) (Z)-5,12-dioxa-3,9-diaza-2(7,2)-pyrrolo[2,1-f][1,2,4]triazina-1 ,4(1,4)-dibenzenacyclododecaphane;
(Compound No. 31) (Z)-1-(5,12-dioxa-3,9-diaza-2(7,2)-pyrrolo[2,1-f][1,2,4]tria zina-1,4(1,4)-dibenzenacyclododecapan-9-yl)propan-1-one;
(Compound No. 32) (Z)-5,12-dioxa-3,9-diaza-2(7,2)-pyrrolo[2,1-f][1,2,4]triazina-1,4(1,4)-dibenzenacyclododecaphan-9-yl(4-chlorophenyl)methanone;
(Compound No. 33) (2¹Z,7Z)-5,10-dioxa-3-aza-2(7,2)-pyrrolo[2,1-f][1,2,4]tr i az ina-1,4(1,4)-dibenzenacyclodecaphan-7-ene;
(Compound No. 34) (Z)-42-methoxy-5,12,15-trioxa-3,9-diaza-2(7,2)-pyrrolo[2,1-f][1,2 ,4]triazina-1,4(1,4)-dibenzenacyclopentadecaphan-10-one;
(Compound No. 35) (2¹Z,4⁴E)-4¹*H-*11,14-dioxa-3,8-diaza-2(7,2)-pyrrolo[2,1-f][1,2,4]triazina-4(4,1)-pyrazola-1(1,4)-benzenacyclotetradecaphan-9-one;
(Compound No. 36): (2¹Z,4⁴E)-4¹H-14-oxa-3,8-diaza-2(7,2)-pyrrolo[2,1-f][1,2,4]triazina -4(4,1)-pyrazola-1(1,4)-benzenacyclotetradecaphan-9-one;
(Compound No. 37): (2¹Z,4⁴E)-4¹*H*-16-oxa-3,8-diaza-2(7,2)-pyrrolo[2,1-f][1,2,4]triazina-4(4,1)-pyrazola-1(1,4)-benzenacyclohexadecaphan-9-one;
(Compound No. 38): (Z)-5,15-dioxa-3,9-diaza-2(7,2)-pyrrolo[2,1-f][1,2,4]triazina- 1,4(1,4)-dibenzenacyclopentadecaphan-10-one;
(Compound No. 39) *N*-(3-(2-((4-(4-(5-((2-(2,6-dioxopiperidin-3-yl)-1,3-dioxoisoindolin-4-yl)oxy)pentyl)piperidin-1-yl)phenyl)amino)pyrrolo[2,1-f][1,2,4]triazin-7-yl)phenyl)methanesulfonamide;
(Compound No. 40) *N*-(3-(2-((4-(4-(3-((2-(2,6-dioxopiperidin-3-yl)-1,3-dioxoisoindolin-4-yl)oxy)propyl)piperidin-1-yl)phenyl)amino)pyrrolo[2,1-f][1,2,4]triazin-7-yl)phenyl)methanesulfonamide;
(Compound No. 41) *N*-(3-(2-((4-(4-(7-((2-(2,6-dioxopiperidin-3-yl)-1,3-dioxoisoindolin-4-yl)oxy)heptyl)piperidin-1-yl)phenyl)amino)pyrrolo[2,1-f][1,2,4]triazin-7-yl)phenyl)methanesulfonamide;
(Compound No. 42) *N*-(3-(2-((4-(4-(11-((2-(2,6-dioxopiperidin-3-yl)-1,3-dioxoisoindolin-4-yl)oxy)undecyl)piperidin-1-yl)phenyl)amino)pyrrolo [2,1-f][1,2,4]triazin-7-yl)phenyl)methanesulfonamide;
(Compound No. 43) *N*-(3-(2-((4-(4-(2-((2-(2,6-dioxopiperidin-3-yl)-1,3-dioxoisoindolin-4-yl)oxy)ethyl)piperidin-1-yl)phenyl)amino)pyrrolo[2,1-f][1,2,4]triazin-7-yl)phenyl)methanesulfonamide;
(Compound No. 44) *N*-(3-(2-((4-(4-(4-((2-(2,6-dioxopiperidin-3-yl)-1,3-dioxoisoindolin-4-yl)oxy)butyl)piperidin-1-yl)phenyl)amino)pyrrolo[2,1-f][1,2,4]triazin-7-yl)phenyl)methanesulfonamide;
(Compound No. 45) *N*-(3-(2-((4-(4-(6-((2-(2,6-dioxopiperidin-3-yl)-1,3-dioxoisoindolin-4-yl)oxy)hexyl)piperidin-1-yl)phenyl)amino)pyrrolo[2,1-f][1,2,4]triazin-7-yl)phenyl)methanesulfonamide;
(Compound No. 46) *N*-(3-(2-((4-(4-(8-((2-(2,6-dioxopiperidin-3-yl)-1,3-dioxoisoindolin-4-yl)oxy)octyl)piperidin-1-yl)phenyl)amino)pyrrolo[2,1-f][1,2,4]triazin-7-yl)phenyl)methanesulfonamide;
(Compound No. 47) *N*-(3-(2-((4-(4-(8-((2-(2,6-dioxopiperidin-3-yl)-1,3-dioxoisoindolin-4-yl)oxy)nonyl)piperidin-1-yl)phenyl)amino)pyrrolo[2,1-f][1,2,4]triazin-7-yl)phenyl)methanesulfonamide;
(Compound No. 48) *N*-(3-(2-((4-(4-(2-(2-((1,3-dioxo-2-(2-oxopiperidin-3-yl)isoindolin-4-yl)oxy)ethoxy)ethyl)piperazin-1-yl)phenyl)amino)pyrrolo[2,1-f][1,2,4]triazin-7-yl)phenyl) methanesulfonamide;
(Compound No. 49) *N*-(3-(2-((4-(4-(6-((1,3-dioxo-2-(2-oxopiperidin-3-yl)isoindolin-4-yl)oxy)hexyl)piperazin-1-yl)phenyl)amino)pyrrolo[2,1-f][1,2,4]triazin-7-yl)phenyl)methanesulfonamide;
(Compound No. 50) *N*-(3-(2-((4-(4-(8-((1,3-dioxo-2-(2-oxopiperidin-3-yl)isoindolin-4-yl)oxy)octyl)piperazin-1-yl)phenyl)amino)pyrrolo[2,1-f][1,2,4]triazin-7-yl)phenyl)methanesulfonamide;
(Compound No. 51) *N*-(3-(2-((4-(4-((1-(2-(2,6-dioxopiperidin-3-yl)-6-fluoro-1,3-dioxoisoindolin-5-yl)piperidin-4-yl)methyl)piperazin-1-yl)phenyl)amino)pyrrolo[2,1-f][1,2,4]trizine-7-yl)phenyl)methanesulfonamide;
(Compound No. 52) *N*-(3-(2-((1-(1-(9-((2-(2,6-dioxopiperidin-3-yl)-1,3-dioxoindolin-4-yl)oxy)nonyl)piperidin-4-yl)-1*H*-pyrazol-4-yl)amino)pyrrolo[2,1-f][1,2,4]triazin-7-yl)phenyl)methanesulfonamide;
(Compound No. 53) 3-(2-((1-(1-(9-((2-(2,6-dioxopiperidin-3-yl)-1,3-dioxoindolin-4-yl)oxy)nonyl)piperidin-4-yl)-1H-pyrazol-4-yl)amino)pyrrolo[2,1-f][1,2,4]triazin-7-yl)-*N*-methylbenzamide;
(Compound No. 54) 2-(2,6-dioxopiperidin-3-yl)-4-((9-(4-(4-((7-phenylpyrrolo[2,1-f][1,2,4]triazin-2-yl)amino)-1*H-*pyrazol-1-yl)piperidin-1-yl)nonyl)oxy)isoindolin-1,3-dione;
(Compound No. 55) 2-(2,6-dioxopiperidin-3-yl)-4-((9-(4-(4-((7-(1-methyl-1*H*-pyrazole-4-1)pyrrolo[2,1-f][1,2,4]triazin-2-yl)amino)-1*H*-pyrazol-1-yl)piperidin-1-yl)nonyl)oxy)isoindolin-1,3-dione;
(Compound No. 56) 4-((9-(4-(4-((7-(1*H*-indol-6-yl)pyrrolo[2,1-f][1,2,4]triazine-2-yl)amino)-1*H-*pyrazol-1-yl)piperidin-1-yl)nonyl)oxy)-2-(2,6-dioxopiperidin-3-yl)isoindolin-1,3-dione;
(Compound No. 57) 2-(2,6-dioxopiperidin-3-yl)-4-((9-(4-(4-((7-(4-hydroxyphenyl)pyrrolo[2,1-f][1,2,4]triazin-2-yl)amino)-1*H-*pyrazol-1-yl)piperidin-1-yl)nonyl)oxy)isoindolin-1,3-dione;
(Compound No. 58) Methyl-7-(4-(2-((1-(1-(9-((2-(2,6-dioxopiperidin-3-yl)-1,3-dioxoisoindolin-4-yl)oxy)nonyl)piperidin-4-yl)-1*H-*pyrazol-4-yl)amino)pyrrolo[2,1-f][1,2,4]triazine -7-day)phenoxy)heptanoate;
(Compound No. 59) *N*-(3-(2-((4-(4-(5-((2-(2,6-dioxopiperidin-3-yl)-1,3-dioxoisoindolin-4-yl)oxy)pentyl)piperazin-1-yl)-2-methoxyphenyl)amino)pyrrolo[2,1-f][1,2,4]triazin-7-yl)phenyl)methanesulfonamide;
(Compound No. 60) *N*-(3-(2-((4-(4-(3-((2-(2,6-dioxopiperidin-3-yl)-1,3-dioxoindolin-4-yl)oxy)propyl)piperazin-1-yl)-2-methoxyphenyl)amino)pyrrolo[2,1-f][1,2,4]triazin-7-yl)phenyl)methanesulfonamide;
(Compound No. 61) *N*-(3-(2-((4-(4-(7-((2-(2,6-dioxopiperidin-3-yl)-1,3-dioxoindolin-4-yl)oxy)heptyl)piperazin-1-yl)-2-methoxyphenyl)amino)pyrrolo[2,1-f][1,2,4]triazin-7-yl)phenyl)methanesulfonamide;
(Compound No. 62) *N*-(3-(2-((4-(4-(11-((2-(2,6-dioxopiperidin-3-yl)-1,3-dioxoindolin-4-yl)oxy)undecyl)piperazin-1-yl)-2-methoxyphenyl)amino)pyrrolo[2,1-f][1,2,4]triazin-7-yl)phenyl)methanesulfonamide;
(Compound No. 63) *N*-(3-(2-((4-(4-(2-((2-(2,6-dioxopiperidin-3-yl)-1,3-dioxoindolin-4-yl)oxy)ethyl)piperazin-1-yl)-2-methoxyphenyl)amino)pyrrolo[2,1-f][1,2,4]triazin-7-yl)phenyl)methanesulfonamide;
(Compound No. 64) *N*-(3-(2-((4-(4-(4-((2-(2,6-dioxopiperidin-3-yl)-1,3-dioxoindolin-4-yl)oxy)butyl)piperazin-1-yl)-2-methoxyphenyl)amino)pyrrolo[2,1-f][1,2,4]triazin-7-yl)phenyl)methanesulfonamide;
(Compound No. 65) *N*-(3-(2-((4-(4-(6-((2-(2,6-dioxopiperidin-3-yl)-1,3-dioxoindolin-4-yl)oxy)hexyl)piperazin-1-yl)-2-methoxyphenyl)amino)pyrrolo[2,1-f][1,2,4]triazin-7-yl)phenyl)methanesulfonamide;
(Compound No. 66) *N*-(3-(2-((4-(4-(8-((2-(2,6-dioxopiperidin-3-yl)-1,3-dioxoindolin-4-yl)oxy)octyl)piperazin-1-yl)-2-methoxyphenyl)amino)pyrrolo[2,1-f][1,2,4]triazin-7-yl)phenyl)methanesulfonamide;
(Compound No. 67) *N*-(3-(2-((4-(4-(9-((2-(2,6-dioxopiperidin-3-yl)-1,3-dioxoindolin-4-yl)oxy)nonyl)piperazin-1-yl)-2-methoxyphenyl)amino)pyrrolo[2,1-f][1,2,4]triazin-7-yl)phenyl)methanesulfonamide;
(Compound No. 68) *N*-(3-(2-((4-(4-(2-(2-((1,3-dioxo-2-(2-oxopiperidin-3-yl)isoindolin-4-yl)oxy)ethoxy)ethyl)piperazin-1-yl)-2-methoxyphenyl)amino)pyrrolo[2,1-f][1,2,4]triazine-7-1)phenyl)methanesulfonamide;
(Compound No. 69) *N*-(3-(2-((4-(4-(6-((1,3-dioxo-2-(2-oxopiperidin-3-yl)isoindolin-4-yl)oxy)hexyl)piperazin-1-yl)-2-methoxyphenyl)amino)pyrrolo[2,1-f][1,2,4]triazin-7-yl)phenyl)methanesulfonamide;
(Compound No. 70) *N*-(3-(2-((4-(4-(8-((1,3-dioxo-2-(2-oxopiperidin-3-yl)isoindoline-4-yl)oxy)octyl)piperazin-1-yl)-2-methoxyphenyl)amino)pyrrolo[2,1-f][1,2,4]triazin-7-yl)phenyl)methanesulfonamide;
(Compound No. 71) *N*-(3-(2-((6-(4-(5-((2-(2,6-dioxopiperidin-3-yl)-1,3-dioxoindolin-4-yl)oxy)pentyl)piperazin-1-yl)pyridin-3-yl)amino)pyrrolo[2,1-f][1,2,4]triazin-7-yl)phenyl)methanesulfonamide;
(Compound No. 72) *N*-(3-(2-((6-(4-(3-((2-(2,6-dioxopiperidin-3-yl)-1,3-dioxoindoline-4-yl)oxy)propyl)piperazin-1-yl)pyridin-3-yl)amino)pyrrolo[2,1-f][1,2,4]triazin-7-yl)phenyl)methanesulfonamide;
(Compound No. 73) *N*-(3-(2-((6-(4-(7-((2-(2,6-dioxopiperidin-3-yl)-1,3-dioxoindolin-4-yl)oxy)heptyl)piperazin-1-yl)pyridin-3-yl)amino)pyrrolo[2,1-f][1,2,4]triazin-7-yl)phenyl)methanesulfonamide;
(Compound No. 74) *N*-(3-(2-((6-(4-(11-((2-(2,6-dioxopiperidin-3-yl)-1,3-dioxoindoline-4-yl)oxy)undecyl)piperazin-1-yl)pyridin-3-yl)amino)pyrrolo[2,1-f][1,2,4]triazin-7-yl)phenyl)methanesulfonamide;
(Compound No. 75) *N*-(3-(2-((6-(4-(2-((2-(2,6-dioxopiperidin-3-yl)-1,3-dioxoindolin-4-yl)oxy)ethyl)piperazin-1-yl)pyridin-3-yl)amino)pyrrolo[2,1-f][1,2,4]triazin-7-yl)phenyl)methanesulfonamide;
(Compound No. 76) *N*-(3-(2-((6-(4-(4-((2-(2,6-dioxopiperidin-3-yl)-1,3-dioxoindoline-4-yl)oxy)butyl)piperazin-1-yl)pyridin-3-yl)amino)pyrrolo[2,1-f][1,2,4]triazin-7-yl)phenyl)methanesulfonamide;
(Compound No. 77) *N*-(3-(2-((6-(4-(6-((2-(2,6-dioxopiperidin-3-yl)-1,3-dioxoindolin-4-yl)oxy)hexyl)piperazin-1-yl)pyridin-3-yl)amino)pyrrolo[2,1-f][1,2,4]triazin-7-yl)phenyl)methanesulfonamide;
(Compound No. 78) *N*-(3-(2-((6-(4-(8-((2-(2,6-dioxopiperidin-3-yl)-1,3-dioxoindolin-4-yl)oxy)octyl)piperazin-1-yl)pyridin-3-yl)amino)pyrrolo[2,1-fl[1,2,4]triazin-7-yl)phenyl)methanesulfonamide;
(Compound No. 79) *N*-(3-(2-((6-(4-(9-((2-(2,6-dioxopiperidin-3-yl)-1,3-dioxoindolin-4-yl)oxy)nonyl)piperazin-1-yl)pyridin-3-yl)amino)pyrrolo[2,1-fl[1,2,4]triazin-7-yl)phenyl)methanesulfonamide;
(Compound No. 80) *N*-(3-(2-((6-(4-(2-(2-((1,3-dioxo-2-(2-oxopiperidin-3-yl)isoindolin-4-yl)oxy)ethoxy)ethyl)piperazin-1-yl)pyridin-3-yl)amino)pyrrolo[2,1-f][1,2,4]triazin-7-yl)phenyl)methanesulfonamide;
(Compound No. 81) *N*-(3-(2-((6-(4-(6-((1,3-dioxo-2-(2-oxopiperidin-3-yl)isoindolin-4-yl)oxy)hexyl)piperazin-1-yl)pyridin-3-yl)amino)pyrrolo[2,1-f][1,2,4]triazin-7-yl)phenyl)methanesulfonamide;
(Compound No. 82) *N*-(3-(2-((6-(4-(8-((1,3-dioxo-2-(2-oxopiperidin-3-yl)isoindolin-4-yl)oxy)octyl)piperazin-1-yl)pyridin-3-yl)amino)pyrrolo[2,1-f][1,2,4]triazin-7-yl)phenyl)methanesulfonamide;
(Compound No. 83) (2S,4R)-1-((S)-3,3-dimethyl-2-(2-(2-(4-(4-((7-(3-(methanesulfonamido)phenyl)pyrrolo[2,1-f][1,2,4]triazin-2-yl)amino)phenyl) piperazin-1-yl)ethoxy)acetaamino)butanoyl)-4-hydroxy-*N*-(4-(4-methylthiazol-5-yl)benzyl)pyrrolidin-2-carboxamide;
(Compound No. 84) (2S,4R)-1-((S)-3,3-dimethyl-2-(3-(2-(2-(4-(4-((7-(3-(methanesulfonamido)phenyl)pyrrolo[2,1-f][1,2,4]triazin-2-yl)amino) phenyl)piperazin-1-yl)ethoxy)ethoxy)propanamido)butanoyl)-4-hydroxy-*N*-(4-(4-methylthiazol-5-yl)benzyl)pyrrolidin-2-carboxamide;
(Compound No. 85) (2S,4R)-1-((S)-3,3-dimethyl-2-(3-(2-(2-(4-(4-((7-(3-(methylsulfonamido)phenyl)pyrrolo[2,1-f][1,2,4]triazin-2-yl)amino) phenyl)piperazin-1-yl)ethoxy)ethoxy)propanamido)butanoyl)-4-hydroxy-*N*-((S)-1-(4-(4-methylthiazol-5-yl)phenyl)ethyl)pyrrolidin-2-carboxamide;
(Compound No. 86) 2-(2,6-dioxopiperidin-3-yl)-4-((5-(4-(5-((7-(4-(trifluoromethoxy)phenyl)pyrrolo[2,1-f][1,2,4]triazin-2-yl)amino)pyridin-2-yl)piperazin-1-yl)pentyl)oxy)isoindolin-1,3-dione;
(Compound No. 87) 4-((5-(4-(5-((7-(3,5-difluorophenyl)pyrrolo[2,1-f][1,2,4]triazine-2-yl)amino)pyridin-2-yl)piperazin-1-yl)pentyl)oxy)-2-(2,6-dioxopiperidin-3-yl)isoindolin-1,3-dione;
(Compound No. 88) 4-((5-(4-(5-((7-(2-aminopyridin-5-yl)pyrrolo[2,1-f][1,2,4]triazine-2-yl)amino)pyridin-2-yl)piperazin-1-al)pentyl)oxy)-2-(2,6-dioxopiperidin-3-yl)isoindolin-1,3-dione;
(Compound No. 89) 2-(2,6-dioxopiperidin-3-yl)-4-((5-(4-(5-((7-(quinolin-3-yl)pyrrolo[2,1-f][1,2,4]triazin-2-yl)amino)pyridin-2-yl)piperazin-1-yl)pentyl)oxy)isoindoline-1,3-dione;
(Compound No. 90) *tert*-butyl4-(3-(2-((6-(4-(5-((2-(2,6-dioxopiperidin-3-yl)-1,3-dioxoisoindolin-4-yl)oxy)pentyl)piperazin-1-yl)pyridin-3-yl)amino)pyrrolo[2,1-f][1,2,4]triazin-7-yl)benzyl)piperazin-1-carboxylate;
(Compound No. 91) (E)-2-(2,6-dioxopiperidin-3-yl)-4-((5-(4-(5-((7-styrylpyrrolo[2,1-f][1,2,4]triazin-2-yl)amino)pyridin-2-yl)piperazin-1-yl)pentyl)oxy)isoindolin-1,3-dione;
(Compound No. 92) *tert*-butyl4-(4-(2-((6-(4-(5-((2-(2,6-dioxopiperidin-3-yl)-1,3-dioxoisoindolin-4-yl)oxy)pentyl)piperazin-1-yl)pyridin-3-yl)amino)pyrrolo[2,1-f][1,2,4]triazin-7-yl)-1*H*-pyrazol-1-yl)piperidin-1-carboxylate;
(Compound No. 93) 4-((5-(4-(5-((7-(4-(dimethylamino)phenyl)pyrrolo[2,1-f][1,2,4]triazine-2-yl)amino)pyridin-2-yl) piperazin-1-yl)pentyl)oxy)-2-(2,6-dioxopiperidin-3-yl)isoindolin-1,3-dione;
(Compound No. 94) 2-(2,6-dioxopiperidin-3-yl)-4-((5-(4-(5-((7-(pyridin-4-yl)pyrrolo[2,1-fl[1,2,4]triazin-2-yl)amino)pyridin-2-yl)piperazin-1-yl)pentyl)oxy)isoindolin-1,3-dione;
(Compound No. 95) 2-(2,6-dioxopiperidin-3-yl)-4-((5-(4-(5-((7-(thiazol-5-yl)pyrrolo[2,1-f][1,2,4]triazin-2-yl)amino)pyridin-2-yl)piperazin-1-yl)pentyl)oxy)isoindolin-1,3-dione;
(Compound No. 96) 2-(2,6-dioxopiperidin-3-yl)-4-((5-(4-(5-((7-(1-(tetrahydro-2*H*-pyran-2 -yl)-1*H-*pyrazol-5-yl)pyrrolo[2,1-f][1,2,4]triazin-2-yl)amino)pyridin-2-yl)piperazin-1-yl)pentyl)oxy)isoindolin-1,3-dione;
(Compound No. 97) 4-((5-(4-(5-((7-((3,5-difluorophenyl)ethynyl)pyrrolo[2,1-f][1,2,4]triazin-2-yl)amino)pyridin-2-yl)piperazin-1-yl)pentyl)oxy)-2-(2,6-dioxopiperidin-3-yl)isoindolin-1,3-dione;
(Compound No. 98) 2-(2,6-dioxopiperidin-3-yl)-4-((5-(4-(5-((7-((3-methoxyphenyl)ethynyl)pyrrolo[2,1-f][1,2,4]triazin-2-yl)amino)aminopyridin-2-yl)piperazin-1-yl)pentyl)oxy)isoindolin-1,3-dione;
(Compound No. 99) *N-*(3-(2-((4-(4-(2-(2-(3-(4-((4-(2-(2,6-dioxopiperidin-3-yl)-6-fluoro-1,3-dioxoisoindolin-5-yl)piperazin-1-yl)methyl)piperidin-1-yl)-3-oxopropoxy)ethoxy)ethyl)piperazin-1-yl)phenyl) amino)pyrrolo[2,1-f][1,2,4]triazin-7-yl)phenyl)methanesulfonamide;
(Compound No. 100) *N-*(3-(4-(3-(2,4-dihydroxy-5-isopropylphenyl)-5-hydroxy-4*H*-1,2,4-triazol-4-yl)phenoxy)propyl)-3-(2-(2-(4-(4-((7-(3-(methylsulfonamido)phenyl)pyrrolo[2,1-f][1,2,4]triazin-2-yl)amino)phenyl)piperazin-1-yl)ethoxy)ethoxy)propanamide;
(Compound No. 101) *N*-(2-((3-(4-(3-(2,4-dihydroxy-5-isopropylphenyl)-5-hydroxy-4*H*-1,2,4-triazol-4-yl)phenoxy)propyl)amino)-2-oxoethyl)-3-(2-(2-(4-(4-((7-(3-(methylsulfonamido)phenyl)pyrrolo[2,1-f][1,2,4]triazin-2-yl)amino)phenyl)piperazin-1-yl)ethoxy)ethoxy)propanamide;
(Compound No. 102) *N*-(3-(2-((4-(4-(2-(2-(3-(4-(4-(3-(2,4-dihydroxy-5-isopropylphenyl)-5-hydroxy-4*H-*1,2,4-triazol-4-yl)benzyl)piperazin-1-yl)-3-oxopropoxy)ethoxy)ethyl)piperazine-1-yl)phenyl)amino)pyrrolo[2,1-f][1,2,4] triazin-7-yl)phenyl)methanesulfonamide;
(Compound No. 103) *N*-(3-(2-((4-(4-(2-(2-(3-(4-((4-(4-(3-(2,4-dihydroxy-5-isopropylphenyl)-5-hydroxy-4*H-*1,2,4-triazol-4-yl)benzyl)piperazin-1-yl)methyl)piperidin-1-yl)-3-oxopropoxy)ethoxy)ethyl) piperazin-1-yl)phenyl)amino)pyrrolo[2,1-f][1,2,4]triazin-7-yl)phenyl) methanesulfonamide;
(Compound No. 104) *N*-(3-(2-((4-(4-(2-(2-(3-(4-((2-(2,4-dihydroxy-5-isopropylbenzoyl)isoindolin-5-yl)methyl)piperazin-1-yl)-3-oxopropoxy) ethoxy)ethyl)piperazin-1-yl)phenyl)amino)pyrrolo[2,1-fl[1,2,4]triazin-7-yl)phenyl)methanesulfonamide;
(Compound No. 105) *N*-(3-(2-((4-(4-(2-(2-(3-(4-((4-((2-(2,4-dihydroxy-5 -isopropylbenzoyl)isoindolin-5-yl)methyl)piperazin-1-yl)methyl)piperidin-1-yl)-3-oxopropoxy)ethoxy)ethyl)piperazin-1-yl)phenyl)amino)pyrrolo[2,1-f][1,2,4]triazin-7-yl)phenyl)methanesulfonamide;
(Compound No. 106) 2-amino-4-(2,4-dichloro-5-(2-(4-(3-(2-(2-(4-(4-((7-(3-(methylsulfonamido)phenyl)pyrrolo[2,1-f][1,2,4]triazin-2-yl)amino) phenyl)piperazin-1-yl)ethoxy)ethoxy)propanoyl)piperazin-1-yl)ethoxy)phenyl)-*N*-ethylthieno[2,3-d]pyrimidin-6-carboxamide;
(Compound No. 107) *N*-(3-(2-((4-(4-(4-((4-((2-(2,4-dihydroxy-5-isopropylbenzoyl)isoindolin-5-yl)methyl)piperazin-1-yl)methyl)benzoic)piperazin-1-yl)phenyl)amino)pyrrolo[2,1-f][1,2,4]triazine-7-1)phenyl)methanesulfonamide;
Compound No. 108) *N*-(3-(2-((4-(4-(1-((2-(2,4-dihydroxy-5-isopropylbenzoic)isoindolin-5-yl)methyl)piperidin-4-carbonyl)piperazin-1-yl) phenyl)amino)pyrrolo[2,1-f][1,2,4]triazin-7-yl)phenyl)methanesulfonamide
(Compound No. 109) 4-(2-((2¹Z,4⁴E)-4¹*H*-11-oxa-3,8-diaza-2(7,2)-pyrrolo[2,1-f][1,2,4]triazin-4(4,1)-pyrazolo-1(1,4)-benzenacycloundecaphan -8-yl)ethoxy)-2-(2,6-dioxopiperidin-3- 1)isoindolin-1,3-dione;
(Compound No. 110) 4-(3-((2¹Z,4⁴E)-4¹*H*-11-oxa-3,8-diaza-2(7,2)-pyrrolo[2,1-f][1,2,4]triazin-4(4,1)-pyrazolo-1(1,4)-benzenacycloundecaphan -8-yl)propoxy)-2-(2,6-dioxopiperidin-3-yl)isoindolin-1,3-dione;
(Compound No. 111) 4-(2-(2-((2¹Z,4⁴E)-4¹*H*-11-oxa-3,8-diaza-2(7,2)-pyrrolo[2,1-f][1,2,4]triazin-4(4,1)-pyrazolo-1(1,4)-benzenacycloundecaphan -8-yl)ethoxy)ethoxy)-2-(2-oxopiperidin-3-yl)isoindolin-1,3-dione;
(Compound No. 112) 4-((6-((2¹Z,4⁴E)-4¹*H*-11-oxa-3,8-diaza-2(7,2)-pyrrolo[2,1-f][1,2,4]triazin-4(4,1)-pyrazol-1(1,4)-benzenacycloundecaphan - 8-yl)hexyl)oxy)-2-(2,6-dioxopiperidin-3-yl)isoindolin-1,3-dione;
(Compound No. 113) 4-((8-((2¹Z,4⁴E)-4¹*H-*11-oxa-3,8-diaza-2(7,2)-pyrrolo[2,1-f][1,2,4]triazin-4(4,1)-pyrazol-1(1,4)-benzenacycloundecaphan - 8-yl)octyl)oxy)-2-(2,6-dioxopiperidin-3-1)isoindolin-1,3-dione;
(Compound No. 114) 4-((8-((2¹Z,4⁴E)-4¹*H*-11-oxa-3,8-diaza-2(7,2)-pyrrolo[2,1-fl[1,2,4]triazin-4(4,1)-pyrazol-1(1,4)-benzenacycloundecaphan - 8-yl)octyl)oxy)-2-(2-oxopiperidin-3-yl)isoindolin-1,3-dione;
(Compound No. 115) 4-((11-((2¹Z,4⁴E)-4¹*H*-11-oxa-3,8-diaza-2(7,2)-pyrrolo[2,1-fl[1,2,4]triazin-4(4,1)-pyrazol-1(1,4)-benzenacycloundecaphan - 8-yl)undecyl)oxy)-2-(2,6-dioxopiperidin-3-yl)isoindolin-1,3-dione;
(Compound No. 116) 4-((9-((2¹Z,4⁴E)-4¹*H*-11-oxa-3,8-diaza-2(7,2)-pyrrolo[2,1-f][1,2,4]triazin-4(4,1)-pyrazola-1(1,4)-benzenacycloundecaphan -8-yl)nonyl)oxy)-2-(2,6-dioxopiperidin-3-yl)isoindolin-1,3-dione;
(Compound No. 117) 5-(4-((1-(3-(2-(2-((2¹Z,4⁴E)-4¹*H*-11-oxa-3,8-diaza-2(7,2)-pyrrolo[2,1-f][1,2,4]triazin-4(4,1)-pyrazola-1(1,4)-benzenacycloundecaphan-8-yl)ethoxy)ethoxy))propanoyl)piperidin-4-yl)methyl)piperazin-1-yl)-2-(2,6-dioxopiperidin-3-yl)-6-fluoroisoindolin-1, 3-dione;
(Compound No. 118) 3-(2-(2-((2¹Z,4⁴E)-4¹H-11-oxa-3,8-diaza-2(7,2)-pyrrolo[2,1-f][1,2,4]triazin-4(4,1)-pyrazola-1(1,4)-benzenacycloundecaphan -8-yl)ethoxy)ethoxy)-*N-*(3-(4-(3-(2,4-dihydroxy-5-isopropylphenyl)-5-hydroxy-4*H*-1,2,4-triazol-4-yl)phenoxy)propyl)propanamide;
(Compound No. 119) 3-(2-(2-((2¹Z,4⁴E)-4¹*H*-11-oxa-3,8-diaza-2(7,2)-pyrrolo[2,1-f][1,2,4]triazin-4(4,1)-pyrazol-1(1,4)-benzenacycloundecaphan-8-yl)ethoxy)ethoxy)-*N-*(2-((3-(4-(3-(2,4-dihydroxy-5-isopropylphenyl)-5-hydroxy-4*H*-1,2,4-triazol-4-yl)phenoxy)propyl)amino)-2-oxoethyl)propanamide;
(Compound No. 120) 3-(2-(2-((2¹Z,4⁴E)-4¹*H*-11-oxa-3,8-diaza-2(7,2)-pyrrolo[2,1-f][1,2,4]triazin-4(4,1)-pyrazolo-1(1,4)-benzenacycloundecaphan -8-yl)ethoxy)ethoxy)-1-(4-(4-(3-(2,4-dihydroxy-5-isopropylphenyl)-5-hydroxy-4*H*-1,2,4-triazol-4-yl)benzyl)piperazin-1-yl)propan-1-one;
(Compound No. 121) 3-(2-(2-((2¹Z,4⁴E)-4¹*H*-11-oxa-3,8-diaza-2(7,2)-pyrrolo[2,1-f][1,2,4]triazin-4(4,1)-pyrazolo-1(1,4)-benzenacycloundecaphan-8-yl)ethoxy)ethoxy)-1-(4-((4-(4-(3-(2,4-dihydroxy-5-isopropylphenyl)-5-hydroxy-4*H*-1,2,4-triazol-4-yl)benzyl)piperazin-1-yl)methyl)piperidin-1-yl)propan-1-one;
(Compound No. 122) 3-(2-(2-((2¹Z,4⁴E)-4¹*H*-11-oxa-3,8-diaza-2(7,2)-pyrrolo[2,1-f][1,2,4]triazin-4(4,1)-pyrazol-1(1,4)-benzenacycloundecaphan-8-yl)ethoxy)ethoxy)-1-(4-((2-(2,4-dihydroxy-5-isopropylbenzoyl)isoindolin-5-yl)methyl)piperazin-1-yl)propan-1-one;
(Compound No. 123) 3-(2-(2-((2¹Z,4⁴E)-4¹*H*-11-oxa-3,8-diaza-2(7,2)-pyrrolo[2,1-f][1,2,4]triazin-4(4,1)-pyrazola-1(1,4)-benzenacycloundecaphan-8-yl)ethoxy)ethoxy)-1-(4-((4-((2-(2,4-dihydroxy-5-isopropylbenzoyl)isoindolin-5-yl)methyl)piperazin-1-yl)methyl)piperidin-1-yl)propan-1-one;
(Compound No. 124) 4-(5-(2-(4-(3-(2-(2-((2¹Z,4⁴E)-4¹*H-*11-oxa-3,8-diaza-2(7,2)-pyrrolo[2,1-f][1,2,4]triazin-4(4,1)-pyrazola-1(1,4)-benzenacycloundecaphan-8-yl)ethoxy)ethoxy)propanoyl)piperazin-1-yl)ethoxy)-2,4-dichlorophenyl)-2-amino-*N*-ethylthieno[2,3-d]pyrimidin-6-carboxamide;
(Compound No. 125) ((2¹Z,4⁴E)-4¹*H*-11-oxa-3,8-diaza-2(7,2)-pyrrolo[2,1-f][1,2,4]triazin-4(4,1)-pyrazola-1(1,4)-benzenacycloundecaphan-8-yl)(4-((4-((2-(2,4-dihydroxy-5-isopropylbenzoyl)isoindolin-5-yl)methyl)piperazin-1-yl) methyl)phenyl)methanone;
(Compound No. 126) ((2¹Z,4⁴E)-4¹*H*-11-oxa-3,8-diaza-2(7,2)-pyrrolo[2,1-f][1,2,4]triazin-4(4,1)-pyrazola-1(1,4)-benzenacycloundecaphane-8-yl)(1-((2-(2,4-dihydroxy-5-isopropylbenzoyl)isoindolin-5-yl)methyl)piperidin-4-yl)methanone.

The pharmaceutical composition of the present invention comprises, as an effetive ingredient, the 2,7-substituted pyrrolo[2,1-f][1,2,4]triazine compound represented by chemical formula 1, a pharmaceutically acceptable salt thereof, a solvate thereof, or a hydrate thereof, along with commonly used inert pharmaceutical carriers, diluents, and excipients. These can be formulated into pharmaceutical preparations commonly used in the field of pharmacy, such as oral dosage forms like tablets, capsules, troches, liquids, suspensions, etc., or non-oral dosage forms.

The excipients that can be used in the pharmaceutical composition of the present invention include sweeteners, binders, solubilizers, solubility enhancers, lubricants, emulsifiers, surfactants, disintegrants, antioxidants, preservatives, stabilizers, fillers, flavoring agents, and fragrances. Examples include lactose, dextrose, sucrose, mannitol, sorbitol, cellulose, glycine, silica, talc, stearic acid, stearin, magnesium stearate, magnesium aluminum silicate, starch, gelatin, tragacanth gum, alginic acid, sodium alginate, methylcellulose, sodium carboxymethylcellulose, agar, water, ethanol, polyethylene glycol, polyvinylpyrrolidone, sodium chloride, calcium chloride, orange essence, strawberry essence, vanilla flavor, etc.

Furthermore, the dosages for administering the compounds according to the present invention to humans may vary depending on factors such as the patient's age, weight, gender, route of administration, health status, and severity of the condition. For instance, for an adult patient weighing 70 kg, the typical dosage ranges from 0.01 to 1,000 mg/day; however, it can also be administered once daily or divided into multiple doses at regular intervals as determined by a physician or pharmacist.

In another aspect, the present invention provides a method for preparing the compound, a pharmaceutically acceptable salt thereof, a hydrate thereof, or a solvate thereof, comprising a step of reacting an intermediate compound of any one of the 2,7-substituted pyrrolo[2,1-f][1,2,4]triazine compounds selected from the group consisting of the following compounds:
N-(3-(2-chloropyrrolo[2,1-f][1,2,4]triazin-7-yl)phenyl)methanesulfonamide;
N-(3-(2-((4-(piperazin-1-yl)phenyl)amino)pyrrolo[2,1-f][1,2,4]triazin-7-yl)phenyl)methanesulfonamide;
N-(3-(2-((6-(piperazin-1-yl)pyridin-3-yl)amino)pyrrolo[2,1-f][1,2,4]triazin-7-yl)phenyl)methanesulfonamide;
N-(3-(2-((2-methoxy-4-(piperazin-1-yl)phenyl)amino)pyrrolo[2,1-f][1,2,4]triazin-7-yl)phenyl)methanesulfonamide;
N-(3-(2-((1-(piperidin-4-yl)-1H-pyrazol-4-yl)amino)pyrrolo[2,1-f][1,2,4]triazin-7-yl)phenyl)methanesulfonamide;
4-((7-(4-(4-amino-1H-pyrazol-1-yl)piperidin-1-yl)heptyl)oxy)-2-(2,6-dioxopiperidin-3-yl)isoindolin-1,3-dione;
4-((5-(4-(4-aminophenyl)piperazin-1-yl)pentyl)oxy)-2-(2,6-dioxopiperidin-3-yl)isoindolin-1,3-dione;
3-(2-(2-(4-(4-((7-(3-(methylsulfonamido)phenyl)pyrrolo[2,1-f][1,2,4]triazine-2-yl)amino)phenyl)piperazin-1-yl)ethoxy)ethoxy)propanoic acid;
4-(4-(4-(3-aminopropoxy)phenyl)-5-hydroxy-4H-1,2,4-triazol-3-yl)-6-isopropylbenzene-1,3-diol;
2-amino-N-(3-(4-(3-(2,4-dihydroxy-5-isopropylphenyl)-5-hydroxy-4H-1,2,4-triazol-4-yl)phenoxy)propyl)acetamide;
4-(5-hydroxy-4-(4-(piperazin-1-ylmethyl)phenyl)-4H-1,2,4-triazol-3-yl)-6-isopropylbenzene-1,3-diol;
4-(5-hydroxy-4-(4-((4-(piperidin-4-ylmethyl)piperazin-1-yl)methyl)phenyl)-4H-1,2,4-triazol- 3-yl)-6-isopropylbenzene-1,3-diol;
(2,4-dihydroxy-5-isopropylphenyl)(5-(piperazin-1-ylmethyl)isoindolin-2-yl)methanone;
(2,4-dihydroxy-5-isopropylphenyl)(5-((4-(piperidin-4-ylmethyl)piperazin-1-yl)methyl)isoindolin-2-yl)methanon;
2-amino-4-(2,4-dichloro-5-(2-(piperazin-1-yl)ethoxy)phenyl)-N-ethylthieno[2,3-d]pyrimidin-6-carboxamide;
4-(3-isopropyl-4-(4-(1-methyl-1H-pyrazol-4-yl)-1H-imidazol-1-yl)-1H-pyrazolo[3,4-b]pyridine-1-yl)-2-((4-(piperidin-4-ylmethyl)piperazin-1-yl)methyl)benzamide;
4-chloro-7-((4-methoxy-3,5-dimethylpyridin-2-yl)methyl)-5-(3-(4-(piperidin-4-ylmethyl)piperazin-1-yl)propy-1-yn-1-yl)-7H-pyrrolo[2,3-d]pyrimidin-2-amine;
4-((4-((2-(2,4-dihydroxy-5-isopropylbenzoic)isoindolin-5-yl)methyl)piperazin-1-yl)methyl)benzoic acid; and
1-((2-(2,4-dihydroxy-5-isopropylbenzoic)isoindolin-5-yl)methyl)piperidin-4-carboxylic acid;

Meanwhile, the present invention is characterized by the method for preparing the 2,7-substituted pyrrolo[2,1-f][1,2,4]triazine compound represented by Chemical Formulae 2 and 3. The representative method for preparation is as follows.

The compounds according to the above Chemical Formulae feature pyrrolo[2,1-f][1,2,4]triazine as a scaffold.

Chemical Formula 2 introduces a cyclic hydrocarbon, such as a benzene ring, at position 7 from the scaffold through Suzuki reaction. Subsequently, at position 2, a cyclic compound containing an amine functional group is substituted via Buchwald reaction. From the introduced amine functional group at position 2, Linker + E3 ligase binder is incorporated.

Chemical Formula 3 introduces a cyclic hydrocarbon, such as a benzene ring, at position 7 from the scaffold through Suzuki reaction. Then, an alkyl group containing a ring-closure-capable functional group is connected. Next, at position 2, an alkyl group containing a ring-closure-capable functional group is connected, and a cyclic compound containing an amine functional group is substituted via Buchwald reaction. After introducing substituents at positions 2 and 7, ring-closure reactions are employed to synthesize cyclic compounds.

### Embodiment of the Invention

The invention as described above will be further elucidated with reference to the following embodiments, formulation examples, and experimental examples. However, it should be understood that these embodiments, formulation examples, and experimental examples are provided for illustration purposes only and do not limit the scope of the invention.

### [Example]

### Example 1: (2¹Z,4⁴E)-4¹H-11-oxa-3,8-diaza-2(7,2)-pyrrolo[2,1-f][1,2,4]triazina- 4(4,1)-pyrazola-1(1,4)-benzenacycloundecaphane

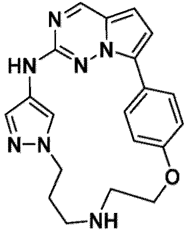

### Step 1) Preparation of 4-(2-chloropyrrole[2,1-f][1,2,4]triazin-7-yl)phenol

To a solution of 7-Bromo-2-chloropyrrole[2,1-f][1,2,4]triazine (1.0 g, 4.3 mmol) and (4-hydroxyphenyl)boronic acid (623 mg, 4.5 mmol) in 1,4-dioxane/distilled water (16/4 mL) was added sodium carbonate (1.37 g, 12.9 mmol) and t-ButylXphos (0.2 g, 0.47 mmol). After flowing nitrogen through the mixture for 10 minutes, Pd(PPh3)Cl2 (1.45 g, 2.07 mmol) was added and stirred at 60 ° C for 6 hours. When the reaction was complete, the mixture was filtered through Celite, diluted with ethyl acetate, and washed with saturated saline solution. The resulting separated organic layer was dried over anhydrous sodium sulfate, filtered under reduced pressure, and distilled under reduced pressure. The resulting residue was purified by chromatography to obtain 0.95 g of the title compound (yield: 90%). MS m/z: 246 [M+1].

### Step 2) Preparation of 7-(4-(2-bromoethoxy)phenyl)-2-chloropyrrole[2,1-f][1,2,4]triazine

To a solution of 4-(2-Chloropyrrole[2,1-f][1,2,4]triazin-7-yl)phenol (4.1 g, 16.69 mmol) in toluene (50 mL) was added triphenylphosphine ( 6.57 g, 25.0 mmol) and 2-bromoethanol (2.4 mL, 7.64 mmol). After stirring for 5 minutes, diisopropyl azocarboxylate (1.13 mL, 5.73 mmol) was slowly added thereto and stirred at 50 ° C. When the reaction was complete, the mixture was diluted in ethyl acetate and washed with distilled water and saturated sodium chloride solution. The resulting separated organic layer was dried with anhydrous sodium sulfate, filtered under reduced pressure, and distilled under reduced pressure. The resulting residue was purified by chromatography to obtain 5 g of the title compound (yield: 85%). MS m/z: 353 [M+1].

### Step 3) Preparation of tert-butyl (3-(4-((7-(4-(2-bromoethoxy)phenyl)pyrrolo[2,1-f][1,2,4]triazin-2-yl)amino)-1H-pyrazol-1-yl)propyl)carbamate

To a solution of 7-(4-(2-bromoethoxy)phenyl)-2-chloropyrrole[2,1-f][1,2,4]triazine (1 g, 2.83 mmol) in 20 mL of see-butanol was added potassium carbonate (1.2 g, 8.5 mmol) and tert-butyl (3-(4-amino-1H-pyrrozyl-1-yl)propyl)carbamate (0.75 g, 3.11 mmol). After flowing nitrogen through the mixture for 10 minutes, Pd2(dba)3 (0.51 g, 0.57 mmol) and Xphos (0.31 g, 0.62 mmol) were added and stirred at 80 ° C for 4 hours. When the reaction was complete, the mixture was filtered through Celite, diluted with ethyl acetate, and washed with saturated saline solution. The resulting separated organic layer was dried over anhydrous sodium sulfate, filtered under reduced pressure, and distilled under reduced pressure. The resulting residue was purified by chromatography to obtain 0.8 g of the title compound (yield: 57%). MS m/z: 557 [M+1].

### Step 4) Preparation of N-(1-(3-aminopropyl)-1-pyrazol-4-yl)-7-(4-(2-bromoethoxy)phenyl)pyrrolo[2,1-f] [1,2,4]triazine-2-amine

To a solution of tert-butyl (3-(4-((7-(4-(2-bromoethoxy)phenyl)pyrrolo[2,1-f] [1,2,4]triazine-2-yl)amino)-1H-pyrazol-1-yl)propyl)carbamate (2.1 g, 3.77 mmol) in DCM (50 mL) was added 4 M hydrochloric acid-dioxane solution (5 mL). The mixture was stirred at 40° C for 4 hours. When the reaction was complete, the mixture was diluted with dichloromethane and neutralized with sodium bicarbonate to pH 5. The resulting separated organic layer was dried over anhydrous sodium sulfate, filtered under reduced pressure, and distilled under reduced pressure. 1.53 g of the resulting title compound (yield: 88%) was used in the next reaction without further purification. MS m/z: 457[M+1].

### Step 5) Preparation of (2¹Z,4⁴E)-4¹H-11-oxa-3,8-diaza-2(7,2)-pyrrolo[2,1-f][1,2,4]triazina-4(4,1)-pyrazola-1(1,4)-benzenacycloundecaphane

To a solution of N-(1-(3-aminopropyl)-1H-pyrazol-4-yl)-7-(4-(2-bromoethoxy)phenyl)pyrrolo[2,1-f][1,2,4]triazine-2-amine (0.42 g, 0.92 mmol)in 2-ethoxymethanol (40 mL) was added diisopropylethylamine (1.0 mL), and stirred at 80 ° C for two days. When the reaction was completed, it was purified by chromatography to obtain 100 mg (29% yield) of the title compound. 1H NMR (400 MHz, DMSO-d6) δ 9.41 (s, 1H), 8.89 (s, 1H), 7.97 (s, 1H), 7.78-7.73 (m, 2H), 7.35-7.30 (m, 2H), 7.26 (s, 1H), 6.92-6.86 (m, 2H), 4.44 (m, 2H), 4.11 (m, 2H), 2.82 (m, 4H), 1.73 (m, 2H). MS m/z: 376 [M+1].

### Example 2: ((2¹Z,4⁴E)-8-methyl-4¹H-11-oxa-3,8-diaza-2(7,2)-pyrrolo[2,1-f][1,2,4]triazina-4(4,1)-pyrazola-1(1,4)-benzenacycloundecaphane

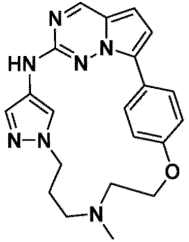

To a solution of (2¹Z,4⁴E)-4¹*H*-11-oxa-3,8-diaza-2(7,2)-pyrrolo[2,1-f][1,2,4]triazina- 4(4,1)-pyrazola-1(1,4)-benzenacycloundecaphane (0.42 g, 0.92 mmol) (40 mg, 0.11 mmol) in dichloromethane (2.1 mL) was added acetic acid (1 drop) and formaldehyde (0.01 mL, 0.26 mmol). After stirring for 30 minutes, sodium triacetoxyborohydride (29 mg, 0.14 mmol) was slowly added and stirred for one day. When the reaction was complete, the mixture was washed with dichloromethane and saturated brine. The resulting separated organic layer was dried over anhydrous sodium sulfate, filtered under reduced pressure, and distilled under reduced pressure. The resulting residue was purified by chromatography to obtain 16 mg of the title compound (yield: 38%). 1H NMR (400 MHz, DMSO-d6) δ 9.41 (s, 1H), 8.89 (s, 1H), 7.97 (s, 1H), 7.78-7.73 (m, 2H), 7.35-7.30 (m, 2H), 7.26 (s, 1H), 6.92-6.86 (m, 2H), 4.44 (m, 2H), 4.11 (m, 2H), 2.82 (m, 4H), 2.21 (s, 3H), 1.73 (m, 2H). MS m/z: 390[M+1].

### Example 3: 1-((2¹Z,4⁴E)-4¹H-11-oxa-3,8-diaza-2(7,2)-pyrrolo[2,1-f][1,2,4]triazina-4(4,1)-pyrazola-1(1,4)-benzenacycloundecafphane-8-yl)ethan-1-one

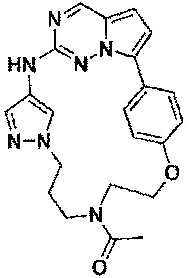

To a solution of (2¹Z,4⁴E)-4¹*H*-11-oxa-3,8-diaza-2(7,2)-pyrrolo[2,1-f][1,2,4]triazina- 4(4,1)-pyrazola-1(1,4)-benzenacycloundecaphane (0.42 g, 0.92 mmol) (40 mg, 0.11 mmol) in dichloromethane (2.1 mL) was added acetyl chloride (0.015 mL, 0.22 mmol) and triethylamine ( 0.2 mL, 0.52 mmol) at 0 ° C, and stirred at room temperature for 30 minutes. When the reaction was completed, the mixture was diluted with ethyl acetate and washed with saturated saline solution. The resulting separated organic layer was dried over anhydrous sodium sulfate, filtered under reduced pressure, and distilled under reduced pressure. The resulting residue was purified by chromatography to obtain 14 mg of the title compound (yield: 31%). MS m/z: 418 [M+1].

### Example 4: 1-((2¹Z,4⁴E)-4¹H-11-oxa-3,8-diaza-2(7,2)-pyrrolo[2,1-f][1,2,4]triazina-4(4,1)-pyrazola-1(1,4)-benzenacycloundecaphane-8-yl)butan-1-one

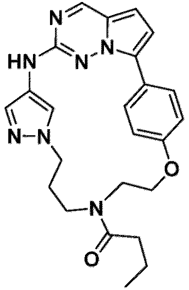

The title compound was obtained by carrying out the same process as Example 3 above using the corresponding acyl chloride. MS m/z: 446 [M+1].

### Example 5: ((2¹Z,4⁴E)-4¹H-11-oxa-3,8-diaza-2(7,2)-pyrrolo[2,1-f][1,2,4]triazina-4(4,1)-pyrazola-1(1,4)-benzenacycloundecaphane-8-yl)(4-chlorophenyl)methanone

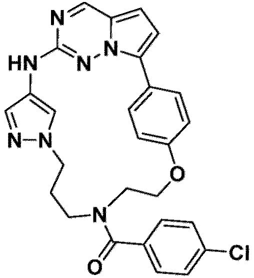

The title compound was obtained by carrying out the same process as Example 3 above using the corresponding acyl chloride. MS m/z: 514 [M+1].

### Example 6: (2¹Z,4⁴E)-N-cyclohexyl-4¹H-11-oxa-3,8-diaza-2(7,2)-pyrrolo[2,1-f][1,2,4]triazina-4(4,1)-pyrazola-1(1,4)-benzenacycloundecaphane-8-carboxamide

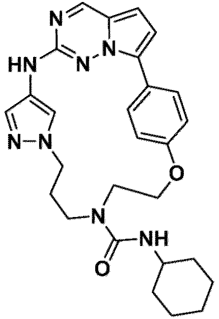

To a solution of (2¹Z,4⁴E)-4¹*H*-11-oxa-3,8-diaza-2(7,2)-pyrrolo[2,1-f][1,2,4]triazina- 4(4,1)-pyrazola-1(1,4)-benzenacycloundecaphane (40 mg, 0.11 mmol) in tetrahydrofuran (2.6 mL) was added isocyanate (32 mg, 0.22 mmol) and stirred at room temperature for 30 minutes. When the reaction was complete, the mixture was diluted with ethyl acetate and washed with brine. The resulting separated organic layer was dried over anhydrous sodium sulfate, filtered under reduced pressure, and distilled under reduced pressure. The resulting residue was purified by chromatography to obtain 18 mg of the title compound (yield: 32%). MS m/z: 501 [M+1].

### Example 7: (2¹Z,4⁴E)-N-(3-methoxyphenyl)-4¹H-11-oxa-3,8-diaza-2(7,2)-pyrrolo[2,1-f][1,2,4]triazina-4(4,1)-pyrazola-1(1,4)-benzenecycloundecaphane-8-carboxamide

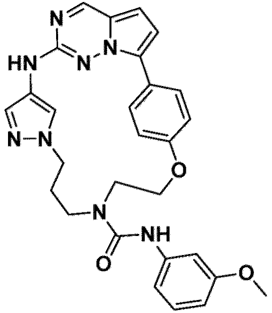

The title compound was obtained by carrying out the same process as Example 6 above using the corresponding isocyanate. MS m/z: 524 [M+1].

### Example 8: (2¹Z,4⁴E)-N-ethyl-4¹H-11-oxa-3,8-diaza-2(7,2)-pyrrolo[2,1-f][1,2,4]triazina-4(4,1)-pyrazola-1(1,4)-benzenacycloundecaphane-8-carboxamide

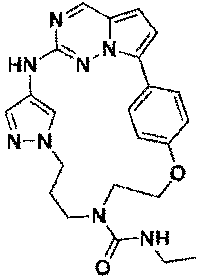

The title compound was obtained by carrying out the same process as Example 6 above using the corresponding isocyanate. MS m/z: 477 [M+1].

### Example 9: (2¹Z,4⁴E)-8-(methylsulfonyl)-4¹H-11-oxa-3,8-diaza-2(7,2)-pyrrolo[2,1-f][1,2,4]triazina-4(4,1)-pyrazola-1(1,4)-benzenecycloundecaphane

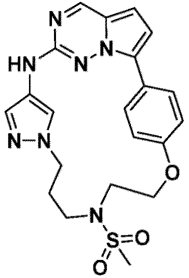

To a solution of (2¹Z,4⁴E)-4¹*H*-11-oxa-3,8-diaza-2(7,2)-pyrrolo[2,1-f][1,2,4]triazina- 4(4,1)-pyrazola-1(1,4)-benzenacycloundecaphane ( (40 mg, 0.11 mmol) in dichloromethane (2.6 mL) was added methanesulfonyl chloride (0.016 mL, 0.39 mmol). When the reaction was complete, the mixture was washed with dichloromethane and saturated brine. The resulting separated organic layer was dried over anhydrous sodium sulfate, filtered under reduced pressure, and distilled under reduced pressure. The resulting residue was purified by chromatography to obtain 16 mg of the title compound (yield: 33%). MS m/z: 454 [M+1].

### Example 10: (2¹Z,4⁴E)-8-((3,4-difluorophenyl)sulfonyl)-4¹H-11-oxa-3,8-diaza-2(7,2)-pyrrolo[2,1-f][1,2,4]triazina-4(4,1)-pyrazola-1(1,4)-benzenecycloundecaphane

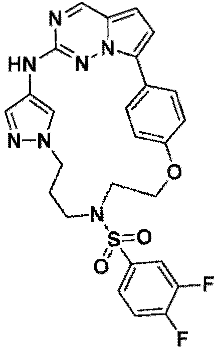

The title compound was obtained by carrying out the same process as Example 9 above using the corresponding sulfonyl chloride. MS m/z: 552 [M+1].

### Example 11: (2¹Z,4⁴E)-8-(phenylsulfonyl)-4¹H-11-oxa-3,8-diaza-2(7,2)-pyrrolo[2,1-f][1,2,4]triazina-4(4,1)-pyrazola-1(1,4)-benzenacycloundecaphane

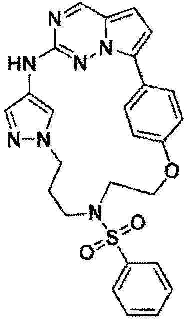

The title compound was obtained by carrying out the same process as Example 9 above using the corresponding sulfonyl chloride. MS m/z: 516 [M+1].

### Example 12: (2¹Z,4⁴E)-8-(cyclopropylsulfonyl)-4¹H-11-oxa-3,8-diaza-2(7,2)-pyrrolo[2,1-f][1,2,4]triazina-4(4,1)-pyrazola-1(1,4)-benzenacycloundecaphane

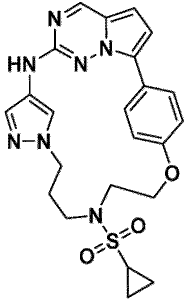

The title compound was obtained by carrying out the same process as Example 9 above using the corresponding sulfonyl chloride. MS m/z: 480 [M+1].

### Example 13: (2¹Z,4⁴E)-8-(propylsulfonyl)-4¹H-11-oxa-3,8-diaza-2(7,2)-pyrrolo[2,1-f][1,2,4]triazina-4(4,1)-pyrazola-1(1,4)-benzenacycloundecaphane

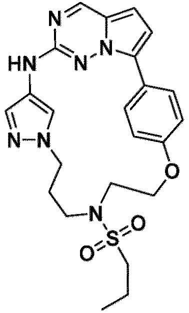

The title compound was obtained by carrying out the same process as Example 9 above using the corresponding sulfonyl chloride. MS m/z: 482 [M+1].

### Example 14: (2¹Z,4⁴E)-8-((5-chlorothiophen-2-yl)sulfonyl)-4¹H-11-oxa-3,8-diaza-2(7,2)-pyrrolo[2,1-f][1,2,4]triazina-4(4,1)-pyrazola-1(1,4)-benzenacycloundecaphane

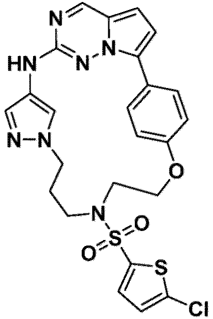

The title compound was obtained by carrying out the same process as Example 9 above using the corresponding sulfonyl chloride. MS m/z: 556 [M+1].

### Example 15: (2¹Z,4⁴E)-8-(cyclohexylsulfonyl)-4¹H-11-oxa-3,8-diaza-2(7,2)-pyrrolo[2,1-f][1,2,4]triazina-4(4,1)-pyrazola-1(1,4)-benzenacycloundecaphane

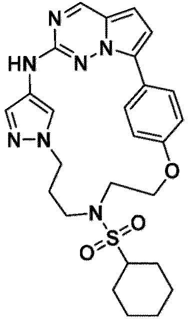

The title compound was obtained by carrying out the same process as Example 9 above using the corresponding sulfonyl chloride. MS m/z: 522 [M+1].

### Example 16: (Z)-5-oxa-3,9,15-triaza-2(7,2)-pyrrolo[2,1-f][1,2,4]triazina-1, 4(1,4)-dibenzenacyclopentadecaphan-10-one

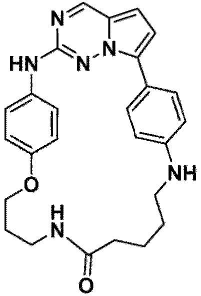

### Step 1) Preparation of 2-chloro-7-(4-nitrophenyl)pyrrolo[2,1-f][1,2,4]triazine

The title compound was obtained by carrying out the same process as Example 1 Step 1 above using the corresponding boronic acid. MS m/z: 275 [M+1].

### Step 2) Preparation of 4-(2-chloropyrrolo[2,1-f][1,2,4]triazin-7-yl)aniline

To a solution of 2-Chloro-7-(4-nitrophenyl)pyrrolo[2,1-f][1,2,4]triazine (1.2 g, 4.4 mmol) in EtOH (10 ml) was added and tin dichloride dihydrate (2.82 g, 11.0 mmol) and stirred at 80 ° C for 3 hours. When the reaction was complete, the mixture was diluted with dichloromethane and neutralized with sodium bicarbonate to pH 5. The resulting separated organic layer was dried over anhydrous sodium sulfate, filtered under reduced pressure, and distilled under reduced pressure. 700 mg (yield: 65%) of the resulting title compound was used in the next reaction without further purification. MS m/z: 245 [M+1].

### Step 3) Preparation of ethyl 5-((4-(2-chloropyrrolo[2,1-f][1,2,4]triazin-7-yl)phenyl)amino)pentanoate

To a solution of 4-(2-Chloropyrrolo[2,1-f][1,2,4]triazin-7-yl)aniline (630 mg, 2.58 mmol) in acetonitrile (8.6 ml) was added potassium carbonate (1.07 g, 7.74 mmol) and ethyl pentanoate (0.61 ml, 3.84 mmol) and stirred at 80 ° C for 3 hours. When the reaction was complete, the mixture was washed with dichloromethane and saturated brine. The resulting separated organic layer was dried over anhydrous sodium sulfate, filtered under reduced pressure, and distilled under reduced pressure. The resulting residue was purified by chromatography to obtain 800 mg of the title compound (yield: 85%). MS m/z: 373 [M+1].

### Step 4) Preparation of Ethyl 5-((4-(2-((4-(3-((tert-butoxycarbonyl)amino)propoxy)phenyl)amino)pyrrolo[2,1-f][1,2,4]triazin-7-yl)phenyl)amino)pentanoate)

To a solution of ethyl 5-((4-(2-chloropyrrolo[2,1-f][1,2,4]triazin-7-yl)phenyl)amino)pentanoate (90 mg, 0.23 mmol) in 2.4 mL of see-butanol was added potassium carbonate (0.60 g, 0.60 mmol) and tert-butyl (3-(4-aminophenoxy)propyl)carbamate (97 mg, 0.36 mmol). After flowing nitrogen through the mixture for 10 minutes, Pd2(dba)3 (44 mg, 0.05 mmol) and Xphos (48 mg, 0.10 mmol) were added and stirred at 80 ° C for 4 hours. When the reaction was complete, the mixture was filtered through Celite, diluted with ethyl acetate, and washed with saturated sodium chloride solution. The resulting separated organic layer was dried over anhydrous sodium sulfate, filtered under reduced pressure, and distilled under reduced pressure. The resulting residue was purified by chromatography to obtain 60 mg of the title compound (yield: 42%). MS m/z: 603 [M+1].

### Step 5) Preparation of 5-((4-(2-((4-(3-((tert-butoxycarbonyl)amino)propoxy)phenyl)amino)pyrrolo[2,1-f][1,2,4]triazin-7-yl)phenyl)amino)pentanoic acid

To a solution of ethyl 5-((4-(2-((4-(3-((tert-butoxycarbonyl)amino)propoxy)phenyl)amino)pyrrolo[2,1-f][1,2,4 ]Triazin-7-yl)phenyl)amino)pentanoate (90 mg) in methanol/water (4:1, v/v) was added lithium hydroxide at 0 ° C, and then stirred at room temperature. When the reaction was completed, the reaction mixture was diluted with dichloromethane, the pH was adjusted to 3-4 with 1 N HCl aqueous solution, and extracted with dichloromethane. The resulting separated organic layer was dried over anhydrous sodium sulfate, filtered under reduced pressure, and distilled under reduced pressure. The resulting title compound was used in the next reaction without further purification. MS m/z: 575 [M+1].

### Step 6) Preparation of 5-((4-(2-((4-(3-aminopropoxy)phenyl)amino)pyrrolo[2,1-f][1,2,4]triazin-7-yl) Phenyl)amino)pentanoic acid

5-((4-(2-((4-(3-((tert-butoxycarbonyl)amino)propoxy)phenyl)amino)pyrrolo[2,1-f][1,2,4] Triazin-7-yl)phenyl)amino)pentanoic acid was dissolved in a 4 M hydrochloric acid-dioxane solution. The mixture was stirred at 40° C for 4 hours. When the reaction was complete, the mixture was diluted with dichloromethane and neutralized with sodium bicarbonate to pH 5. The resulting separated organic layer was dried over anhydrous sodium sulfate, filtered under reduced pressure, and distilled under reduced pressure. The resulting title compound was used in the next reaction without further purification. MS m/z: 475 [M+1].

### Step 7) Preparation of (Z)-5-oxa-3,9,15-triaza-2(7,2)-pyrrolo[2,1-f][1,2,4]triazina-1, 4(1,4)-dibenzenacyclopentadecaphan-10-one

To a solution of 5-((4-(2-((4-(3-aminopropoxy)phenyl)amino)pyrrolo[2,1-fl[1,2,4]triazin-7-yl)phenyl)amino)pentanoic acid in dimethylformamide was added diisopropylethylamine and HATU at 0 ° C, and stirred at room temperature for 1 hour. When the reaction was complete, the mixture was diluted with dichloromethane and washed with saturated brine. The resulting separated organic layer was dried over anhydrous sodium sulfate, filtered under reduced pressure, and distilled under reduced pressure. The resulting title compound was used in the next reaction without further purification. MS m/z: 457 [M+1].

### Example 17: (1⁴Z,2¹Z)-1¹H-5-oxa-3,9-diaza-2(7,2)-pyrrolo[2,1-f][1,2,4]triazina- 1(4,1)-pyrazola-4(1,4)-benzenacyclohexadecaphan-10-one

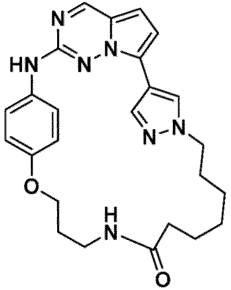

### Step 1) Preparation of tert-butyl 4-(2-chloropyrrolo[2,1-f][1,2,4]triazin-7-yl)-1H-pyrazolee-1-carboxylate

The title compound was obtained by carrying out the same process as Step 1 of Example 1 above using the corresponding boronic acid. MS m/z: 320 [M+1].

### Step 2) Preparation of 2-Chloro-7-(1H-pyrazol-4-yl)pyrrolo[2,1-f][1,2,4]triazine

To a solution of tert-butyl 4-(2-chloropyrrolo[2,1-f][1,2,4]triazin-7-yl)-1H-pyrazolee-1-carboxylate (500 mg, 2.38 mmol) in dichloromethane (2.3 ml) was added trifluoroacetic acid (2.3 ml) was added at 0 ° C, and stirred at room temperature for 12 hours. When the reaction was complete, the mixture was diluted with dichloromethane and neutralized with sodium bicarbonate to pH 5. The resulting separated organic layer was dried over anhydrous sodium sulfate, filtered under reduced pressure, and distilled under reduced pressure. The resulting title compound was used in the next reaction without further purification. MS m/z: 220 [M+1].

### Step 3) Preparation of Methyl 6-(4-(2-chloropyrrolo[2,1-f][1,2,4]triazin-7-yl)-1H-pyrazol-1-yl)heptanoate

To a solution of 4-(2-Chloropyrrolo[2,1-f][1,2,4]triazin-7-yl)aniline (280 mg, 1.28 mmol) in acetonitrile (2.6 ml) was addded potassium carbonate (442 mg, 3.2 mmol) and methyl hexanoate (0.35 ml, 1.92 mmol) and stirred at 80 ° C for 3 hours. When the reaction was complete, the mixture was washed with dichloromethane and saturated sodium chloride solution. The resulting separated organic layer was dried over anhydrous sodium sulfate, filtered under reduced pressure, and distilled under reduced pressure. The resulting residue was purified by chromatography to obtain 200 mg of the title compound (yield: 45%).MS m/z: 348 [M+1].

### Step 4) Preparation of Methyl 6-(4-(2-((4-(3-((tert-butoxycarbonyl)amino)propoxy)phenyl)amino)pyrrolo[2,1-f][1,2 4]triazine-7-yl)-1H-pyrazol-1-yl)heptanoate

The title compound was obtained by carrying out the same process as Step 4 of Example 16 above using the corresponding aniline. MS m/z: 578 [M+1].

### Step 5) Preparation of (1⁴Z,2¹Z)-1¹H-5-oxa-3,9-diaza-2(7,2)-pyrrolo[2,1-f][1,2,4]triazina-1(4,1)-pyrazola-4(1,4)-benzenacyclohexadecaphan-10-one

The title compound was obtained by carrying out the same process as steps 5 to 7 of Example 16 using the compound prepared in step 4 above. MS m/z: 460 [M+1].

### Example 18: (1⁴Z,2¹Z,4⁴E)-1¹H,4¹H-3,8-diaza-2(7,2)-pyrrolo[2,1-f][1,2,4]triazina-1,4(4,1)-dipyrazolacyclopentadecaphan-9-one

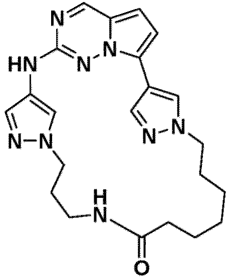

The title compound was obtained by carrying out the same process as Example 17 above. MS m/z: 434 [M+1].

### Example 19: (1⁴Z,2¹Z)-1¹H-3-aza-2(7,2)-pyrrolo[2,1-f][1,2,4]triazina-5(1,4)-piperazina-1(4,1)-pyrazola-4(1,4)-benzenacyclododecaphane-9-one

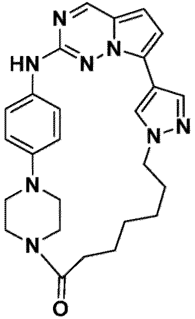

The title compound was obtained by carrying out the same process as Example 17 above. MS m/z: 471 [M+1].

### Example 20: (Z)-13-fluoro-5,17-dioxa-3,9-diaza-2(7,2)-pyrrolo[2,1-f][1,2,4 ]triazina-1,4(1,4)-dibenzenacycloheptadecaphan-10-one

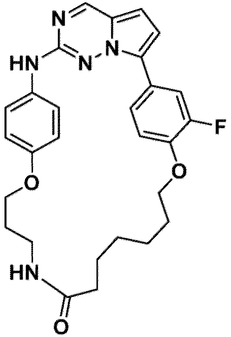

### Step 1) Preparation of 4-(2-chloropyrrolo[2,1-f][1,2,4]triazin-7-yl)-2-fluorophenol

The title compound was obtained by carrying out the same process as Step 1 of Example 1 above using the corresponding boronic acid MS m/z: 264 [M+1].

### Step 2) Preparation of Methyl 7-(4-(2-chloropyrrolo[2,1-f][1,2,4]triazin-7-yl)-2-fluorophenoxy)heptanoate

To a solution of 4-(2-chloropyrrolo[2,1-f][1,2,4]triazin-7-yl)-2-fluorophenol (400 mg, 1.52 mmol) acetonitrile (5.5 ml) was added potassium carbonate (570 mg, 3.8 mmol) and methyl heptanoate (0.34 ml, 1.82 mmol) and stirred at 80 ° C for 3 hours. When the reaction was complete, the mixture was washed with dichloromethane and saturated sodium chloride solution. The resulting separated organic layer was dried over anhydrous sodium sulfate, filtered under reduced pressure, and distilled under reduced pressure. The resulting residue was purified by chromatography to obtain 514 mg of the title compound (yield: 83%). MS m/z: 406 [M+1].

### Step 3) Preparation of Methyl 7-(4-(2-((4-(3-((tert-butoxycarbonyl)amino)propoxy)phenyl)amino)pyrrolo[2,1-f][1,2,4]triazin-7-yl)-2-fluorophenoxy)heptanoate

The title compound was obtained by carrying out the same process as Step 4 of Example 16 above using the corresponding aniline. MS m/z: 636 [M+1].

### Step 4) Preparation of (Z)-13-fluoro-5,17-dioxa-3,9-diaza-2(7,2)-pyrrolo[2,1-f][1,2,4 Itriazina-1,4(1,4)-dibenzenacycloheptadecaphan-10-one

The title compound was obtained by carrying out the same process as steps 5 to 7 of Example 16 using the compound prepared in step 3 above. MS m/z: 504 [M+1].

### Example 21: (2¹Z,4⁴E)-13-fluoro-4¹H-16-oxa-3,8-diaza-2(7,2)-pyrrolo[2,1-f][1,2,4]triazina-4(4,1)-pyrazola-1(1,4)-benzenacyclohexadecaphan-9-one

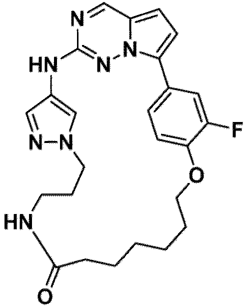

The title compound was obtained by carrying out the same process as Example 20 above. MS m/z: 478 [M+1].

### Example 22: (Z)-1³-fluoro-13-oxa-3-aza-2(7,2)-pyrrolo[2,1-f][1,2,4]triazina-5(1,4)-piperazina-1,4(1,4)-dibenzenacyclotridecaphan-6-one

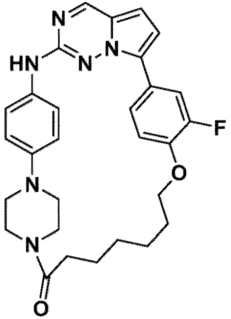

The title compound was obtained by carrying out the same process as Example 20 above. MS m/z: 504 [M+1].

### Example 23: (Z)-17-oxa-3,9-diaza-2(7,2)-pyrrolo[2,1-f][1,2,4]triazina-5(1,4)-piperazina-1,4(1,4)-dibenzenacycloheptadecaphan-10-one

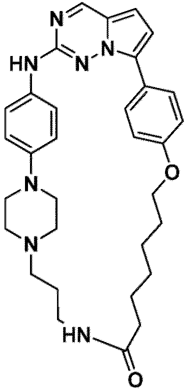

### Step 1) Preparation of 4-(2-chloropyrrolo[2,1-f][1,2,4]triazin-7-yl)phenol

The title compound was obtained by carrying out the same process as Step 1 of Example 1 using the corresponding boronic acid (yield: 90%). MS m/z: 246 [M+1].

### Step 2) Preparation of Methyl 7-(4-(2-chloropyrrolo[2,1-f][1,2,4]triazin-7-yl)phenoxy)heptanoate

The title compound was obtained by carrying out the same process as Step 2 of Example 20 using 4-(2-chloropyrrolo[2,1-f][1,2,4]triazin-7-yl)phenol. MS m/z: 388 [M+1].

### Step 3) Preparation of Methyl 7-(4-(2-((4-(4-(3-((tert-butoxycarbonyl)amino)propyl)piperazin-1-yl)phenyl)amino)pyrrolo[2 ,1-f][1,2,4]triazine-7-yl)phenoxy)heptanoate

The title compound was obtained by carrying out the same process as Step 4 of Example 16 above using the corresponding aniline. MS m/z: 686 [M+1].

### Step 4) Preparation of (Z)-17-oxa-3,9-diaza-2(7,2)-pyrrolo[2,1-f][1,2,4]triazina-5(1,4)-piperazina-1,4(1,4)-dibenzenacycloheptadecaphan-10-one

The title compound was obtained by performing the same process as steps 5 to 7 of Example 16 using the compound prepared in step 3 above. MS m/z: 504 [M+1].

### Example 24: (Z)-13-oxa-3-aza-2(7,2)-pyrrolo[2,1-f][1,2,4]triazina-5(1,4)-piperazina-1,4(1,4)-dibenzenacyclotridecaphan-6-one

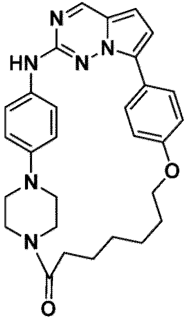

### Step 1) Preparation of tert-butyl 4-(4-((7-(4-((7-methoxy-7-oxoheptyl)oxy)phenyl)pyrrolo[2,1-f][1,2,4]triazine-2-yl)amino)phenyl)piperazine-1-carboxylate

The title compound was obtained by carrying out the same process as Step 4 of Example 16 using the compound prepared in Step 2 of Example 23 and the corresponding aniline. MS m/z: 497[M+1].

### Step 2) Preparation of (Z)-13-oxa-3-aza-2(7,2)-pyrrolo[2,1-f][1,2,4]triazina-5(1,4)-piperazina-1,4(1,4)-dibenzenacyclotridecaphan-6-one

The title compound was obtained by carrying out the same process as steps 5 to 7 of Example 16 using the compound prepared in step 1 above. MS m/z: 629 [M+1].

### Example 25: (2¹Z,4⁴E)-4¹H-11-oxa-3-aza-2(7,2)-pyrrolo[2,1-f][1,2,4]triazina-5(4,1)-piperidina-4(4,1)-pyrazola-1(1,4)-benzenacycloundecaphan-6-one

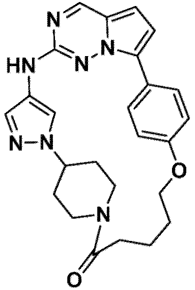

### Step 1) Preparation of Ethyl 5-(4-(2-chloropyrrolo[2,1-f][1,2,4]triazin-7-yl)phenoxy)pentanoate

The title compound was obtained using ethyl 5-bromopentanoate in Step 1 of Example 23 above. MS m/z: 373 [M+1].

### Step 2) Preparation of (2¹Z,4⁴E)-4¹H-11-oxa-3-aza-2(7,2)-pyrrolo[2,1-f][1,2,4]triazina-5(4,1)-piperidina-4(4,1)-pyrazola-1(1,4)-benzenacycloundecaphan-6-one

The title compound was obtained by carrying out the same process as Steps 4 to 7 of Example 16 using the compound prepared in Step 1 above. MS m/z: 458 [M+1].

### Example 26: (2¹Z,4⁴E)-4¹H-15-oxa-3,9-diaza-2(7,2)-pyrrolo[2,1-f][1,2,4]triazina-5(4,1)-piperidina-4(4,1)-pyrazola-1(1,4)-benzenacyclopentadecaphan-10-one

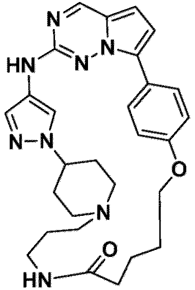

The title compound was obtained by carrying out the same process as Example 25 above using the corresponding aniline. MS m/z: 515 [M+1].

### Example 27: (2¹Z,4⁴E)-4¹H-17-oxa-3,9-diaza-2(7,2)-pyrrolo[2,1-f][1,2,4]triazina-5(4,1)-piperidina-4(4,1)-pyrazola-1(1,4)-benzenacycloheptadecaphan-10-one

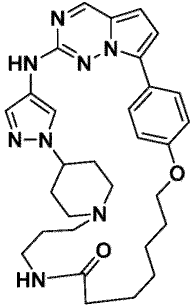

The title compound was obtained by carrying out the same process as Example 25 above using the corresponding aniline. MS m/z: 675 [M+1].

### Example 28: (Z)-5,12,15-trioxa-3,9-diaza-2(7,2)-pyrrolo[2,1-f][1,2,4]triazina -1,4(1,4)-dibenzenacyclopentadecaphan-10-one

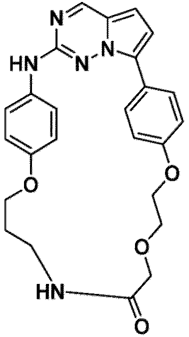

### Step 1) Preparation of Ethyl 2-(2-(4-(2-chloropyrrolo[2,1-f][1,2,4]triazin-7-yl)phenoxy)ethoxy)acetate

The title compound was obtained by carrying out the same process as Step 2 of Example 23 using ethyl 2-(2-bromoethoxy)acetate. MS m/z: 376 [M+1].

### Step 2) Preparation of (Z)-5,12,15-trioxa-3,9-diaza-2(7,2)-pyrrolo[2,1-f][1,2,4]triazina -1,4(1,4)-dibenzenacyclopentadecaphan-10-one

The title compound was obtained by performing the same process as Example 24 above using the corresponding aniline. MS m/z: 460 [M+1].

### Example 29: (Z)-5,17-dioxa-3,9-diaza-2(7,2)-pyrrolo[2,1-f][1,2,4]triazina-1 ,4(1,4)-dibenzenacycloheptadecaphan-10-one

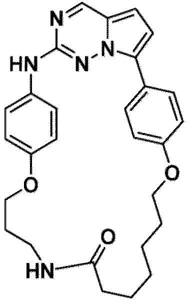

The title compound was obtained by carrying out same process as Example 71 above using the corresponding aniline. MS m/z: 486[M+1].

### Example 30: (Z)-5,12-dioxa-3,9-diaza-2(7,2)-pyrrolo[2,1-f][1,2,4]triazina-1 ,4(1,4)-dibenzenacyclododecaphane

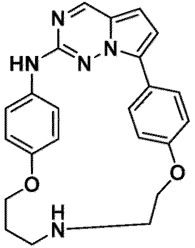

### Step 1) Preparation of tert-butyl (3-(4-((7-(4-(2-bromoethoxy)phenyl)pyrrolo[2,1-f][1,2,4]triazin-2-yl)amino)phenoxy)propyl)carbamate

The title compound was obtained by carrying out the same process as Step 3 of Example 1 using the corresponding aniline. MS m/z: 582 [M+1].

### Step 2) Preparation of (Z)-5,12-dioxa-3,9-diaza-2(7,2)-pyrrolo[2,1-f][1,2,4]triazina-1 ,4(1,4)-dibenzenacyclododecaphane

To a solution of the compound prepared in step 1 in dichloromethane (5 ml) was added 4 M hydrochloric acid-dioxane solution. The mixture was stirred at 40° C for 4 hours. When the reaction was complete, the mixture was diluted with dichloromethane, neutralized with sodium bicarbonate to pH 5, and extracted with isopropyl alcohol/chloroform (1:4, v/v) solution. The organic layer was dried over anhydrous sodium sulfate, filtered under reduced pressure, and distilled under reduced pressure. The resulting title compound was used in the next reaction without further purification (yield: 88%). MS m/z: 483 [M+1]. The resulting bromoamine was diluted in dimethylformamide (4 ml), diisopropylethylamine (5 equivalents) was added, and stirred at 80 ° C for 12 hours. When the reaction was complete, it was diluted with ethyl acetate and washed with saturated sodium chloride solution. The resulting organic layer was dried over anhydrous sodium sulfate and purified by chromatography to obtain the title compound (yield: 29%). MS m/z: 402 [M+1].

### Example 31: (Z)-1-(5,12-dioxa-3,9-diaza-2(7,2)-pyrrolo[2,1-f][1,2,4]tria zina-1,4(1,4)-dibenzenacyclododecapan-9-yl)propan-1-one

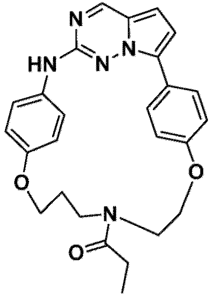

To a solution of the compound prepared in step 2 of Example 30 in tetrahydrofuran (1 ml) was added triethylamine (3 equivalents) at 0 ° C. Afterwards, acetyl chloride (1.2 equivalents) was added and stirred at 0 ° C for 20 minutes. When the reaction was complete, it was diluted with ethyl acetate and washed with saturated sodium chloride solution. The resulting organic layer was dried over anhydrous sodium sulfate and purified by chromatography to obtain the title compound (yield: 57%). MS m/z: 582 [M+1].

### Example 32: (Z)-5,12-dioxa-3,9-diaza-2(7,2)-pyrrolo[2,1-f][1,2,4]triazina-1,4(1,4)-dibenzenacyclododecaphan-9-yl(4-chlorophenyl)methanone

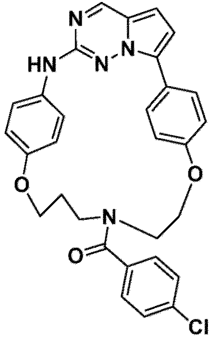

The title compound was obtained by performing the same process as Example 31 using the corresponding acyl chloride (yield: 88%). MS m/z: 540 [M+1].

### Example 33: (2¹Z,7Z)-5,10-dioxa-3-aza-2(7,2)-pyrrolo[2,1-f][1,2,4]triazina-1,4(1,4)-dibenzenacyclodecaphan-7-ene

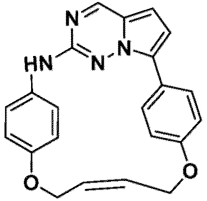

### Step 1) Preparation of 4-(2-((4-hydroxyphenyl)amino)pyrrolo[2,1-f][1,2,4]triazin-7-yl)phenol

The title compound was obtained by carrying out the same process as Step 4 of Example 1 using aniline corresponding to the compound prepared in Step 1 of Example 1 (yield: 62%). MS m/z: 319 [M+1].

### Step 2) Preparation of N,7-bis(4-(allyloxy)phenyl)pyrrolo[2,1-f][1,2,4]triazine-2-amine

To a solution of 4-(2-((4-hydroxyphenyl)amino)pyrrolo[2,1-f][1,2,4]triazin-7-yl)phenol in dimethylformamide (5 ml) was added potassium carbonate (5 equivalents) and allyl bromide (3 equivalents) and stirred at 50 ° C for 2 hours. When the reaction was complete, it was diluted with ethyl acetate and washed with saturated sodium chloride solution. The resulting organic layer was dried over anhydrous sodium sulfate and purified by chromatography to obtain the title compound (yield: 78%). MS m/z: 399 [M+1].

### Step 3) Preparation (2¹Z,7Z)-5,10-dioxa-3-aza-2(7,2)-pyrrolo[2,1-f][1,2,4]triazina-1,4(1,4)-dibenzenacyclodecaphan-7-ene

To a solution of N,7-bis(4-(allyloxy)phenyl)pyrrolo[2,1-f][1,2,4]triazine-2-amine in toluene (10 ml) was added Grubbs second catalyst(10 mol%) and stirred at 100 ° C for 40 hours. When the reaction was completed, it was distilled under reduced pressure and purified by chromatography to obtain the title compound (yield: 12%). MS m/z: 371 [M+1].

### Example 34: (Z)-4²-methoxy-5,12,15-trioxa-3,9-diaza-2(7,2)-pyrrolo[2,1-f][1,2 4]triazina-1,4(1,4)-dibenzenacyclopentadecaphan-10-one

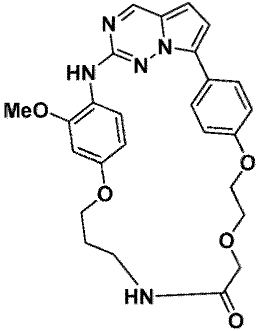

The title compound was obtained by carrying out the same process as Example 24 above using the corresponding aniline. MS m/z: 490 [M+1].

### Example 35: (2¹Z,4⁴E)-4¹H-11,14-dioxa-3,8-diaza-2(7,2)-pyrrolo[2,1-f][1,2,4]triazina-4(4,1)-pyrazola-1(1,4)-benzenacyclotetradecaphan-9-one

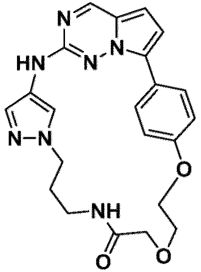

### Step 1) Preparation of Ethyl 2-(2-(4-(2-chloropyrrolo[2,1-f][1,2,4]triazin-7-yl)phenoxy)ethoxy)acetate

The title compound was obtained by carrying out the same process as Step 2 of Example 1 using tert-butyl (3-(4-aminophenoxy)propyl)carbamate (yield: 58%). MS m/z: 376 [M+1].

### Step 2) Preparation of (2¹Z,4⁴E)-4¹H-11,14-dioxa-3,8-diaza-2(7,2)-pyrrolo[2,1-f][1,2,4]triazina-4(4,1)-pyrazola-1(1,4)-benzenacyclotetradecaphan-9-one

The title compound was obtained by carrying out the same process as steps 4 to 7 of Example 1 using the corresponding aniline. MS m/z: 434 [M+1].

### Example 36: (2¹Z,4⁴E)-4¹H-14-oxa-3,8-diaza-2(7,2)-pyrrolo[2,1-f][1,2,4]triazina -4(4,1)-pyrazola-1(1,4)-benzenacyclotetradecaphan-9-one

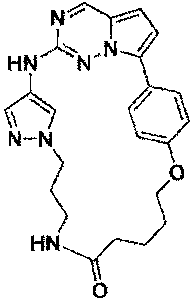

The title compound was obtained by carrying out the same process as Example 25 above using the corresponding aniline. MS m/z: 432 [M+1].

### Example 37: (2¹Z,4⁴E)-4¹H-16-oxa-3,8-diaza-2(7,2)-pyrrolo[2,1-f][1,2,4]triazina-4(4,1)-pyrazola-1(1,4)-benzenacyclohexadecaphan-9-one

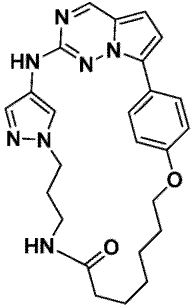

The title compound was obtained by carrying out the same process as Example 24 above using the corresponding aniline. MS m/z: 460 [M+1].

### Example 38: (Z)-5,15-dioxa-3,9-diaza-2(7,2)-pyrrolo[2,1-f][1,2,4]triazina- 1,4(1,4)-dibenzenacyclopentadecaphan-10-one

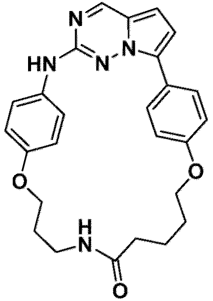

The title compound was obtained by carrying out the same process as Example 25 above using the corresponding aniline. MS m/z: 458 [M+1].

### Example 39: N-(3-(2-((4-(4-(5-((2-(2,6-dioxopiperidin-3-yl)-1,3-dioxoisoindolin-4-yl)oxy)pentyl)piperidin-1-yl)phenyl)amino)pyrrolo[2,1-f][1,2,4]triazin7-yl)phenyl)methanesulfonamide

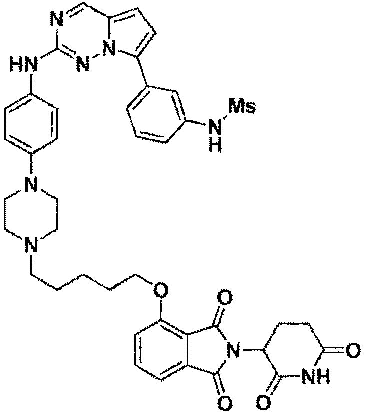

### Step 1: Preparation of N-(3-(2-chloropyrrolo[2,1-f][1,2,4]triazin-7-yl)phenyl)methanesulfonamide

To a solution of 7-bromo-2-chloropyrrole[2,1-f][1,2,4]triazine (1.0 g, 4.31 mmol) and (3-(methylsulfonamide)phenyl)boronic acid (972 mg, 4.53 mmol) in 1,4-dioxane (28 mL) was added 2.0 N sodium carbonate (13 mL) and t-ButylXphos (182 mg, 0.43 mmol) was added. After flowing nitrogen through the mixture for 10 minutes, Pd(PPh3)Cl2 (302 mg, 0.43 mmol) was added and stirred at 60 ° C for 6 hours. When the reaction was complete, the mixture was filtered using Celite, the filtrate was diluted with ethyl acetate, and then washed with saturated sodium chloride soluiton. The organic layer was dried over magnesium sulfate and concentrated. The residue was purified by chromatography to obtain the target compound (1.1 g, 86% yield). MS m/z: 323 [M+1].

### Step 2: Preparation of tert-butyl4-(4-((7-(3-(methylsulfonamide)phenyl)pyrrolo[2,1-f][1,2,4]triazin-2-yl)amino) Phenyl)piperazine-1-carboxylate

To a solution of N-(3-(2-chloropyrrolo[2,1-f][1,2,4]triazin-7-yl)phenyl)methanesulfonamide (1.0 g, 3.11 mmol) in sec-butanol (17 mL) was cesium carbonate (1.8 g, 12.8 mmol) and tert-butyl 4-(4-aminophenyl)piperazine-1-carboxylate (861 mg, 3.11 mmol). After flowing nitrogen into the solution for 10 minutes, Pd2(dba)3 (570 mg, 0.62 mmol) and Xphos (300 mg, 0.62 mmol) were added. The reaction mixture was stirred at 80° C for 6 hours. When the reaction was complete, the mixture was filtered using Celite, the filtrate was diluted with ethyl acetate, and then washed with saturated sodium chloride solution. The organic layer was dried over magnesium sulfate and concentrated. The residue was purified by chromatography to obtain the target compound (1.17 g, 67% yield). MS m/z: 564 [M+1].

### Step 3: Preparation of N-(3-(2-((4-(piperazin-1-yl)phenyl)amino)pyrrolo[2,1-f][1,2,4]triazin-7-yl)phenyl) methanesulfonamide

To a solution of tert-butyl4-(4-((7-(3-(methylsulfonamide)phenyl)pyrrolo[2,1-f][1,2,4]triazin-2-yl)amino)phenyl)piperazin-1-carboxylate (0.1 g, 0.18 mmol) in dichloromethane (2 mL) was added trifluoroacetic acid (0.3 ml). When the reaction was complete, the reaction mixture was concentrated under reduced pressure and used in the next reaction without purification. MS m/z: 464 [M+1].

### Step 4: Preparation of N-(3-(2-((4-(4-(5-((2-(2,6-dioxopiperidin-3-yl)-1,3-dioxoisoindolin-4-yl)oxy)pentyl)piperidin-1-yl)phenyl)amino)pyrrolo[2,1-f][1,2,4]triazin-7-yl)phenyl)methanesulfonamide

To a solution of N-(3-(2-((4-(piperazin-1-yl)phenyl)amino)pyrrolo[2,1-f][1,2,4]triazin-7-yl)phenyl)methanesulfonamide (100 mg, 0.22 mmol) in in dimethylformamide (2 mL) was added potassium iodide (73 mg, 0.22 mmol), diisopropylethylamine (0.12 mL, 0.66 mmol), and 4-((5-bromopentyl)oxy)-2- (2,6-dioxopiperidin-3-yl)isoindoline-1,3-dione (186 mg, 0.44 mmol). The reaction mixture solution was stirred at 60° C for 12 hours. When the reaction was complete, the reaction mixture solution was diluted with ethyl acetate and washed with saturated sodium chloride solution. The organic layer was dried over magnesium sulfate and concentrated. The resulting residue purified by chromatography to obtain the target compound (101 mg, 57% yield). MS m/z: 806 [M+1].

### Example 40: N-(3-(2-((4-(4-(3-((2-(2,6-dioxopiperidin-3-yl)-1,3-dioxoisoindolin-4-yl)oxy)propyl)piperidin-1-yl)phenyl)amino)pyrrolo[2,1-f][1,2,4]triazin-7-yl)phenyl)methanesulfonamide

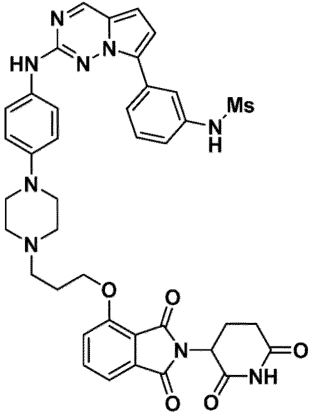

The procedure was identical to that of Example 39, except that 4-((5-bromopentyl)oxy)-2-(2,6-dioxopiperidin-3-yl)isoindoline-1,3-dione was used instead of 4-((5-bromopropyl)oxy)-2-(2,6-dioxopiperidin-3-yl)isoindoline-1,3-dione in step 4 of Example 39.

### Example 41: N-(3-(2-((4-(4-(7-((2-(2,6-dioxopiperidin-3-yl)-1,3-dioxoisoindolin-4-yl)oxy)heptyl)piperidin-1-yl)phenyl)amino)pyrrolo[2,1-f][1,2,4]triazin-7-yl)phenyl)methanesulfonamide

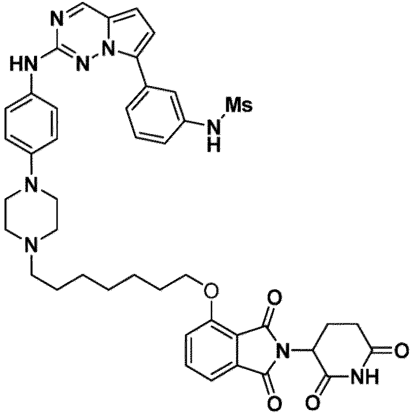

The procedure was identical to that of Example 39, except that 4-((5-bromoheptyl)oxy)-2-(2,6-dioxopiperidin-3-yl)isoindoline-1,3-dione was used instead of 4-((5-bromoheptyl)oxy)-2-(2,6-dioxopiperidin-3-yl)isoindoline-1,3-dione in step 4 of Example 1.

### Example 42: N-(3-(2-((4-(4-(11-((2-(2,6-dioxopiperidin-3-yl)-1,3-dioxoisoindolin-4-yl)oxy)undecyl)piperidin-1-yl)phenyl)amino)pyrrolo [2,1-f][1,2,4]triazin-7-yl)phenyl)methanesulfonamide

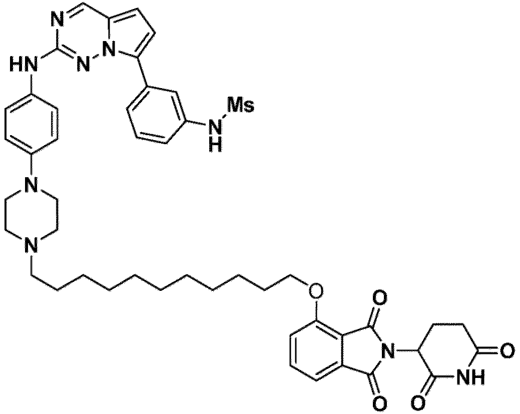

The procedure was identical to that of Example 39, except that 4-((5-bromoundecyl)oxy)-2-(2,6-dioxopiperidin-3-yl)isoindoline-1,3-dione was used instead of 4-((5-bromoheptyl)oxy)-2-(2,6-dioxopiperidin-3-yl)isoindoline-1,3-dione in step 4 of Example 1.

### Example 43: N-(3-(2-((4-(4-(2-((2-(2,6-dioxopiperidin-3-yl)-1,3-dioxoisoindolin-4-yl)oxy)ethyl)piperidin-1-yl)phenyl)amino)pyrrolo[2,1-f][1,2,4]triazin-7-yl)phenyl)methanesulfonamide

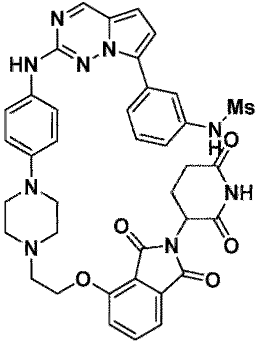

The procedure was identical to that of Example 39, except that 4-((5-bromoethyl)oxy)-2-(2,6-dioxopiperidin-3-yl)isoindoline-1,3-dione was used instead of 4-((5-bromoheptyl)oxy)-2-(2,6-dioxopiperidin-3-yl)isoindoline-1,3-dione in step 4 of Example 1.

### Example 44: N-(3-(2-((4-(4-(4-((2-(2,6-dioxopiperidin-3-yl)-1,3-dioxoisoindolin-4-yl)oxy)butyl)piperidin-1-yl)phenyl)amino)pyrrolo[2,1-f][1,2,4]triazin-7-yl)phenyl)methanesulfonamide

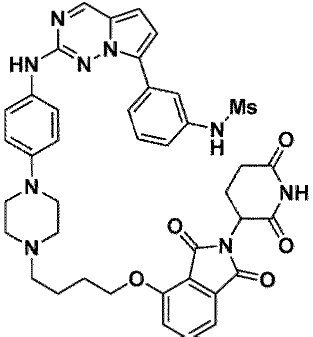

The procedure was identical to that of Example 39, except that 4-((5-bromobutyl)oxy)-2-(2,6-dioxopiperidin-3-yl)isoindoline-1,3-dione was used instead of 4-((5-bromoheptyl)oxy)-2-(2,6-dioxopiperidin-3-yl)isoindoline-1,3-dione in step 4 of Example 39.

### Example 45: N-(3-(2-((4-(4-(6-((2-(2,6-dioxopiperidin-3-yl)-1,3-dioxoisoindolin-4-yl)oxy)hexyl)piperidin-1-yl)phenyl)amino)pyrrolo[2,1-f][1,2,4]triazin-7-yl)phenyl)methanesulfonamide

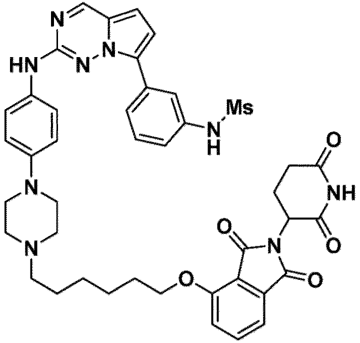

The procedure was identical to that of Example 39, except that 4-((5-bromohexyl)oxy)-2-(2,6-dioxopiperidin-3-yl)isoindoline-1,3-dione was used instead of 4-((5-bromoheptyl)oxy)-2-(2,6-dioxopiperidin-3-yl)isoindoline-1,3-dione in step 4 of Example 39.

### Example 46: N-(3-(2-((4-(4-(8-((2-(2,6-dioxopiperidin-3-yl)-1,3-dioxoisoindolin-4-yl)oxy)octyl)piperidin-1-yl)phenyl)amino)pyrrolo[2,1-f][1,2,4]triazin-7-yl)phenyl)methanesulfonamide

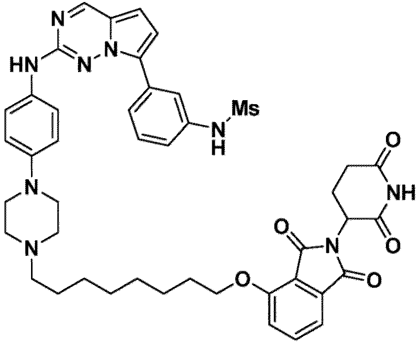

The procedure was identical to that of Example 39, except that 4-((5-bromooctyl)oxy)-2-(2,6-dioxopiperidin-3-yl)isoindoline-1,3-dione was used instead of 4-((5-bromoheptyl)oxy)-2-(2,6-dioxopiperidin-3-yl)isoindoline-1,3-dione in step 4 of Example 39.

### Example 47: N-(3-(2-((4-(4-(8-((2-(2,6-dioxopiperidin-3-yl)-1,3-dioxoisoindolin-4-yl)oxy)nonyl)piperidin-1-yl)phenyl)amino)pyrrolo[2,1-f][1,2,4]triazin-7-yl)phenyl)methanesulfonamide

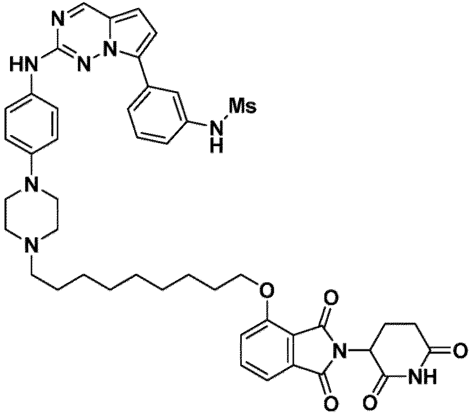

The procedure was identical to that of Example 39, except that 4-((5-bromononyl)oxy)-2-(2,6-dioxopiperidin-3-yl)isoindoline-1,3-dione was used instead of 4-((5-bromoheptyl)oxy)-2-(2,6-dioxopiperidin-3-yl)isoindoline-1,3-dione in step 4 of Example 39.

### Example 48: N-(3-(2-((4-(4-(2-(2-((1,3-dioxo-2-(2-oxopiperidin-3-yl)isoindolin-4-yl)oxy)ethoxy)ethyl)piperazin-1-yl)phenyl)amino)pyrrolo[2,1-f][1,2,4]triazin-7-yl)phenyl) methanesulfonamide

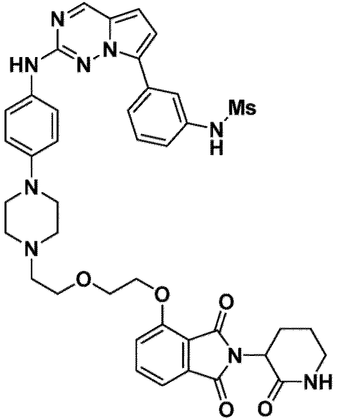

The procedure was identical to that of Example 39, except that 4-(2-(2-bromoethoxy)ethoxy)-2-(2-oxopiperidin-3-yl)isoindoline-1,3-dione was used instead of 4-((5-bromoheptyl)oxy)-2-(2,6-dioxopiperidin-3-yl)isoindoline-1,3-dione in step 4 of Example 39.

### Example 49: N-(3-(2-((4-(4-(6-((1,3-dioxo-2-(2-oxopiperidin-3-yl)isoindolin-4-yl)oxy)hexyl)piperazin-1-yl)phenyl)amino)pyrrolo[2,1-f][1,2,4]triazin-7-yl)phenyl)methanesulfonamide

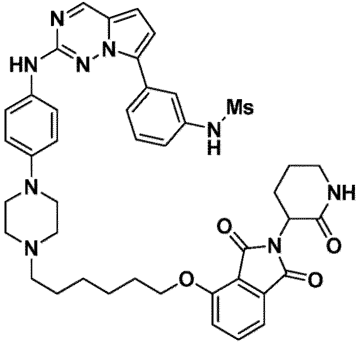

The procedure was identical to that of Example 39, except that 4-((6-bromohexyl)oxy)-2-(2-oxocyclohexyl)isoindoline-1,3-dione was used instead of 4-((5-bromoheptyl)oxy)-2-(2,6-dioxopiperidin-3-yl)isoindoline-1,3-dione in step 4 of Example 39.

### Example 50: N-(3-(2-((4-(4-(8-((1,3-dioxo-2-(2-oxopiperidin-3-yl)isoindolin-4-yl)oxy)octyl)piperazin-1-yl)phenyl)amino)pyrrolo[2,1-f][1,2,4]triazin-7-yl)phenyl)methanesulfonamide

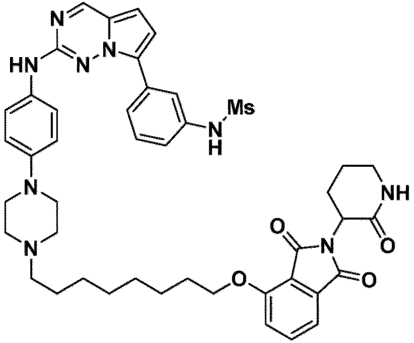

The procedure was identical to that of Example 39, except that 4-((6-bromooctyl)oxy)-2-(2-oxocyclohexyl)isoindoline-1,3-dione was used instead of 4-((5-bromoheptyl)oxy)-2-(2,6-dioxopiperidin-3-yl)isoindoline-1,3-dione in step 4 of Example 39.

### Example 51: N-(3-(2-((4-(4-((1-(2-(2,6-dioxopiperidin-3-yl)-6-fluoro-1,3-dioxoisoindolin-5-yl)piperidin-4-yl)methyl)piperazin-1-yl)phenyl)amino)pyrrolo[2,1-f][1,2,4]trizine-7-yl)phenyl)methanesulfonamide

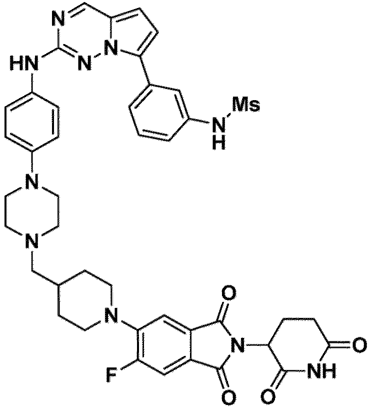

To a solution of N-(3-(2-((4-(piperazin-1-yl)phenyl)amino)pyrrolo[2,1-f][1,2,4]triazin-7-yl)phenyl)methanesulfonamide (100 mg, 0.22 mmol) in dichloromethane (2 mL) was added 1-(2-(2,6-dioxopiperidin-3-yl)-6-fluoro-1,3-dioxoisoindolin-5-yl)piperidin-4-carbaldehyde (128 mg, 0.33 mmol), acetic acid (1 drop). After adding sodium triacetoxyborohydride (140 mg, 0.66 mmol), the reaction mixture was stirred at room temperature for 3 hours. When the reaction was complete, water was added to quench the reaction, diluted with dichloromethane, and then washed with saturated sodium chloride solution. The organic layer was dried with magnesium sulfate and concentrated under reduced pressure. The resulting residue was purified by chromatography to obtain the target compound (113 mg, 62% yield). MS m/z: 835 [M+1].

### Example 52: N-(3-(2-((1-(1-(9-((2-(2,6-dioxopiperidin-3-yl)-1,3-dioxoindolin-4-yl)oxy)nonyl)piperidin-4-yl)-1H-pyrazol-4-yl)amino)pyrrolo[2,1-f][1,2,4]triazin-7-yl)phenyl)methanesulfonamide

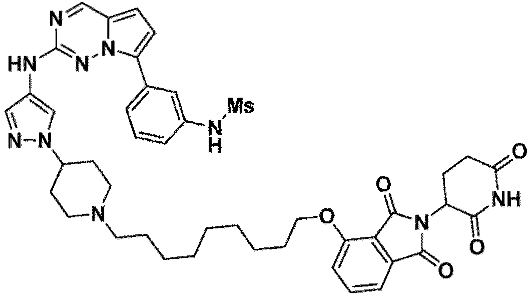

### Step 1: Preparation of 2-(2,6-dioxopiperidin-3-yl)-4-((9-(4-(4-nitro-1H-pyrazol-1-yl)piperidin-1- 1)nonyl)oxy)isoindoline-1,3-dione

To a solution of 4-(4-nitro-1H-pyrazol-1-yl)piperidine (1.5 g, 7.61 mmol) dimethylformamide (15 mL) was added potassium iodide (2.5 g, 15.22 mmol), diisopropylethylamine (4 mL, 22.83 mmol), and 4-((9-bromononyl)oxy)-2-(2,6-dioxopiperidin-3-yl)isoindoline-1,3-dione (186 mg, 0.44 mmol). The reaction mixture was stirred at 60° C for 12 hours. When the reaction was complete, the reaction mixture was diluted with ethyl acetate and washed with saturated sodium chloride solution. The organic layer was dried over magnesium sulfate and concentrated under reduced pressure. The resulting residue was purified by chromatography to obtain the target compound. MS m/z: 595 [M+1].

### Step 2: Preparation of 4-((9-(4-(4-amino-1H-pyrazol-1-yl)piperidin-1-yl)nonyl)oxy)-2-(2,6-dioxopiperidin-3-yl) Isoindoline-1,3-dione

To a solution of 2-(2,6-dioxopiperidin-3-yl)-4-((9-(4-(4-nitro-1H-pyrazol-1-yl)piperidin-1-yl)nonyl)oxy)isoindolin-1,3-dione (1 g, 1.68 mmol) in ethyl acetate (20 mL) was added Pd/C (100 mg) was added. The mixture was stirred at room temperature for one day under the pressure of a balloon filled with hydrogen gas. This reaction mixture solution was filtered through Celite and concentrated under reduced pressure. The target compound was used in the next reaction without purification. MS m/z : 565 [M+1].

Step 3: Preparation of *N*-(3-(2-((1-(1-(9-((2-(2,6-dioxopiperidin-3-yl)-1,3-dioxoindolin-4-yl)oxy)nonyl)piperidin-4-yl)-1*H*-pyrazol-4-yl)amino)pyrrolo[2,1-f][1,2,4]triazin-7-yl)phenyl)methanesulfonamide

The procedure was identical to that of step 2 of Example 1, except that 4-((9-(4-(4-amino-1H-pyrazol-1-yl)piperidin-1-yl)nonyl)oxy)-2-(2,6-dioxopiperidin-3-yl)isoindoline-1,3-dione was used instead of tert-butyl 4-(4-aminophenyl)piperazin-1-carboxylate. MS m/z : 851 [M+1].

### Example 53: 3-(2-((1-(1-(9-((2-(2,6-dioxopiperidin-3-yl)-1,3-dioxoindolin-4-yl)oxy)nonyl)piperidin-4-yl)-1H-pyrazol-4-yl)amino)pyrrolo[2,1-f][1,2,4]triazin-7-yl)-N-methylbenzamide

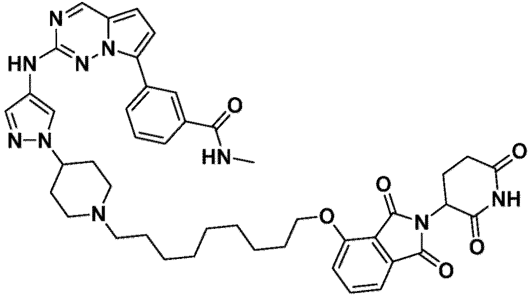

The procedure was identical to that of step 1 of Example 39, except that (3-(methylcarbamoyl)phenyl)boronic acid was used instead of (3-(methylsulfonamide)phenyl)boronic acid.

### Example 54: 2-(2,6-dioxopiperidin-3-yl)-4-((9-(4-(4-((7-phenylpyrrolo[2,1-f][1,2,4]triazin-2-yl)amino)-1H-pyrazol-1-yl)piperidin-1-yl)nonyl)oxy)isoindolin-1,3-dione

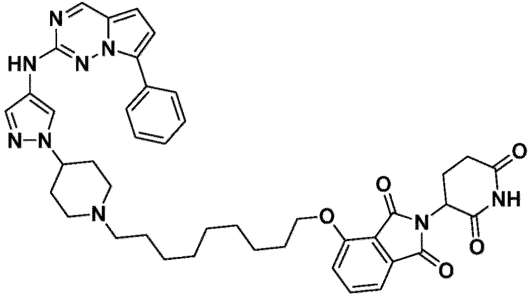

The procedure was identical to that of step 1 of Example 39, except that phenylboronic acid was used instead of (3-(methylsulfonamide)phenyl)boronic acid.

### Example 55: 2-(2,6-dioxopiperidin-3-yl)-4-((9-(4-(4-((7-(1-methyl-1H-pyrazole-4-1)pyrrolo[2,1-f][1,2,4]triazin-2-yl)amino)-1H-pyrazol-1-yl)piperidin-1-yl)nonyl)oxy)isoindolin-1,3-dione

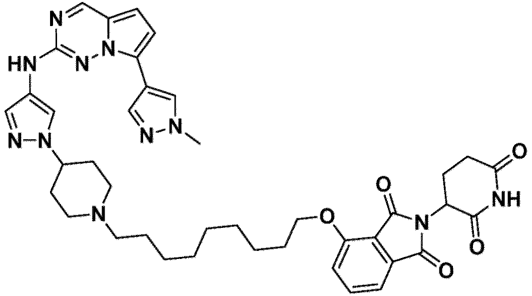

The procedure was identical to that of step 1 of Example 39, except that (1-methyl-1H-pyrazol-4-yl)boronic acid was used instead of (3-(methylsulfonamide)phenyl)boronic acid.

### Example 56: 4-((9-(4-(4-((7-(1H-indol-6-yl)pyrrolo[2,1-f][1,2,4]triazine-2-yl)amino)-1H-pyrazol-1-yl)piperidin-1-yl)nonyl)oxy)-2-(2,6-dioxopiperidin-3-yl)isoindolin-1,3-dione

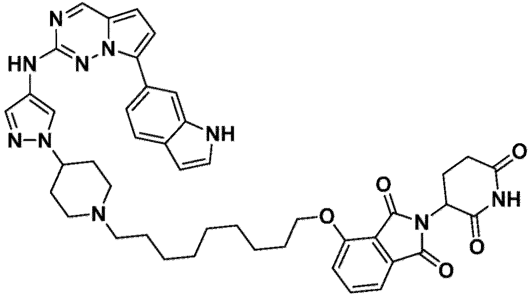

The procedure was identical to that of step 1 of Example 39, except that (1H-indole-6-yl)boronic acid was used instead of (3-(methylsulfonamide)phenyl)boronic acid.

### Example 57: 2-(2,6-dioxopiperidin-3-yl)-4-((9-(4-(4-((7-(4-hydroxyphenyl)pyrrolo[2,1-f][1,2,4]triazin-2-yl)amino)-1H-pyrazol-1-yl)piperidin-1-yl)nonyl)oxy)isoindolin-1,3-dione

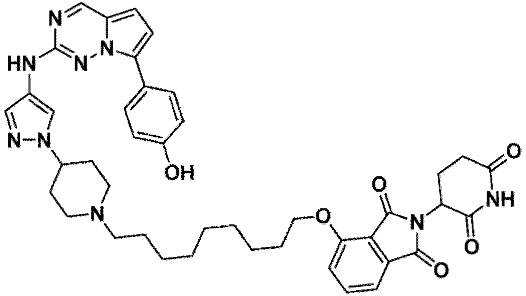

The procedure was identical to that of step 1 of Example 39, except that (4-Hydroxyphenyl)boronic acid was used instead of (3-(methylsulfonamide)phenyl)boronic acid.

### Example 58: Methyl-7-(4-(2-((1-(1-(9-((2-(2,6-dioxopiperidin-3-yl)-1,3-dioxoisoindolin-4-yl)oxy)nonyl)piperidin-4-yl)-1H-pyrazol-4-yl)amino)pyrrolo[2,1-f][1,2,4]triazine -7-day)phenoxy)heptanoate

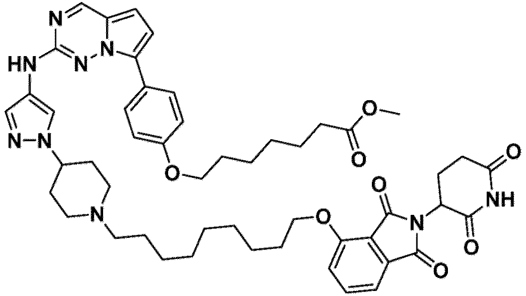

The procedure was identical to that of step 3 of Example 52, except that methyl-7-(4-(2-chloropyrrolo[2,1-f][1,2,4]triazin-7-yl)phenoxy)heptanoate was used instead of N-(3-(2-chloropyrrolo[2,1-f][1,2,4]triazin-7-yl)phenyl)methanesulfonamide.

### Example 59: N-(3-(2-((4-(4-(5-((2-(2,6-dioxopiperidin-3-yl)-1,3-dioxoisoindolin-4-yl)oxy)pentyl)piperazin-1-yl)-2-methoxyphenyl)amino)pyrrolo[2,1-f][1,2,4]triazin-7-yl)phenyl)methanesulfonamide

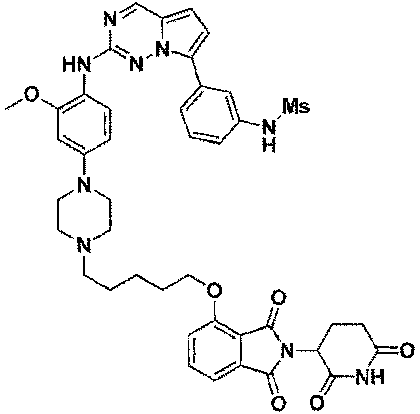

The procedure was identical to that of step 2 of Example 39, except that tert-butyl-4-(4-amino-3-methoxyphenyl)piperazine-1-carboxylate was used instead of tert-butyl 4-(4-aminophenyl)piperazine-1-carboxylate.

### Example 60: N-(3-(2-((4-(4-(3-((2-(2,6-dioxopiperidin-3-yl)-1,3-dioxoindolin-4-yl)oxy)propyl)piperazin-1-yl)-2-methoxyphenyl)amino)pyrrolo[2,1-f][1,2,4]triazin-7-yl)phenyl)methanesulfonamide

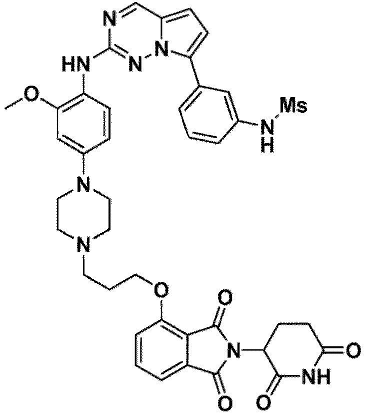

The procedure was identical to that of Example 40, except that tert-butyl-4-(4-amino-3-methoxyphenyl)piperazine-1-carboxylate was used instead of tert-butyl 4-(4-aminophenyl)piperazine-1-carboxylate.

### Example 61: N-(3-(2-((4-(4-(7-((2-(2,6-dioxopiperidin-3-yl)-1,3-dioxoindolin-4-yl)oxy)heptyl)piperazin-1-yl)-2-methoxyphenyl)amino)pyrrolo[2,1-f][1,2,4]triazin-7-yl)phenyl)methanesulfonamide

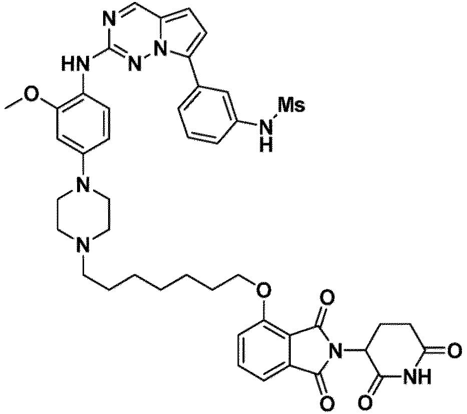

The procedure was identical to that of Example 41, except that tert-butyl-4-(4-amino-3-methoxyphenyl)piperazine-1-carboxylate was used instead of tert-butyl 4-(4-aminophenyl)piperazine-1-carboxylate.

### Example 62: N-(3-(2-((4-(4-(11-((2-(2,6-dioxopiperidin-3-yl)-1,3-dioxoindolin-4-yl)oxy)undecyl)piperazin-1-yl)-2-methoxyphenyl)amino)pyrrolo[2,1-f][1,2,4]triazin-7-yl)phenyl)methanesulfonamide

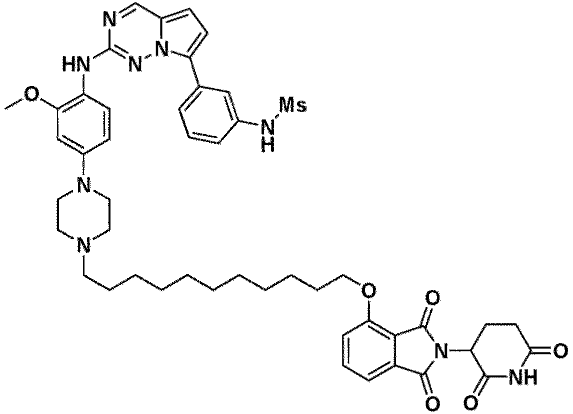

The procedure was identical to that of Example 42, except that tert-butyl-4-(4-amino-3-methoxyphenyl)piperazine-1-carboxylate was used instead of tert-butyl 4-(4-aminophenyl)piperazine-1-carboxylate.

### Example 63: N-(3-(2-((4-(4-(2-((2-(2,6-dioxopiperidin-3-yl)-1,3-dioxoindolin-4-yl)oxy)ethyl)piperazin-1-yl)-2-methoxyphenl)amino)pyrrolo[2,1-f][1,2,4]triazin-7-yl)phenyl)methanesulfonamide

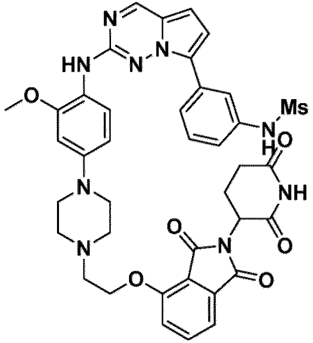

The procedure was identical to that of Example 43, except that tert-butyl-4-(4-amino-3-methoxyphenyl)piperazine-1-carboxylate was used instead of tert-butyl 4-(4-aminophenyl)piperazine-1-carboxylate.

### Example 64: N-(3-(2-((4-(4-(4-((2-(2,6-dioxopiperidin-3-yl)-1,3-dioxoindolin-4-yl)oxy)butyl)piperazin-1-yl)-2-methoxyphenl)amino)pyrrolo[2,1-f][1,2,4]triazin-7-yl)phenyl)methanesulfonamide

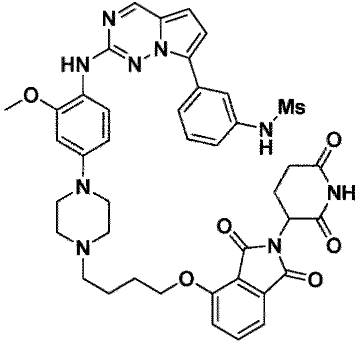

The procedure was identical to that of Example 44, except that tert-butyl-4-(4-amino-3-methoxyphenyl)piperazine-1-carboxylate was used instead of tert-butyl 4-(4-aminophenyl)piperazine-1-carboxylate.

### Example 65: N-(3-(2-((4-(4-(6-((2-(2,6-dioxopiperidin-3-yl)-1,3-dioxoindolin-4-yl)oxy)hexyl)piperazin-1-yl)-2-methoxyphenyl)amino)pyrrolo[2,1-f][1,2,4]triazin-7-yl)phenyl)methanesulfonamide

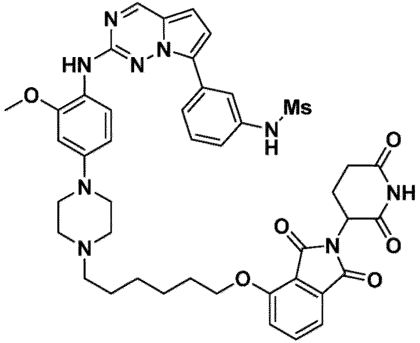

The procedure was identical to that of Example 45, except that tert-butyl-4-(4-amino-3-methoxyphenyl)piperazine-1-carboxylate was used instead of tert-butyl 4-(4-aminophenyl)piperazine-1-carboxylate.

### Example 66: N-(3-(2-((4-(4-(8-((2-(2,6-dioxopiperidin-3-yl)-1,3-dioxoindolin-4-yl)oxy)octyl)piperazin-1-yl)-2-methoxyphenl)amino)pyrrolo[2,1-f][1,2,4]triazin-7-yl)phenyl)methanesulfonamide

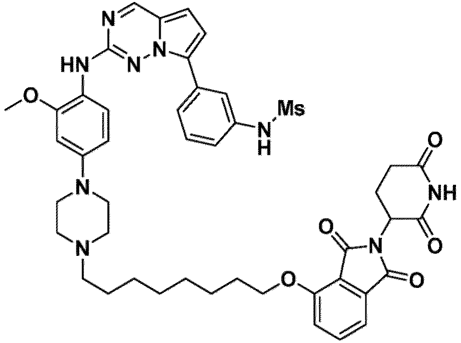

The procedure was identical to that of Example 46, except that tert-butyl-4-(4-amino-3-methoxyphenyl)piperazine-1-carboxylate was used instead of tert-butyl 4-(4-aminophenyl)piperazine-1-carboxylate.

### Example 67: N-(3-(2-((4-(4-(9-((2-(2,6-dioxopiperidin-3-yl)-1,3-dioxoindolin-4-yl)oxy)nonyl)piperazin-1-yl)-2-methoxyphenl)amino)pyrrolo[2,1-f][1,2,4]triazin-7-yl)phenyl)methanesulfonamide

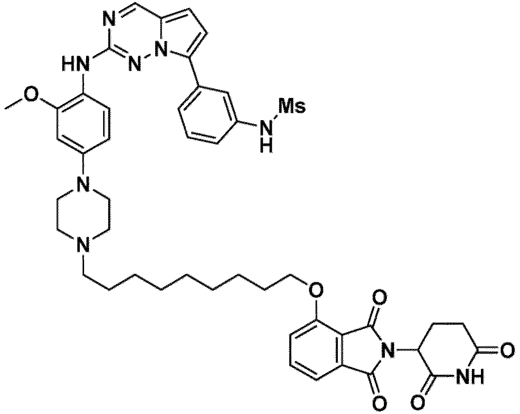

The procedure was identical to that of Example 47, except that tert-butyl-4-(4-amino-3-methoxyphenyl)piperazine-1-carboxylate was used instead of tert-butyl 4-(4-aminophenyl)piperazine-1-carboxylate.

### Example 68: N-(3-(2-((4-(4-(2-(2-((1,3-dioxo-2-(2-oxopiperidin-3-yl)isoindolin-4-yl)oxy)ethoxy)ethyl)piperazin-1-yl)-2-methoxyphenyl)amino)pyrrolo[2,1-f][1,2,4]triazine-7-1)phenyl)methanesulfonamide

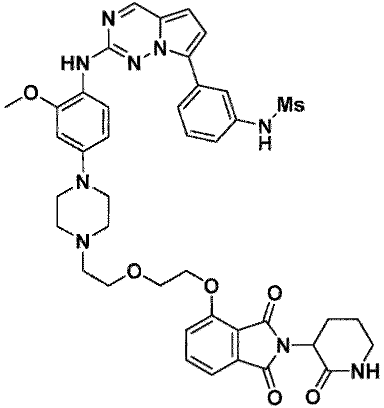

The procedure was identical to that of Example 48, except that tert-butyl-4-(4-amino-3-methoxyphenyl)piperazine-1-carboxylate was used instead of tert-butyl 4-(4-aminophenyl)piperazine-1-carboxylate.

### Example 69: N-(3-(2-((4-(4-(6-((1,3-dioxo-2-(2-oxopiperidin-3-yl)isoindolin-4-yl)oxy)hexyl)piperazin-1-yl)-2-methoxyphenyl)amino)pyrrolo[2,1-f][1,2,4]triazin-7-yl)phenyl)methanesulfonamide

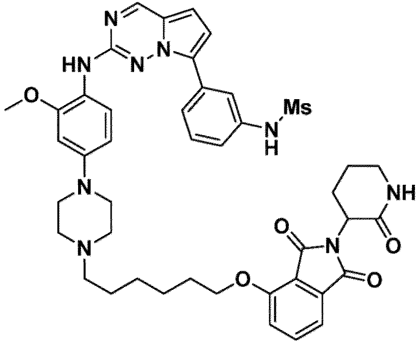

The procedure was identical to that of Example 49, except that tert-butyl-4-(4-amino-3-methoxyphenyl)piperazine-1-carboxylate was used instead of tert-butyl 4-(4-aminophenyl)piperazine-1-carboxylate.

### Example 70: N-(3-(2-((4-(4-(8-((1,3-dioxo-2-(2-oxopiperidin-3-yl)isoindoline-4-yl)oxy)octyl)piperazin-1-yl)-2-methoxyphenl)amino)pyrrolo[2,1-f][1,2,4]triazin-7-yl)phenyl)methanesulfonamide

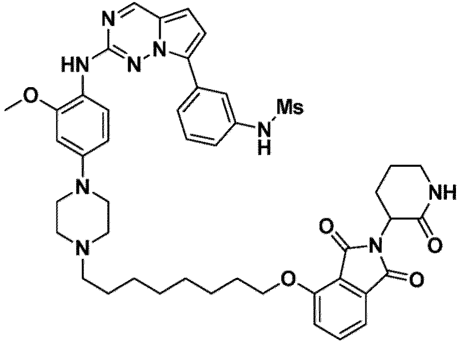

The procedure was identical to that of Example 50, except that tert-butyl-4-(4-amino-3-methoxyphenyl)piperazine-1-carboxylate was used instead of tert-butyl 4-(4-aminophenyl)piperazine-1-carboxylate.

### Example 71: N-(3-(2-((6-(4-(5-((2-(2,6-dioxopiperidin-3-yl)-1,3-dioxoindolin-4-yl)oxy)pentyl)piperazin-1-yl)pyridin-3-yl)amino)pyrrolo[2,1-f][1,2,4]triazin-7-yl)phenyl)methanesulfonamide

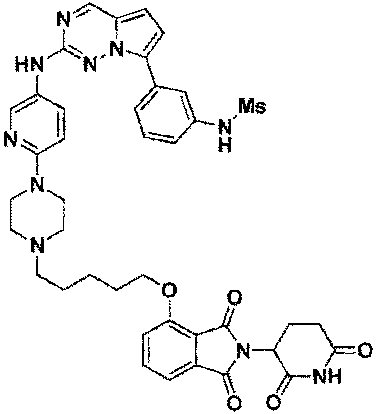

The procedure was identical to that of Example 51, except that tert-butyl-4-(5-aminopyridin-2-yl)piperazine-1-carboxylate was used instead of tert-butyl 4-(4-aminophenyl)piperazine-1-carboxylate.

### Example 72: N-(3-(2-((6-(4-(3-((2-(2,6-dioxopiperidin-3-yl)-1,3-dioxoindoline-4-yl)oxy)propyl)piperazin-1-yl)pyridin-3-yl)amino)pyrrolo[2,1-f][1,2,4]triazin-7-yl)phenyl)methanesulfonamide

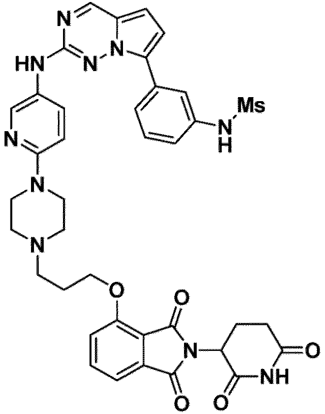

The procedure was identical to that of Example 40, except that tert-butyl-4-(5-aminopyridin-2-yl)piperazine-1-carboxylate was used instead of tert-butyl 4-(4-aminophenyl)piperazine-1-carboxylate.

### Example 73: N-(3-(2-((6-(4-(7-((2-(2,6-dioxopiperidin-3-yl)-1,3-dioxoindolin-4-yl)oxy)heptyl)piperazin-1-yl)pyridin-3-yl)amino)pyrrolo[2,1-f][1,2,4]triazin-7-yl)phenyl)methanesulfonamide

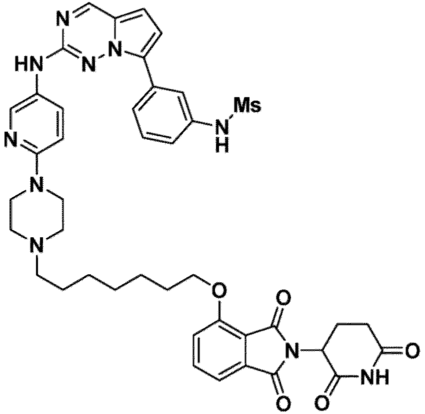

The procedure was identical to that of Example 42, except that tert-butyl-4-(5-aminopyridin-2-yl)piperazine-1-carboxylate was used instead of tert-butyl 4-(4-aminophenyl)piperazine-1-carboxylate.

### Example 74: N-(3-(2-((6-(4-(11-((2-(2,6-dioxopiperidin-3-yl)-1,3-dioxoindoline-4-yl)oxy)undecyl)piperazin-1-yl)pyridin-3-yl)amino)pyrrolo[2,1-f][1,2,4]triazin-7-yl)phenyl)methanesulfonamide

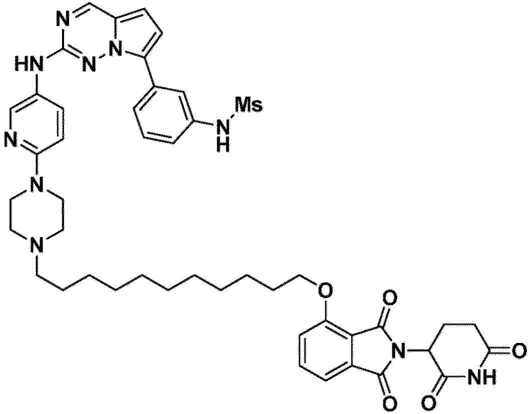

The procedure was identical to that of Example 43, except that tert-butyl-4-(5-aminopyridin-2-yl)piperazine-1-carboxylate was used instead of tert-butyl 4-(4-aminophenyl)piperazine-1-carboxylate.

### Example 75: N-(3-(2-((6-(4-(2-((2-(2,6-dioxopiperidin-3-yl)-1,3-dioxoindolin-4-yl)oxy)ethyl)piperazin-1-yl)pyridin-3-yl)amino)pyrrolo[2,1-f][1,2,4]triazin-7-yl)phenyl)methanesulfonamide

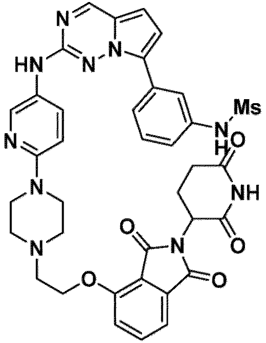

The procedure was identical to that of Example 44, except that tert-butyl-4-(5-aminopyridin-2-yl)piperazine-1-carboxylate was used instead of tert-butyl 4-(4-aminophenyl)piperazine-1-carboxylate.

### Example 76: N-(3-(2-((6-(4-(4-((2-(2,6-dioxopiperidin-3-yl)-1,3-dioxoindoline-4-yl)oxy)butyl)piperazin-1-yl)pyridin-3-yl)amino)pyrrolo[2,1-f][1,2,4]triazin-7-yl)phenyl)methanesulfonamide

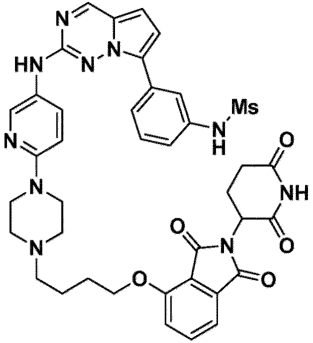

The procedure was identical to that of Example 45, except that tert-butyl-4-(5-aminopyridin-2-yl)piperazine-1-carboxylate was used instead of tert-butyl 4-(4-aminophenyl)piperazine-1-carboxylate.

### Example 77: N-(3-(2-((6-(4-(6-((2-(2,6-dioxopiperidin-3-yl)-1,3-dioxoindolin-4-yl)oxy)hexyl)piperazin-1-yl)pyridin-3-yl)amino)pyrrolo[2,1-f][1,2,4]triazin-7-yl)phenyl)methanesulfonamide

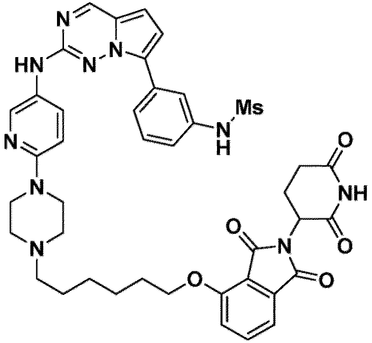

The procedure was identical to that of Example 46, except that tert-butyl-4-(5-aminopyridin-2-yl)piperazine-1-carboxylate was used instead of tert-butyl 4-(4-aminophenyl)piperazine-1-carboxylate.

### Example 78: N-(3-(2-((6-(4-(8-((2-(2,6-dioxopiperidin-3-yl)-1,3-dioxoindolin-4-yl)oxy)octyl)piperazin-1-yl)pyridin-3-yl)amino)pyrrolo[2,1-f][1,2,4]triazin-7-yl)phenyl)methanesulfonamide

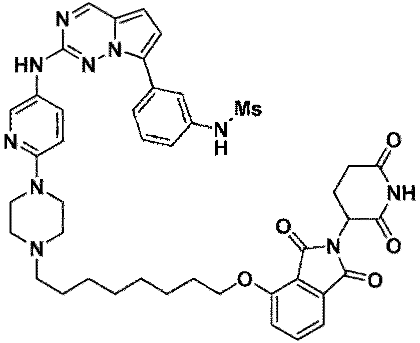

The procedure was identical to that of Example 47, except that tert-butyl-4-(5-aminopyridin-2-yl)piperazine-1-carboxylate was used instead of tert-butyl 4-(4-aminophenyl)piperazine-1-carboxylate.

### Example 79: N-(3-(2-((6-(4-(9-((2-(2,6-dioxopiperidin-3-yl)-1,3-dioxoindolin-4-yl)oxy)nonyl)piperazin-1-yl)pyridin-3-yl)amino)pyrrolo[2,1-f][1,2,4]triazin-7-yl)phenyl)methanesulfonamide

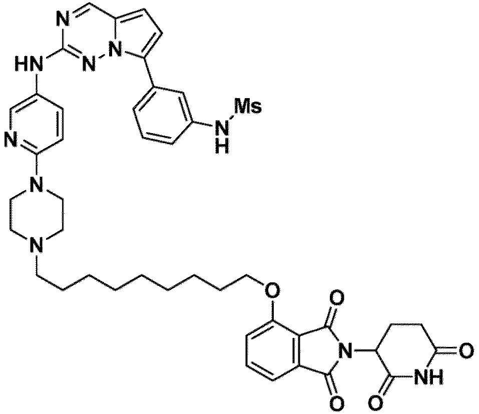

The procedure was identical to that of Example 48, except that tert-butyl-4-(5-aminopyridin-2-yl)piperazine-1-carboxylate was used instead of tert-butyl 4-(4-aminophenyl)piperazine-1-carboxylate.

### Example 80: N-(3-(2-((6-(4-(2-(2-((1,3-dioxo-2-(2-oxopiperidin-3-yl)isoindolin-4-yl)oxy)ethoxy)ethyl)piperazin-1-yl)pyridin-3-yl)amino)pyrrolo[2,1-f][1,2,4]triazin-7-yl)phenyl)methanesulfonamide

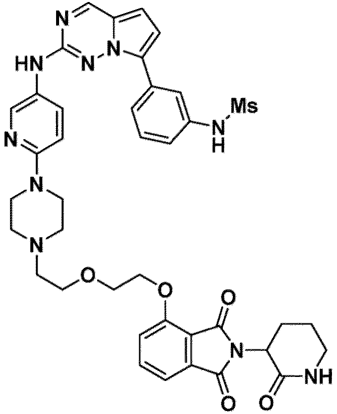

The procedure was identical to that of Example 49, except that tert-butyl-4-(5-aminopyridin-2-yl)piperazine-1-carboxylate was used instead of tert-butyl 4-(4-aminophenyl)piperazine-1-carboxylate.

### Example 81: N-(3-(2-((6-(4-(6-((1,3-dioxo-2-(2-oxopiperidin-3-yl)isoindolin-4-yl)oxy)hexyl)piperazin-1-yl)pyridin-3-yl)amino)pyrrolo[2,1-f][1,2,4]triazin-7-yl)phenyl)methanesulfonamide

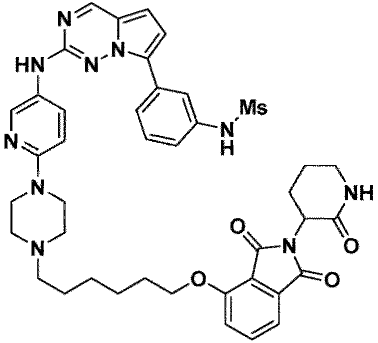

The procedure was identical to that of Example 50, except that tert-butyl-4-(5-aminopyridin-2-yl)piperazine-1-carboxylate was used instead of tert-butyl 4-(4-aminophenyl)piperazine-1-carboxylate.

### Example 82: N-(3-(2-((6-(4-(8-((1,3-dioxo-2-(2-oxopiperidin-3-yl)isoindolin-4-yl)oxy)octyl)piperazin-1-yl)pyridin-3-yl)amino)pyrrolo[2,1-f][1,2,4]triazin-7-yl)phenyl)methanesulfonamide

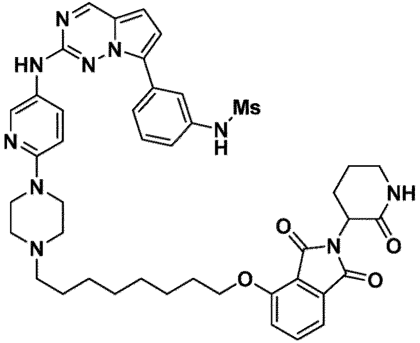

The procedure was identical to that of Example 51, except that tert-butyl-4-(5-aminopyridin-2-yl)piperazine-1-carboxylate was used instead of tert-butyl 4-(4-aminophenyl)piperazine-1-carboxylate.

### Example 83: (2S,4R)-1-((S)-3,3-dimethyl-2-(2-(2-(4-(4-((7-(3-(methanesulfonamido)phenyl)pyrrolo[2,1-f][1,2,4]triazin-2-yl)amino)phenyl) piperazin-1-yl)ethoxy)acetaamino)butanoyl)-4-hydroxy-N-(4-(4-methylthiazol-5-yl)benzyl)pyrrolidin-2-carboxamide

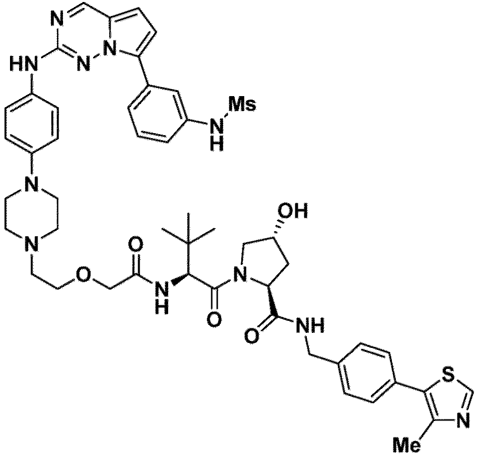

### Step 1: Preparation of Ethyl-2-(2-(4-(4-((7-(3-(methylselponamido)phenyl)pyrrolo[2,1-f][1,2,4]triazine-2-yl)amino)phenyl)piperazin-1-yl)ethoxy)acetate

To a solution of N-(3-(2-((4-(piperazin-1-yl)phenyl)amino)pyrrolo[2,1-f][1,2,4]triazin-7-yl)phenyl)methanesulfonamide (100 mg, 0.22 mmol) in dimethylformamide (2 mL) was added potassium iodide (73 mg, 0.22 mmol), diisopropylethylamine (0.12 mL, 0.66 mmol) and ethyl-2-(2-chloroethoxy)acetate (73) mg, 0.44 mmol). The reaction mixture was stirred at 60° C for 24 hours. When the reaction was complete, the reaction mixture solution was diluted with ethyl acetate and washed with saturated sodium chloride solution. The organic layer was dried over magnesium sulfate and concentrated. The residue was purified by chromatography to obtain the target compound (99 mg, 76% yield). MS m/z: 594 [M+1].

### Step 2: Preparation of 2-(2-(4-(4-((7-(3-(methylsulfonamido)phenyl)pyrrolo[2,1-f][1,2,4]triazine-2-1)amino)phenyl) piperazin-1-yl) ethoxy) acetic acid

To a solution of ethyl-2-(2-(4-(4-((7-(3-(methylsulfonamido)phenyl)pyrrolo[2,1-f][1,2,4]triazin-2-yl)amino)phenyl)piperazin-1-yl)ethoxy)acetate (99 mg, 0.17 mmol) in tetrohydrofuran (1 mL), methanol (1 mL), and water (1 mL) was added LiOH-H₂O (15 mg 0.34 mmol). The reaction mixture solution was stirred at room temperature for 12 hours. The reaction mixture was diluted with ethyl acetate, neutralized with 1 N aqueous hydrogen chloride solution to reach pH 5, and then washed with saturated sodium chloride solution. The organic layer was dried over magnesium sulfate and concentrated to obtain the target compound (92 mg, 94% yield), which was used in the next reaction without purification. MS m/z : 566 [M+1].

### Step 3: Preparation of (2S,4R)-1-((S)-3,3-dimethyl-2-(2-(2-(4-(4-((7-(3-(methanesulfonamido)phenyl)pyrrolo[2,1-f][1,2,4]triazin-2-yl)amino)phenyl) piperazin-1-yl)ethoxy)acetaamino)butanoyl)-4-hydroxy-N-(4-(4-methylthiazol-5-yl)benzyl)pyrrolidin-2-carboxamide

To a solution of 2-(2-(4-(4-((7-(3-(methylsulfonamido)phenyl)pyrrolo[2,1-f][1,2,4]triazin-2-yl)amino )phenyl)piperazin-1-yl)ethoxy)acetic acid (92 mg, 0.16 mmol),PyBOP (166 mg, 0.32 mmol), and (2S,4R)-1-((S)-2-amino- 3,3-dimethylbutanoyl)-4-hydroxy-N-(4-(4-methylthiazol-5-yl)benzyl)pyrrolidine-2-carboxamide (103 mg, 0.24 mmol) in dimethylormamide (2 mL) was added diisopropylethylamine (0.1 mL, 0.48 mmol). The reaction mixture was stirred at room temperature for 3 hours. When the reaction was complete, the reaction mixture solution was diluted with ethyl acetate and washed with saturated sodium chloride solution. The organic layer was dried over magnesium sulfate and concentrated. The resulting residue was purified by chromatography to obtain the target compound (88 mg, 56% yield). MS m/z: 978 [M+1].

### Example 84: (2S,4R)-1-((S)-3,3-dimethyl-2-(3-(2-(2-(4-(4-((7-(3-(methanesulfonamido)phenyl)pyrrolo[2,1-f][1,2,4]triazin-2-yl)amino) phenyl)piperazin-1-yl)ethoxy)ethoxy)propanamido)butanoyl)-4-hydroxy-N-(4-(4-methylthiazol-5-yl)benzyl)pyrrolidin-2-carboxamide

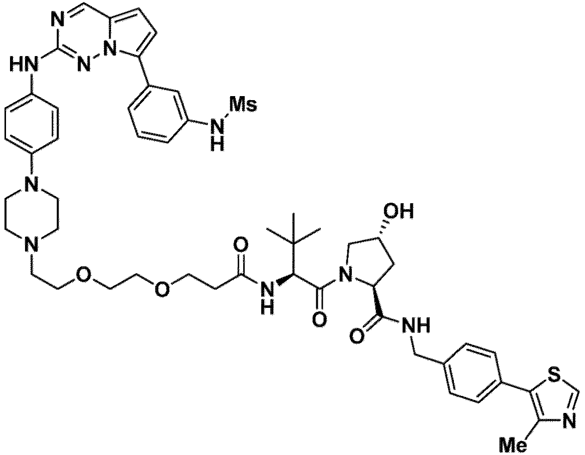

### Step 1: Preparation of tert-butyl-3-(2-(2-(4-(4-((7-(3-(methylsulfonamido)phenyl)pyrrolo[2,1-f][1,2,4]triazin-2-yl)amino)phenyl)piperazin-1-yl)ethoxy)ethoxy)propanate

The procedure was identical to that of step 1 of Example 45, except that tert-butyl-3-(2-(2-iodoethoxy)ethoxy)propanoate was used instead of ethyl-2-(2-chloroethoxy)acetate.

### Step 2: Preparation of 3-(2-(2-(4-(4-((7-(3-(methylsulfonamido)phenyl)pyrrolo[2,1-f][1,2,4]triazine-2-yl)amino)phenyl)piperazin-1-yl)ethoxy)ethoxy)propanoic acid

The procedure was identical to that of step 3 of Example 1, except that tert-butyl-3-(2-(2-(4-(4-((7-(3-(methylsulfonamido)phenyl)pyrrolo[2,1-f][1,2,4]tri azin-2-yl) amino) phenyl) piperazin-1-yl) ethoxy) ethoxy) propanate was used instead of tert-butyl4-(4-((7-(3-(methylsulfonamido)phenyl)pyrrolo[2,1-f][1,2,4]triazin- 2-yl)amino)phenyl)piperazin-1-carboxylate.

### Step 3: Preparation of (2S,4R)-1-((S)-3,3-dimethyl-2-(3-(2-(2-(4-(4-((7-(3-(methanesulfonamido)phenyl)pyrrolo[2,1-f][1,2,4]triazin-2-yl)amino) phenyl)piperazin-1-yl)ethoxy)ethoxy)propanamido)butanoyl)-4-hydroxy-N-(4-(4-methylthiazol-5-yl)benzyl)pyrrolidin-2-carboxamide

The procedure was identical to that of step 3 of Example 45, except that 3-(2-(2-(4-(4-((7-(3-(methylsulfonamido)phenyl)pyrrolo[2,1-f][1,2,4]triazine-2- 1) amino) phenyl) piperazin-1-yl) ethoxy) ethoxy) propanoic acid was used instead of 2-(2-(4-(4-((7-(3-(methylsulfonamido)phenyl)pyrrolo[2,1-f][1,2,4]triazin-2-yl)amino)phenyl)piperazin-1-yl)ethoxy)acetic acid.

### Example 85: (2S,4R)-1-((S)-3,3-dimethyl-2-(3-(2-(2-(4-(4-((7-(3-(methylsulfonamido)phenyl)pyrrolo[2,1-f][1,2,4]triazin-2-yl)amino) phenyl)piperazin-1-yl)ethoxy)ethoxy)propanamido)butanoyl)-4-hydroxy-N-((S)-1-(4-(4-methylthiazol-5-yl)phenyl)ethyl)pyrrolidin-2-carboxamide

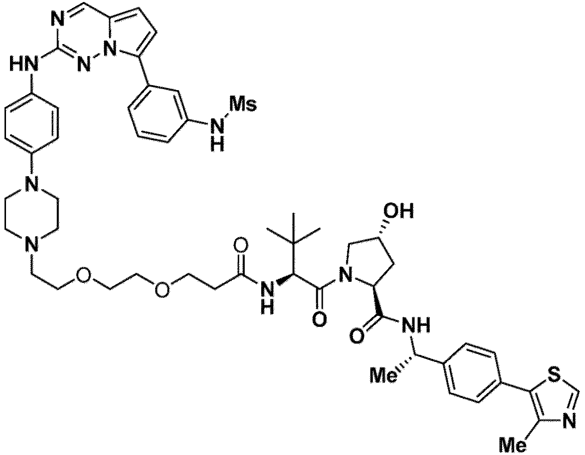

The procedure was identical to that of step 3 of Example 84, except that (2S,4R)-1-((S)-2-amino-3,3-dimethylbutanoyl)-4-hydroxy-N-((S)-1-(4-(4-methylthiazole -5-yl) phenyl) ethyl) pyrrolidine-2-carboxamide was used instead of (2S,4R)-1-((S)-2-amino-3,3-dimethylbutanoyl)-4-hydroxy-N-(4-(4-methylthiazol-5-yl)benzyl)pyrrollidine-2-carboxamide.

### Example 86: 2-(2,6-dioxopiperidin-3-yl)-4-((5-(4-(5-((7-(4-(trifluoromethoxy)phenyl)pyrrolo[2,1-f][1,2,4]triazin-2-yl)amino)pyridin-2-yl)piperazin-1-yl)pentyl)oxy)isoindolin-1,3-dione

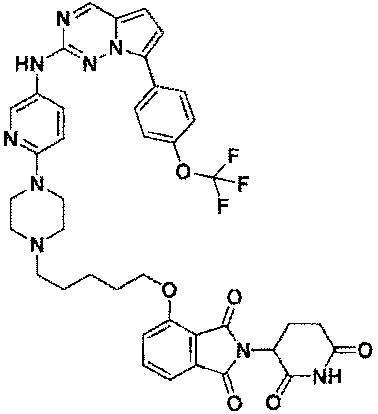

### Step 1) Preparation of 2-Bromo-7-(4-(trifluoromethoxy)pyrrolo[2,1-f][1,2,4]triazine

The procedure was identical to that of step 1 of Example 39, except that (4-(trifluoromethoxyphenyl)boronic acid was used instead of (2S,4R)-1(3-(methylsulfonamide)phenyl)boronic acid. MS m/z: 359 [M+1]

### Step 2) Preparation of 2-(2,6-dioxopiperidin-3-yl)-4-((5-(4-(5-nitropyridin-2-yl)piperazin-1-yl)pentyl)oxy)isoindolin-1,3-dione

1-(5-nitropyridin-2-yl)piperazine (1 equivalent), 4-((5-bromopentyl)oxy)-2-(2,6-dioxopiperidin-3-yl)isoindolin-1,3-dione (1.2 equivalents), potassium iodide (1 equivalent), and diisopropylethylamine (2.5 equivalents) were dissolved in dimethylformamide. The reaction mixture was stirred at 65 °C for 2 hours. When the reaction was complete, the reaction mixture was diluted with ethyl acetate and washed with saturated sodium chloride solution. The separated organic layer was dried with anhydrous sodium sulfate, filtered, and concentrated under reduced pressure. The resulting residue was purified by chromatography to obtain the title compound. MS m/z: 550 [M+1].

### Step 3) Preparation of 4-((5-(4-(4-aminophenyl)piperazin-1-yl)pentyl)oxy)-2-(2,6-dioxopiperidin-3-yl)isoindoline- 1,3-dione

2-(2,6-dioxopiperidin-3-yl)-4-((5-(4-(5-nitropyridin-2-yl)piperazin-1-yl)pentyl)oxy)isoindolin-1,3-dione (1 equivalent), ammonium chloride (10 equivalents), and iron powder (10 equivalents) were dissolved in tetrahydrofuran/methanol/water (4:2:1). The reaction mixture was stirred at 80 °C for 1 hour. When the reaction was complete, the reaction mixture was diluted with ethyl acetate and washed with sodium bicarbonate aqueous solution and saturated sodium chloride solution. The separated organic layer was dried with anhydrous sodium sulfate, filtered, and concentrated under reduced pressure. MS m/z: 520 [M+1].

### Step 4) Preparation of 2-(2,6-dioxopiperidin-3-yl)-4-((5-(4-(5-((7-(4-(trifluoromethoxy)phenyl)pyrrolo[2,1-f][1,2,4]triazin-2-yl)amino)pyridin-2-yl)piperazin-1-yl)pentyl)oxy)isoindolin-1,3-dione

To a solution of 2-chloro-7-(4-(trifluoromethoxy)phenyl)pyrrolo[2,1-f][1,2,4]triazine (1 equivalent) in sec-butanol/toluene (1:1)) was added cesium carbonate (4 equivalents) and 4-((5-(4-(4-aminophenyl)piperazin-1-yl)pentyl)oxy)-2-(2,6-dioxopiperidin-3-yl)isoindolin-1,3-dione (1 equivalent). Nitrogen was flowed into this solution for 10 minutes, and then Pd2(dba)3 (0.1 equivalent) and Xphos (0.1 equivalent) were added. The reaction mixture solution was stirred at 85 °C for 6 hours. When the reaction was complete, the mixture was filtered using Celite, the filtrate was diluted with ethyl acetate, and then washed with saturated sodium chloride solution. The organic layer was dried over magnesium sulfate and concentrated. The residue was purifed by column chromatography to obtain the title compound. MS m/z: 798 [M+1]

### Example 87: 4-((5-(4-(5-((7-(3,5-difluorophenyl)pyrrolo[2,1-f][1,2,4]triazine-2-yl)amino)pyridin-2-yl)piperazin-1-yl)pentyl)oxy)-2-(2,6-dioxopiperidin-3-yl)isoindolin-1,3-dione

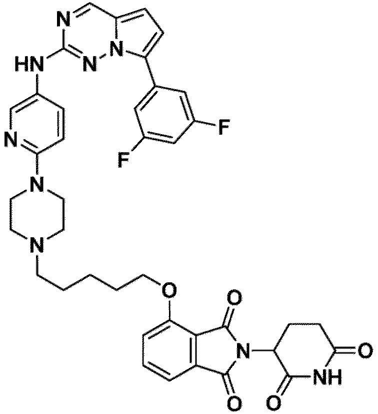

The procedure was identical to that of step 1 of Example 86, except (3,5-difluorophenyl)boronic acid was used instead of (4-(trifluoromethoxyphenyl)boronic acid. MS m/z: 750 [M+1]

### Example 88: 4-((5-(4-(5-((7-(2-aminopyridin-5-yl)pyrrolo[2,1-f][1,2,4]triazine-2-yl)amino)pyridin-2-yl)piperazin-1-al)pentyl)oxy)-2-(2,6-dioxopiperidin-3-yl)isoindolin-1,3-dione

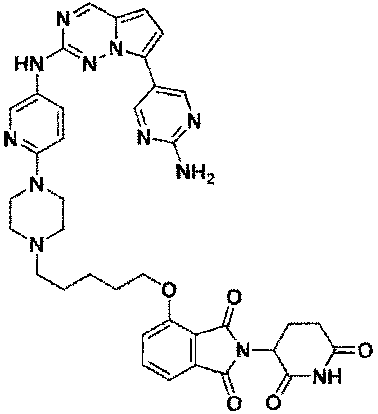

The procedure was identical to that of step 1 of Example 86, except that (2-aminopyrimidin-5-yl)boronic acid was used instead of (4-(trifluoromethoxyphenyl)boronic acid. MS m/z: 731 [M+1]

### Example 89: 2-(2,6-dioxopiperidin-3-yl)-4-((5-(4-(5-((7-(quinolin-3-yl)pyrrolo[2,1-f][1,2,4]triazin-2-yl)amino)pyridin-2-yl)piperazin-1-yl)pentyl)oxy)isoindoline-1,3-dione

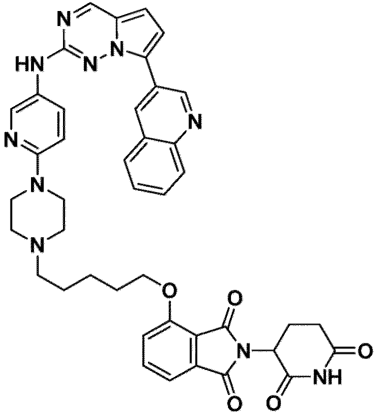

The procedure was identical to that of step 1 of Example 86, except that quinolin-3-ylboronic acid was used instead of (4-(trifluoromethoxyphenyl)boronic acid. MS m/z: 765 [M+1]

### Example 90: tert-butyl4-(3-(2-((6-(4-(5-((2-(2,6-dioxopiperidin-3-yl)-1,3-dioxoisoindolin-4-yl)oxy)pentyl)piperazin-1-yl)pyridin-3-yl)amino)pyrrolo[2,1-f][1,2,4]triazin-7-yl)benzyl)piperazin-1-carboxylate

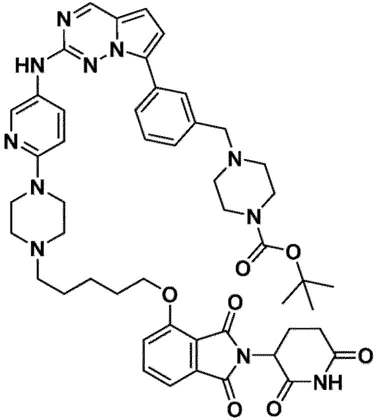

The procedure was identical to that of step 1 of Example 86, except that *tert*-butyl4-(3-(4,4,5,5-tetramethyl-1,3,2-dioxaboron-2-yl)benzyl)piperazine-1-carboxylate was used instead of (4-(trifluoromethoxyphenyl)boronic acid. MS m/z: 913 [M+1]

### Example 91: (E)-2-(2,6-dioxopiperidin-3-yl)-4-((5-(4-(5-((7-styrylpyrrolo[2,1-f][1,2,4]triazin-2-y1)amino)pyridin-2-y1)piperazin-1-yl)pentyl)oxy)isoindolin-1,3-dione

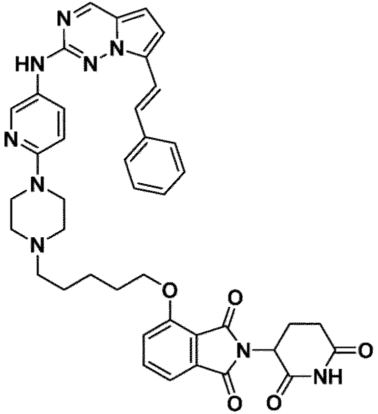

The procedure was identical to that of step 1 of Example 86, except that (E)-styrylboronic acid was used instead of (4-(trifluoromethoxyphenyl)boronic acid. MS m/z: 740 [M+1]

### Example 92: tert-butyl4-(4-(2-((6-(4-(5-((2-(2,6-dioxopiperidin-3-yl)-1,3-dioxoisoindolin-4-yl)oxy)pentyl)piperazin-1-yl)pyridin-3-yl)amino)pyrrolo[2,1-f][1,2,4]triazin-7-yl)-1H-pyrazol-1-yl)piperidin-1-carboxylate

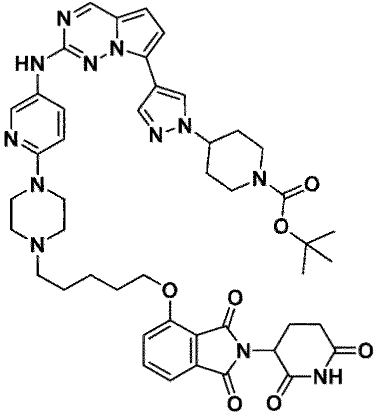

The procedure was identical to that of step 1 of Example 86, except that tert-butyl4-(4-(4,4,5,5-tetramethyl-1,3,2-dioxabororen-2-yl)-1H-pyrazol-1-yl)piperidine-1- carboxylate was used instead of (4-(trifluoromethoxyphenyl)boronic acid. MS m/z: 888 [M+1]

### Example 93: 4-((5-(4-(5-((7-(4-(dimethylamino)phenyl)pyrrolo[2,1-f][1,2,4]triazine-2-yl)amino)pyridin-2-yl)piperazin-1-yl)pentyl)oxy)-2-(2,6-dioxopiperidin-3-yl)isoindolin-1,3-dione

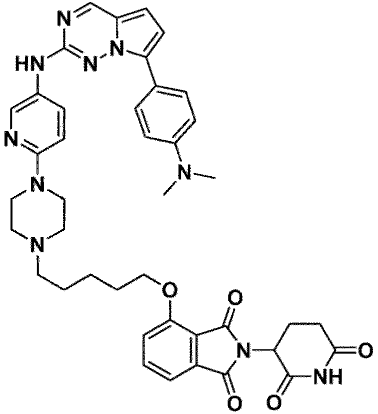

The procedure was identical to that of step 1 of Example 86, except that *N,N*-dimethyl-4-(4,4,5,5-tetramethyl-1,3,2-dioxadibororen-2-yl)aniline was used instead of (4-(trifluoromethoxyphenyl)boronic acid. MS m/z: 757 [M+1]

### Example 94: 2-(2,6-dioxopiperidin-3-yl)-4-((5-(4-(5-((7-(pyridin-4-yl)pyrrolo[2,1-f][1,2,4]triazin-2-yl)amino)pyridin-2-yl)piperazin-1-yl)pentyl)oxy)isoindolin-1,3-dione

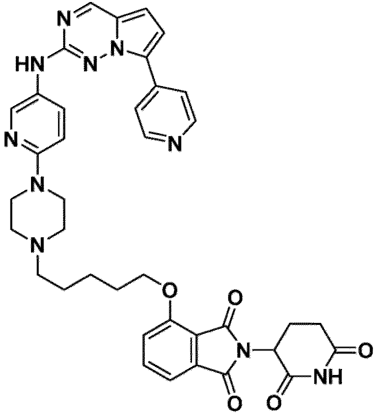

The procedure was identical to that of step 1 of Example 86, except that pyridine-4-ylboronic acid was used instead of (4-(trifluoromethoxyphenyl)boronic acid. MS m/z: 715 [M+1]

### Example 95: 2-(2,6-dioxopiperidin-3-yl)-4-((5-(4-(5-((7-(thiazol-5-yl)pyrrolo[2,1-f][1,2,4]triazin-2-yl)amino)pyridin-2-yl)piperazin-1-yl)pentyl)oxy)isoindolin-1,3-dione

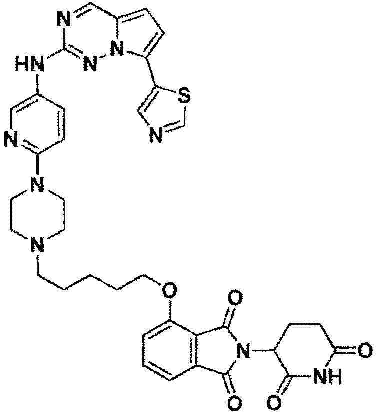

The procedure was identical to that of step 1 of Example 86, except that 5-(4,4,5,5-tetramethyl-1,3,2-dioxabororen-2-yl)thiazole was used instead of (4-(trifluoromethoxyphenyl)boronic acid. MS m/z: 721 [M+1]

### Example 96: 2-(2,6-dioxopiperidin-3-yl)-4-((5-(4-(5-((7-(1-(tetrahydro-2H-pyran-2-yl)-1H-pyrazol-5-yl)pyrrolo[2,1-f][1,2,4]triazin-2-yl)amino)pyridin-2-yl)piperazin-1-yl)pentyl)oxy)isoindolin-1,3-dione

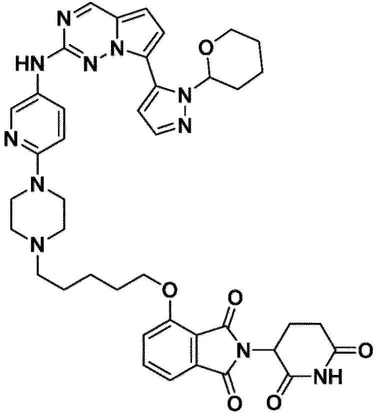

The procedure was identical to that of step 1 of Example 86, except that 1-(tetrahydro-2*H*-pyran-2-yl)-5-(4,4,5,5-tetramethyl-1,3,2-dioxabororen-2-yl)-1*H-*pyrazole was used instead of (4-(trifluoromethoxyphenyl)boronic acid. MS m/z: 788 [M+1]

### Example 97: 4-((5-(4-(5-((7-((3,5-difluorophenyl)ethynyl)pyrrolo[2,1-f][1,2,4]triazin-2-yl)amino)pyridin-2-yl)piperazin-1-yl)pentyl)oxy)-2-(2,6-dioxopiperidin-3-yl)isoindolin-1,3-dione

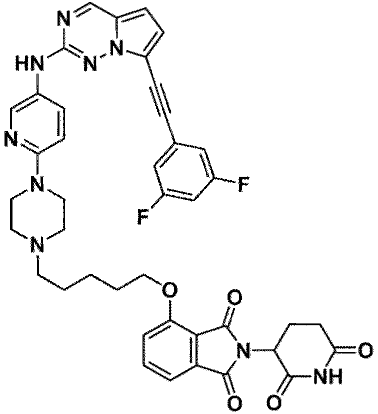

### Step 1) Preparation of 2-chloro-7-((3,5-difluorophenyl)ethynyl)pyrrolo[2,1-f][1,2,4]triazine

7-Bromo-2-chloropyrrolo[2,1-f][1,2,4]triazine (1 equivalent), 1-ethynyl-3,5-difluorobenzene (1.5 equivalent), CuI (0.1 equivalent), diisopropylamine (3 equivalent), and PdCl2(Ph3)2 (0.1 equivalent) were dissolved in sec-butanol/toluene (1:1). Nitrogen was flowed into this reaction mixture for 10 minutes, and then stirred at 85 °C for 6 hours. When the reaction was complete, the mixture was filtered using Celite, the filtrate was diluted with ethyl acetate, and then washed with saturated sodium chloride solution. The organic layer was dried over magnesium sulfate and concentrated. The title compound was obtained by purification by chromatography. MS m/z: 290 [M+1]

### Step 2) Preparation of 4-((5-(4-(5-((7-((3,5-difluorophenyl)ethynyl)pyrrolo[2,1-f][1,2,4]triazin-2-yl)amino)pyridin-2-yl)piperazin-1-yl)pentyl)oxy)-2-(2,6-dioxopiperidin-3-yl)isoindolin-1,3-yl)dione

The procedure was identical to that of step 4 of Example 86, except that 2-chloro-7-((3,5-difluorophenyl) ethynyl)pyrrolo[2,1-f][1,2,4]triazine was used instead of 2-chloro-7-(4-(trifluoromethoxy)phenyl)phyllolo[2,1-f][1,2,4]triazine. MS m/z: 774 [M+1]

### Example 98: 2-(2,6-dioxopiperidin-3-yl)-4-((5-(4-(5-((7-((3-methoxyphenyl)ethynyl)pyrrolo[2,1-f][1,2,4]triazin-2-yl)amino)aminopyridin-2-yl)piperazin-1-yl)pentyl)oxy)isoindolin-1,3-dione

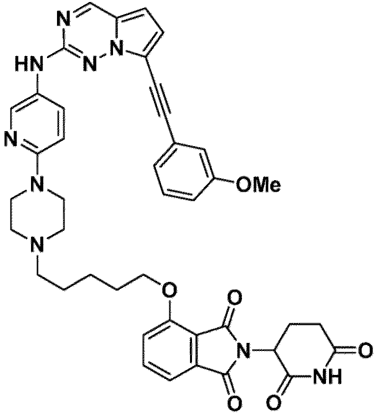

The procedure was identical to that of Example 97, except that 1-ethynyl-3-methoxybenzene was used instead of 1-ethynyl-3,5-difluorobenzene. MS m/z: 678 [M+1]

### Example 99: N-(3-(2-((4-(4-(2-(2-(3-(4-((4-(2-(2,6-dioxopiperidin-3-yl)-6-fluoro-1,3-dioxoisoindolin-5-yl)piperazin-1-yl)methyl)piperidin-1-yl)-3-oxopropoxy)ethoxy)ethyl)piperazin-1-yl)phenyl)amino)pyrrolo[2,1-f][1,2,4]triazin-7-yl)phenyl)methanesulfonamide

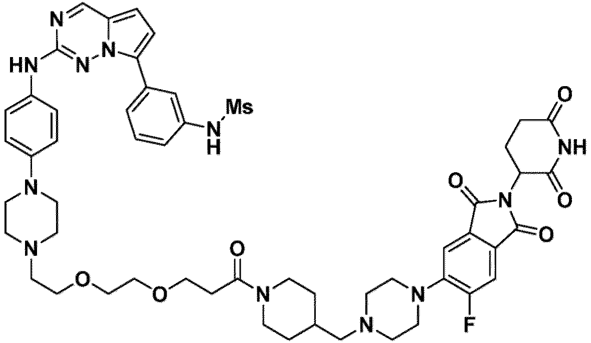

To a solution of 3-(2-(2-(4-(4-((7-(3-(methylsulfonamido)phenyl)pyrrolo[2,1-f][1,2,4]triazine-2-yl)amino)phenyl)piperazin-1-yl)ethoxy)ethoxy) propanoic acid (60 mg, 0.10 mmol), EDCI (56 mg, 0.29 mmol), HOAt (40 mg, 0.29 mmol) in dimethylforamide (2 mL) was diisopropylethylamine (0.08 mL, 0.49 mmol). After stirring the reaction mixture solution at room temperature for 30 minutes, 2-(2,6-dioxopiperidin-3-yl)-5-fluoro-6-(4-(piperidin-4-ylmethyl)piperazine-1-yl) Isoindoline-1,3-dione (50 mg, 0.11 mmol) was added and stirred at 50 °C for 6 hours. When the reaction was complete, the reaction mixture was diluted with dichloromethane and washed with saturated sodium chloride solution. The organic layer was dried over magnesium sulfate and concentrated. The title compound was obtained by purification by chromatography. MS m/z: 1064 [M+1].

### Example 100: N-(3-(4-(3-(2,4-dihydroxy-5-isopropylphenyl)-5-hydroxy-4H-1,2,4-triazol-4-yl)phenoxy)propyl)-3-(2-(2-(4-(4-((7-(3-(methylsulfonamido)phenyl)pyrrolo[2,1-f][1,2,4]triazin-2-yl)amino)phenyl)piperazin-1-yl)ethoxy)ethoxy)propanamide

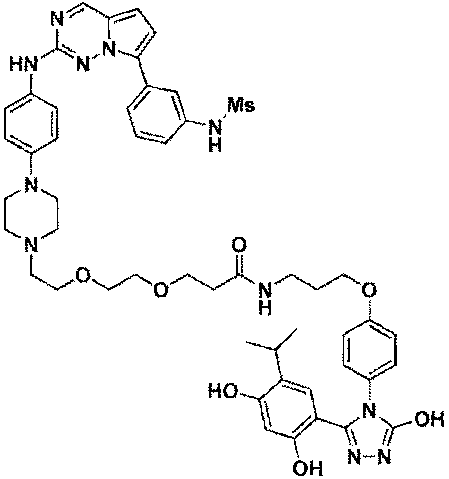

### Step 1) Preparation of tert-butyl (3-(4-nitrophenoxy)propyl)carbamate

To a solution of 4-nitrophenol (4 g, 28.75 mmol) and tert-butyl (3-bromopropyl) carbamate (8.22 g, 34.505 mmol) in 9.6 mL of acetonitrile was added potassium carbonate (7.95 g, 57.51 mmol). The reaction solution was stirred at 80 °C for 20 hours. When the reaction was complete, the mixture was filtered using Celite, the filtrate was diluted with ethyl acetate, and then washed with saturated sodium chloride solution. The organic layer was dried over sodium sulfate and concentrated. The resulting residue was used in the next reaction without purification. MS m/z: 297 [M+1].

### Step 2) Preparation of t-butyl (3-(4-aminophenoxy)propyl)carbamate

To a solution of t-butyl (3-(4-nitrophenoxy)propyl)carbamate (6 g, 22.53 mmol) in acetonitrile (45 mL) was added Pd/C (10% wt, 600 mg. The reaction mixture was stirred for 12 hours under hydrogen pressure. When the reaction was completed, the mixture was filtered through Celite and purified by chromatography to obtain the title compound (4.01 g, 67% yield). MS m/z: 267 [M+1].

### Step 3) Preparation of t-Butyl (3-(4-(2,4-dihydroxy-5-isopropylphenylcydoamido)phenoxy)propyl)carbamate

To a solution of 2,4-dihydroxy-5-isopropylbenzodithionic acid (3.4 g, 14.9 mmol) in 12 mL of N, N-dimethylformamide was added sodium bicarbonate (3.78 g, 44.97 mmol) and sodium chloroacetate (2.6 g, 22.48 mmol) at 0° C. To this mixture was added *t*-utyl (3-(4-aminophenoxy)propyl)carbamate (972 mg, 4.53 mmol) dissolved in 12 mL of N, N-dimethylformamide dropwise, and stirred at 70 ° C for 2 hours. When the reaction was complete, the reaction mixture was diluted with ethyl acetate and washed with saturated sodium chloride solution. The organic layer was dried over sodium sulfate and concentrated. The resulting residue was purified by chromatography to obtain the title compound (5.2 g, 75% yield). 1H NMR (400 MHz, DMSO-d6) δ 11.41 (s, 1H), 11.27 (s, 1H), 10.07 (s, 1H), 7.78 (s, 1H), 7.59 - 7.51 (m, 2H), 7.01 - 6.90 (m, 3H), 6.40 (s, 1H), 3.98 (t, 3.13 - 3.01 (m, 3H), 1.83 (p, 2H), 1.38 (s, 9H), 1.16 (d, 6H). MS m/z: 461 [M+1].

### Step 4) Preparation of tert-butyl (3-(4-(7-hydroxy-6-isopropyl-2-oxo-4-thioxo-2H-benzo[e][1,3]oxazin-3(4H)-1)phenoxy)propyl)Carbamate

To a solution of 2,4-dihydroxy-5-isopropylbenzodithionic acid (5.1 g, 11.01 mmol) in 22 mL of tetrahydrofuran was added carbonyldiimidazole (3.57 g, 22.02 mmol. The reaction mixture solution was stirred at room temperature for 4 hours. When the reaction was complete, the reaction solution was diluted with ethyl acetate and washed with saturated sodium chloride solution. The organic layer was dried over sodium sulfate and concentrated. The resulting residue was used in the next reaction without purification. MS m/z: 487 [M+1].

### Step 5) Preparation of t-Butyl (3-(4-(3-(2,4-dihydroxy-5-isopropylphenyl)-5-hydroxy-4H-1,2,4-triazol-4-yl)phenoxy)propyl)carbamate

To a solution of *tert*-butyl(3-(4-(7-hydroxy-6-isopropyl-2-oxo-4-thioxo-2*H*-benzo[e][1,3]oxazin-3(4*H*)-yl)phenoxy)propyl)carbamate (5.6 g, 11.50 mmol) in anhydrous ethanol (57 mL) was added hydrazine hydrate (1.15 g, 22.995 mmol). When the reaction was complete, the reaction mixture was diluted with ethyl acetate and washed with saturated sodium chloride solution. The organic layer was dried over sodium sulfate and concentrated. The resulting residue was purified by chromatography to obtain the title compound (3.86 g, 69% yield). 1H NMR (300 MHz, DMS0-d6) δ 11.85 (s, 1H), 9.56 (s, 1H), 9.38 (s, 1H), 7.14 - 7.03 (m, 2H), 6.95 - 6.85 (m, 3H), 6.80 (s, 1H), 6.25 (s, 1H), 3.94 (t, 2H), 3.12 - 2.87 (m, 3H), 1.80 (p, 2H), 1.37 (s, 9H), 0.98 (d, 6H). MS m/z: 485 [M+1].

### Step 6) Preparation of 4-(4-(4-(3-aminopropoxy)phenyl)-5-hydroxy-4H-1,2,4-triazol-3-yl)-6-isopropylbenzene-1,3-diol

To a solution of *t*-butyl (3-(4-(3-(2,4-dihydroxy-5-isopropylphenyl)-5-hydroxy-4*H*-1,2,4-triazol-4-yl)phenoxy)propyl)carbamate (2.75 g, 6.19 mmol) in dichloromethane (30 mL) was added 4M HCl 1,4-dioxane (27 mL) at 0 ° C. The reaction mixture was stirred at room temperature for 4 hours. When the reaction was complete, the reaction mixture was dried under reduced pressure. The resulting residue was used in the next reaction without purification. 1H NMR (400 MHz, DMSO-d6) δ 11.88 (s, 1H), 9.65 (s, 1H), 9.41 (s, 1H), 7.95 (s, 3H), 7.14 - 7.05 (m, 2H), 6.98 - 6.87 (m, 2H), 6.83 (s, 1H), 6.28 (s, 1H), 4.03 (t, 2H), 3.06 - 2.86 (m, 3H), 1.99 (p, 2H), 0.99 (d, 6H). MS m/z: 385 [M+1].

### Step 7) Preparation of N-(3-(4-(3-(2,4-dihydroxy-5-isopropylphenyl)-5-hydroxy-4H,2,4-triazol-4-yl)phenoxy)propyl)-3-(2-(2-(4-(4-((7-(3-(methylsulfonamido)phenyl)pyrrolo[2,1-f][1,2,4]triazin-2-yl)amino)phenyl)piperazin-1-yl)ethoxy)ethoxy)propanamide

The procedure was identical to that of Example 99, except that 2-amino-*N*-(3-(4-(3-(2,4-adihydroxy-5-isopropylphenyl)-5-hydroxy-4*H*-1,2,4-triazol-4-yl)phenoxy)propyl)acetamide was used instead of 2-(2,6-dioxopiperidin-3-yl)-5-fluoro-6-(4-(piperidin-4-ylmethyl)piperazin-1-yl)isoindoline-1,3-dione. MS m/z: 991 [M+1].

### Example 101: N-(2-((3-(4-(3-(2,4-dihydroxy-5-isopropylphenyl)-5-hydroxy-4H-1,2,4-triazol-4-yl)phenoxy)propyl)amino)-2-oxoethyl)-3-(2-(2-(4-(4-((7-(3-(methylsulfonamido)phenyl)pyrrolo[2,1-f][1,2,4]triazin-2-yl)amino)phenyl)piperazin-1-yl)ethoxy)ethoxy)propanamide

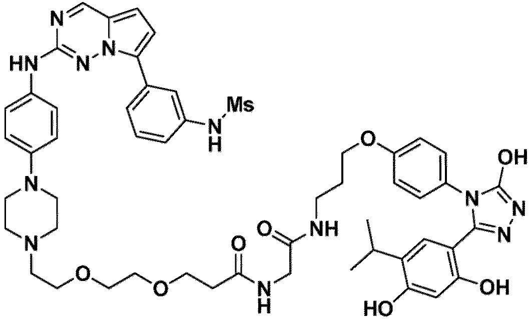

### Step 1) Preparation of tert-butyl(2-((3-(4-(3-(2,4-dihydroxy-5-isopropylphenyl)-5-hydroxy-4H-1,2,4-triazol-4-yl)phenoxy)propyl)amino)-2-oxaethyl)carbamate

To a solution of 4-(4-(4-(3-aminopropoxy)phenyl)-5-hydroxy-4*H*-1,2,4-triazol-3-yl)-6-isopropylbenzene-1,3-diol (500 mg, 1.10 mmol) and (t-butoxycarbonyl)glycine (175 mg, 1.51 mmol) in dimethylformamide (3.7 mL) was slowly added diisopropylethylamine (0.76 mL, 4.39 mmol) dropwise. The reaction mixture was stirred at room temperature for 1 hour. When the reaction was complete, the reaction mixture was diluted with dichloromethane and washed with saturated sodium chloride solution. The separated organic layer was dried with anhydrous sodium sulfate, filtered, and distilled under reduced pressure. The resulting residue was purified by chromatography to obtain 495 mg of the title compound (yield: 83%). MS m/z: 542 [M+1].

### Step 2) Preparation of 2-Amino-N-(3-(4-(3-(2,4-dihydroxy-5-isopropylphenyl)-5-hydroxy-4H-1,2,4-triazol-4-yl)phenoxy)propyl)acetamide

To a solution of 2-amino-*N*-(3-(4-(3-(2,4-dihydroxy-5-isopropylphenyl)-5-hydroxy-4*H*-1,2,4-triazol-4-yl)phenoxy)propyl)acetamide (495 mg) in 5 mL of dichloromethane was slowly added 4 M hydrochloric acid-dioxane solution (5 mL) dropwise at 0 ° C. The reaction mixture was stirred at room temperature for 1 hour. When the reaction was completed, the title compound was obtained by distillation under reduced pressure. MS m/z: 442 [M+1].

### Step 3) Preparation of N-(2-((3-(4-(3-(2,4-dihydroxy-5-isopropylphenyl)-5-hydroxy-4H-1,2,4-triazol-4-yl)phenoxy)propyl)amino)-2-oxoethyl)-3-(2-(2-(4-(4-((7-(3-(methylsulfonamido)phenyl)pyrrolo[2,1-f][1,2,4]triazin-2-yl)amino)phenyl)piperazin-1-yl)ethoxy)ethoxy)propanamide

The procedure was identical to that of Example 99, except that 2-amino-*N*-(3-(4-(3-(2,4-adihydroxy-5-isopropylphenyl)-5-hydroxy-4*H*-1,2,4-triazol-4-yl)phenoxy)propyl)acetamide was used instead of 2-(2,6-dioxopiperidin-3-yl)-5-fluoro-6-(4-(piperidin-4-ylmethyl)piperazin-1-yl)isoindolin-1,3-dione. MS m/z: 1048 [M+1].

### Example 102: N-(3-(2-((4-(4-(2-(2-(3-(4-(4-(3-(2,4-dihydroxy-5-isopropylphenyl)-5-hydroxy-4H-1,2,4-triazol-4-yl)benzyl)piperazin-1-yl)-3-oxopropoxy)ethoxy)ethyl)piperazine-1-yl)phenyl)amino)pyrrolo[2,1-f][1,2,4] triazin-7-yl)phenyl)methanesulfonamide

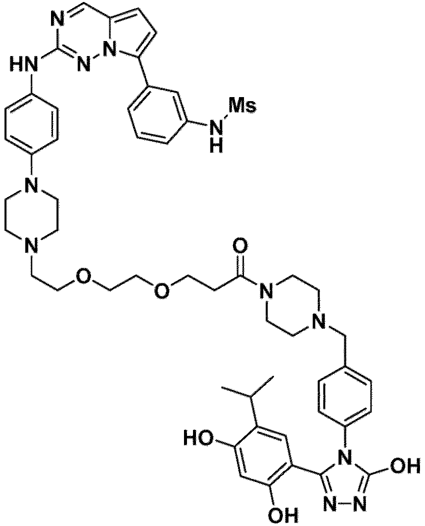

### Step 1) Preparation of 4-(5-hydroxy-4-(4-(piperazin-1-ylmethyl)phenyl)-4H-1,2,4-triazol-3-yl)-6-isopropylbenzene- 1,3-diol

The procedure was identical to that of step 3 of Example 100, except that *t*-butyl 4-(4-aminobenzyl)piperazine-1-carboxylate (972 mg, 4.53 mmol) was used instead of t-butyl (3-(4-aminophenoxy)propyl)carbamate. MS m/z: 410 [M+1].

### Step 2) Preparation of N-3-(2-((4-(4-(2-(2-(3-(4-(4-(3-(2,4-dihydroxy-5-isopropylphenyl)-5-hydroxy-4H-1,2,4-triazol-4-yl)benzyl)piperazin-1-yl)-3-oxopropoxy)ethoxy)ethyl)piperazine-1-yl)phenyl)amino)pyrrolo[2,1-f][1,2,4]triazin-7-yl)phenyl)methanesulfonamide

The procedure was identical to that of Example 99, except that 4-(5-hydroxy-4-(4-(piperazin-1-ylmethyl)phenyl)-4*H*-1,2,4-triazol-3-yl)-6-isopropylbenzene-1,3-diol was used instead of 2-(2,6-dioxopiperidin-3-yl)-5-fluoro-6-(4-(piperidin-4-ylmethyl)piperazin-1-yl)isoindolin-1,3-dione. MS m/z: 1016 [M+1].

### Example 103: N-(3-(2-((4-(4-(2-(2-(3-(4-((4-(4-(3-(2,4-dihydroxy-5-isopropylphenyl)-5-hydroxy-4H-1,2,4-triazol-4-yl)benzyl)piperazin-l-yl)methyl)piperidin-1-yl)-3-oxopropoxy)ethoxy)ethyl)piperazin-1-yl)phenyl)amino)pyrrolo[2,1-f][1,2,4]triazin-7-yl)phenyl) methanesulfonamide

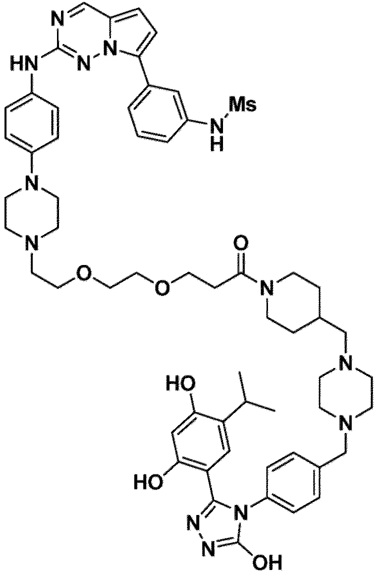

### Step 1) Preparation of 4-(5-hydroxy-4-(4-((4-(piperidin-4-ylmethyl)piperazin-1-yl)methyl)phenyl)-4H-1,2,4- triazol-3-yl)-6-isopropylbenzine-1,3-diol

To a solution of 4-(5-hydroxy-4-(4-(piperazin-1-ylmethyl)phenyl)-4*H-*1,2,4-triazol-3-yl)-6-isopropylbenzene-1,3-diol (300 mg, 0.73 mmol), tert-butyl4-formylpiperidine-1-carboxylate (312 mg, 1.47 mmol), and acetic acid (4 mg, 0.07 mmol) in dichloroethene (5 mL) was slowly added sodium triacetoxyborohydride (309 mg, 1.47 mmol) was portionwise at 0 ° C. The reaction mixture was stirred at room temperature for 6 hours. When the reaction was complete, the mixture was diluted with dichloromethane and neutralized with sodium bicarbonate. The resulting separated organic layer was dried over anhydrous sodium sulfate, filtered, and distilled under reduced pressure. The resulting residue purified by chromatography to obtain the title compound (360 mg, 67% yield). MS m/z: 609 [M+1].

### Step 2) To a solution of 4-(5-hydroxy-4-(4-((4-(piperidin-4-ylmethyl)piperazin-1-yl)methyl)phenyl)-4H-1,2,4-triazole-3-yl)-6-isopropylbenzene-1,3-diol (360 mg) in 4 mL of dichloromethane was added 4 M hydrochloric acid-dioxane solution (4 mL) dropwise at 0 °C. The reaction mixture was stirred at room temperature for 1 hour. When the reaction was completed, the title compound was obtained by distillation under reduced pressure. MS m/z: 509 [M+1].

### Step 3) Preparation of N-(3-(2-((4-(4-(2-(2-(3-(4-((4-(4-(3-(2,4-dihydroxy-5-isopropylphenyl)-5-hydroxy-4H-1,2,4-triazol-4-yl)benzyl)piperazin-1-yl)methyl)piperidin-1-yl)-3-oxopropoxy)ethoxy)ethyl) piperazin-1-yl)phenyl)amino)pyrrolo[2,1-f][1,2,4]triazin-7-yl)phenyl) methanesulfonamide

The procedure was identical to that of Example 99, except that 4-(5-hydroxy-4-(4-((4-(piperidin-4-ylmethyl)piperazin-1-yl)methyl)phenyl)-4H-1,2,4-triazole- 3-yl)-6-isopropylbenzene-1,3-diol was used instead of 2-(2,6-dioxopiperidin-3-yl)-5-fluoro-6-(4-(piperidin-4-ylmethyl)piperazin-1-yl)isoindolin-1,3-dione. MS m/z: 1113 [M+1].

### Example 104: N-(3-(2-((4-(4-(2-(2-(3-(4-((2-(2,4-dihydroxy-5-isopropylbenzoyl)isoindolin-5-yl)methyl)piperazin-1-yl)-3-oxopropoxy) ethoxy)ethyl)piperazin-1-yl)phenyl)amino)pyrrolo[2,1-f][1,2,4]triazin-7-yl)phenyl)methanesulfonamide

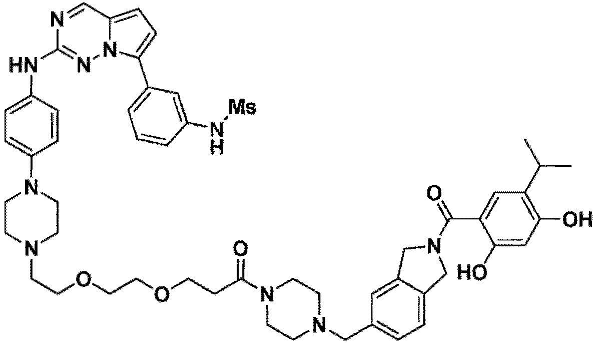

### Step 1) Preparation of 1-(2,4-dihydroxy-5-isopropylphenyl)ithen-1-one

To a solution of 4-isopropylbenzene-1,3-diol (121 mg, 0.80 mmol) was added boron trifluoride diethyl etherate (0.6 mL) and acetic acid (48 mg, 0.80 mol) at room temperature. The reaction mixture was stirred at 90 °C for 4 hours. When the reaction was complete, an aqueous solution of sodium acetate was added, stirred vigorously for 2.5 hours, filtered, and the obtained solid was dried to obtain the title compound. MS m/z: 195 [M+1].

### Step 2) Preparation of 1-(2,4-bis(benzyloxyl)-5-isopropylphenyl)ethen-1-one

To a solution of 1-(2,4-dihydroxy-5-isopropylphenyl)ethen-1-one (115 mg, 0.59 mmol) in acetonitrile (2 mL) was added benzyl bromide (236 mg, 1.38 mmol) and potassium carbonate (204 mg, 1.48 mmol) The reaction mixture was stirred at 80 °C for 12 hours. When the reaction was completed, acetonitrile was distilled under reduced pressure, water was added, and the resulting solid was filtered and dried. The resulting solid was was washed with hexane to obtain the title compound. MS m/z: 375 [M+1].

### Step 3) Preparation of 2,4-bis(benzyloxy)-5-isopropylbenzoic acid

To a solution of 1-(2,4-bis(benzyloxyl)-5-isopropylphenyl)ethen-1-one (1.5 g, 4.01 mmol) in to 1,4-dioxane (15 mL)/water (15 mL) was added sodium hydroxide aqueous solution (1.60 g, 40.10 mmol) and bromine (0.04 ml, 0.80 mmol) dropwise. The reaction mixture solution was stirred at room temperature for 12 hours. When the reaction was completed, the pH was adjusted to 2 using aqueous hydrogen chloride solution, and the resulting solid was filtered, washed with water, and dried to obtain the title compound. 1H NMR (300 MHz, DMS0-d6) δ 7.57 (s, 1H), 7.55-7.28 (m, 10H), 6.89 (s, 1H), 5.20 (s, 4H), 3.24-3.13 (m, 1H), 1.18-1.13 (m, 6H). MS m/z: 377 [M+1].

### Step 4) Preparation of (2,4-bis(benzyloxy)-5-isopropylphenyl)(5-bromoisoindolin-2-yl)methanone

To a solution of 2,4-bis(benzyloxy)-5-isopropylbenzoic acid (138 mg, 0.37 mmol) in in dimethylformamide (1.5 mL) was added 1-ethyl-3-(3-dimethylaminopropyl)carbodiimide (140 mg, 0.73 mmol), Hydroxybenzotriazole (100 mg, 0.74 mmol). After stirring the reaction mixture solution at room temperature for 10 minutes, diisopropylethylamine (0.260 mL, 1.47 mmol) was added to the reaction mixture solution at 0 °C, and 5-bromoisoindoline (95 mg, 0.4 mmol) was slowly added. The reaction mixture was stirred at room temperature for 8 hours. When the reaction was complete, the reaction mixture solution was diluted with ethyl acetate and washed with saturated sodium chloride solution. The organic layer was dried over magnesium sulfate and concentrated. The resulting residue was purified by chromatography to obtain the title compound (205 mg, 64% yield). 1H NMR (300 MHz, DMS0-d6) δ 7.64-7.18 (m, 13H), 7.10 (s, 1H), 6.96 (s, 1H), 5.19 (s, 4H), 4.76 (d, J=13.9, 2H), 4.51 (d, J=16.3, 2H), 3.27-3.16 (m, 1H), 1.16 (s, 3H), 1.14 (s, 3H). MS m/z: 556 [M+H].

### Step 5) Preparation of Benzyl 4-(trifluoromethylborato)piperazine-1-carboxylate potassium salt

Benzyl piperazine-1-carboxylate (100 mg, 0.45 mmol) and potassium (bromomethyl)trifluoroborate (90 mg, 0.45 mmol) were dissolved in tetrahydrofuran (1.5 mL) and the mixed solution was istirred at 80 °C and stirred for 12 hours. After the reaction was completed, the reaction mixture was cooled and concentrated. Acetone and potassium carbonate were added to the concentrated reaction mixture, stirred for 30 minutes, filtered through Celite, and concentrated to obtain the title compound (116 mg, 76% yield). MS m/z: 301 [M-K].

### Step 6) Preparation of Benzyl 4-((2-(2,4-bis(benzyloxy)-5-isopropylbenzoyl)isoindolin-5-yl)methyl)piperazine-1-carboxylate

To a solution of (2,4-bis(benzyloxy)-5-isopropylphenyl)(5-bromoisoindolin-2-yl)methanone (100 mg, 0.18 mmol) and benzyl 4-(trifluoromethylborato)piperazin-1-carboxylate potassium salt (61 mg, 0.18 mmol), XPhos (5 mg, 0.011 mmol), and cesium carbonate (176 mg, 0.54 mmol) in tetrahydrofuran (0.9 mL)/water (0.1 mL) solution was added palladium acetate (1.2 mg, 0.0054 mmol) under nitrogen environment. The reaction mixture was stirred at 80 °C for 12 hours. When the reaction was complete, the mixture was filtered using Celite, the filtrate was diluted with ethyl acetate, and then washed with saturated sodium chloride solution. The organic layer was dried over magnesium sulfate and concentrated. The resulting residue was purified by chromatography to obtain the title compound (107 mg, 84% yield).MS m/z: 710 [M+H].

### Step 7) Preparation of (2,4-dihydroxy-5-isopropylphenyl)(5-(piperazin-1-ylmethyl)isoindolin-2-yl)methanone hydrochloride

To a solution of benzyl 4-((2-(2,4-bis(benzyloxy)-5-isopropylbenzoyl)isoindolin-5-yl)methyl)piperazine-1-carboxylate (100 mg, 0.25 mmol) in a tetrahydrofuran (1 mL)/methanol (1 mL) was added Palladium hydroxide (20 mg) and stirred for 12 hours under hydrogen pressure. When the reaction was completed, the mixture was filtered using Celite, and the filtrate was concentrated. The resulting residue was diluted with ethyl acetate, 1N hydrochloric acid solution was added. The solid was filtered and washed twice with ethyl acetate to obtain the title compound (80 mg, 74% yield).1H NMR (300 MHz, DMS0-d6) δ 10.08 (s, 1H), 7.35-7.14 (m, 3H), 7.04 (s, 1H), 6.39 (s, 1H), 4.76 (s, 4H), 3.40 (s, 2H), 3.15-3.03 (m, 1H), 2.71-2.62 (m, 4H), 2.26 (s, 4H), 1.16 (s, 3H), 1.14 (s, 3H). MS m/z: 396 [M+H] .

### Step 8) Preparation of N-(3-(2-((4-(4-(2-(2-(3-(4-((2-(2,4-dihydroxy-5-isopropylbenzoyl)isoindolin-5-yl)methyl)piperazin-1-yl)-3-oxopropoxy) ethoxy)ethyl)piperazin-1-yl)phenyl)amino)pyrrolo[2,1-f][1,2,4]triazin-7-yl)phenyl)methanesulfonamide

The procedure was identical to that of Example 99, except that 4(2,4-dihydroxy-5-isopropylphenyl)(5-(piperazin-1-ylmethyl)isoindolin-2-yl)methanone was used instead of 2-(2,6-dioxopiperidin-3-yl)-5-fluoro-6-(4-(piperidin-4-ylmethyl)piperazin-1-yl)isoindolin-1,3-dione. MS m/z: 1002 [M+1].

### Exmaple 105: N-(3-(2-((4-(4-(2-(2-(3-(4-((4-((2-(2,4-dihydroxy-5 - isopropylbenzoyl)isoindolin-5-yl)methyl)piperazin-1-yl)methyl)piperidin-1-yl)-3-oxopropoxy)ethoxy)ethyl)piperazin-1-yl)phenyl)amino)pyrrolo[2,1-f][1,2,4]triazin-7-yl)phenyl)methanesulfonamide

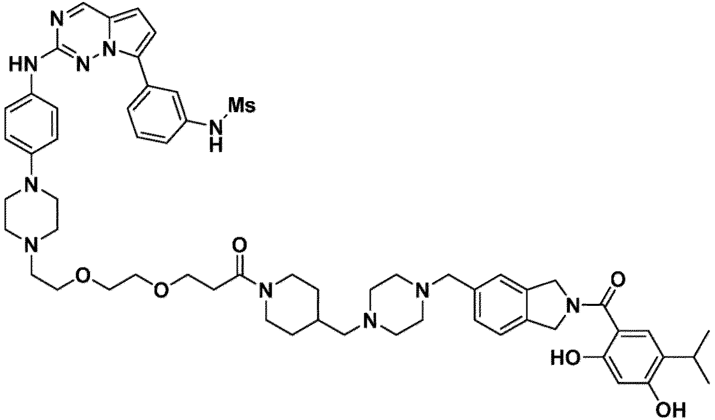

The procedure was identical to that of Example 99, except that (2,4-dihydroxy-5-isopropylphenyl)(5-((4-(piperidin-4-ylmethyl)piperazin-1-yl)methyl)isoindolin-2-yl)methanone was used instead of 2-(2,6-dioxopiperidin-3-yl)-5-fluoro-6-(4-(piperidin-4-ylmethyl)piperazin-1-yl)isoindolin-1,3-dione. MS m/z: 1099 [M+1].

### Example 106: 2-amino-4-(2,4-dichloro-5-(2-(4-(3-(2-(2-(4-(4-((7-(3-(methylsulfonamido)phenyl)pyrrolo[2,1-f][1,2,4]triazin-2-yl)amino) phenyl)piperazin-1-yl)ethoxy)ethoxy)propanoyl)piperazin-1-yl)ethoxy)phenyl)-N-ethylthieno[2,3-d]pyrimidin-6-carboxamide

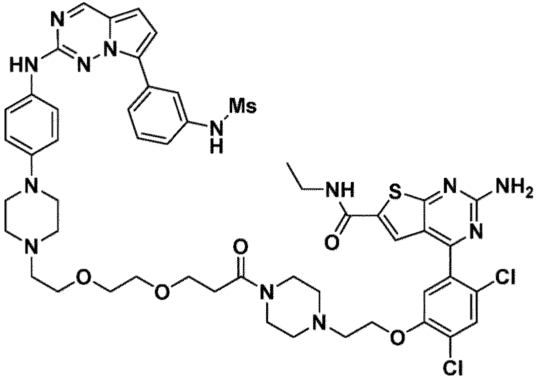

### Step 1) Preparation of N-(2,4-dichloro-5-hydroxyphenyl)acetamide

To a solutio nof *N*-(3-hydroxyphenyl)acetamide (8 g, 52.9 mmol) in acetic acid (100 mL) was slowly thionyl chloride (1.0 M in MC, 111 mL. 111 mmol) over 1 hour dropwise at room temperature. The reaction mixture was stirred at room temperature for 8 hours. When the reaction was complete, ice water was added, and the resulting solid was filtered and washed with water. The solid was dried at 50 °C to obtain the title compound as a yellow solid (yield 92%).

### Step 2) Preparation of N-(5-(benzyloxy)-2,4-dichlorophenyl)acetamide

To a solution of *N*-(2,4-dichloro-5-hydroxyphenyl)acetamide (10g, 45.4 mmol) and potassium carbonate (7.27g, 52.7 mmol) in acetonitrile (100 mL) was slowly added benzyl bromide (7.765 g, 45.4 mmol) dropwise. The reaction mixture was stirred at 80 °C for 3 hours. When the reaction was complete, the reaction mixture was diluted with dichloromethane and washed with saturated sodium chloride solution, sodium hydroxide aqueous solution, and ammonium aqueous solution. The separated organic layer was dried over anhydrous sodium sulfate, filtered, and distilled under reduced pressure to obtain the title compound (yield: 95%).

### Step 3) Preparation of 5-(benzyloxy)-2,4-dichloroaniline

To a solution of *N*-(5-(benzyloxy)-2,4-dichlorophenyl)acetamide in methanol (100 mL)/water(100 mL) was added saturated hydrochloric acid (15 g, 0.15 mol). The reaction mixture solution was stirred at 60 °C for 12 hours. When the reaction was completed, the solid produced by distillation under reduced pressure was filtered, washed with water, dried at 50 °C to obtain the title compound (yield: 85%).

### Step 4) Preparation of 1-(benzyloxyl)-2,4-dichloro-5-iodobenzene

To a solution of 5-(benzyloxy)-2,4-dichloroaniline (16.2 g, 60 mmol) in acetic acid (240 mL) was slowly added hydrogen chloride (60 mL, 6M) dropwise. To this soluiton was added aqueous solution of sodium nitrite (4.8 g, 69 mmol) (40 mL) dropwise at 5 °C. After stirring the reaction mixture at the same temperature for 30 minutes, potassium iodide (20 g, 120 mmol), iodine (4 g, 16 mmol), and water (200 mL) were added slowly. The reaction mixture was stirred at the same temperature for 90 minutes. When the reaction was complete, the reaction mixture was diluted with dichloromethane and washed water, sodium thiosulfate aqueous solution, sodium hydroxide aqueous solution, and saturated sodium chloride solution. The separated organic layer was dried over anhydrous sodium sulfate, filtered, and distilled under reduced pressure to obtain the title compound (yield: 90%).

### Step 5) Preparation of Ethyl2-amino-4-(5-(bezyloxyl)-2,4-dichlorophenyl)thieno[2,3-d]pyrimidine-6-carboxylate

1-(benzyloxyl)-2,4-dichloro-5-iodobenzene (20.6 g, 54 mmol), bis(pinacholate)biboron (14.5 g, 57 mmol), potassium acetoxide (16 g, 163 mmol) was diluted with 50 mL of dimethylformamide, and palladium(II) acetate (450 mg, cat.) was added. The reaction mixture was stirred at 90 °C for 18 hours. When the reaction was complete, the reaction mixture was diluted with ethyl acetate and washed with saturated sodium chloride solution. As a result, the separated organic layer was dried over anhydrous sodium sulfate, filtered, and distilled under reduced pressure to obtain the title compound. To a solution of 2-amino-4-chloro-thieno[2,3-d]pyrimidine-6-carboxylic acid ethyl ester (12.85 g, 50 mmol) in 4-dioxane (160 mL) was added aqueous solution of potassium phosphate (40 mL, 2M) and palladium(II) acetate (450 mg, cat.). The reaction mixture was stirred at 100 °C for 3 hours. When the reaction was completed, the reaction mixture was diluted with ethyl acetate and washed with saturated sodium chloride solution. The separated organic layer was dried with anhydrous sodium sulfate, filtered, and distilled under reduced pressure. The resulting residue was purified by chromatography to obtain the title compound (yield: 45%).

### Step 6) Preparation of 2-Amino-4-(5-(bezyloxyl)-2,4-dichlorophenyl)-N-ethylthieno[2,3-d]pyrimidine-6-carboxamide

To a solution of ethyl2-amino-4-(5-(bezyloxyl)-2,4-dichlorophenyl)thieno[2,3-d]pyrimidine-6-carboxylate (500 mg, 1.05 mmol) in tetrahydrofuran (4 mL)/methanol (4 mL)/water (4 mL) was added lithium hydroxide (88 mg, 2.00 mmol). When the reaction was completed, it was diluted with ethyl acetate and washed with 1N hydrogen chloride solution. The separated organic layer was dried over anhydrous sodium sulfate, filtered, and distilled under reduced pressure to obtain the title compound. To this compound in dimethylformamide was added HATU (798 mg, 2.10 mmol) and diisopropylethylamine (0.55 mL, 3.15 mmol). After stirring at room temperature for 30 minutes, ethylamine (145 mg, 3.15 mmol) was slowly added dropwise. The reaction mixture solution was stirred at room temperature for 1 hour. When the reaction was completed, the reaction mixture was diluted with ethyl acetate and washed with salt water. The separated organic layer was dried with anhydrous sodium sulfate, filtered, and distilled under reduced pressure. The resulting residue was purified by chromatography to obtain the title compound (50%).

### Step 7) Preparation of 2-Amino-4-(2,4-dichloro-5-hydroxyphenyl)-N-ethylthieno[2,3-d]pyrimidine-6-carboxamide

To a solution of 2-amino-4-(5-(bezyloxyl)-2,4-dichlorophenyl)-N-ethylthieno[2,3-d]pyrimidine-6-carboxamide (280 mg, 0.59 mmol in 3 mL of dichloromethane was slowly added 3 mL of boron trichloride solution (1M dichloromethane) at -78 °C. The reaction mixture solution was stirred at room temperature for 3 hours. When the reaction was completed, the reaction mixture was distilled under reduced pressure, diluted with ethyl acetate, and washed with aqueous sodium acetate solution and saturated sodium chloride solution. As a result, the separated organic layer was dried with anhydrous sodium sulfate, filtered, and distilled under reduced pressure. The resulting residue was solidified with using hexane and filtered to obtain the title compound.

### Step 8) Preparation of tert-butyl4-(2-(5-(2-amino-6-(ethylcarbamoyl)thieno[2,3-d]pyrimidin-4-yl)-2,4-dichloro phenoxy)ethyl)pyrerazine-1-carboxylate

To a solution of 2-amino-4-(2,4-dichloro-5-hydroxyphenyl)-N-ethylcyeno[2,3-d]pyrimidine-6-carboxamide (500 mg, 1.05 mmol (88 mg, 0.23 mmol), triphenylphosphine (91 mg, 0.35 mmol), and tert-butyl-4-(2-hydroxyethyl)piperazine-lcarboxylate (65 mg, 0.28 mmol) in 10 mL of was slowly added DEAD dropwise at room temperature. When the reaction was completed, the reaction mixture was diluted with ethyl acetate and washed with aqueous ammonium chloride solution and saturated sodium chloride solution. The organic layer was dried over anhydrous sodium sulfate, filtered, and the resulting residue was purified by chromatography to obtain the title compound (55%).

### Step 9) Preparation of 2-amino-4-(2,4-dichloro-5-(2-(piperazin-1-yl)ethoxy)phenyl)-N-ethylthieno[2,3-d]pyrimydin-6-carboxamide

To a solution of *tert*-butyl4-(2-(5-(2-amino-6-(ethylcarbamoyl)thieno[2,3-d]pyrimidin-4-yl)-2,4-dichlorophenoxy) ethyl)pyrerazine-1-carboxylate (300 mg) in 4 mL of ethyl acetate was added 4 mL of 4N hydrogen chloride 1,4-dioxane solution dropwise. The reaction mixture solution was stirred at room temperature for 4 hours. When the reaction was completed, the title compound (95%) was obtained by distillation under reduced pressure.

### Step 10) Preparation of 2-amino-4-(2,4-dichloro-5-(2-(4-(3-(2-(2-(4-(4-((7-(3-(methylsulfonamido)phenyl)pyrrolo[2,1-f][1,2,4]triazin-2-yl)amino) phenyl)piperazin-1-yl)ethoxy)ethoxy)propanoyl)piperazin-1-yl)ethoxy)phenyl)-N-ethylthieno[2,3-d]pyrimidin-6-carboxamide

The procedure was identical to that of Example 99, except that 2-amino-4-(2,4-dichloro-5-(2-(piperazin-1-yl)ethoxy)phenyl)-N-ethylthieno[2,3-d]pyrimidin-6-carboxamide was used instead of 2-(2,6-dioxopiperidin-3-yl)-5-fluoro-6-(4-(piperidin-4-ylmethyl)piperazin-1-yl)isoindolin-1,3-dione. MS m/z: 1048 [M+1].

### Example 107: N-(3-(2-((4-(4-(4-((4-((2-(2,4-dihydroxy-5-isopropylbenzoyl)isoindolin-5-yl)methyl)piperazin-1-yl)methyl)benzoic)piperazin-1-yl)phenyl)amino)pyrrolo[2,1-f][1,2,4]triazine-7-1)phenyl)methanesulfonamide

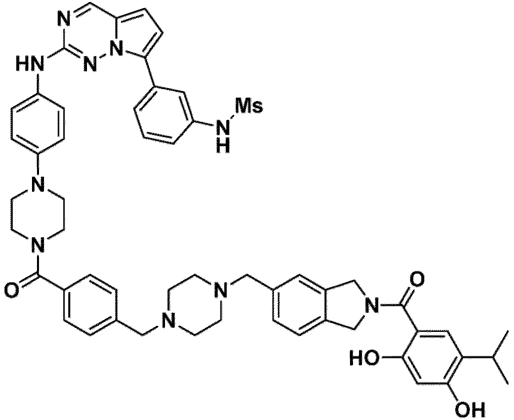

### Step 1) Preparation of Methyl-4-((4-((2-(2,4-bis(benzyloxy)-5-isopropylbenzoyl)isoindolin-5-yl)methyl)piperazin-1-yl)methyl) benzoate

The procedure was identical to that of step 5-6 of Example 104, except that methyl 4-(piperazin-1-ylmethyl)benzoate was used benzyl piperazine-1-carboxylate. MS m/z: 724 [M+1].

### Step 2) Preparation of 4-((4-((2-(2,4-bis(benzyloxy)-5-isopropylbenzoyl)isoindolin-5-yl)methyl)piperazin-1-yl)methyl)benzoic acid

The procedure was identical to that of step 3 of Example 108. MS m/z: 709 [M+1].

### Step 3) Preparation of 4-((4-((2-(2,4-dihydroxy-5-isopropylbenzoic)isoindolin-5-yl)methyl)piperazin-1-yl)methyl)benzoic acid

The procedure was identical to that of step 4 of Example 108. MS m/z: 530 [M+1].

### Step 4) Preparation of N-(3-(2-((4-(4-(4-((4-((2-(2,4-dihydroxy-5-isopropylbenzoyl)isoindolin-5-yl)methyl)piperazin-1-yl)methyl)benzoic)piperazin-1-yl)phenyl)amino)pyrrolo[2,1-f][1,2,4]triazine-7-1)phenyl)methanesulfonamide

To a solution of 4-((4-((2-(2,4-dihydroxy-5-isopropylbenzoic)isoindolin-5-yl)methyl)piperazin-1-yl)methyl)benzoic acid (50 mg, 0.09 mmol), EDCI (54 mg, 0.28 mmol), and HOAt (37 mg, 0.29 mmol) in dimethylormamide (2 mL) was added then diisopropylethylamine (0.08 mL, 0.45 mmol) After stirring the reaction mixture solution at room temperature for 30 minutes, *N*-(3-(2-((4-(piperazin-1-yl)phenyl)amino)pyrrolo[2,1-f][1,2,4] triazin-7-yl)phenyl)methanesulfonamide (46 mg, 0.10 mmol) was added, and stirred at 50 °C for 6 hours. When the reaction was complete, the reaction mixture s was diluted with dichloromethane and washed with saturated sodium chloride solution. The organic layer was dried over magnesium sulfate and concentrated. The title compound was obtained by purification by column chromatography. MS m/z: 976 [M+1].

### Example 108: N-(3-(2-((4-(4-(l-((2-(2,4-dihydroxy-5-isopropylbenzoic)isoindolin-5-yl)methyl)piperidin-4-carbonyl)piperazin-1-yl)phenyl)amino)pyrrolo[2,1-f][1,2,4]triazin-7-yl)phenyl)methanesulfonamide

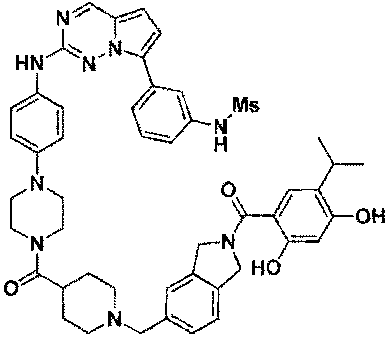

### Step 1) Preparation of Methyl 1-(trifluoromethylborato)piperidine-4-carboxylate potassium salt

Methyl piperidine-4-carboxylate (100 mg, 0.70 mmol) and potassium (bromomethyl)trifluoroborate (141 mg, 0.70 mmol) were dissolved in tetrahydrofuran (2 mL) and the mixture was stirred at 80 °C for 12 hours. After the reaction was completed, the reaction mixture was cooled and concentrated. Acetone and potassium carbonate were added to the concentrated reaction mixture, stirred for 30 minutes, filtered through Celite, and concentrated to obtain the target compound (109 mg, 59% yield). MS m/z: 224 [M-K] .

### Step 2) Preparation of Methyl 1-((2-(2,4-bis(benzyloxy)-5-isopropylbenzoyl)isoindolin-5-yl)methyl)piperidine-4-carboxylate

To a solution of (2,4-bis(benzyloxy)-5-isopropylphenyl)(5-bromoisoindolin-2-yl)methanone (100 mg, 0.18 mmol) and methyl 1-(trifluoromethylborato) Piperidine-4-carboxylate potassium salt (47 mg, 0.18 mmol), XPhos (5 mg, 0.011 mmol), and cesium carbonate (176 mg, 0.54 mmol) in tetrahydrofuran (0.9 mL)/water (0.1 mL) was added palladium acetate (1.2 mg, 0.0054 mmol). The reaction mixture solution was stirred at 80 °C for 12 hours. When the reaction was complete, the reaction mixture was filtered using Celite, the filtrate was diluted with ethyl acetate, and then washed with saturated sodium chloride solution. The organic layer was dried over magnesium sulfate and concentrated. The resulting residue was purified by chromatography to obtain the target compound (49 mg, 43% yield). MS m/z: 633 [M+H].

### Step 3) Preparation of 1-((2-(2,4-bis(benzyloxy)-5-isopropylbenzoyl)isoindolin-5-yl)methyl)piperidine-4-carboxylic acid

To a solution of Methyl 1-((2-(2,4-bis(benzyloxy)-5-isopropylbenzoyl)isoindolin-5-yl)methyl)piperidine-4-carboxylate (660 mg, 1.0 mmol) in a solution of water (1 mL)/tetrahydrofuran (4 mL)/methanol (2 mL) was added lithium hydroxide hydrate (130 mg, 3.1 mmol) was added at 0 °C. The reaction mixture was stirred at room temperature for 2 hours and concentrated upon completion of the reaction. The concentrated reaction mixture was diluted with water and dichloromethane, and then 1 N hydrochloric acid solution was slowly added to adjust the pH of the reaction mixture to 4, which was then extracted three times with dichloromethane. The organic layer was collected, dried over sodium sulfate, and concentrated to obtain the target compound (590 mg, 92% yield). MS m/z 619 [M+H].

### Step 4) Preparation of 1-((2-(2,4-dihydroxy-5-isopropylbenzoic)isoindolin-5-yl)methyl)piperidine-4-carboxylic acid

To a solution of benzyl 1-((2-(2,4-bis(benzyloxy)-5-isopropylbenzoyl)isoindolin-5-yl)methyl)piperidine-4-carboxylic acid (155 mg, 0.25 mmol) in a solution of tetrahydrofuran (1 mL)/methanol (1 mL) was added palladium hydroxide (20 mg) and stirred for 12 hours under with hydrogen atmosphere. When the reaction was completed, the reaction mixture was filtered using Celite, and the filtrate was concentrated to obtain the target compound (81 mg, 74% yield). MS m/z 439 [M+H].

### Step 5) Preparation of N-(3-(2-((4-(4-(1-((2-(2,4-dihydroxy-5-isopropylbenzoic)isoindolin-5-yl)methyl)piperidin-4-carbonyl)piperazin-1-yl) phenyl)amino)pyrrolo[2,1-f][1,2,4]triazin-7-yl)phenyl)methanesulfonamide

The procedure was identical to that of Example 108, except that 1-((2-(2,4-dihydroxy-5-isopropylbenzoic)isoindolin-5-yl)methyl)piperidin-4-carboxylic acid was used instead 4-((4-((2-(2,4-dihydroxy-5-isopropylbenzoic)isoindolin-5-yl)methyl)piperazin-1-yl)methyl)benzoic acid. MS m/z 885 [M+H].

### Example 109: 4-(2-((2¹Z,4⁴E)-4¹H-11-oxa-3,8-diaza-2(7,2)-pyrrolo[2,1-f][1,2,4]triazin-4(4,1)-pyrazolo-1(1,4)-benzenacycloundecaphan-8-yl)ethoxy)-2-(2,6-dioxopiperidin-3- 1)isoindolin-1,3-dione

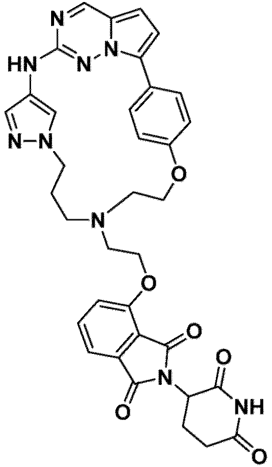

(2¹Z,4⁴E)-4¹*H*-11-oxa-3,8-diaza-2(7,2)-pyrrolo[2,1-f][1,2,4]triazina-4(4,1)-pyrazola-1(1,4)-benzenacycloundecaphane (83 mg, 0.22 mmol), potassium iodide (219 mg, 0.66 mmol), diisopropylethylamine (0.12 mL, 0.66 mmol), and 4-(2-bromoethoxy)-2-(2,6-dioxopiperidin-3-yl)isoindoline-1,3-dione (167 mg, 0.44 mmol) weredissolved in dimethylformamide (2) mL). The reaction mixture solution was stirred at 60 °C for 12 hours. When the reaction was complete, the reaction mixture solution was diluted with ethyl acetate and washed with saturated sodium chloride solution. The organic layer was dried over magnesium sulfate and concentrated. The resulting residue was purified by chromatography to obtain the title compound (75 mg, 51% yield). MS m/z: 676 [M+1].

### Example 110: 4-(3-((2¹Z,4⁴E)-4¹H-11-oxa-3,8-diaza-2(7,2)-pyrrolo[2,1-f][1,2,4]triazin-4(4,1)-pyrazolo-1(1,4)-benzenacycloundecaphan-8-yl)propoxy)-2-(2,6-dioxopiperidin-3-yl)isoindolin-1,3-dione

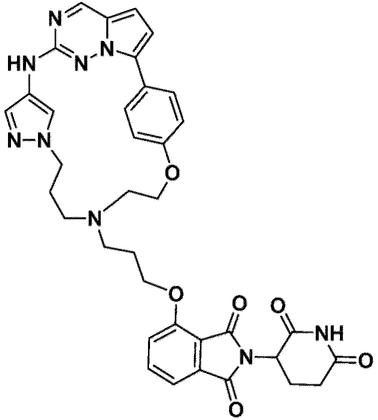

The procedure was identical to that of Example 109, except that 4-(3-bromopropoxy)-2-(2,6-dioxopiperidin-3-yl)isoindolin-1,3-dione (167 mg, 0.44 mmol) was used instead of 4-(2-bromoethoxy)-2-(2,6-dioxopiperidin-3-yl)isoindoline-1,3-dione. MS m/z: 690 [M+1].

### Example 111: 4-(2-(2-((2¹Z,4⁴E)-4¹H-11-oxa-3,8-diaza-2(7,2)-pyrrolo[2,1-f][1,2,4]triazin-4(4,1)-pyrazolo-1(1,4)-benzenacycloundecaphan -8-yl)ethoxy)ethoxy)-2-(2-oxopiperidin-3-yl)isoindolin-1,3-dione

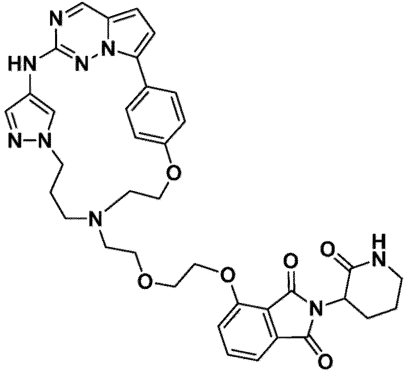

The procedure was identical to that of Example 109, except that 4-(2-(2-bromoethoxy)ethoxy)-2-(2-oxopiperidin-3-yl)isoindoline-1,3-dione was used instead of 4-(2-bromoethoxy)-2-(2,6-dioxopiperidin-3-yl)isoindoline-1,3-dione. MS m/z: 706 [M+1].

### Example 112: 4-((6-((2¹Z,4⁴E)-4¹H-11-oxa-3,8-diaza-2(7,2)-pyrrolo[2,1-f][1,2,4]triazin-4(4,1)-pyrazol-1(1,4)-benzenacycloundecaphan-8-yl)hexyl)oxy)-2-(2,6-dioxopiperidin-3-yl)isoindolin-1,3-dione

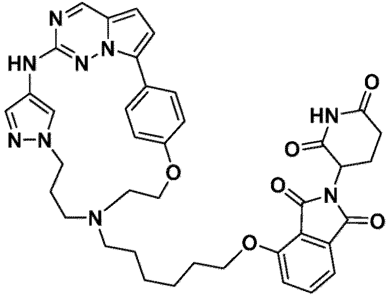

The procedure was identical to that of Example 109, except that 4-((6-bromohexyl)oxy)-2-(2,6-dioxopiperidin-3-yl)isoindoline-1,3-dione was used instead of 4-(2-bromoethoxy)-2-(2,6-dioxopiperidin-3-yl)isoindoline-1,3-dione. MS m/z: 732 [M+1].

### Example 113: 4-((8-((2¹Z,4⁴E)-4¹H-11-oxa-3,8-diaza-2(7,2)-pyrrolo[2,1-f][1,2,4]triazin-4(4,1)-pyrazol-1(1,4)-benzenacycloundecaphan-8-yl)octyl)oxy)-2-(2,6-dioxopiperidin-3-1)isoindolin-1,3-dione

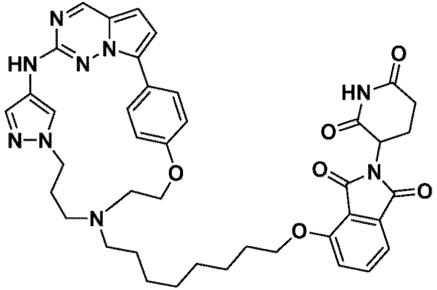

The procedure was identical to that of Example 109, except that 4-((8-bromooctyl)oxy)-2-(2,6-dioxopiperidin-3-yl)isoindoline-1,3-dione was used instead of 4-(2-bromoethoxy)-2-(2,6-dioxopiperidin-3-yl)isoindoline-1,3-dione. MS m/z: 690 [M+1].

### Example 114: 4-((8-((2¹Z,4⁴E)-4¹H-11-oxa-3,8-diaza-2(7,2)-pyrrolo[2,1-f][1,2,4]triazin-4(4,1)-pyrazo1-1(1,4)-benzenacycloundecaphan-8-yl)octyl)oxy)-2-(2-oxopiperidin-3-yl)isoindolin-1,3-dione

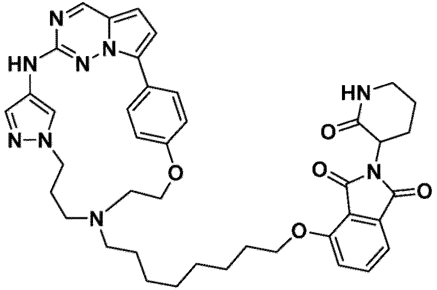

The procedure was identical to that of Example 109, except that 4-((8-bromooctyl)oxy)-2-(2-oxopiperidin-3-yl)isoindoline-1,3-dione was used instead of 4-(2-bromoethoxy)-2-(2,6-dioxopiperidin-3-yl)isoindoline-1,3-dione. MS m/z: 746 [M+1].

### Example 115: 4-((11-((2¹Z,4⁴E)-4¹H-11-oxa-3,8-diaza-2(7,2)-pyrrolo[2,1-f][1,2,4]triazin-4(4,1)-pyrazol-1(1,4)-benzenacycloundecaphan-8-yl)undecyl)oxy)-2-(2,6-dioxopiperidin-3-yl)isoindolin-1,3-dione

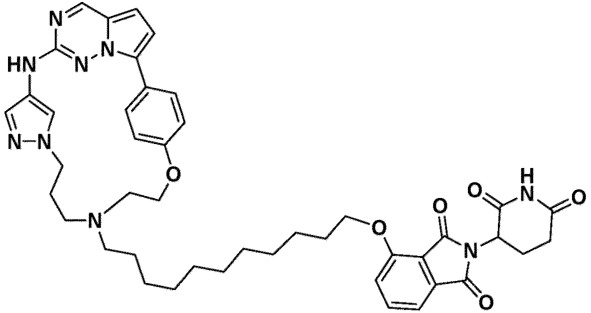

The procedure was identical to that of Example 109, except that 4-((11-bromoundecyl)oxy)-2-(2,6-dioxamperidin-3-yl)isoindoline-1,3-dione was used instead of 4-(2-bromoethoxy)-2-(2,6-dioxopiperidin-3-yl)isoindoline-1,3-dione. MS m/z: 802 [M+1].

### Example 116: 4-((9-((2¹Z,4⁴E)-4¹H-11-oxa-3,8-diaza-2(7,2)-pyrrolo[2,1-f][1,2,4]triazin-4(4,1)-pyrazola-1(1,4)-benzenacycloundecaphan-8-yl)nonyl)oxy)-2-(2,6-dioxopiperidin-3-yl)isoindolin-1,3-dione

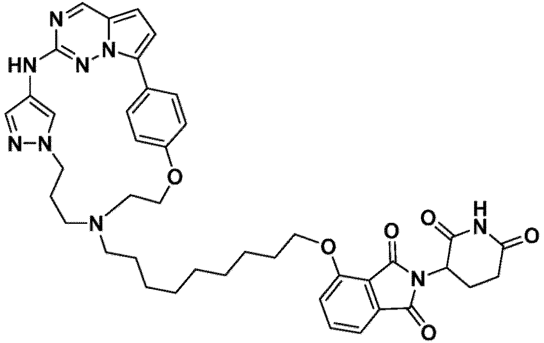

The procedure was identical to that of Example 109, except that 4-((9-bromononyl)oxy)-2-(2,6-dioxopiperidin-3-yl)isoindoline-1,3-dione was used instead of 4-(2-bromoethoxy)-2-(2,6-dioxopiperidin-3-yl)isoindoline-1,3-dione. MS m/z: 774 [M+1].

### Example 117: 5-(4-((1-(3-(2-(2-((2¹Z,4⁴E)-4¹H-11-oxa-3,8-diaza-2(7,2)-pyrrolo[2,1-f][1,2,4]triazin-4(4,1)-pyrazola-1(1,4)-benzenacycloundecaphan-8-yl)ethoxy)ethoxy))propanoyl)piperidin-4-yl)methyl)piperazin-1-yl)-2-(2,6-dioxopiperidin-3-yl)-6-fluoroisoindolin-1,3-dione

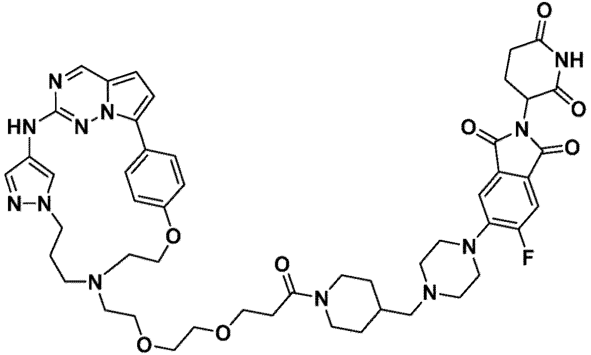

### Step 1) Preparation of t-Butyl 3-(2-(2-((2¹Z,4⁴E)-4¹H-11-oxa-3,8-diaza-2(7,2)-pyrrolo[2,1-f][1,2,4]triazin-4(4,1)-pyrazol-1(1,4)-benzenecycloundecaphan-8-yl)ethoxy)ethoxy)propanoate

(2¹Z,4⁴E)-4¹*H-*11-oxa-3,8-diaza-2(7,2)-pyrrolo[2,1-f][1,2,4]triazina-4(4,1)-pyrazola-1(1,4)-benzenacycloundecaphane (83 mg, 0.22 mmol), potassium iodide (73 mg, 0.22 mmol), diisopropylethylamine (0.12 mL, 0.66 mmol) and t-butyl 3-(2-(2-iodoethoxy)ethoxy)propanoate (151 mg, 0.44 mmol) were dissolved in dimethylformamide (2 mL). The reaction mixture was stirred at 60 °C for 24 hours. When the reaction was complete, the reaction mixture was diluted with ethyl acetate and washed with saturated sodium chloride solution. The organic layer was dried over magnesium sulfate and concentrated. The resulting residue was purified by column chromatography to obtain the title compound (66 mg, 51% yield). MS m/z: 592 [M+1].

### Step 2) Preparation of 3-(2-(2-((2¹Z,4⁴E)-4¹H-11-oxa-3,8-diaza-2(7,2)-pyrrolo[2,1-f][1,2,4]triazin-4(4,1)-pyrazole-1(1,4)-benzenecycloundecaphan-8-yl)ethoxy)ethoxy)propanoic acid

To a solution of *t*-butyl 3-(2-(2-((2¹Z,4⁴E)-4¹*H*-11-oxa-3,8-diaza-2(7,2)-pyrrolo[2,1-f][1,2,4]triazin-4(4,1)-pyrazol-1(1,4)-benzenecycloundecaphan-8-yl)ethoxy)ethoxy)propanoate (90 mg, 0.17 mmol) in tetrohydrofuran (1 mL), methanol (1 mL), and water (1 mL) was added LiOH-H2O (15 mg 0.34 mmol). The reaction mixture was stirred at room temperature for 12 hours. The reaction mixture solution was diluted with ethyl acetate, neutralized with 1 N aqueous hydrogen chloride solution to reach pH 5, and then washed with saturated sodium chloride solution. The organic layer was dried over magnesium sulfate and concentrated to obtain the title compound (92 mg, 94% yield), which was used in the next reaction without purification. MS m/z : 536 [M+1].

### Step 3) Preparation of 5-(4-((1-(3-(2-(2-((2¹Z,4⁴E)-4¹H-11-oxa-3,8-diaza-2(7,2)-pyrrolo[2,1-f][1,2,4]triazin-4(4,1)-pyrazola-1(1,4)-benzenacycloundecaphan-8-yl)ethoxy)ethoxy))propanoyl)piperidin-4-yl)methyl)piperazin-1-yl)-2-(2,6-dioxopiperidin-3-yl)-6-fluoroisoindolin-1, 3-dione

To a solution fo 3-(2-(2-((2¹Z,4⁴E)-4¹*H-*11-oxa-3,8-diaza-2(7,2)-pyrrolo[2,1-f][1,2,4]triazin-4(4,1)-pyrazole-1(1,4)-benzenecycloundecaphan-8-yl)ethoxy)ethoxy)propanoic acid (55 mg, 0.10 mmol), EDCI (56 mg, 0.29 mmol) and HOAt (40 mg, 0.29 mmol) in dimethylolamide (2 mL) was added diisopropylethylamine (0.08 mL, 0.49 mmol). After stirring the reaction mixture solution at room temperature for 30 minutes, 2-(2,6-dioxopiperidin-3-yl)-5-fluoro-6-(4-(piperidin-4-ylmethyl)piperazin-1-yl)isoindoline-1,3-dione (50 mg, 0.11 mmol) was added and stirred at 50 °C for 6 hours. When the reaction was complete, the reaction mixture was diluted with dichloromethane and washed with saturated sodium chloride soluiton. The organic layer was dried over magnesium sulfate and concentrated. The resulting residue was purifed by column chromatography to obtain the title compound. MS m/z: 976 [M+1].

### Example 118: 3-(2-(2-((2¹Z,4⁴E)-4¹H-11-oxa-3,8-diaza-2(7,2)-pyrrolo[2,1-f][1,2,4]triazin-4(4,1)-pyrazola-1(1,4)-benzenacycloundecaphan -8-yl)ethoxy)ethoxy)-N-(3-(4-(3-(2,4-dihydroxy-5-isopropylphenyl)-5-hydroxy-4H-1,2,4-triazol-4-yl)phenoxy)propyl)propanamide

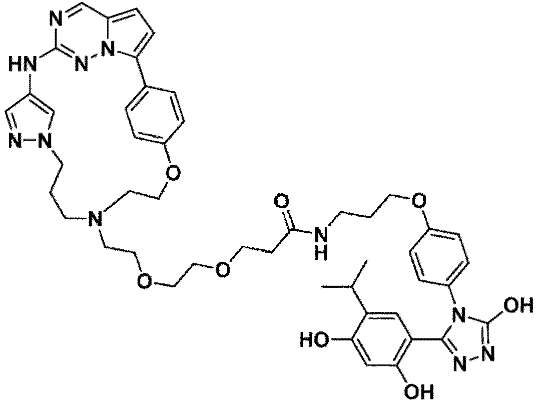

The procedure was identical to that of Example 117, except that 4-(4-(4-(3-aminopropoxy)phenyl)-5-hydroxy-4*H*-1,2,4-triazol-3-yl)-6-isopropylbenzene-1,3-diol was used instead of 2-(2,6-dioxopiperidin-3-yl)-5-fluoro-6-(4-(piperidin-4-ylmethyl)piperazin-1-yl)isoindolin-1,3-dione. MS m/z: 903 [M+1].

### Example 119: 3-(2-(2-((2¹Z,4⁴E)-4¹H-11-oxa-3,8-diaza-2(7,2)-pyrrolo[2,1-f][1,2,4]triazin-4(4,1)-pyrazol-1(1,4)-benzenacycloundecaphan-8-yl)ethoxy)ethoxy)-N-(2-((3-(4-(3-(2,4-dihydroxy-5-isopropylphenyl)-5-hydroxy-4H-1,2,4-triazol-4-yl)phenoxy)propyl)amino)-2-oxoethyl)propanamide

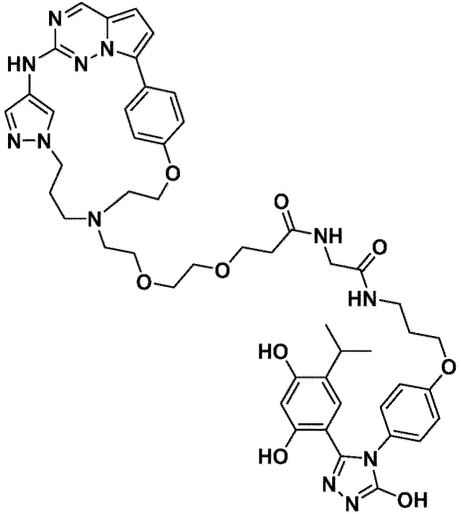

The procedure was identical to that of Example 117, except that 2-amino-*N*-(3-(4-(3-(2,4-adihydroxy-5-isopropylphenyl)-5-hydroxy-4*H*-1,2,4-triazol-4-yl)phenoxy)propyl)acetamide was used instead of 2-(2,6-dioxopiperidin-3-yl)-5-fluoro-6-(4-(piperidin-4-ylmethyl)piperazin-1-yl)isoindolin-1,3-dione. MS m/z: 960 [M+1].

### Example 120: 3-(2-(2-((2¹Z,4⁴E)-4¹H-11-oxa-3,8-diaza-2(7,2)-pyrrolo[2,1-f][1,2,4]triazin-4(4,1)-pyrazolo-1(1,4)-benzenacycloundecaphan -8-yl)ethoxy)ethoxy)-1-(4-(4-(3-(2,4-dihydroxy-5-isopropylphenyl)-5-hydroxy-4H-1,2,4-triazol-4-yl)benzyl)piperazin-1-yl)propan-1-one

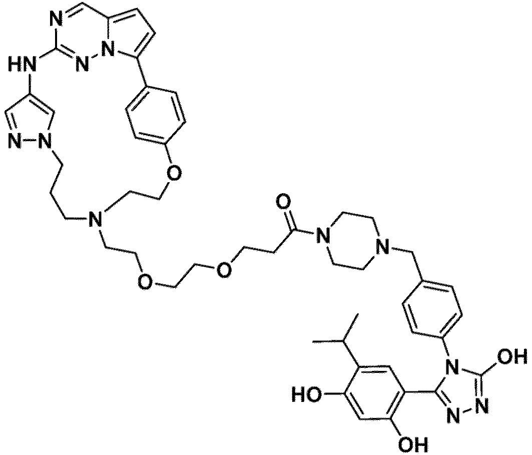

The procedure was identical to that of Example 117, except that 4-(5-hydroxy-4-(4-(piperazin-1-ylmethyl)phenyl)-4*H*-1,2,4-triazol-3-yl)-6-isopropylbenzene-1,3-diol was used instead of 2-(2,6-dioxopiperidin-3-yl)-5-fluoro-6-(4-(piperidin-4-ylmethyl)piperazin-1-yl)isoindolin-1,3-dione. MS m/z: 928 [M+1].

### Example 121: 3-(2-(2-((2¹Z,4⁴E)-4¹H-11-oxa-3,8-diaza-2(7,2)-pyrrolo[2,1-f][1,2,4]triazin-4(4,1)-pyrazolo-1(1,4)-benzenacycloundecaphan-8-yl)ethoxy)ethoxy)-l-(4-((4-(4-(3-(2,4-dihydroxy-5-isopropylphenyl)-5-hydroxy-4H-1,2,4-triazol-4-yl)benzyl)piperazin-1-yl)methyl)piperidin-1-yl)propan-1-one

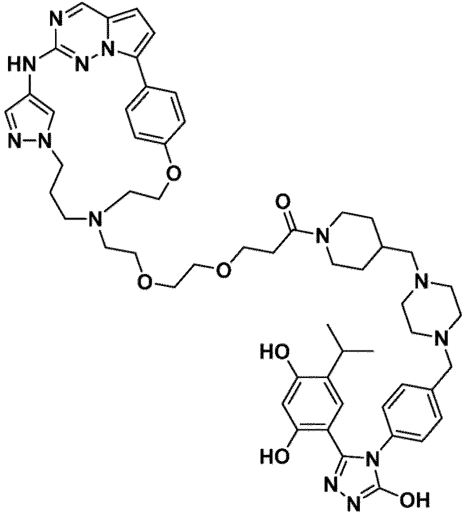

The procedure was identical to that of Example 117, except that 4-(5-hydroxy-4-(4-((4-(piperidin-4-ylmethyl)piperazin-1-yl)methyl)phenyl)-4*H* 1,2,4-triazol-3-yl)-6-isopropylbenzene-1,3-diol was used instead of 2-(2,6-dioxopiperidin-3-yl)-5-fluoro-6-(4-(piperidin-4-ylmethyl)piperazin-1-yl)isoindolin-1,3-dione. MS m/z: 1025 [M+1].

### Example 122: 3-(2-(2-((2¹Z,4⁴E)-4¹H-11-oxa-3,8-diaza-2(7,2)-pyrrolo[2,1-f][1,2,4]triazin-4(4,1)-pyrazol-1(1,4)-benzenacycloundecaphan-8-yl)ethoxy)ethoxy)-1-(4-((2-(2,4-dihydroxy-5-isopropylbenzoyl)isoindolin-5-yl)methyl)piperazin-1-yl)propan-1-one

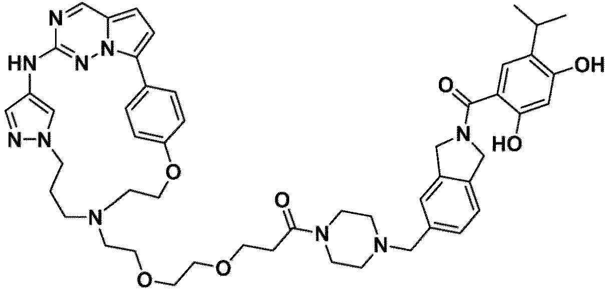

The procedure was identical to that of Example 117, except that (2,4-dihydroxy-5-isopropylphenyl)(5-(piperazin-1-ylmethyl)isoindolin-2-yl)methanone was used instead of 2-(2,6-dioxopiperidin-3-yl)-5-fluoro-6-(4-(piperidin-4-ylmethyl)piperazin-1-yl)isoindolin-1,3-dione. MS m/z: 914 [M+1].

### Example 123: 3-(2-(2-((2¹Z,4⁴E)-4¹H-11-oxa-3,8-diaza-2(7,2)-pyrrolo[2,1-f][1,2,4]triazin-4(4,1)-pyrazola-1(1,4)-benzenacycloundecaphan-8-yl)ethoxy)ethoxy)-1-(4-((4-((2-(2,4-dihydroxy-5-isopropylbenzoyl)isoindolin-5-yl)methyl)piperazin-1-yl)methyl)piperidin-1-yl)propan-1-one

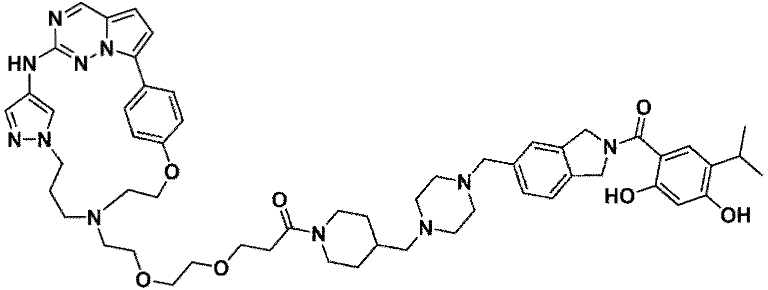

The procedure was identical to that of Example 117, except that (2,4-dihydroxy-5-isopropylphenyl)(5-((4-(piperidin-4-ylmethyl)piperazin-1-yl)methyl)isoindolin-2-yl)methanone was used instead of 2-(2,6-dioxopiperidin-3-yl)-5-fluoro-6-(4-(piperidin-4-ylmethyl)piperazin-1-yl)isoindolin-1,3-dione. MS m/z: 1011 [M+1].

### Example 124: 4-(5-(2-(4-(3-(2-(2-((2¹Z,4⁴E)-4¹H-11-oxa-3,8-diaza-2(7,2)-pyrrolo[2,1-f][1,2,4]triazin-4(4,1)-pyrazola-1(1,4)-benzenacycloundecaphan-8-yl)ethoxy)ethoxy)propanoyl)piperazin-1-yl)ethoxy)-2,4-dichlorophenyl)-2-amino-N-ethylcyano[2,3-d]pyrimidin-6-carboxamide

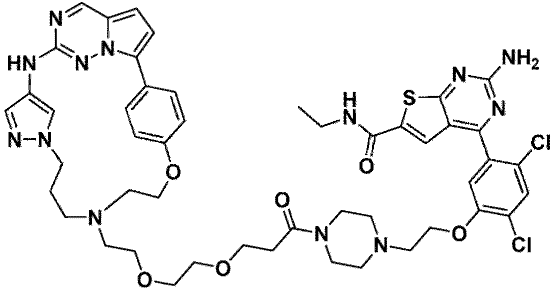

The procedure was identical to that of Example 117, except that 2-amino-4-(2,4-dichloro-5-(2-(piperazin-1-yl)ethoxy)phenyl)-*N-*ethylthieno[2,3-d]pyrimidin-6-carboxamide was used instead of 2-(2,6-dioxopiperidin-3-yl)-5-fluoro-6-(4-(piperidin-4-ylmethyl)piperazin-1-yl)isoindolin-1,3-dione. MS m/z: 1014 [M+1].

### Example 125: ((2¹Z,4⁴E)-4¹H-11-oxa-3,8-diaza-2(7,2)-pyrrolo[2,1-f][1,2,4]triazin-4(4,1)-pyrazola-1(1,4)-benzenacycloundecaphan-8-yl)(4-((4-((2-(2,4-dihydroxy-5-isopropylbenzoyl)isoindolin-5-yl)methyl)piperazin-1-yl) methyl)phenyl)methanone

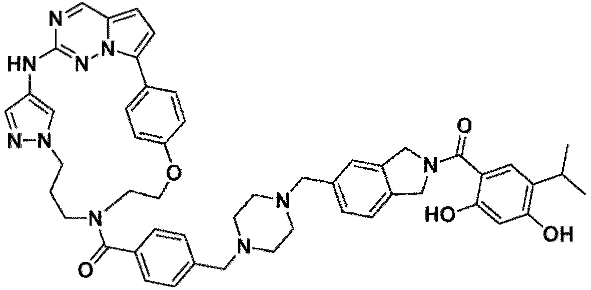

To a solution of 4-((4-((2-(2,4-dihydroxy-5-isopropylbenzoic)isoindolin-5-yl)methyl)piperazin-1-yl)methyl)benzoic acid (50 mg, 0.09 mmol), EDCI (54 mg, 0.28 mmol), and HOAt (37 mg, 0.29 mmol) were dissolved in dimethylormamide (2 mL) in dimethylolamide (2 mL) was added diisopropylethylamine (0.08 mL, 0.45 mmol). After stirring the reaction mixture at room temperature for 30 minutes, (2¹Z,4⁴E)-4¹*H-*11-oxa-3,8-diaza-2(7,2)-pyrrolo[2,1-f][1,2,4]triazine-4(4,1)-pyrazola-1(1,4)-benzenecycloundecaphane (38 mg, 0.10 mmol) was added and stirred at 50 °C for 6 hours. When the reaction was complete, the reaction mixture was diluted with dichloromethane and washed with saturated sodium chloride solution. The organic layer was dried over magnesium sulfate and concentrated. The resultin residue was purified by column chromatography to obtain the title compound. MS m/z: 888 [M+1].

### Example 126: ((2¹Z,4⁴E)-4¹H-11-oxa-3,8-diaza-2(7,2)-pyrrolo[2,1-f][1,2,4]triazin-4(4,1)-pyrazola-1(1,4)-benzenacycloundecaphane-8-yl)(1-((2-(2,4-dihydroxy-5-isopropylbenzoyl)isoindolin-5-yl)methyl)piperidin-4-yl)methanone

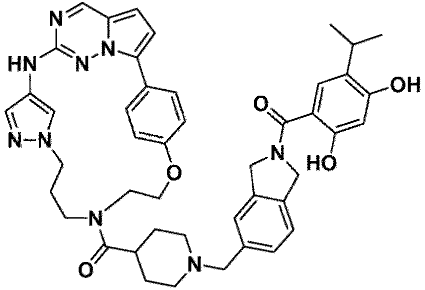

The procedure was identical to that of Example 125, except that 1-((2-(2,4-dihydroxy-5-isopropylbenzoic)isoindolin-5-yl)methyl)piperidine-4-carboxylic acid was used instead of 4-((4-((2-(2,4-dihydroxy-5-isopropylbenzoic)isoindolin-5-yl)methyl)piperazin-1-yl)methyl)benzoic acid. MS m/z: 796 [M+1].

Upon organizing the structures and LC-MS data of Examples 1 to 126 as described above, the resulting table is as follows.

| | Structure | L C M S | | Structure | L C M S |
|---|---|---|---|---|---|
| E x. 1 | 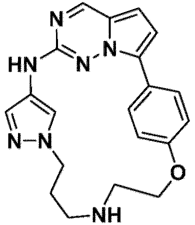 | 3 7 6 | E x. 2 | 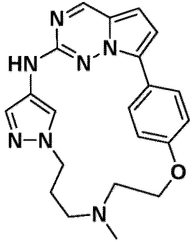 | 4 9 0 |
| E x. 3 | 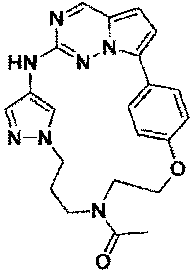 | 4 1 8 | E x. 4 | 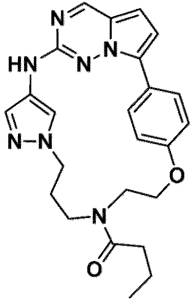 | 4 4 6 |
| E x. 5 | 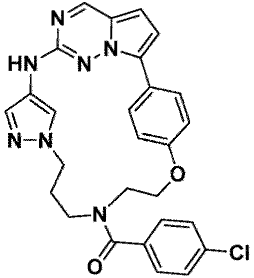 | 5 1 4 | E x. 6 | 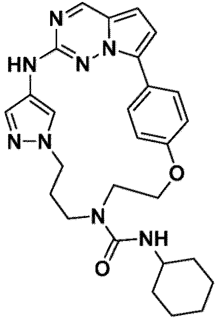 | 5 0 1 |
| E x. 7 | 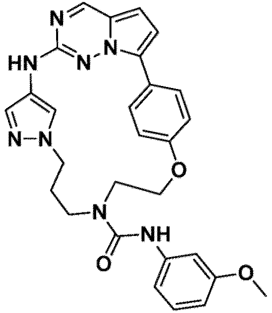 | 5 2 4 | E x. 8 | 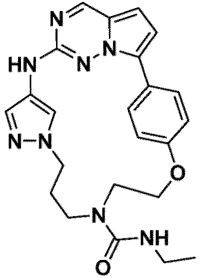 | 4 4 7 |

| | | | | | |
|---|---|---|---|---|---|
| E x. 9 | 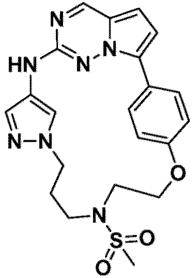 | 4 5 4 | E x. 1 0 | 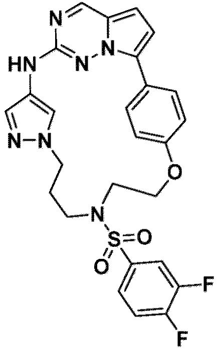 | 5 5 2 |
| E x. 1 1 | 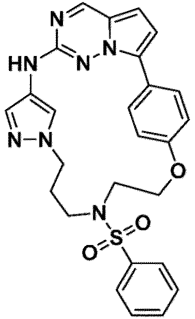 | 5 1 6 | E x. 1 2 | 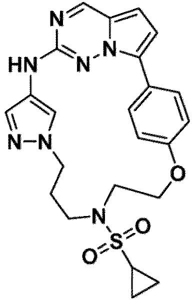 | 4 8 0 |
| E x. 1 3 | 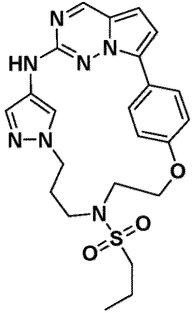 | 4 8 2 | E x. 1 4 | 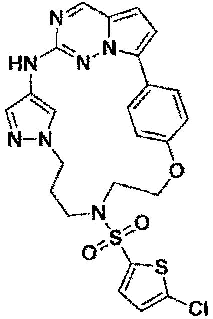 | 5 5 6 |

| | | | | | |
|---|---|---|---|---|---|
| E x. 1 5 | 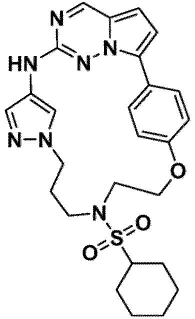 | 522 | Ex.16 | 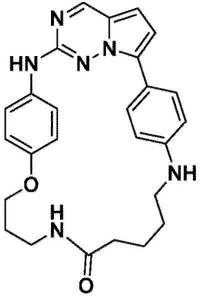 | 4 5 7 |
| E x. 1 7 | 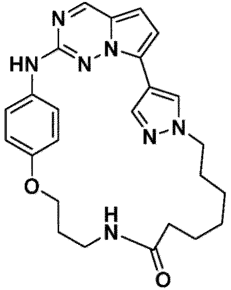 | 4 6 0 | E x. 1 8 | 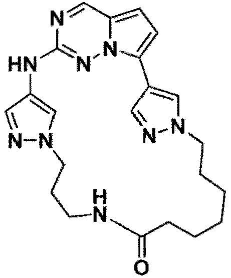 | 4 3 4 |
| E x. 1 9 | 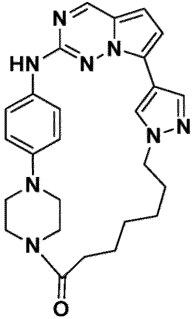 | 4 7 1 | E x. 2 0 | 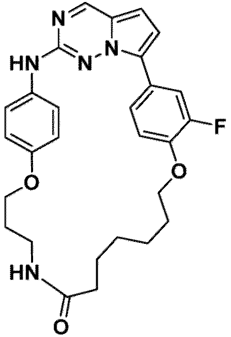 | 5 0 4 |

| | | | | | |
|---|---|---|---|---|---|
| E x. 2 1 | 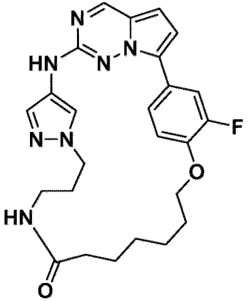 | 4 7 8 | E x. 2 2 | 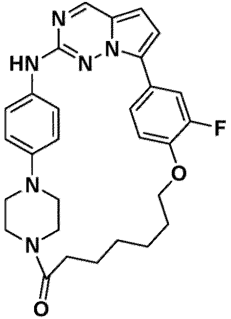 | 5 0 4 |
| E x. 2 3 | 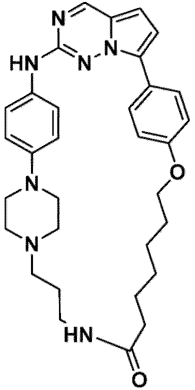 | 5 5 4 | E x. 2 4 | 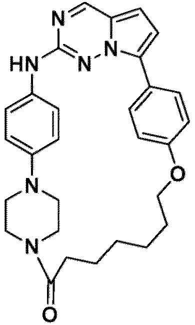 | 4 9 7 |
| E x. 2 5 | 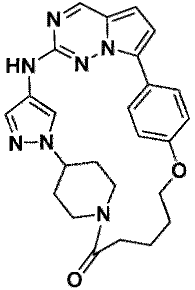 | 4 5 8 | E x. 2 6 | 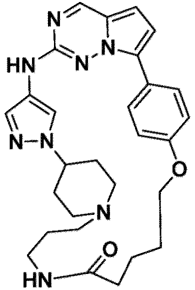 | 5 1 5 |

| | | | | | |
|---|---|---|---|---|---|
| E x .2 7 | 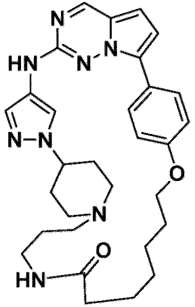 | 5 4 3 | E x. 2 8 | 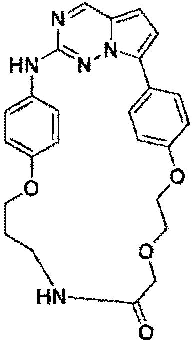 | 4 6 0 |
| E x. 2 9 | 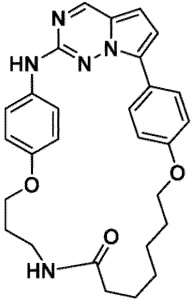 | 4 8 6 | E x. 3 0 | 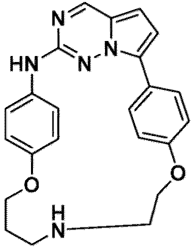 | 4 0 2 |
| E x. 3 1 | 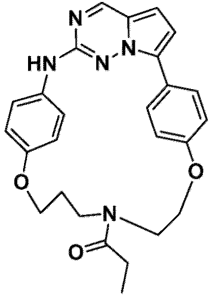 | 4 5 8 | E x. 3 2 | 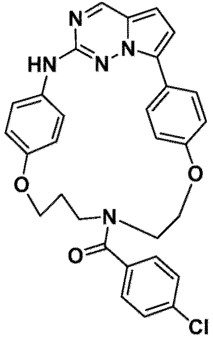 | 5 4 0 |

| | | | | | |
|---|---|---|---|---|---|
| E x. 3 3 | 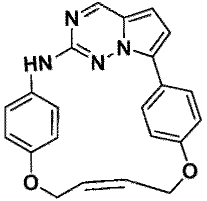 | 3 7 1 | E x . 3 4 | 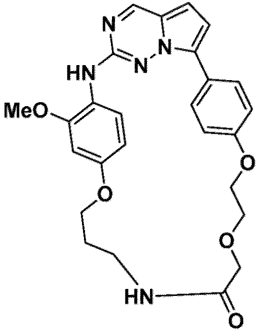 | 4 9 0 |
| E x. 3 5 | 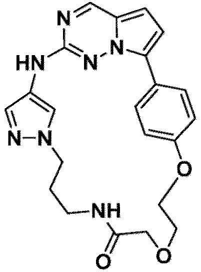 | 4 3 4 | E x. 3 6 | 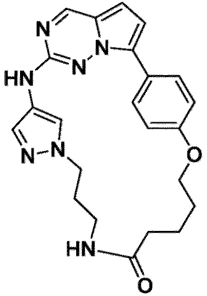 | 4 3 2 |
| E x. 3 7 | 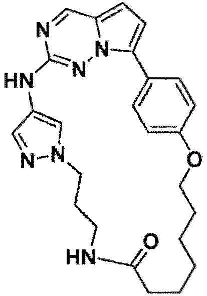 | 4 6 0 | E x. 3 8 | 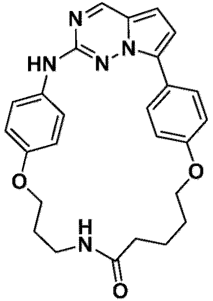 | 4 5 8 |

| | | | | | |
|---|---|---|---|---|---|
| E x. 3 9 | 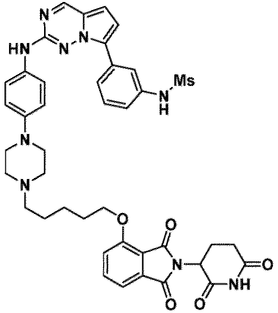 | 8 0 6 | E x. 4 0 | 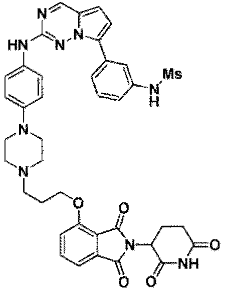 | 7 7 8 |
| E x. 4 1 | 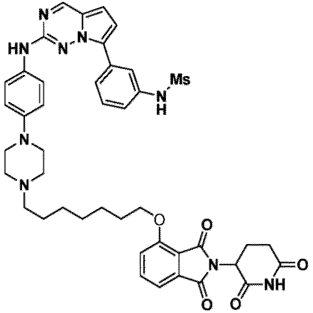 | 8 3 4 | E x. 4 2 | 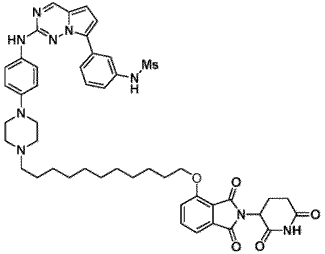 | 8 9 0 |
| E x. 4 3 | 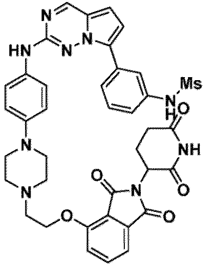 | 7 6 4 | E x. 4 4 | 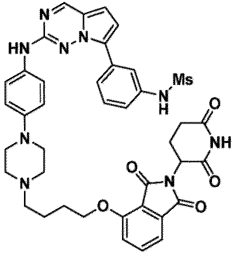 | 7 9 2 |

| | | | | | |
|---|---|---|---|---|---|
| E x. 4 5 | 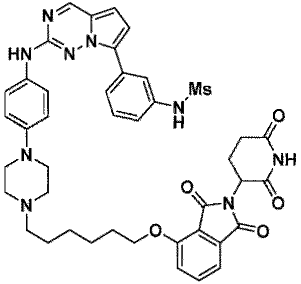 | 8 2 0 | E x. 4 6 | 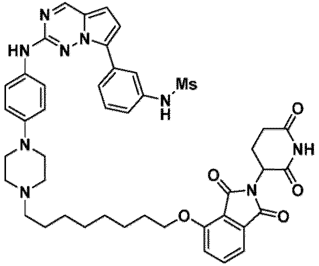 | 8 4 8 |
| E x. 4 7 | 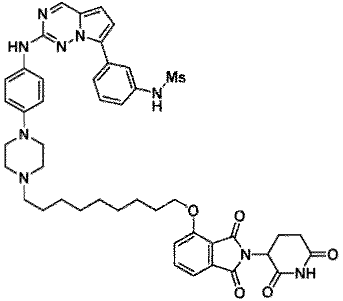 | 8 6 2 | E x. 4 8 | 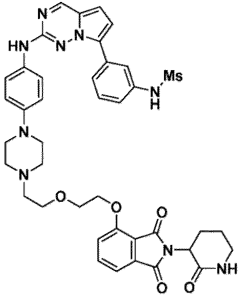 | 7 9 4 |
| E x. 4 9 | 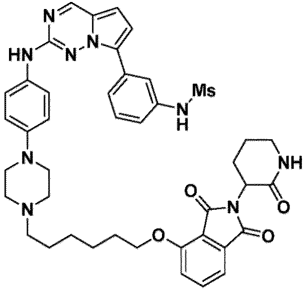 | 8 0 6 | E x. 5 0 | 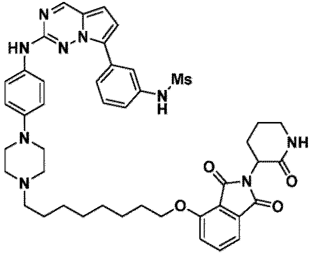 | 8 3 4 |

| | | | | | |
|---|---|---|---|---|---|
| E x. 5 1 | 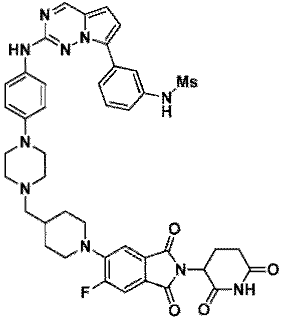 | 8 3 5 | E x. 5 2 | 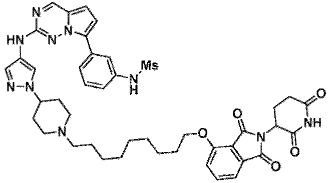 | 8 5 1 |
| E x. 5 3 | 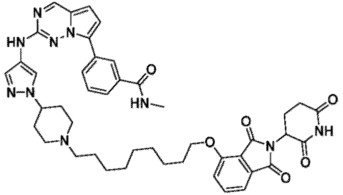 | 8 1 5 | E x. 5 4 | 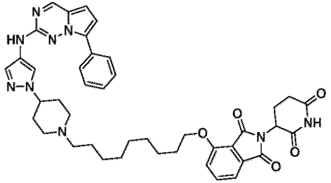 | 7 5 8 |
| E x. 5 5 | 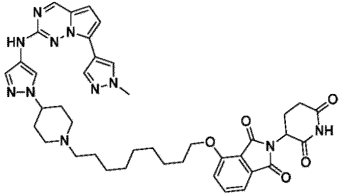 | 7 6 2 | E x. 5 6 | 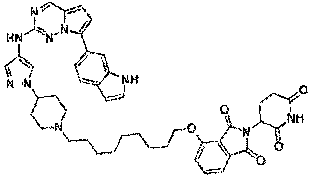 | 7 9 7 |

| | | | | | |
|---|---|---|---|---|---|
| E x. 5 7 | 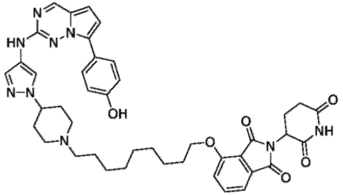 | 7 7 4 | E x. 5 8 | 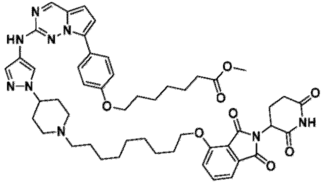 | 9 1 6 |
| E x. 5 9 | 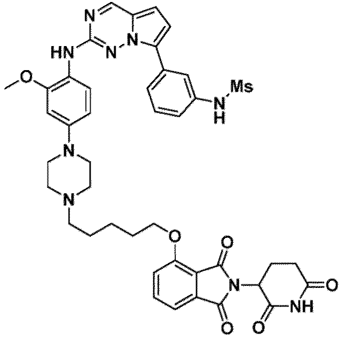 | 8 3 6 | E x. 6 0 | 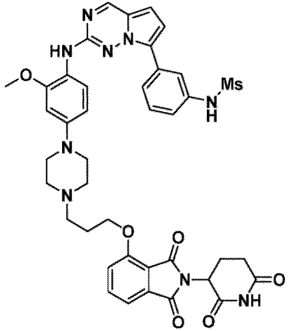 | 8 0 8 |
| E x. 6 1 | 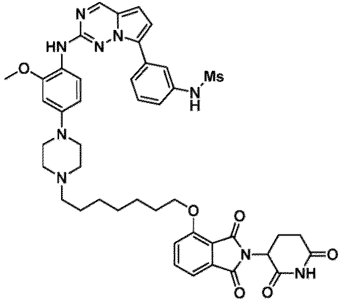 | 8 6 4 | E x. 6 2 | 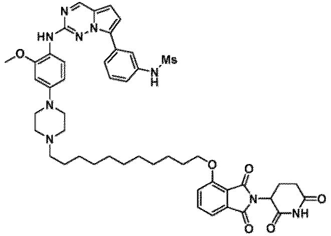 | 9 2 0 |

| | | | | | |
|---|---|---|---|---|---|
| E x. 6 3 | 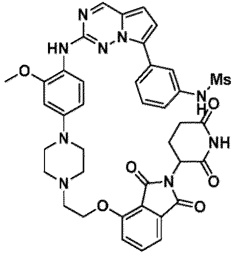 | 7 9 4 | E x. 6 4 | 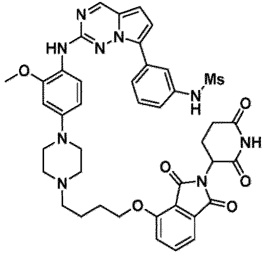 | 8 2 2 |
| E x. 6 5 | 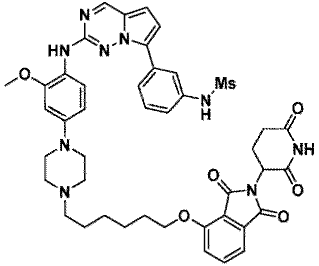 | 8 5 0 | E x. 6 6 | 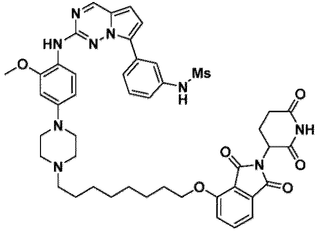 | 8 7 8 |
| E x. 6 7 | 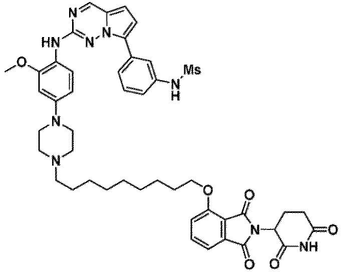 | 8 9 2 | E x. 6 8 | 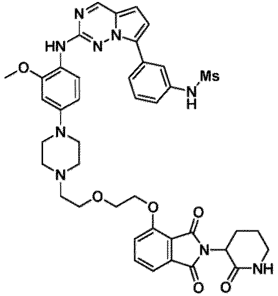 | 8 2 4 |

| | | | | | |
|---|---|---|---|---|---|
| E x. 6 9 | 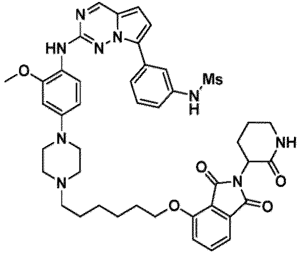 | 8 3 6 | E x. 7 0 | 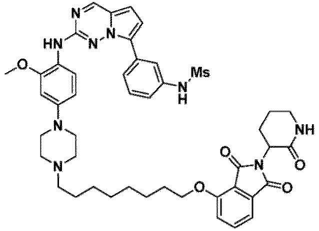 | 8 6 4 |
| E x. 7 1 | 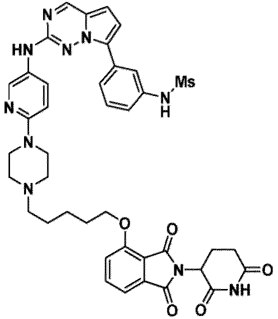 | 8 0 7 | E x. 7 2 | 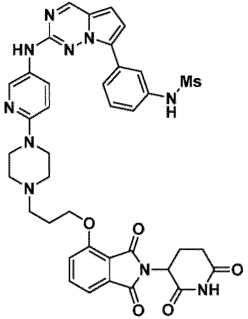 | 7 7 9 |
| E x. 7 3 | 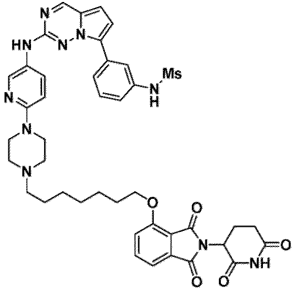 | 8 3 5 | E x. 7 4 | 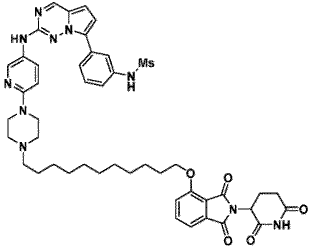 | 8 9 1 |

| | | | | | |
|---|---|---|---|---|---|
| E x. 7 5 | 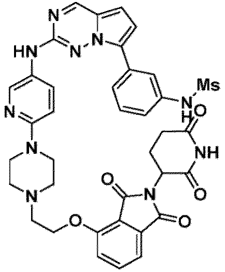 | 7 6 5 | E x. 7 6 | 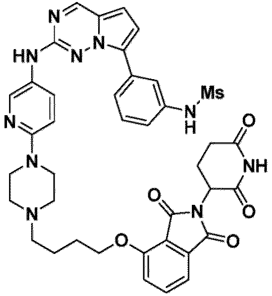 | 7 9 3 |
| E x. 7 7 | 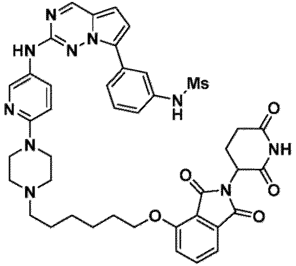 | 8 2 1 | E x. 7 8 | 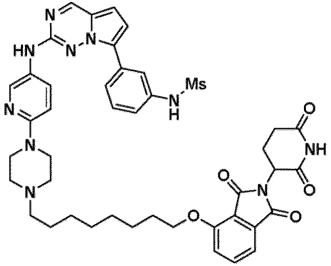 | 8 4 9 |
| E x. 7 9 | 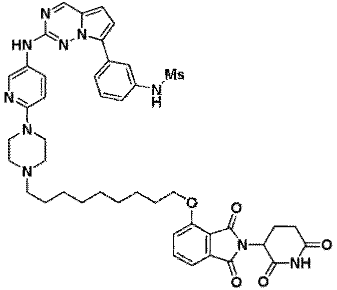 | 8 6 3 | E x. 8 0 | 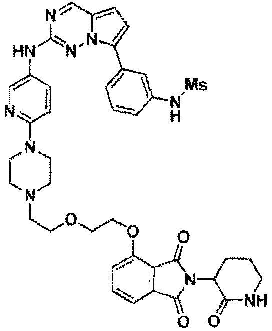 | 7 9 5 |

| | | | | | |
|---|---|---|---|---|---|
| E x. 8 1 | 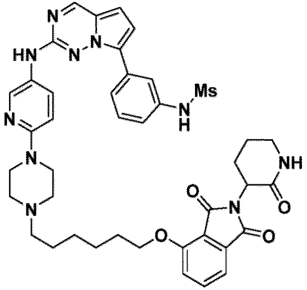 | 8 0 7 | E x. 8 2 | 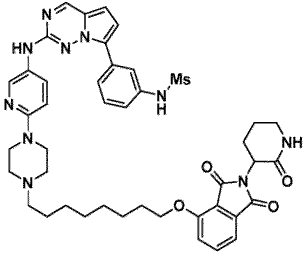 | 8 3 5 |
| E x. 8 3 | 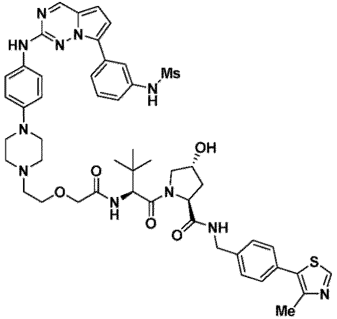 | 9 7 8 | E x. 8 4 | 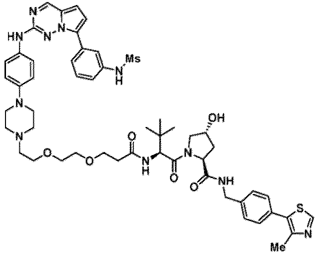 | 1 0 3 6 |
| E x. 8 5 | 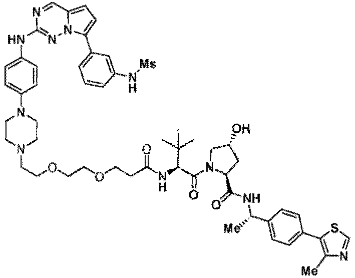 | 1 0 5 0 | E x. 8 6 | 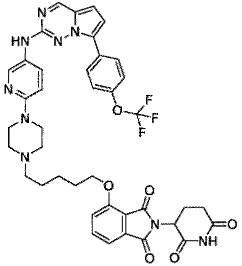 | 7 9 8 |

| | | | | | |
|---|---|---|---|---|---|
| E x. 8 7 | 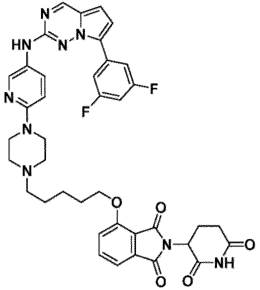 | 7 5 0 | E x. 8 8 | 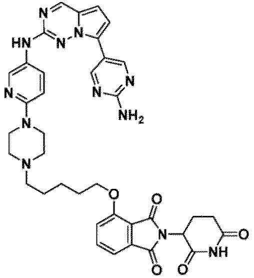 | 7 3 1 |
| E x. 8 9 | 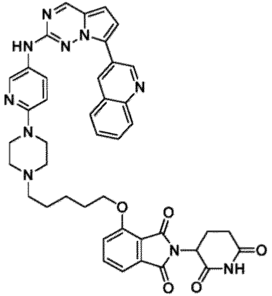 | 7 6 5 | E x. 9 0 | 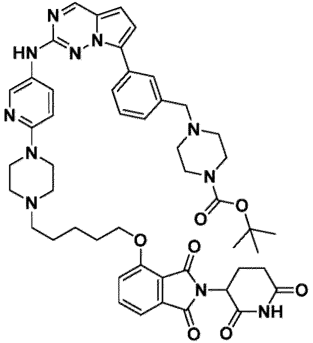 | 9 1 3 |
| E x. 9 1 | 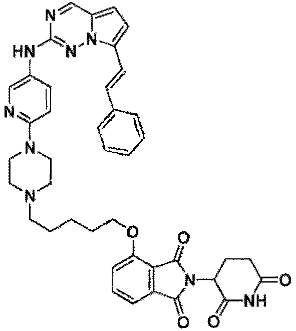 | 7 4 0 | E x. 9 2 | 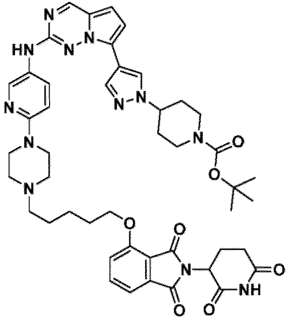 | 8 8 8 |

| | | | | | |
|---|---|---|---|---|---|
| E x. 9 3 | 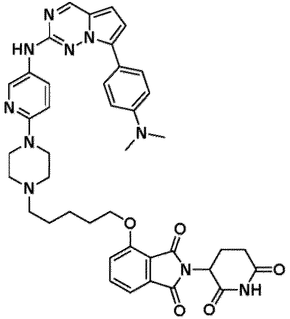 | 7 5 7 | E x. 9 4 | 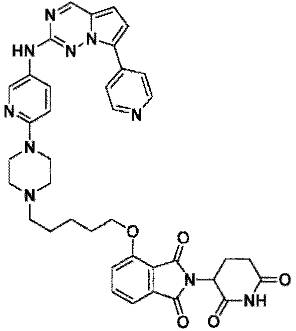 | 7 1 5 |
| E x. 9 5 | 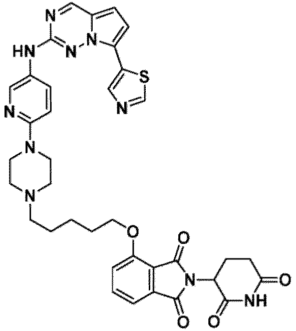 | 7 2 1 | E x. 9 6 | 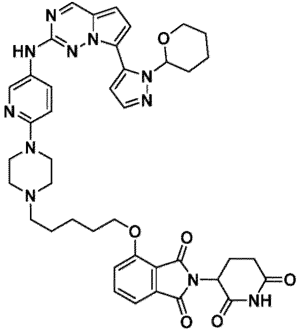 | 7 8 8 |
| E x. 9 7 | 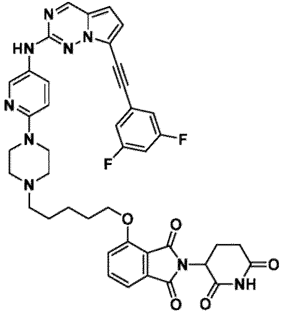 | 7 7 4 | E x. 9 8 | 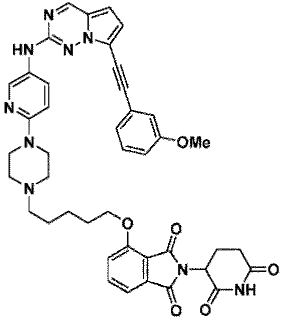 | 7 6 8 |

| | | | | | |
|---|---|---|---|---|---|
| E x. 9 9 | 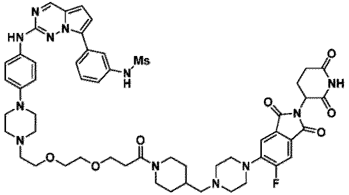 | 1 0 6 4 | E x. 1 0 0 | 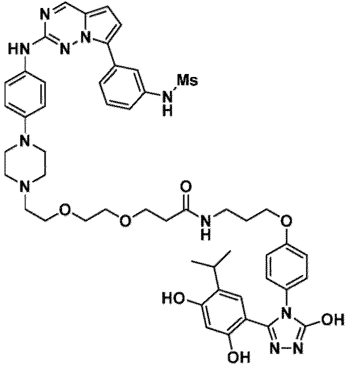 | 9 9 1 |
| E x. 1 0 1 | 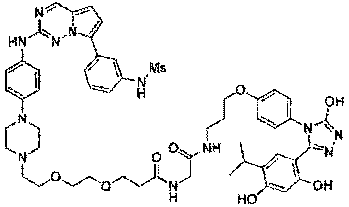 | 1 0 4 8 | E x. 1 0 2 | 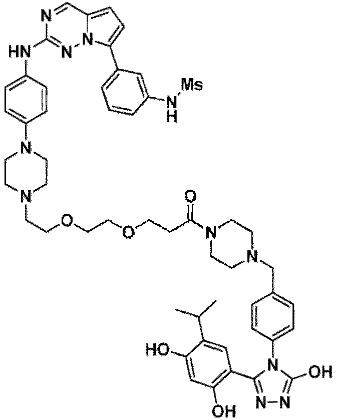 | 1 0 1 6 |
| E x. 1 0 3 | 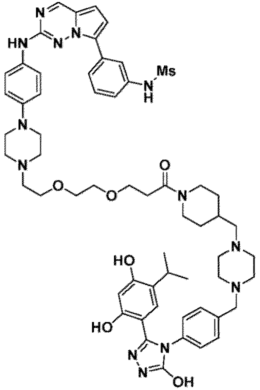 | 1 1 1 3 | E x. 1 0 4 | 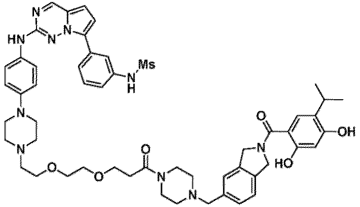 | 1 0 0 2 |

| | | | | | |
|---|---|---|---|---|---|
| E x. 1 0 5 | 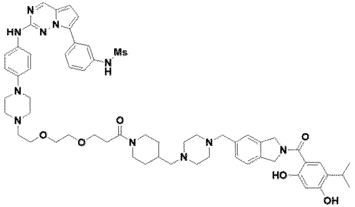 | 1 0 9 9 | E x. 1 0 6 | 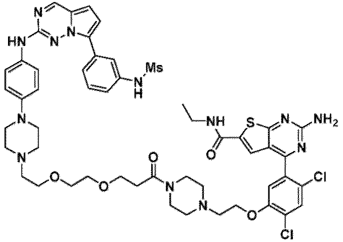 | 1 1 0 2 |
| E x. 1 0 7 | 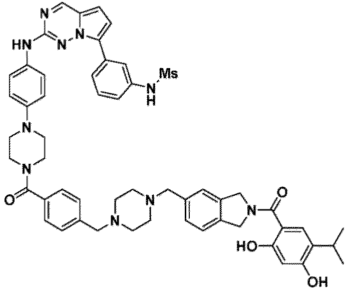 | 9 7 6 | E x. 1 0 8 | 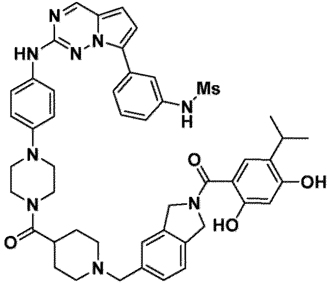 | 8 8 5 |
| E x. 1 0 9 | 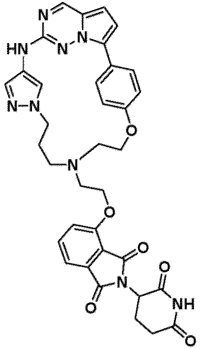 | 6 7 6 | E x. 1 1 0 | 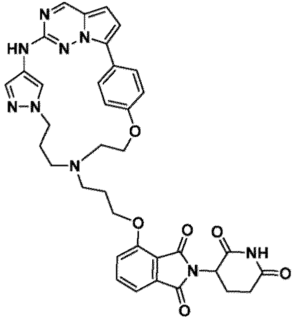 | 6 9 0 |

| | | | | | |
|---|---|---|---|---|---|
| E x. 1 1 1 | 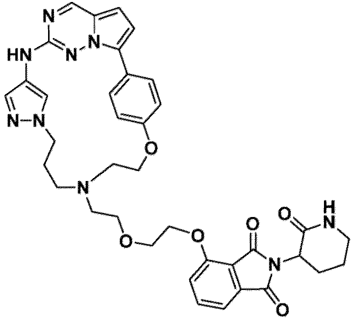 | 7 0 6 | E x. 1 1 2 | 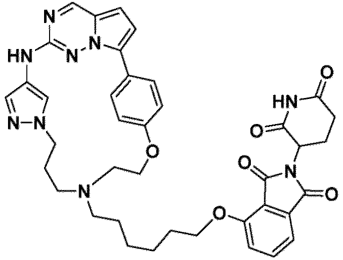 | 7 3 2 |
| E x. 1 1 3 | 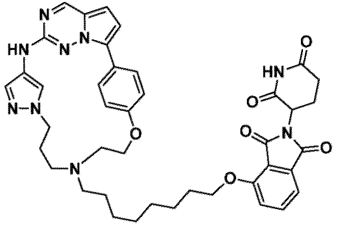 | 7 6 0 | E x. 1 1 4 | 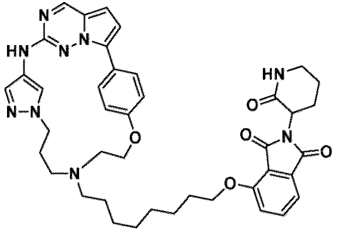 | 7 4 6 |
| E x. 1 1 5 | 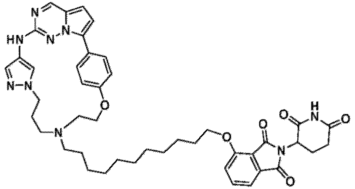 | 8 0 2 | E x. 1 1 6 | 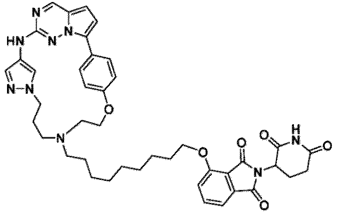 | 7 7 4 |

| | | | | | |
|---|---|---|---|---|---|
| E x. 1 1 7 | 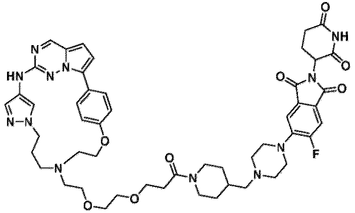 | 9 7 6 | E x. 1 1 8 | 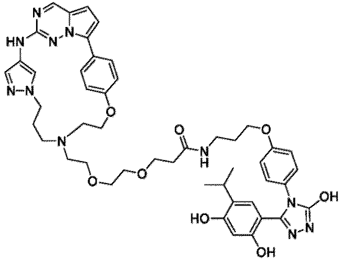 | 9 0 3 |
| E x. 1 1 9 | 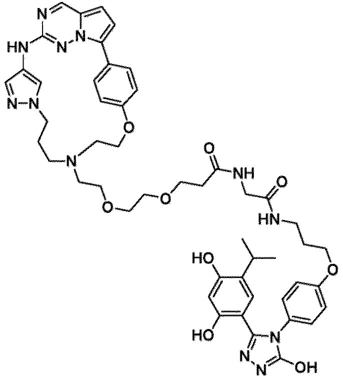 | 9 6 0 | E x. 1 2 0 | 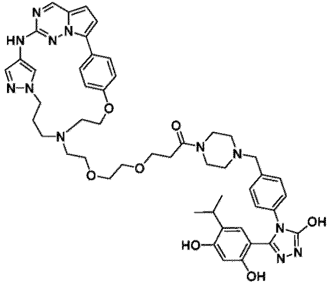 | 9 2 8 |
| E x. 1 2 1 | 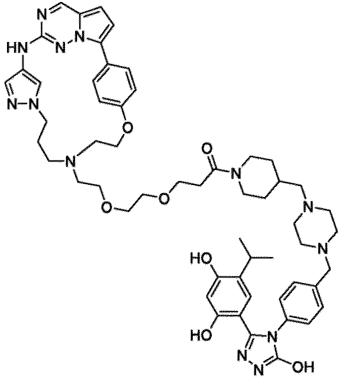 | 1 0 2 5 | E x. 1 2 2 | 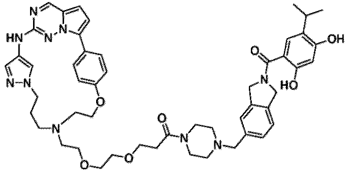 | 9 1 4 |

| | | | | | |
|---|---|---|---|---|---|
| E x. 1 2 3 | 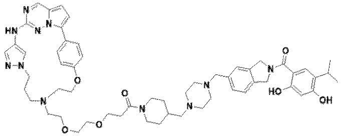 | 1 0 1 1 | E x. 1 2 4 | 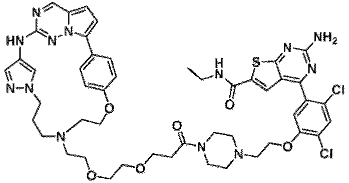 | 1 0 1 4 |
| E x. 1 2 5 | 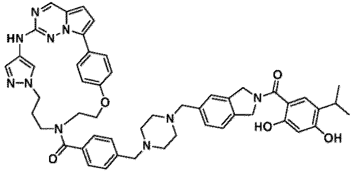 | 8 8 8 | E x. 1 2 6 | 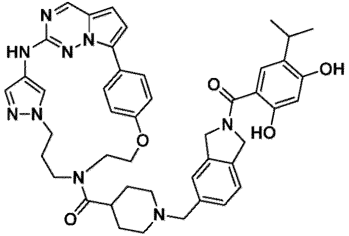 | 7 9 6 |

Meanwhile, the novel compounds represented by the Chemical Formula 1 according to the present invention can be formulated into various forms depending on the purpose. The following are examples of some formulation methods containing the compounds represented by Chemical Formula 1 as active ingredients according to the present invention, but it should be understood that the present invention is not limited thereto.

### [Formulation Example]

### Formulation Example 1 : Tablet (direct compression)

A mixture of 5.0 mg of the active ingredient, 14.1 mg of lactose, 0.8 mg of crospovidone USNF, and 0.1 mg of magnesium stearate was compressed to make a tablet.

### Formulation Example 2 : Tablet (wet graunlation)

The active ingredient (5.0 mg) was sieved, then mixed with lactose (16.0 mg) and starch (4.0 mg). Polysorbate 80 (0.3 mg) was dissolved in distilled water, and an appropriate amount of this solution was added. The mixture was granulated, dried, and then sifted. Colloidal silicon dioxide (2.7 mg) and magnesium stearate (2.0 mg) were added and mixed with the granules. The granules were compressed to make tablets.

### Formulation Example 3 : Powder and Capsules

The active ingredient (5.0 mg) was sieved and mixed with lactose (14.8 mg), polyvinyl pyrrolidone (10.0 mg), and magnesium stearate (0.2 mg). The mixture was then filled into hard No. 5 gelatin capsules using a suitable device.

### Formulation Example 4 : Injectable

An injectable formulation was prepared containing 100 mg of the active ingredient, along with 180 mg of mannitol, 26 mg of Na2HPO4.12H2O, and 2974 mg of distilled water.

### [Experimental Example]

### Experimental Example 1. Enzyme activity measurement in cancer cell

Seeding 3000 cells per well in a 96-well plate. After cell stabilization, treat the cells with compounds diluted in 0.5% DMSO at a 1/3 serial dilution and culture for 72 hours. Following treatment with CellTiter-Glo, luminescence is produced only in the presence of ATP, as luciferase degrades luciferin. Luminescence values were measured using Envision (PerkinElmer). Based on the measured luminescence values, the concentration of compounds causing cell death was calculated to determine the GI50 value.

The experimental results were as shown in Table 1 below. The activity values were categorized into three levels
A: IC₅₀ < 1 µM
B: 1 µM ≤ IC₅₀ < 10 µM,
C: IC₅₀ ≥ 10 µM

**TABLE 1**

| GI₅₀s (µM) | | | | | | |
|---|---|---|---|---|---|---|
| | HCT116 | colo205 | MDA-MB-231 | H1975 | H23 | H358 |
| Example 2 | A | B | C | B | A | A |
| Example 3 | A | B | B | B | B | B |
| Example 7 | B | C | C | B | B | B |
| Example 9 | A | B | C | B | B | A |
| Example 17 | B | C | C | B | B | C |
| Example 20 | C | C | C | C | C | C |
| Example 23 | B | B | C | B | A | B |
| Example 24 | B | B | C | B | B | B |
| Example 25 | A | C | C | C | B | C |
| Example 26 | A | A | C | A | A | A |
| Example 27 | C | C | B | C | B | B |
| Example 28 | B | B | C | B | B | B |
| Example 29 | B | B | C | B | B | B |
| Example 30 | B | B | B | B | B | B |
| Example 34 | C | C | B | C | C | C |
| Example 35 | B | B | C | B | B | B |
| Example 36 | A | B | C | B | A | B |
| Example 37 | B | C | C | C | B | C |
| Example 38 | B | C | C | C | C | B |
| Example 39 | C | C | C | C | C | C |
| Example 40 | B | C | C | C | C | C |
| Example 41 | C | C | C | C | C | C |
| Example 42 | C | C | C | C | C | C |
| Example 83 | B | C | C | B | B | B |
| Example 84 | B | C | C | B | B | B |

### Experimental Example 2. Enzyme activity measurement in Ba/F3 cell

Seeding 3000 cells per well in a 96-well plate. After cell stabilization, treat the cells with compounds diluted in 0.5% DMSO at a 1/3 serial dilution and culture for 72 hours. Following treatment with CellTiter-Glo, luminescence is produced only in the presence of ATP, as luciferase degrades luciferin. Luminescence values were measured using Envision (PerkinElmer). Based on the measured luminescence values, the concentration of compounds causing cell death was calculated to determine the GI50 value.

The experimental results were as shown in Table 2 below. The activity values were categorized into three levels.
A: IC₅₀ < 1 µM
B: 1 µM ≤ IC₅₀ < 10 µM,
C: IC₅₀ ≥ 10 µM

**TABLE 2**

| Ba/F3 GI₅₀s (µM) | | | | |
|---|---|---|---|---|
| | FLT3 (ITD/D835YF691L) | FLT3 (ITD) | FLT3 (ITD/D835Y) | parental |
| Example 2 | A | A | A | B |
| Example 3 | A | A | A | B |
| Example 7 | B | A | A | C |
| Example 9 | A | A | A | B |
| Example 17 | B | B | B | C |
| Example 20 | C | B | B | C |
| Example 23 | B | A | A | C |
| Example 24 | C | B | B | B |
| Example 25 | B | A | A | C |
| Example 26 | A | A | A | C |
| Example 27 | A | A | A | B |
| Example 28 | B | A | A | A |
| Example 29 | B | A | B | C |
| Example 30 | A | A | A | C |
| Example 34 | B | B | B | C |
| Example 35 | A | A | A | B |
| Example 36 | A | A | A | B |
| Example 37 | B | A | A | C |
| Example 38 | B | A | A | C |
| Example 39 | C | C | C | C |
| Example 40 | A | A | A | B |
| Example 41 | A | A | B | B |
| Example 42 | B | A | B | B |
| Example 83 | C | A | A | B |
| Example 84 | C | B | A | B |

### Experimental Example 3. Target protein degradation activity measurement in cancer cells.

1×10⁶ cells per well were seeded into each well of a 6-well plate. The cells were treated with compounds at various concentrations and incubated for 24 hours. After cell lysis, protein quantification was performed using the BCA assay, and samples were prepared for Western blot by adding Laemmli sample buffer (reducing) and boiling at 99° C for 5 minutes.

Samples were loaded onto an acrylamide gel for electrophoresis, and proteins were transferred onto a membrane via transfer. To prevent nonspecific binding between antibodies and proteins, the membrane was incubated with 5% skim milk for 30 minutes with agitation. The membrane was then incubated with a primary antibody solution diluted 1:1000 in solution for 10 hours at 4° C with agitation. Unbound primary antibodies were removed by washing the membrane with TBS/T buffer for 5 minutes three times. The membrane was then incubated with a secondary antibody solution diluted 1:10000 with HRP (horse radish peroxidase) at room temperature for 1 hour with agitation. Unbound secondary antibodies were removed by washing the membrane with TBS/T buffer for 5 minutes three times. Chemiluminescence was measured using an ECL solution, and the signal was detected using LAS.

The experimental results are shown in Figure 1.

## Claims

1. A 2,7-substituted pyrrolo[2,1-f][1,2,4]triazine compound represented by Chemical Formula 1, a pharmaceutically acceptable salt thereof, a hydrate thereof, or a solvate thereof: 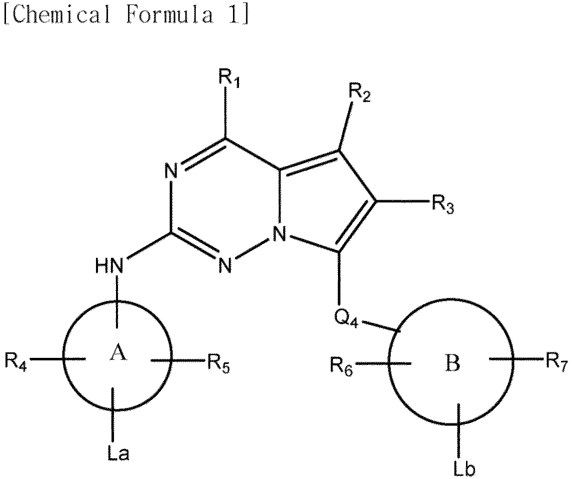 wherein
R₁, R₂, and R₃ each independently represent hydrogen; hydroxy; halogen; C₁-C₁₃ alkyl; C₁-C₆ alkoxy; C₃-C₁₀ cyclyl; C₃-C₁₀ heterocyclyl; -C(O)-(C₁-C₁₃ alkyl); amino(-NR₈R₉); nitro(-N(O)₂); amide(-(C=O)NR₈R₉); ester(-C(O)OR₈); carboxylic acid(-C(O)OH); nitrile(-CN); sulfonamide(-NHS(O)₂R₈); sulfide(-SR₈); sulfonyl(-S(O)₂R₈);or phosphinyl(-P(O)R₃R₉);
A and B each independently represent C₆-C₁₀ aryl; C₃-C₁₀ cyclyl; C₃-C₁₀ heteroaryl; or C₃-C₁₀ heterocyclyl;
R₄, R₅, R₆, and R₇ each independently represent hydrogen; hydroxy; halogen; C₁-C₁₃ alkyl; C₁-C₆ alkoxy; C₃-C₁₀ cyclyl; C₃-C₁₀ heterocyclyl; -C(O)-(C₁-C₁₃ alkyl); amino(-NR₃R₉); nitro(-N(O)₂); amide(-(C=O)NR₈R₉); ester(-C(O)OR₈); carboxylic acid(-C(O)OH); nitrile(-CN); sulfonamide(-NHS(O)₂R₈); sulfide(-SR₈); sulfonyl(-S(O)₂R₈);or phosphinyl(-P(O)R₈R₉);
Lₐ represents hydrogen; hydroxy; halogen; C₁-C₁₃ alkyl; -Zₐ-Q₃-E; or - Zₐ-Q₁-M, wherein in -Zₐ-Q₁-M, when L_{b} is -Z_{b}-Q₂, M is connected to Q₂, therby forming a ring;
L_{b} represents hydrogen; hydroxy; halogen; C₁-C₁₃ alkyl; C₁-C₆ alkoxy; C₃-C₁₀ cyclyl; C₃-C₁₀ heterocyclyl; -C(O)-(C₁-C₁₃ alkyl); amino(-NR₈R₉); nitro(-N(O)₂); amide(-(C=O)NR₈R₉): ester(-C(O)OR₈); carboxylic acid(-C(O)OH); nitrile(-CN); sulfonamide(-NHS(O)₂R₈); sulfide(-SR₈); sulfonyl(-S(O)₂R₈);or-Z_{b}-Q₂, wherein in -Z_{b}-Q₂, when Lₐ is -Zₐ-Q₁-M, Q₂ is connected to M, therby forming a ring;
Zₐ or Z_{b} may each independently be present or absent, and if Zₐ or Z_{b} is present, Zₐ or Z_{b} each independently represents -O-, -CO-, -COO-, -CₙHₙ₊₂-, -O(CₙHₙ₊₂)-, -(CₙHₙ₊₂)O-, -(CₙHₙ₊₂)O(CₘHₘ₊₂)-, -C₃-C₁₀ cyclyl-; or -C₃-C₁₀ heterocyclyl;
Q₁, Q₂, and Q₃ each independently represent -CₙHₙ₊₂-, -O(CₙHₙ₊₂)-, - (OC₂H₄)ₙ-, -(C₂H₄O)ₙ-, -(CₙHₙ₊₂)O-,-(CₙHₙ₊₂)CO-, -NR₈(CₙHₙ₊₂)-, -(NR₈C₂H₄)ₙ-, - (C₂H₄NR₈)ₙ-, -(CₙHₙ₊₂)NR₈-, -(CₙHₙ₊₂)O(CₘHₘ₊₂)-, -(CₙHₙ₊₂)O(CₘHₘ₊₂)CO-, -C₃-C₁₀ cyclyl-; or -C₃-C₁₀ heterocyclyl-;
Q₄ may be present or absent, and if present, Q₄ represents -CₙHₙ₊₂-, - CₙHₙ-, or -CₘHₙ₋₂- ;
M represents -O-, -CO-, -COO-, -C₂H₂-, -CₙHₙ₊₂-, -O(CₙHₙ₊₂)-, -(CₙHₙ₊₂)O-, -(CₙHₙ₊₂)O(CₘHₘ₊₂)-, -NR₈-; -NR₈CH₂-; -NHR₈-; -NR₈C(O)-; -NR₈C(O)O-; -C(O)NR₈-; -NR₈C(O)NR₈-; -NC(O)R₈-; -NS(O)₂R₈-; -S(O)₂-; -NR₈S(O)₂-; and -S(O)₂NR₈- ;
n and m each independently represent an integer from 0 to 15;
E represents 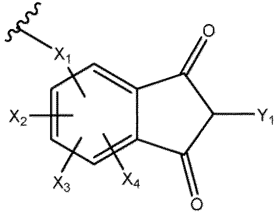 or 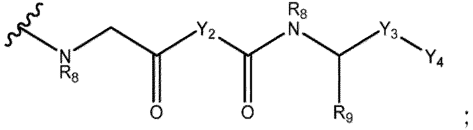
X₂, X₃, and X₄ each independently represent hydrogen; hydroxy; halogen; C₁-C₁₃ alkyl; C₁-C₆ alkoxy; C₃-C₁₀ cyclyl; C₃-C₁₀ heterocyclyl; -C(O)-(C₁-C₁₃ alkyl); amino(-NR₃R₉); nitro(-N(O)₂); amide(-(C=O)NR₃R₉); ester(-C(O)OR₈); carboxylic acid(-C(O)OH); nitrile(-CN); sulfonamide(-NHS(O)₂R₈); sulfide(-SR₈); sulfonyl(-S(O)₂R₈);or phosphinyl(-P(O)R₈R₉);
X₁ may be present or absent, and if present, X₁ represents -CR₈R₉-, - O- or -NR₈-;
Y₁, Y₂, Y₃, and Y₄ each independently represent C₆-C₁₀ aryl; C₃-C₁₀ heteroaryl; C₃-C₁₀ cyclyl; or C₃-C₁₀ heterocyclyl;
R₈ and R₉ each independently represent hydrogen; C₁-C₆ alkyl; C₁-C₆ alkenyl; C₁-C₆ alkynyl; C₆-C₁₀ aryl; C₃-C₁₀ heteroaryl; C₃-C₁₀ cyclyl; C₃-C₁₀ heterocyclyl; or R₈ may arbitrarily include at least one of N, O, S, NH, C=N, C=O, -NHC(O)-, -NHC(O)NH-, -NHS(O)₂-, or SO₂, along with a nitrogen or carbon atom connected to R₉, and form a 3 to 7 membered saturated ring arbitrarily substituted with at least one of hydrogen, C₁-C₁₃ alkyl, C₆-C₁₀ aryl, C₃-C₁₀ heteroaryl, hydroxyl, halide, and cyano groups;
C₁-C₆ alkoxy, C₁-C₁₃ alkyl, or C₃-C₁₀ cyclyl includes 1 or more substituent selected from the group consisting of hydrogen; hydroxy; halogen; C₁-C₁₃ alkyl; C₁-C₆ alkoxy; amino(-NR₈R₉); nitro(-N(O)₂); amide(-(C=O)NR₈R₉); carboxylic acid(-C(O)OH); ester(-C(O)OR₈); nitrile(-CN); urea(-NR₈(C=O)NR₉-); sulfonamide(-NHS(O)₂-); sulfide(-S-); sulfonyl(-S(O)₂-); phosphinyl(-P(O)R₈R₉); C₆-C₁₀ aryl; C₃-C₁₀ heteroaryl; and C₃-C₁₀ heterocyclyl;
C₆-C₁₀ aryl, C₃-C₁₀ heteroaryl, or C₃-C₁₀ heterocyclyl includes 1 or more substituent selected from the group consisting of hydrogen; hydroxy; halogen; carbonyl(-(C=O)R₈R₉); halogen, or C₁-C₃ alkyl either substituted with C₃-C₁₀ heterocyclyl or unsubstituted; halogen or C₁-C₃ alkoxy either substituted or unsubstituted with C₃-C₁₀ heterocyclyl; C₆-C₁₀ phenoxy; amino(-NR₈R₉); amide(-(C=O)NR₈R₉); C₆-C₁₀ aryl; C₃-C₁₀ heteroaryl and C₃-C₁₀ heterocyclyl;
C₃-C₁₀ heteroaryl and C₃-C₁₀ heterocyclyl include 1 or more heteroatoms selected from the group consisting of N, O, or S.

2. The 2,7-substituted pyrrolo[2,1-f][1,2,4]triazine compound represented by Chemical Formula 1 according to claim 1, a pharmaceutically acceptable salt thereof, a hydrate thereof, or a solvate thereof, wherein A and B each independently represent furan, benzofuran, pyrrole, indole, thiophene, benzothiophene, imidazole, benzimidazole, purine, pyrazole, indazole, oxazole, benzoxazole, thiazole, benzisoxazole, benzene, naphthalene, anthracene, pyridine, quinoline, pyrazine, quinoxaline, acridine, pyrimidine, quinazoline, pyridazine, quinoline, phthalazine, or triazine.

3. The 2,7-substituted pyrrolo[2,1-f][1,2,4]triazine compound represented by Chemical Formula 1 according to claim 1, a pharmaceutically acceptable salt thereof, a hydrate thereof, or a solvate thereof, wherein A and B each independently represent benzene, pyrazole, pyridine, indazole, pyrimidine, quinoline, or thiazole, and wherein R1, R2, and R3 each independently represent hydrogen, halogen, C1-C13 alkyl, or C3-C10 cycloalkyl.

4. The 2,7-substituted pyrrolo[2,1-f][1,2,4]triazine compound represented by Chemical Formula 1 according to claim 1, a pharmaceutically acceptable salt thereof, a hydrate thereof, or a solvate thereof, wherein R4, R5, R6, and R7 each independently represent hydrogen; hydroxy; halogen; -NHCH3; -NHMs; -CO(NH)CH3; -OCH3; -OC2H5; or -OC6H12COOCH3.

5. The 2,7-substituted pyrrolo[2,1-f][1,2,4]triazine compound of claim 1, wherein the compound of Chemical Formula 1 has a structure represented by Chemical Formula 2, a pharmaceutically acceptable salt thereof, a hydrate thereof, or a solvate thereof: 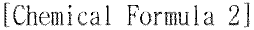 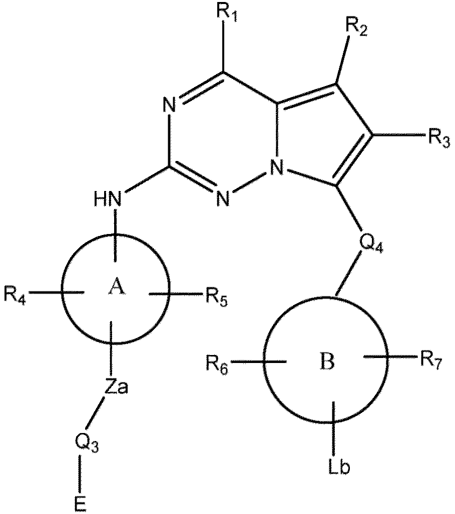 wherein
R₁, R₂, and R₃ each independently represent hydrogen; hydroxy; halogen; C₁-C₁₃ alkyl; C₁-C₆ alkoxy; C₃-C₁₀ cyclyl; C₃-C₁₀ heterocyclyl; -C(O)-(C₁-C₁₃ alkyl); amino(-NR₈R₉); nitro(-N(O)₂); amide(-(C=O)NR₈R₉); ester(-C(O)OR₈); carboxylic acid(-C(O)OH); nitrile(-CN); sulfonamide(-NHS(O)₂R₈); sulfide(-SR₈); sulfonyl(-S(O)₂R₈);or phosphinyl(-P(O)R₈R₉);
A and B each independently represent C₆-C₁₀ aryl; C₃-C₁₀ cyclyl; C₃-C₁₀ heteroaryl; or C₃-C₁₀ heterocyclyl;
R₄, R₅, R₆, and R₇ each independently represent hydrogen; hydroxy; halogen; C₁-C₁₃ alkyl; C₁-C₆ alkoxy; C₃-C₁₀ cyclyl; C₃-C₁₀ heterocyclyl; -C(O)-(C₁-C₁₃ alkyl); amino(-NR₃R₉); nitro(-N(O)₂); amide(-(C=O)NR₈R₉); ester(-C(O)OR₈); carboxylic acid(-C(O)OH); nitrile(-CN); sulfonamide(-NHS(O)₂R₈); sulfide(-SR₈); sulfonyl(-S(O)₂R₈);or phosphinyl(-P(O)R₈R₉);
L_{b} represents hydrogen; hydroxy; halogen; C₁-C₁₃ alkyl; C₁-C₆ alkoxy; C₃-C₁₀ cyclyl; C₃-C₁₀ heterocyclyl; -C(O)-(C₁-C₁₃ alkyl); amino(-NR₈R₉); nitro(-N(O)₂); amide(-(C=O)NR₈R₉); carboxylic acid(-C(O)OH); ester(-C(O)OR₈); nitrile(-CN); sulfonamide(-NHS(O)₂R₈); sulfide(-SR₈); or sulfonyl(-S(O)₂R₈);
Zₐ may be present or absent, and if present, Zₐ represents -O-, -CO-, -COO-, -CₙHₙ₊₂-, -O(CₙHₙ₊₂)-, -(CₙHₙ₊₂)O-, -(CₙHₙ₊₂)O(CₘHₘ₊₂)-, -C₃-C₁₀ cyclyl-; or -C₃-C₁₀ heterocyclyl;
Q₃ represents -CₙHₙ₊₂-, -O(CₙHₙ₊₂)-, -(OC₂H₄)ₙ-, -(C₂H₄O)ₙ-, -(CₙHₙ₊₂)O-,-(CₙHₙ₊₂)CO-, -NR₈(CₙHₙ₊₂)-, -(NR₈C₂H₄)ₙ-, -(C₂H₄NR₈)ₙ-, -(CₙHₙ₊₂)NR₈-, - (CₙHₙ₊₂)O(CₘHₘ₊₂)-, -(CₙHₙ₊₂)O(CₘHₘ₊₂)CO-, -C₃-C₁₀ cyclyl-; or -C₃-C₁₀ heterocyclyl-;
Q₄ may be present or absent, and if present, it represents -CₙHₙ₊₂-, - CₙHₙ-, or -CₙHₙ₋₂-;
n and m each independently represent an integer from 0 to 15,
E represents 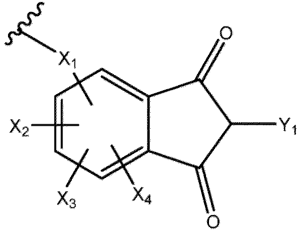 or 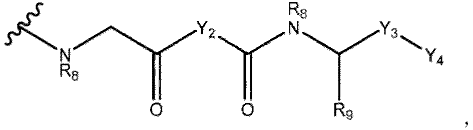
X₂, X₃, and X₄ each independently represent hydrogen; hydroxy; halogen; C₁-C₁₃ alkyl; C₁-C₆ alkoxy; C₃-C₁₀ cyclyl; C₃-C₁₀ heterocyclyl; -C(O)-(C₁-C₁₃ alkyl); amino(-NR₈R₉); nitro(-N(O)₂); amide(-(C=O)NR₈R₉); ester(-C(O)OR₈); carboxylic acid(-C(O)OH); nitrile(-CN); sulfonamide(-NHS(O)₂R₈); sulfide(-SR₈); sulfonyl(-S(O)₂R₈);or phosphinyl(-P(O)R₈R₉);
X₁ may be present or absent, and if present, X₁ represents -CR₈R₉-, - O- or -NR₈-;
Y₁, Y₂, Y₃, and Y₄ each independently represent C₆-C₁₀ aryl; C₃-C₁₀ heteroaryl; C₃-C₁₀ cyclyl; or C₃-C₁₀ heterocyclyl;
R₈ and R₉ each independently represent hydrogen; C₁-C₆ alkyl; C₁-C₆ alkenyl; C₁-C₆ alkynyl; C₆-C₁₀ aryl; C₃-C₁₀ heteroaryl; C₃-C₁₀ cyclyl; C₃-C₁₀ heterocyclyl; or R₈ may arbitrarily include at least one of N, O, S, NH, C=N, C=O, -NHC(O)-, -NHC(O)NH-, -NHS(O)₂-, or SO₂, along with a nitrogen or carbon atom connected to R₉, and form a 3 to 7 membered saturated ring arbitrarily substituted with at least one of hydrogen, C₁-C₁₃ alkyl, C₆-C₁₀ aryl, C₃-C₁₀ heteroaryl, hydroxyl, halide, and cyano groups;
C₁-C₆ alkoxy, C₁-C₁₃ alkyl, or C₃-C₁₀ cyclyl includes 1 or more substituent selected from the group consisting of hydrogen; hydroxy; halogen; C₁-C₁₃ alkyl; C₁-C₆ alkoxy; amino(-NR₃R₉); nitro(-N(O)₂); amide(-(C=O)NR₈R₉); carboxylic acid(-C(O)OH); ester(-C(O)OR₈); nitrile(-CN); urea(-NR₃(C=O)NR₉-); sulfonamide(-NHS(O)₂-); sulfide(-S-); sulfonyl(-S(O)₂-); phosphinyl(-P(O)R₈R₉); C₆-C₁₀ aryl; C₃-C₁₀ heteroaryl; and C₃-C₁₀ heterocyclyl;
C₆-C₁₀ aryl, C₃-C₁₀ heteroaryl, or C₃-C₁₀ heterocyclyl includes 1 or more substituent selected from the group consisting of hydrogen; hydroxy; halogen; carbonyl(-(C=O)R₈R₉); halogen, or C₁-C₃ alkyl either substituted with C₃-C₁₀ heterocyclyl or unsubstituted; halogen or C₁-C₃ alkoxy either substituted or unsubstituted with C₃-C₁₀ heterocyclyl; C₆-C₁₀ phenoxy; amino(-NR₈R₉); amide(-(C=O)NR₈R₉); C₆-C₁₀ aryl; C₃-C₁₀ heteroaryl and C₃-C₁₀ heterocyclyl;
C₃-C₁₀ heteroaryl and C₃-C₁₀ heterocyclyl include 1 or more heteroatoms selected from the group consisting of N, O, or S.

6. The 2,7-substituted pyrrolo[2,1-f][1,2,4]triazine compound of Chemical Formula 1, having a structure represented by Chemical Formula 2 according to claim 5, a pharmaceutically acceptable salt thereof, a hydrate thereof, or a solvate thereof, wherein A and B each independently represent benzene, pyrazole, pyridine, indazole, pyrimidine, quinoline, or thiazole,
R₁, R₂, and R₃ each independently represent hydrogen, halogen, C₁-C₁₃ alkyl or C₃-C₁₀ cyclyl,
R₄, R₅, R₆, and R₇ each independently represent hydrogen; hydroxy; halogen: -NHCH₃; -NHMs; -CO(NH)CH₃; -OCH₃; -OC₂H₅; or -OC₆H₁₂COOCH₃;
Y₁ represents 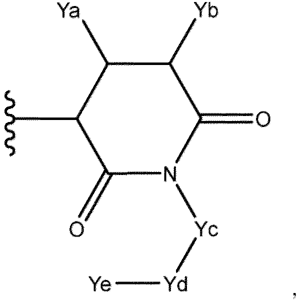
Yₐ and Y_{b} each independently represent hydrogen; halogen; C₁-C₁₃ alkyl; C₁-C₆ haloalkyl; C₁-C₆ alkoxy; C₃-C₁₀ cyclyl; C₃-C₁₀ heterocyclyl; -C(O)-(C₁-C₁₃ alkyl); amino(-NR₃R₉); nitro(-N(O)₂); amide(-(C=O)NR₈R₉); carboxylic acid(-C(O)OH); ester(-C(O)OR₈); nitrile(-CN); sulfonamide(-NHS(O)₂R₈); sulfide(-SR₈); sulfonyl(-S(O)₂R₈); or phosphinyl(-P(O)R₈R₉);
Y_{c} and Y_{d} each independently represent -O-, -CO-, -COO-, -CₙHₙ₊₂-, - O(CₙHₙ₊₂)-, -(CₙHₙ₊₂)O-, -(CₙHₙ₊₂)O(CₘHₘ₊₂)-, -C₃-C₁₀ cyclyl-; or -C₃-C₁₀ heterocyclyl-;
Yₑ represents C₆-C₁₀; C₃-C₁₀ cyclyl; C₃-C₁₀ heteroaryl; or C₃-C₁₀ heterocyclyl.

7. The 2,7-substituted pyrrolo[2,1-f][1,2,4]triazine compound of Chemical Formula 1, having a structure represented by Chemical Formula 2 according to claim 6, a pharmaceutically acceptable salt thereof, a hydrate thereof, or a solvate thereof, wherein E represents E₁, E₂, E₃, E₄, E₅, E₆, E₇, E₈, E₉, E₁₀, E₁₁, E₁₂, E₁₃, E₁₄ or E₁₅.

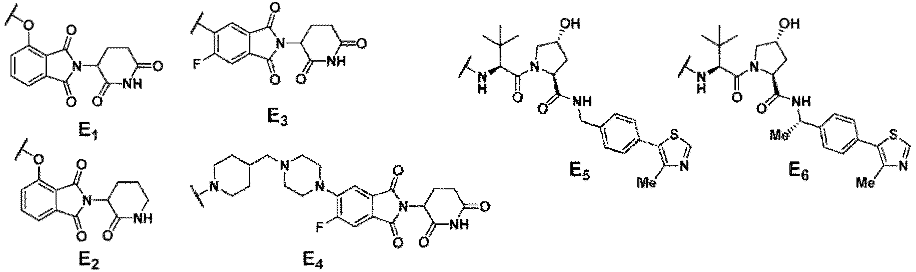

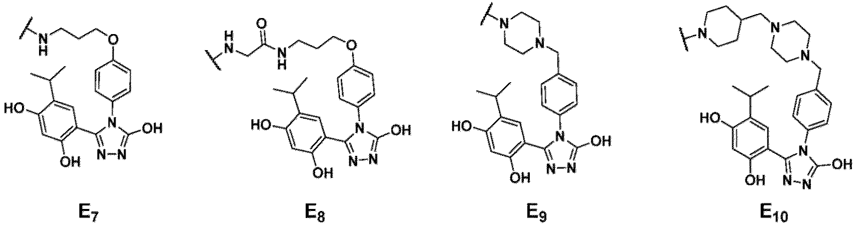

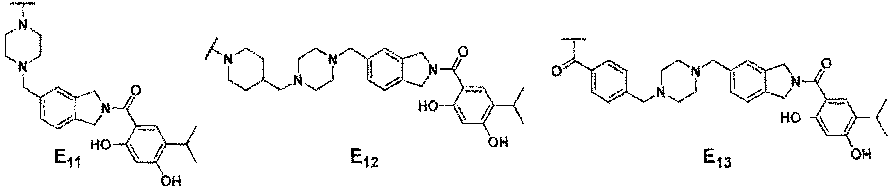

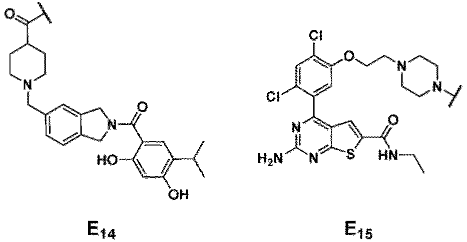

8. The 2,7-substituted pyrrolo[2,1-f][1,2,4]triazine compound of claim 1, wherein the compound of Chemical Formula 1 has a structure represented by Chemical Formula 3, a pharmaceutically acceptable salt thereof, a hydrate thereof, or a solvate thereof: 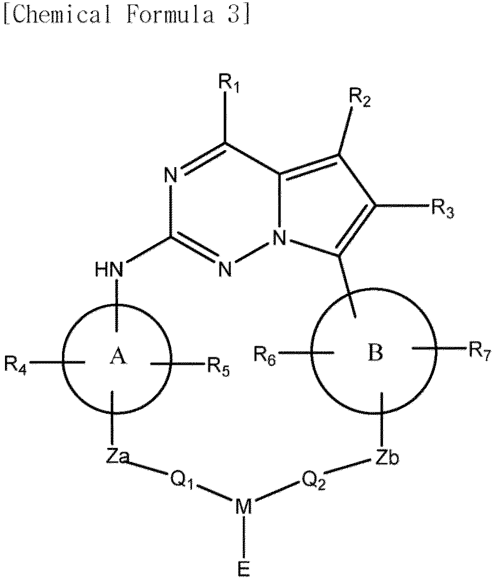 wherein
R₁, R₂, and R₃ each independently represent hydrogen; hydroxy; halogen; C₁-C₁₃ alkyl; C₁-C₆ alkoxy; C₃-C₁₀ cyclyl; C₃-C₁₀ heterocyclyl; -C(O)-(C₁-C₁₃ alkyl); amino(-NR₈R₉); nitro(-N(O)₂); amide(-(C=O)NR₈R₉); ester(-C(O)OR₈); carboxylic acid(-C(O)OH); nitrile(-CN); sulfonamide(-NHS(O)₂R₈); sulfide(-SR₈); sulfonyl(-S(O)₂R₈); or phosphinyl(-P(O)R₈R₉);
A and B each independently represent C₆-C₁₀ aryl; C₃-C₁₀ cyclyl; C₃-C₁₀ heteroaryl; or C₃-C₁₀ heterocyclyl;
R₄, R₅, R₆, and R₇ each independently represent hydrogen; hydroxy; halogen; C₁-C₁₃ alkyl; C₁-C₆ haloalkyl; C₁-C₆ alkoxy; C₃-C₁₀ cyclyl; C₃-C₁₀ heterocyclyl; -C(O)-(C₁-C₁₃ alkyl); amino(-NR₈R₉); nitro(-N(O)₂); amide(-(C=O)NR₈R₉); carboxylic acid(-C(O)OH); ester(-C(O)OR₈); nitrile(-CN); sulfonamide(-NHS(O)₂R₈); sulfide(-SR₈): sulfonyl(-S(O)₂R₈); or phosphinyl(-P(O)R₈R₉);
Zₐ or Z_{b} may each independently be present or absent, and if present, Zₐ or Z_{b} each independently represents -O-, -CO-, -COO-, -CₙHₙ₊₂-, -O(CₙHₙ₊₂)-, -(CₙHₙ₊₂)O-, -(CₙHₙ₊₂)O(CₘHₘ₊₂)-, -C₃-C₁₀ cyclyl-; or -C₃-C₁₀ heterocyclyl-;
Q₁ and Q₂ each independently represent -CₙHₙ₊₂-, -O(CₙHₙ₊₂)-, -(OC₂H₄)ₙ-, -(C₂H₄O)ₙ-, -(CₙHₙ₊₂)O-,-(CₙHₙ₊₂)CO-, -NR₈(CₙHₙ₊₂)-, -(NR₈C₂H₄)ₙ-, -(C₂H₄NR₈)ₙ-, - (CₙHₙ₊₂)NR₈-, -(CₙHₙ₊₂)O(CₘHₘ₊₂)-, -(CₙHₙ₊₂)O(CₘHₘ₊₂)CO-, -C₃-C₁₀ cyclyl-; or -C₃-C₁₀ heterocyclyl-;
M represents -O-, -CO-, -COO-, -C₂H₂-, -CₙHₙ₊₂-, -O(CₙHₙ₊₂)-, -(CₙHₙ₊₂)O-, -(CₙHₙ₊₂)O(CₘHₘ₊₂)-, -NR₈-; -NR₈CH₂-; -NHR₈-; -NR₈C(O)-; -NR₈C(O)O-; -C(O)NR₈-; -NR₈C(O)NR₈-; -NC(O)R₈-; -NS(O)₂R₈-; -S(O)₂-; -NR₈S(O)₂-; and -S(O)₂NR₈- ;
E represents 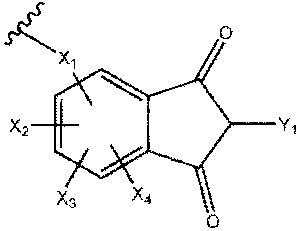 or 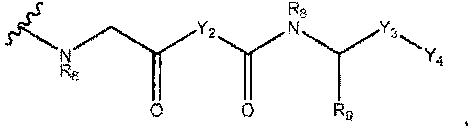
X₂, X₃, and X₄ each independently represent hydrogen; hydroxy; halogen; C₁-C₁₃ alkyl; C₁-C₆ alkoxy; C₃-C₁₀ cyclyl; C₃-C₁₀ heterocyclyl; -C(O)-(C₁-C₁₃ alkyl); amino(-NR₈R₉); nitro(-N(O)₂); amide(-(C=O)NR₈R₉); ester(-C(O)OR₈); carboxylic acid(-C(O)OH); nitrile(-CN); sulfonamide(-NHS(O)₂R₈); sulfide(-SR₈); sulfonyl(-S(O)₂R₈);or phosphinyl(-P(O)R₈R₉);
X₁ may be present or absent, and if present, X₁ represents -CR₈R₉-, - O- or -NR₈-;
Y₁, Y₂, Y₃, and Y₄ each independently represent C₆-C₁₀ aryl; C₃-C₁₀ heteroaryl; C₃-C₁₀ cyclyl; or C₃-C₁₀ heterocyclyl;
n and m each independently represent an integer from 0 to 15;
R₈ and R₉ each independently represent hydrogen; C₁-C₆ alkyl; C₁-C₆ alkenyl; C₁-C₆ alkynyl; C₆-C₁₀ aryl; C₃-C₁₀ heteroaryl; C₃-C₁₀ cyclyl; C₃-C₁₀ heterocyclyl; or R₈ may arbitrarily include at least one of N, O, S, NH, C=N, C=O, -NHC(O)-, -NHC(O)NH-, -NHS(O)₂-, or SO₂, along with a nitrogen or carbon atom connected to R₉, and form a 3 to 7 membered saturated ring arbitrarily substituted with at least one of hydrogen, C₁-C₁₃ alkyl, C₆-C₁₀ aryl, C₃-C₁₀ heteroaryl, hydroxyl, halide, and cyano groups;
C₁-C₆ alkoxy, C₁-C₁₃ alkyl, or C₃-C₁₀ cyclyl includes 1 or more substituent selected from the group consisting of hydrogen; hydroxy; halogen; C₁-C₁₃ alkyl; C₁-C₆ alkoxy; amino(-NR₈R₉); nitro(-N(O)₂); amide(-(C=O)NR₈R₉); carboxylic acid(-C(O)OH); ester(-C(O)OR₈); nitrile(-CN); urea(-NR₃(C=O)NR₉-); sulfonamide(-NHS(O)₂-); sulfide(-S-); sulfonyl(-S(O)₂-); phosphinyl(-P(O)R₈R₉); C₆-C₁₀ aryl; C₃-C₁₀ heteroaryl; and C₃-C₁₀ heterocyclyl;
C₆-C₁₀ aryl, C₃-C₁₀ heteroaryl, or C₃-C₁₀ heterocyclyl includes 1 or more substituent selected from the group consisting of hydrogen; hydroxy; halogen; carbonyl(-(C=O)R₈R₉); halogen, or C₁-C₃ alkyl either substituted with C₃-C₁₀ heterocyclyl or unsubstituted; halogen or C₁-C₃ alkoxy either substituted or unsubstituted with C₃-C₁₀ heterocyclyl; C₆-C₁₀ phenoxy; amino(-NR₈R₉); amide(-(C=O)NR₈R₉); C₆-C₁₀ aryl; C₃-C₁₀ heteroaryl and C₃-C₁₀ heterocyclyl;
C₃-C₁₀ heteroaryl and C₃-C₁₀ heterocyclyl include 1 or more heteroatoms selected from the group consisting of N, O, or S.

9. The 2,7-substituted pyrrolo[2,1-f][1,2,4]triazine compound of Chemical Formula 1, having a structure represented by Chemical Formula 3 according to claim 8, a pharmaceutically acceptable salt thereof, a hydrate thereof, or a solvate thereof, wherein A and B each independently represent benzene, pyrazole, pyridine, indazole, pyrimidine, quinoline, or thiazole,
R₁, R₂, and R₃ each independently represent hydrogen, halogen, C₁-C₁₃ alkyl or C₃-C₁₀ cyclyl,
R₄, R₅, R₆, and R₇ each independently represent hydrogen; hydroxy; halogen; -NHCH3; -NHMs; -CO(NH)CH₃; -OCH₃; -OC₂H₅; or -OC₆H₁₂COOCH₃.

10. The 2,7-substituted pyrrolo[2,1-f][1,2,4]triazine compound of Chemical Formula 1, having a structure represented by Chemical Formula 3 according to claim 8, a pharmaceutically acceptable salt thereof, a hydrate thereof, or a solvate thereof, wherein E represents E₁, E₂, E₃, E₄, E₅, E₆, E₇, E₈, E₉, E₁₀, E₁₁, E₁₂, E₁₃, E₁₄ or E₁₅.

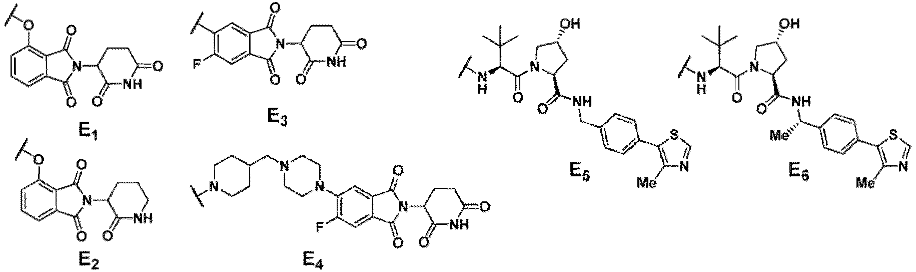

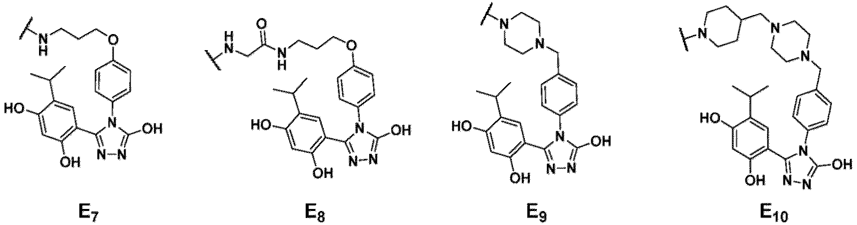

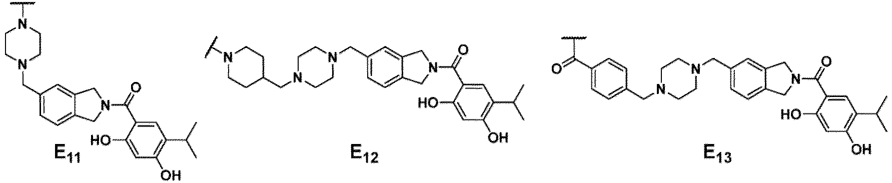

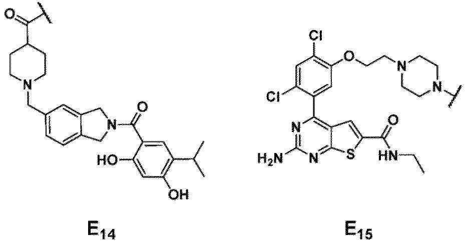

11. The 2,7-substituted pyrrolo[2,1-f][1,2,4]triazine compound represented by Chemical Formula 1 according to claim 1, a pharmaceutially acceptable salt thereof, a hydrate thereof, or a solvate thereof, wherein the compounds **characterized by** being selected from the group consisting of compounds numbered 1 to 126:
(Compound No. 1) (2¹Z,4⁴E)-4¹*H*-11-oxa-3,8-diaza-2(7,2)-pyrrolo[2,1-f][1,2,4]triazina- 4(4,1)-pyrazola-1(1,4)-benzenacycloundecaphane;
(Compound No. 2) ((2¹Z,4⁴E)-8-methyl-4¹*H*-11-oxa-3,8-diaza-2(7,2)-pyrrolo[2,1-f][1,2,4]triazina-4(4,1)-pyrazola-1(1,4)-benzenacycloundecaphane;
(Compound No. 3) 1-((2¹Z,4⁴E)-4¹*H*-11-oxa-3,8-diaza-2(7,2)-pyrrolo[2,1-f][1,2,4]triazina-4(4,1)-pyrazola-1(1,4)-benzenacycloundecafphane-8-yl)ethan-1-one;
(Compound No. 4) 1-((2¹Z,4⁴E)-4¹*H*-11-oxa-3,8-diaza-2(7,2)-pyrrolo[2,1-f][1,2,4]triazina-4(4,1)-pyrazola-1(1,4)-benzenacycloundecaphane-8-yl)butan-1-one;
(Compound No. 5) ((2¹Z,4⁴E)-4¹H-11-oxa-3,8-diaza-2(7,2)-pyrrolo[2,1-f][1,2,4]triazina -4(4,1)-pyrazola-1(1,4)-benzenacycloundecaphane-8-yl)(4-chlorophenyl)methanone;
(Compound No. 6) (2¹Z,4⁴E)-*N*-cyclohexyl-4¹*H*-11-oxa-3,8-diaza-2(7,2)-pyrrolo[2,1-f][1,2,4]triazina-4(4,1)-pyrazola-1(1,4)-benzenacycloundecaphane-8-carboxamide;
(Compound No. 7) (2¹Z,4⁴E)-*N*-(3-methoxyphenyl)-4¹*H*-11-oxa-3,8-diaza-2(7,2)-pyrrolo[2,1-f][1,2,4]triazina-4(4,1)-pyrazola-1(1,4)-benzenecycloundecaphane-8-carboxamide;
(Compound No. 8) (2¹Z,4⁴E)-*N*-ethyl-4¹*H*-11-oxa-3,8-diaza-2(7,2)-pyrrolo[2,1-f][1,2,4]triazina-4(4,1)-pyrazola-1(1,4)-benzenacycloundecaphane-8-carboxamide;
(Compound No. 9) (2¹Z,4⁴E)-8-(methylsulfonyl)-4¹*H*-11-oxa-3,8-diaza-2(7,2)-pyrrolo[2,1-f][1,2,4]triazina-4(4,1)-pyrazola-1(1,4)-benzenecycloundecaphane;
(Compound No. 10) (2¹Z,4⁴E)-8-((3,4-difluorophenyl)sulfonyl)-4¹*H*-11-oxa-3,8-diaza-2(7,2)-pyrrolo [2,1-f][1,2,4]triazina-4(4,1)-pyrazola-1(1,4)-benzenecycloundecaphane;
(Compound No. 11) (2¹Z,4⁴E)-8-(phenylsulfonyl)-4¹*H*-11-oxa-3,8-diaza-2(7,2)-pyrrolo[2,1-f][1,2,4]triazina-4(4,1)-pyrazola-1(1,4)-benzenacycloundecaphane;
(Compound No. 12) (2¹Z,4⁴E)-8-(cyclopropylsulfonyl)-4¹*H*-11-oxa-3,8-diaza-2(7,2)-pyrrolo[2,1-f][1,2,4]triazina-4(4,1)-pyrazola-1(1,4)-benzenacycloundecaphane;
(Compound No. 13) 2¹Z,4⁴E)-8-(propylsulfonyl)-4¹*H*-11-oxa-3,8-diaza-2(7,2)-pyrrolo[2,1-f][1,2,4]triazina-4(4,1)-pyrazola-1(1,4)-benzenacycloundecaphane;
(Compound No. 14) (2¹Z,4⁴E)-8-((5-chlorothiophen-2-yl)sulfonyl)-4¹*H-*11-oxa-3,8-diaza-2(7,2)-pyrrolo[2,1-f][1,2,4]triazina-4(4,1)-pyrazola-1(1,4)-benzenacycloundecaphane;
(Compound No. 15) (2¹Z,4⁴E)-8-(cyclohexylsulfonyl)-4¹*H*-11-oxa-3,8-diaza-2(7,2)-pyrrolo[2,1-f][1,2,4]triazina-4(4,1)-pyrazola-1(1,4)-benzenacycloundecaphane;
(Compound No. 16) (Z)-5-oxa-3,9,15-triaza-2(7,2)-pyrrolo[2,1-f][1,2,4]triazina-1, 4(1,4)-dibenzenacyclopentadecaphan-10-one;
(Compound No. 17) (1⁴Z,2¹Z)-1¹H-5-oxa-3,9-diaza-2(7,2)-pyrrolo[2,1-f][1,2,4]triazina- 1(4,1)-pyrazola-4(1,4)-benzenacyclohexadecaphan-10-one;
(Compound No. 18) (1⁴Z,2¹Z,4⁴E)-1¹*H*,4¹*H*-3,8-diaza-2(7,2)-pyrrolo[2,1-f][1,2,4]triazina-1,4(4,1)-dipyrazolacyclopentadecaphan-9-one;
Compound No. 19) (1⁴Z,2¹Z)-1¹*H*-3-aza-2(7,2)-pyrrolo[2,1-f][1,2,4]triazina-5(1,4)-piperazina-1(4,1)-pyrazola-4(1,4)-benzenacyclododecaphane-9-one;
(Compound No. 20) (Z)-13-fluoro-5,17-dioxa-3,9-diaza-2(7,2)-pyrrolo[2,1-f][1,2,4 Itriazina-1,4(1,4)-dibenzenacycloheptadecaphan-10-one;
(Compound No. 21) (2¹Z,4⁴E)-13-fluoro-4¹H-16-oxa-3,8-diaza-2(7,2)-pyrrolo[2,1-f][1,2,4]triazina-4(4,1)-pyrazola-1(1,4)-benzenacyclohexadecaphan-9-one;
(Compound No. 22) (Z)-1³-fluoro-13-oxa-3-aza-2(7,2)-pyrrolo[2,1-f][1,2,4]triazina-5(1,4)-piperazina-1,4(1,4)-dibenzenacyclotridecaphan-6-one;
(Compound No. 23) (Z)-17-oxa-3,9-diaza-2(7,2)-pyrrolo[2,1-f][1,2,4]triazina-5(1,4)-piperazina-1,4(1,4)-dibenzenacycloheptadecaphan-10-one;
(Compound No. 24) (Z)-13-oxa-3-aza-2(7,2)-pyrrolo[2,1-f][1,2,4]triazina-5(1,4)-piperazina-1,4(1,4)-dibenzenacyclotridecaphan-6-one;
(Compound No. 25) (2¹Z,4⁴E)-4¹*H*-11-oxa-3-aza-2(7,2)-pyrrolo[2,1-f][1,2,4]triazina-5(4,1)-piperidina-4(4,1)-pyrazola-1(1,4)-benzenacycloundecaphan-6-one;
(Compound No. 26) (2¹Z,4⁴E)-4¹*H*-15-oxa-3,9-diaza-2(7,2)-pyrrolo[2,1-f][1,2,4]triazina-5(4,1)-piperidina-4(4,1)-pyrazola-1(1,4)-benzenacyclopentadecaphan-10-one;
(Compound No. 27) (2¹Z,4⁴E)-4¹H-17-oxa-3,9-diaza-2(7,2)-pyrrolo[2,1-f][1,2,4]triazina-5(4,1)-piperidina-4(4,1)-pyrazola-1(1,4)-benzenacycloheptadecaphan-10-one;
(Compound No. 28) (Z)-5,12,15-trioxa-3,9-diaza-2(7,2)-pyrrolo[2,1-f][1,2,4]triazina -1,4(1,4)-dibenzenacyclopentadecaphan-10-one;
(Compound No. 29) (Z)-5,17-dioxa-3,9-diaza-2(7,2)-pyrrolo[2,1-f][1,2,4]triazina-1 ,4(1,4)-dibenzenacycloheptadecaphan-10-one;
(Compound No. 30) (Z)-5,12-dioxa-3,9-diaza-2(7,2)-pyrrolo[2,1-f][1,2,4]triazina-1 ,4(1,4)-dibenzenacyclododecaphane;
(Compound No. 31) (Z)-1-(5,12-dioxa-3,9-diaza-2(7,2)-pyrrolo[2,1-f][1,2,4]triazina-1,4(1,4)-dibenzenacyclododecapan-9-yl)propan-1-one:
(Compound No. 32) (Z)-5,12-dioxa-3,9-diaza-2(7,2)-pyrrolo[2,1-f][1,2,4]triazina-1,4(1,4)-dibenzenacyclododecaphan-9-yl(4-chlorophenyl)methanone;
(Compound No. 33) (2¹Z,7Z)-5,10-dioxa-3-aza-2(7,2)-pyrrolo[2,1-f][1,2,4]triazina-1,4(1,4)-dibenzenacyclodecaphan-7-ene;
(Compound No. 34) (Z)-42-methoxy-5,12,15-trioxa-3,9-diaza-2(7,2)-pyrrolo[2,1-f][1,2 ,4]triazina-1,4(1,4)-dibenzenacyclopentadecaphan-10-one;
(Compound No. 35) (2¹Z,4⁴E)-4¹*H*-11,14-dioxa-3,8-diaza-2(7,2)-pyrrolo[2,1-f][1,2,4]triazina-4(4,1)-pyrazola-1(1,4)-benzenacyclotetradecaphan-9-one;
(Compound No. 36): (2¹Z,4⁴E)-4¹H-14-oxa-3,8-diaza-2(7,2)-pyrrolo[2,1-f][1,2,4]triazina -4(4,1)-pyrazola-1(1,4)-benzenacyclotetradecaphan-9-one;
(Compound No. 37): (2¹Z,4⁴E)-4¹*H*-16-oxa-3,8-diaza-2(7,2)-pyrrolo[2,1-f][1,2,4]triazina-4(4,1)-pyrazola-1(1,4)-benzenacyclohexadecaphan-9-one;
(Compound No. 38): (Z)-5,15-dioxa-3,9-diaza-2(7,2)-pyrrolo[2,1-f][1,2,4]triazina- 1,4(1,4)-dibenzenacyclopentadecaphan-10-one;
(Compound No. 39) N-(3-(2-((4-(4-(5-((2-(2,6-dioxopiperidin-3-yl)-1,3-dioxoisoindolin-4-yl)oxy)pentyl)piperidin-1-yl)phenyl)amino)pyrrolo[2,1-f][1,2,4]triazin-7-yl)phenyl)methanesulfonamide;
(Compound No. 40) *N*-(3-(2-((4-(4-(3-((2-(2,6-dioxopiperidin-3-yl)-1,3-dioxoisoindolin-4-yl)oxy)propyl)piperidin-1-yl)phenyl)amino)pyrrolo[2,1-f][1,2,4]triazin-7-yl)phenyl)methanesulfonamide;
(Compound No. 41) *N*-(3-(2-((4-(4-(7-((2-(2,6-dioxopiperidin-3-yl)-1,3-dioxoisoindolin-4-yl)oxy)heptyl)piperidin-1-yl)phenyl)amino)pyrrolo[2,1-f][1,2,4]triazin-7-yl)phenyl)methanesulfonamide;
(Compound No. 42) *N*-(3-(2-((4-(4-(11-((2-(2,6-dioxopiperidin-3-yl)-1,3-dioxoisoindolin-4-yl)oxy)undecyl)piperidin-1-yl)phenyl)amino)pyrrolo [2,1-f][1,2,4]triazin-7-yl)phenyl)methanesulfonamide;
(Compound No. 43) *N*-(3-(2-((4-(4-(2-((2-(2,6-dioxopiperidin-3-yl)-1,3-dioxoisoindolin-4-yl)oxy)ethyl)piperidin-1-yl)phenyl)amino)pyrrolo[2,1-f][1,2,4]triazin-7-yl)phenyl)methanesulfonamide;
(Compound No. 44) *N*-(3-(2-((4-(4-(4-((2-(2,6-dioxopiperidin-3-yl)-1,3-dioxoisoindolin-4-yl)oxy)butyl)piperidin-1-yl)phenyl)amino)pyrrolo[2,1-f][1,2,4]triazin-7-yl)phenyl)methanesulfonamide;
(Compound No. 45) *N*-(3-(2-((4-(4-(6-((2-(2,6-dioxopiperidin-3-yl)-1,3-dioxoisoindolin-4-yl)oxy)hexyl)piperidin-1-yl)phenyl)amino)pyrrolo[2,1-f][1,2,4]triazin-7-yl)phenyl)methanesulfonamide;
(Compound No. 46) *N*-(3-(2-((4-(4-(8-((2-(2,6-dioxopiperidin-3-yl)-1,3-dioxoisoindolin-4-yl)oxy)octyl)piperidin-1-yl)phenyl)amino)pyrrolo[2,1-f][1,2,4]triazin-7-yl)phenyl)methanesulfonamide;
(Compound No. 47) *N*-(3-(2-((4-(4-(8-((2-(2,6-dioxopiperidin-3-yl)-1,3-dioxoisoindolin-4-yl)oxy)nonyl)piperidin-1-yl)phenyl)amino)pyrrolo[2,1-f][1,2,4]triazin-7-yl)phenyl)methanesulfonamide;
(Compound No. 48) *N*-(3-(2-((4-(4-(2-(2-((1,3-dioxo-2-(2-oxopiperidin-3-yl)isoindolin-4-yl)oxy)ethoxy)ethyl)piperazin-1-yl)phenyl)amino)pyrrolo[2,1-f][1,2,4]triazin-7-yl)phenyl) methanesulfonamide;
(Compound No. 49) *N*-(3-(2-((4-(4-(6-((1,3-dioxo-2-(2-oxopiperidin-3-yl)isoindolin-4-yl)oxy)hexyl)piperazin-1-yl)phenyl)amino)pyrrolo[2,1-f][1,2,4]triazin-7-yl)phenyl)methanesulfonamide;
(Compound No. 50) *N*-(3-(2-((4-(4-(8-((1,3-dioxo-2-(2-oxopiperidin-3-yl)isoindolin-4-yl)oxy)octyl)piperazin-1-yl)phenyl)amino)pyrrolo[2,1-f][1,2,4]triazin-7-yl)phenyl)methanesulfonamide;
(Compound No. 51) *N*-(3-(2-((4-(4-((1-(2-(2,6-dioxopiperidin-3-yl)-6-fluoro-1,3-dioxoisoindolin-5-yl)piperidin-4-yl)methyl)piperazin-1-yl)phenyl)amino)pyrrolo[2,1-f][1,2,4]trizine-7-yl)phenyl)methanesulfonamide;
(Compound No. 52) *N*-(3-(2-((1-(1-(9-((2-(2,6-dioxopiperidin-3-yl)-1,3-dioxoindolin-4-yl)oxy)nonyl)piperidin-4-yl)-1*H*-pyrazol-4-yl)amino)pyrrolo[2,1-f][1,2,4]triazin-7-yl)phenyl)methanesulfonamide;
(Compound No. 53) 3-(2-((1-(1-(9-((2-(2,6-dioxopiperidin-3-yl)-1,3-dioxoindolin-4-yl)oxy)nonyl)piperidin-4-yl)-1H-pyrazol-4-yl)amino)pyrrolo[2,1-f][1,2,4]triazin-7-yl)-*N*-methylbenzamide:
(Compound No. 54) 2-(2,6-dioxopiperidin-3-yl)-4-((9-(4-(4-((7-phenylpyrrolo[2,1-f][1,2,4]triazin-2-yl)amino)-1*H*-pyrazol-1-yl)piperidin-1-yl)nonyl)oxy)isoindolin-1,3-dione
(Compound No. 55) 2-(2,6-dioxopiperidin-3-yl)-4-((9-(4-(4-((7-(1-methyl-1*H*-pyrazole-4-1)pyrrolo[2,1-f][1,2,4]triazin-2-yl)amino)-1*H*-pyrazol-1-yl)piperidin-1-yl)nonyl)oxy)isoindolin-1,3-dione
(Compound No. 56) 4-((9-(4-(4-((7-(1*H*-indol-6-yl)pyrrolo[2,1-f][1,2,4]triazine-2-yl)amino)-1*H*-pyrazol-1-yl)piperidin-1-yl)nonyl)oxy)-2-(2,6-dioxopiperidin-3-yl)isoindolin-1,3-dione;
(Compound No. 57) 2-(2,6-dioxopiperidin-3-yl)-4-((9-(4-(4-((7-(4-hydroxyphenyl)pyrrolo[2,1-f][1,2,4]triazin-2-yl)amino)-1*H*-pyrazol-1-yl)piperidin-1-yl)nonyl)oxy)isoindolin-1,3-dione;
(Compound No. 58) Methyl-7-(4-(2-((1-(1-(9-((2-(2,6-dioxopiperidin-3-yl)-1,3-dioxoisoindolin-4-yl)oxy)nonyl)piperidin-4-yl)-1*H*-pyrazol-4-yl)amino)pyrrolo[2,1-f][1,2,4]triazine -7-day)phenoxy)heptanoate;
(Compound No. 59) *N*-(3-(2-((4-(4-(5-((2-(2,6-dioxopiperidin-3-yl)-1,3-dioxoisoindolin-4-yl)oxy)pentyl)piperazin-1-yl)-2-methoxyphenyl)amino)pyrrolo[2,1-f][1,2,4]triazin-7-yl)phenyl)methanesulfonamide;
(Compound No. 60) *N*-(3-(2-((4-(4-(3-((2-(2,6-dioxopiperidin-3-yl)-1,3-dioxoindolin-4-yl)oxy)propyl)piperazin-1-yl)-2-methoxyphenyl)amino)pyrrolo[2,1-f][1,2,4]triazin-7-yl)phenyl)methanesulfonamide;
(Compound No. 61) *N*-(3-(2-((4-(4-(7-((2-(2,6-dioxopiperidin-3-yl)-1,3-dioxoindolin-4-yl)oxy)heptyl)piperazin-1-yl)-2-methoxyphenyl)amino)pyrrolo[2,1-f][1,2,4]triazin-7-yl)phenyl)methanesulfonamide;
(Compound No. 62) *N*-(3-(2-((4-(4-(11-((2-(2,6-dioxopiperidin-3-yl)-1,3-dioxoindolin-4-yl)oxy)undecyl)piperazin-1-yl)-2-methoxyphenyl)amino)pyrrolo[2,1-f][1,2,4]triazin-7-yl)phenyl)methanesulfonamide;
(Compound No. 63) *N*-(3-(2-((4-(4-(2-((2-(2,6-dioxopiperidin-3-yl)-1,3-dioxoindolin-4-yl)oxy)ethyl)piperazin-1-yl)-2-methoxyphenyl)amino)pyrrolo[2,1-f][1,2,4]tri azin-7-yl)phenyl)methanesulfonamide;
(Compound No. 64) *N*-(3-(2-((4-(4-(4-((2-(2,6-dioxopiperidin-3-yl)-1,3-dioxoindolin-4-yl)oxy)butyl)piperazin-1-yl)-2-methoxyphenyl)amino)pyrrolo[2,1-f][1,2,4]triazin-7-yl)phenyl)methanesulfonamide;
(Compound No. 65) *N*-(3-(2-((4-(4-(6-((2-(2,6-dioxopiperidin-3-yl)-1,3-dioxoindolin-4-yl)oxy)hexyl)piperazin-1-yl)-2-methoxyphenyl)amino)pyrrolo[2,1-f][1,2,4]triazin-7-yl)phenyl)methanesulfonamide;
(Compound No. 66) *N*-(3-(2-((4-(4-(8-((2-(2,6-dioxopiperidin-3-yl)-1,3-dioxoindolin-4-yl)oxy)octyl)piperazin-1-yl)-2-methoxyphenyl)amino)pyrrolo[2,1-f][1,2,4]triazin-7-yl)phenyl)methanesulfonamide;
(Compound No. 67) *N*-(3-(2-((4-(4-(9-((2-(2,6-dioxopiperidin-3-yl)-1,3-dioxoindolin-4-yl)oxy)nonyl)piperazin-1-yl)-2-methoxyphenyl)amino)pyrrolo[2,1-f][1,2,4]triazin-7-yl)phenyl)methanesulfonamide;
(Compound No. 68) *N*-(3-(2-((4-(4-(2-(2-((1,3-dioxo-2-(2-oxopiperidin-3-yl)isoindolin-4-yl)oxy)ethoxy)ethyl)piperazin-1-yl)-2-methoxyphenyl)amino)pyrrolo[2,1-f][1,2,4]triazine-7-1)phenyl)methanesulfonamide;
(Compound No. 69) *N*-(3-(2-((4-(4-(6-((1,3-dioxo-2-(2-oxopiperidin-3-yl)isoindolin-4-yl)oxy)hexyl)piperazin-1-yl)-2-methoxyphenyl)amino)pyrrolo[2,1-f][1,2,4]triazin-7-yl)phenyl)methanesulfonamide;
(Compound No. 70) *N*-(3-(2-((4-(4-(8-((1,3-dioxo-2-(2-oxopiperidin-3-yl)isoindoline-4-yl)oxy)octyl)piperazin-1-yl)-2-methoxyphenyl)amino)pyrrolo[2,1-f][1,2,4]triazin-7-yl)phenyl)methanesulfonamide;
(Compound No. 71) *N*-(3-(2-((6-(4-(5-((2-(2,6-dioxopiperidin-3-yl)-1,3-dioxoindolin-4-yl)oxy)pentyl)piperazin-1-yl)pyridin-3-yl)amino)pyrrolo[2,1-f][1,2,4]triazin-7-yl)phenyl)methanesulfonamide:
(Compound No. 72) *N*-(3-(2-((6-(4-(3-((2-(2,6-dioxopiperidin-3-yl)-1,3-dioxoindoline-4-yl)oxy)propyl)piperazin-1-yl)pyridin-3-yl)amino)pyrrolo[2,1-f][1,2,4]triazin-7-yl)phenyl)methanesulfonamide:
(Compound No. 73) *N*-(3-(2-((6-(4-(7-((2-(2,6-dioxopiperidin-3-yl)-1,3-dioxoindolin-4-yl)oxy)heptyl)piperazin-1-yl)pyridin-3-yl)amino)pyrrolo[2,1-f][1,2,4]triazin-7-yl)phenyl)methanesulfonamide:
(Compound No. 74) *N*-(3-(2-((6-(4-(11-((2-(2,6-dioxopiperidin-3-yl)-1,3-dioxoindoline-4-yl)oxy)undecyl)piperazin-1-yl)pyridin-3-yl)amino)pyrrolo[2,1-f][1,2,4]triazin-7-yl)phenyl)methanesulfonamide;
(Compound No. 75) *N*-(3-(2-((6-(4-(2-((2-(2,6-dioxopiperidin-3-yl)-1,3-dioxoindolin-4-yl)oxy)ethyl)piperazin-1-yl)pyridin-3-yl)amino)pyrrolo[2,1-f][1,2,4]triazin-7-yl)phenyl)methanesulfonamide;
(Compound No. 76) *N*-(3-(2-((6-(4-(4-((2-(2,6-dioxopiperidin-3-yl)-1,3-dioxoindoline-4-yl)oxy)butyl)piperazin-1-yl)pyridin-3-yl)amino)pyrrolo[2,1-f][1,2,4]tri azin-7-yl)phenyl)methanesulfonamide;
(Compound No. 77) *N*-(3-(2-((6-(4-(6-((2-(2,6-dioxopiperidin-3-yl)-1,3-dioxoindolin-4-yl)oxy)hexyl)piperazin-1-yl)pyridin-3-yl)amino)pyrrolo[2,1-f][1,2,4]triazin-7-yl)phenyl)methanesulfonamide;
(Compound No. 78) *N*-(3-(2-((6-(4-(8-((2-(2,6-dioxopiperidin-3-yl)-1,3-dioxoindolin-4-yl)oxy)octyl)piperazin-1-yl)pyridin-3-yl)amino)pyrrolo[2,1-f][1,2,4]triazin-7-yl)phenyl)methanesulfonamide;
(Compound No. 79) *N*-(3-(2-((6-(4-(9-((2-(2,6-dioxopiperidin-3-yl)-1,3-dioxoindolin-4-yl)oxy)nonyl)piperazin-1-yl)pyridin-3-yl)amino)pyrrolo[2,1-f][1,2,4]triazin-7-yl)phenyl)methanesulfonamide;
(Compound No. 80) *N*-(3-(2-((6-(4-(2-(2-((1,3-dioxo-2-(2-oxopiperidin-3-yl)isoindolin-4-yl)oxy)ethoxy)ethyl)piperazin-1-yl)pyridin-3-yl)amino)pyrrolo[2,1-f][1,2,4]triazin-7-yl)phenyl)methanesulfonamide;
(Compound No. 81) *N*-(3-(2-((6-(4-(6-((1,3-dioxo-2-(2-oxopiperidin-3-yl)isoindolin-4-yl)oxy)hexyl)piperazin-1-yl)pyridin-3-yl)amino)pyrrolo[2,1-f][1,2,4]triazin-7-yl)phenyl)methanesulfonamide;
(Compound No. 82) *N*-(3-(2-((6-(4-(8-((1,3-dioxo-2-(2-oxopiperidin-3-yl)isoindolin-4-yl)oxy)octyl)piperazin-1-yl)pyridin-3-yl)amino)pyrrolo[2,1-f][1,2,4]triazin-7-yl)phenyl)methanesulfonamide;
(Compound No. 83) (2S,4R)-1-((S)-3,3-dimethyl-2-(2-(2-(4-(4-((7-(3-(methanesulfonamido)phenyl)pyrrolo[2,1-f][1,2,4]triazin-2-yl)amino)phenyl) piperazin-1-yl)ethoxy)acetaamino)butanoyl)-4-hydroxy-*N*-(4-(4-methylthiazol-5-yl)benzyl)pyrrolidin-2-carboxamide;
(Compound No. 84) (2S,4R)-1-((S)-3,3-dimethyl-2-(3-(2-(2-(4-(4-((7-(3-(methanesulfonamido)phenyl)pyrrolo[2,1-f][1,2,4]triazin-2-yl)amino) phenyl)piperazin-1-yl)ethoxy)ethoxy)propanamido)butanoyl)-4-hydroxy-N-(4-(4-methylthiazol-5-yl)benzyl)pyrrolidin-2-carboxamide;
(Compound No. 85) (2S,4R)-1-((S)-3,3-dimethyl-2-(3-(2-(2-(4-(4-((7-(3-(methylsulfonamido)phenyl)pyrrolo[2,1-f][1,2,4]triazin-2-yl)amino) phenyl)piperazin-1-yl)ethoxy)ethoxy)propanamido)butanoyl)-4-hydroxy-N-((S)-1-(4-(4-methylthiazol-5-yl)phenyl)ethyl)pyrrolidin-2-carboxamide;
(Compound No. 86) 2-(2,6-dioxopiperidin-3-yl)-4-((5-(4-(5-((7-(4-(trifluoromethoxy)phenyl)pyrrolo[2,1-f][1,2,4]triazin-2-yl)amino)pyridin-2-yl)piperazin-1-yl)pentyl)oxy)isoindolin-1,3-dione;
(Compound No. 87) 4-((5-(4-(5-((7-(3,5-difluorophenyl)pyrrolo[2,1-f][1,2,4]triazine-2-yl)amino)pyridin-2-yl)piperazin-1-yl)pentyl)oxy)-2-(2,6-dioxopiperidin-3-yl)isoindolin-1,3-dione;
(Compound No. 88) 4-((5-(4-(5-((7-(2-aminopyridin-5-yl)pyrrolo[2,1-f][1,2,4]triazine-2-yl)amino)pyridin-2-yl)piperazin-1-al)pentyl)oxy)-2-(2,6-dioxopiperidin-3-yl)isoindolin-1,3-dione;
(Compound No. 89) 2-(2,6-dioxopiperidin-3-yl)-4-((5-(4-(5-((7-(quinolin-3-yl)pyrrolo[2,1-fl[1,2,4]triazin-2-yl)amino)pyridin-2-yl)piperazin-1-yl)pentyl)oxy)isoindoline-1,3-dione;
(Compound No. 90) *tert*-butyl4-(3-(2-((6-(4-(5-((2-(2,6-dioxopiperidin-3-yl)-1,3-dioxoisoindolin-4-yl)oxy)pentyl)piperazin-1-yl)pyridin-3-yl)amino)pyrrolo[2,1-f][1,2,4]triazin-7-yl)benzyl)piperazin-1-carboxylate;
(Compound No. 91) (E)-2-(2,6-dioxopiperidin-3-yl)-4-((5-(4-(5-((7-styrylpyrrolo[2,1-f][1,2,4]triazin-2-yl)amino)pyridin-2-yl)piperazin-1-yl)pentyl)oxy)isoindolin-1,3-dione;
(Compound No. 92) *tert*-butyl4-(4-(2-((6-(4-(5-((2-(2,6-dioxopiperidin-3-yl)-1,3-dioxoisoindolin-4-yl)oxy)pentyl)piperazin-1-yl)pyridin-3-yl)amino)pyrrolo[2,1-f][1,2,4]triazin-7-yl)-1*H*-pyrazol-1-yl)piperidin-1-carboxylate;
(Compound No. 93) 4-((5-(4-(5-((7-(4-(dimethylamino)phenyl)pyrrolo[2,1-f][1,2,4]triazine-2-yl)amino)pyridin-2-yl) piperazin-1-yl)pentyl)oxy)-2-(2,6-dioxopiperidin-3-yl)isoindolin-1,3-dione;
(Compound No. 94) 2-(2,6-dioxopiperidin-3-yl)-4-((5-(4-(5-((7-(pyridin-4-yl)pyrrolo[2,1-f][1,2,4]triazin-2-yl)amino)pyridin-2-yl)piperazin-1-yl)pentyl)oxy)isoindolin-1,3-dione;
(Compound No. 95) 2-(2,6-dioxopiperidin-3-yl)-4-((5-(4-(5-((7-(thiazol-5-yl)pyrrolo[2,1-f][1,2,4]triazin-2-yl)amino)pyridin-2-yl)piperazin-1-yl)pentyl)oxy)isoindolin-1,3-dione;
(Compound No. 96) 2-(2,6-dioxopiperidin-3-yl)-4-((5-(4-(5-((7-(1-(tetrahydro-2*H*-pyran-2 -yl)-1*H*-pyrazol-5-yl)pyrrolo[2,1-f][1,2,4]triazin-2-yl)amino)pyridin-2-yl)piperazin-1-yl)pentyl)oxy)isoindolin-1,3-dione;
(Compound No. 97) 4-((5-(4-(5-((7-((3,5-difluorophenyl)ethynyl)pyrrolo[2,1-f][1,2,4]triazin-2-yl)amino)pyridin-2-yl)piperazin-1-yl)pentyl)oxy)-2-(2,6-dioxopiperidin-3-yl)isoindolin-1,3-dione;
(Compound No. 98) 2-(2,6-dioxopiperidin-3-yl)-4-((5-(4-(5-((7-((3-methoxyphenyl)ethynyl)pyrrolo[2,1-f][1,2,4]triazin-2-yl)amino)aminopyridin-2-yl)piperazin-1-yl)pentyl)oxy)isoindolin-1,3-dione;
(Compound No. 99) *N*-(3-(2-((4-(4-(2-(2-(3-(4-((4-(2-(2,6-dioxopiperidin-3-yl)-6-fluoro-1,3-dioxoisoindolin-5-yl)piperazin-1-yl)methyl)piperidin-1-yl)-3-oxopropoxy)ethoxy)ethyl)piperazin-1-yl)phenyl) amino)pyrrolo[2,1-f][1,2,4]triazin-7-yl)phenyl)methane sulfonamide;
(Compound No. 100) *N*-(3-(4-(3-(2,4-dihydroxy-5-isopropylphenyl)-5-hydroxy-4*H*-1,2,4-triazol-4-yl)phenoxy)propyl)-3-(2-(2-(4-(4-((7-(3-(methylsulfonamido)phenyl)pyrrolo[2,1-f][1,2,4]triazin-2-yl)amino)phenyl)piperazin-1-yl)ethoxy)ethoxy)propanamide;
(Compound No. 101) *N*-(2-((3-(4-(3-(2,4-dihydroxy-5-isopropylphenyl)-5-hydroxy-4*H*-1,2,4-triazol-4-yl)phenoxy)propyl)amino)-2-oxoethyl)-3-(2-(2-(4-(4-((7-(3-(methylsulfonamido)phenyl)pyrrolo[2,1-fl[1,2,4]triazin-2-yl)amino)phenyl)piperazin-1-yl)ethoxy)ethoxy)propanamide;
(Compound No. 102) *N*-(3-(2-((4-(4-(2-(2-(3-(4-(4-(3-(2,4-dihydroxy-5-isopropylphenyl)-5-hydroxy-4*H*-1,2,4-triazol-4-yl)benzyl)piperazin-1-yl)-3-oxopropoxy)ethoxy)ethyl)piperazine-1-yl)phenyl)amino)pyrrolo[2,1-f][1,2,4] triazin-7-yl)phenyl)methanesulfonamide;
(Compound No. 103) *N*-(3-(2-((4-(4-(2-(2-(3-(4-((4-(4-(3-(2,4-dihydroxy-5-isopropylphenyl)-5-hydroxy-4*H*-1,2,4-triazol-4-yl)benzyl)piperazin-1-yl)methyl)piperidin-1-yl)-3-oxopropoxy)ethoxy)ethyl) piperazin-1-yl)phenyl)amino)pyrrolo[2,1-f][1,2,4]triazin-7-yl)phenyl) methanesulfonamide;
(Compound No. 104) *N*-(3-(2-((4-(4-(2-(2-(3-(4-((2-(2,4-dihydroxy-5-isopropylbenzoyl)isoindolin-5-yl)methyl)piperazin-1-yl)-3-oxopropoxy) ethoxy)ethyl)piperazin-1-yl)phenyl)amino)pyrrolo[2,1-f][1,2,4]triazin-7-yl)phenyl)methanesulfonamide;
(Compound No. 105) *N*-(3-(2-((4-(4-(2-(2-(3-(4-((4-((2-(2,4-dihydroxy-5 -isopropylbenzoyl)isoindolin-5-yl)methyl)piperazin-1-yl)methyl)piperidin-1-yl)-3-oxopropoxy)ethoxy)ethyl)piperazin-1-yl)phenyl)amino)pyrrolo[2,1-f][1,2,4]triazin-7-yl)phenyl)methanesulfonamide;
(Compound No. 106) 2-amino-4-(2,4-dichloro-5-(2-(4-(3-(2-(2-(4-(4-((7-(3-(methylsulfonamido)phenyl)pyrrolo[2,1-f][1,2,4]triazin-2-yl)amino) phenyl)piperazin-1-yl)ethoxy)ethoxy)propanoyl)piperazin-1-yl)ethoxy)phenyl)-*N*-ethylthieno[2,3-d]pyrimidin-6-carboxamide;
(Compound No. 107) *N*-(3-(2-((4-(4-(4-((4-((2-(2,4-dihydroxy-5-isopropylbenzoyl)isoindolin-5-yl)methyl)piperazin-1-yl)methyl)benzoic)piperazin-1-yl)phenyl)amino)pyrrolo[2,1-f][1,2,4]triazine-7-1)phenyl)methanesulfonamide;
Compound No. 108) *N*-(3-(2-((4-(4-(1-((2-(2,4-dihydroxy-5-isopropylbenzoic)isoindolin-5-yl)methyl)piperidin-4-carbonyl)piperazin-1-yl) phenyl)amino)pyrrolo[2,1-f][1,2,4]triazin-7-yl)phenyl)methanesulfonamide
(Compound No. 109) 4-(2-((2¹Z,4⁴E)-4¹*H*-11-oxa-3,8-diaza-2(7,2)-pyrrolo[2,1-f][1,2,4]triazin-4(4,1)-pyrazolo-1(1,4)-benzenacycloundecaphan -8-yl)ethoxy)-2-(2,6-dioxopiperidin-3-1)isoindolin-1,3-dione;
(Compound No. 110) 4-(3-((2¹Z,4⁴E)-4¹*H*-11-oxa-3,8-diaza-2(7,2)-pyrrolo[2,1-f][1,2,4]triazin-4(4,1)-pyrazolo-1(1,4)-benzenacycloundecaphan -8-yl)propoxy)-2-(2,6-dioxopiperidin-3-yl)isoindolin-1,3-dione;
(Compound No. 111) 4-(2-(2-((2¹Z,4⁴E)-4¹*H*-11-oxa-3,8-diaza-2(7,2)-pyrrolo[2,1-f][1,2,4]triazin-4(4,1)-pyrazolo-1(1,4)-benzenacycloundecaphan -8-yl)ethoxy)ethoxy)-2-(2-oxopiperidin-3-yl)isoindolin-1,3-dione;
(Compound No. 112) 4-((6-((2¹Z,4⁴E)-4¹*H*-11-oxa-3,8-diaza-2(7,2)-pyrrolo[2,1-f][1,2,4]triazin-4(4,1)-pyrazol-1(1,4)-benzenacycloundecaphan - 8-yl)hexyl)oxy)-2-(2,6-dioxopiperidin-3-yl)isoindolin-1,3-dione;
(Compound No. 113) 4-((8-((2¹Z,4⁴E)-4¹*H*-11-oxa-3,8-diaza-2(7,2)-pyrrolo[2,1-f][1,2,4]triazin-4(4,1)-pyrazol-1(1,4)-benzenacycloundecaphan - 8-yl)octyl)oxy)-2-(2,6-dioxopiperidin-3-1)isoindolin-1,3-dione;
(Compound No. 114) 4-((8-((2¹Z,4⁴E)-4¹*H*-11-oxa-3,8-diaza-2(7,2)-pyrrolo[2,1-f][1,2,4]triazin-4(4,1)-pyrazol-1(1,4)-benzenacycloundecaphan - 8-yl)octyl)oxy)-2-(2-oxopiperidin-3-yl)isoindolin-1,3-dione;
(Compound No. 115) 4-((11-((2¹Z,⁴⁴E)-4¹*H*-11-oxa-3,8-diaza-2(7,2)-pyrrolo[2,1-f][1,2,4]triazin-4(4,1)-pyrazol-1(1,4)-benzenacycloundecaphan - 8-yl)undecyl)oxy)-2-(2,6-dioxopiperidin-3-yl)isoindolin-1,3-dione;
(Compound No. 116) 4-((9-((2¹Z,4⁴E)-4¹*H*-11-oxa-3,8-diaza-2(7,2)-pyrrolo[2,1-f][1,2,4]triazin-4(4,1)-pyrazola-1(1,4)-benzenacycloundecaphan -8-yl)nonyl)oxy)-2-(2,6-dioxopiperidin-3-yl)isoindolin-1,3-dione;
(Compound No. 117) 5-(4-((1-(3-(2-(2-((2¹Z,4⁴E)-4¹*H*-11-oxa-3,8-diaza-2(7,2)-pyrrolo[2,1-f][1,2,4]triazin-4(4,1)-pyrazola-1(1,4)-benzenacycloundecaphan-8-yl)ethoxy)ethoxy))propanoyl)piperidin-4-yl)methyl)piperazin-1-yl)-2-(2,6-dioxopiperidin-3-yl)-6-fluoroisoindolin-1, 3-dione;
(Compound No. 118) 3-(2-(2-((2¹Z,4⁴E)-4¹H-11-oxa-3,8-diaza-2(7,2)-pyrrolo[2,1-f][1,2,4]triazin-4(4,1)-pyrazola-1(1,4)-benzenacycloundecaphan -8-yl)ethoxy)ethoxy)-*N*-(3-(4-(3-(2,4-dihydroxy-5-isopropylphenyl)-5-hydroxy-4*H*-1,2,4-triazol-4-yl)phenoxy)propyl)propanamide;
(Compound No. 119) 3-(2-(2-((2¹Z,4⁴E)-4¹*H*-11-oxa-3,8-diaza-2(7,2)-pyrrolo[2,1-f][1,2,4]triazin-4(4,1)-pyrazol-1(1,4)-benzenacycloundecaphan-8-yl)ethoxy)ethoxy)-*N*-(2-((3-(4-(3-(2,4-dihydroxy-5-isopropylphenyl)-5-hydroxy-4*H*-1,2,4-triazol-4-yl)phenoxy)propyl)amino)-2-oxoethyl)propanamide;
(Compound No. 120) 3-(2-(2-((2¹Z,4⁴E)-4¹*H*-11-oxa-3,8-diaza-2(7,2)-pyrrolo[2,1-f][1,2,4]triazin-4(4,1)-pyrazolo-1(1,4)-benzenacycloundecaphan -8-yl)ethoxy)ethoxy)-1-(4-(4-(3-(2,4-dihydroxy-5-isopropylphenyl)-5-hydroxy-4*H*-1,2,4-triazol-4-yl)benzyl)piperazin-1-yl)propan-1-one;
(Compound No. 121) 3-(2-(2-((2¹Z,4⁴E)-4¹*H*-11-oxa-3,8-diaza-2(7,2)-pyrrolo[2,1-f][1,2,4]triazin-4(4,1)-pyrazolo-1(1,4)-benzenacycloundecaphan-8-yl)ethoxy)ethoxy)-1-(4-((4-(4-(3-(2,4-dihydroxy-5-isopropylphenyl)-5-hydroxy-4*H*-1,2,4-triazol-4-yl)benzyl)piperazin-1-yl)methyl)piperidin-1-yl)propan-1-one;
(Compound No. 122) 3-(2-(2-((2¹Z,4⁴E)-4¹*H*-11-oxa-3,8-diaza-2(7,2)-pyrrolo[2,1-f][1,2,4]triazin-4(4,1)-pyrazol-1(1,4)-benzenacycloundecaphan-8-yl)ethoxy)ethoxy)-1-(4-((2-(2,4-dihydroxy-5-isopropylbenzoyl)isoindolin-5-yl)methyl)piperazin-1-yl)propan-1-one;
(Compound No. 123) 3-(2-(2-((2¹Z,4⁴E)-4¹*H*-11-oxa-3,8-diaza-2(7,2)-pyrrolo[2,1-f][1,2,4]triazin-4(4,1)-pyrazola-1(1,4)-benzenacycloundecaphan-8-yl)ethoxy)ethoxy)-1-(4-((4-((2-(2,4-dihydroxy-5-isopropylbenzoyl)isoindolin-5-yl)methyl)piperazin-1-yl)methyl)piperidin-1-yl)propan-1-one;
(Compound No. 124) 4-(5-(2-(4-(3-(2-(2-((2¹Z,4⁴E)-4¹*H*-11-oxa-3,8-diaza-2(7,2)-pyrrolo[2,1-f][1,2,4]triazin-4(4,1)-pyrazola-1(1,4)-benzenacycloundecaphan-8-yl)ethoxy)ethoxy)propanoyl)piperazin-1-yl)ethoxy)-2,4-dichlorophenyl)-2-amino-*N*-ethylthieno[2,3-d]pyrimidin-6-carboxamide;
(Compound No. 125) ((2¹Z,4⁴E)-4¹*H*-11-oxa-3,8-diaza-2(7,2)-pyrrolo[2,1-f][1,2,4]triazin-4(4,1)-pyrazola-1(1,4)-benzenacycloundecaphan-8-yl)(4-((4-((2-(2,4-dihydroxy-5-isopropylbenzoyl)isoindolin-5-yl)methyl)piperazin-1-yl) methyl)phenyl)methanone;
(Compound No. 126) ((2¹Z,4⁴E)-4¹*H*-11-oxa-3,8-diaza-2(7,2)-pyrrolo[2,1-f][1,2,4]triazin-4(4,1)-pyrazola-1(1,4)-benzenacycloundecaphane-8-yl)(1-((2-(2,4-dihydroxy-5-isopropylbenzoyl)isoindolin-5-yl)methyl)piperidin-4-yl)methanone.

12. The 2,7-substituted pyrrolo[2,1-f][1,2,4]triazine compound represented by Chemical Formula 1 according to claim 1, a pharmaceutically acceptable salt thereof, a hydrate thereof, or a solvate thereof, wherein the pharmaceutically acceptable salt is salt of inorganic acids or organic acids selected from the group consisting of hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, hydroxymaleic acid, benzoic acid, hydroxybenzoic acid, phenylacetic acid, salicylic acid, methanesulfonic acid, benzenesulfonic acid, and toluenesulfonic acid.

13. A pharmaceutical composition, for use in prevention, alleviation, or treatment of cancer, comprising the compound according to any one of claims 1 to 12 as an effective ingredient.

14. The pharmaceutical composition according to claim 13, for use in prevention, alleviation, or treatment of cancer, which is a disease induced by abnormal cell growth due to protein kinase activity.

15. The pharmaceutical composition according to claim 13, for use in prevention, alleviation, or treatment of cancer, comprising the compound with inhibition efficacy of over 80% against protein kinases at 1 µM.

16. The pharmaceutical composition according to claim 15, for use in prevention, alleviation, or treatment of cancer, wherein one or more of the protein kinases selected from the group consisting of ABL1, ABL2, ALK, ARK5, Aurora A, Aurora B, Aurora C, AXL, BLK, BMX, BRK, c-Kit, c-MER, c-Src, CAMK2a, CAMK2d, CDK16, , CDK17, CDK18, CDK2/cyclin A, CDK2/cyclin O, CDK4/cyclin D1, CDK4/cyclin D2, CDK4/cyclin D3, CDK6/cyclin D1, CDK6/cyclin D3, CDK7/cyclin H, CDK9/cyclin K, CDK9/cyclin T1, CDK9/cyclin T2, CK2a, CK2a2, CLK1, CLK2, CLK4, DAPK1, DAPK2, DDR1, DDR2, DRAK1, DYRK1/DYRK1A, DYRK1B, DYRK3, EPHA1, EPHA2, EPHA4, EPHA5, EPHA6, EPHA7, EPHB1, EPHB2, ERBB4/HER4, ERK7/MAPK15, ERN2/IRE2, FAK/PTK2, FER, FES/FPS, FGFR1, FGFR2, FGFR3, FGR, FLT1/VEGFR1, FLT3, FLT4/VEGFR3, FMS, FRK/PTK5, FYN, GLK/MAP4K3, GSK3a, HIPK2, HIPK3, HIPK4, HPK1/MAP4K1, IGF1R, IKKe/IKBKE, IR, IRAK1, IRR/INSRR, JAK1, JAK2, JAK3, JNK1, JNK2, JNK3, KDR/VEGFR2, KHS/MAP4K5, LCK, LIMK1, LIMK2, LRRK2, LYN, LYN B, MAK, MARK1, MARK2/PAR-1Ba, MARK3, MARK4, MAST3, MEKK2, MELK, MLCK2/MYLK2, MLK1/MAP3K9, MLK2/MAP3K10, MLK3/MAP3K11, MUSK, MYLK4, NEK1, NEK2, NEK5, NEK9, p70S6K/RPS6KB1, PAK4, PAK5, PAK6, PDGFRa, PDGFRb, PHKg1, PHKg2, PKAcb, PKCa, PKCb2, PKCg, PKCmu/PRKD1, PKCnu/PRKD3, PKD2/PRKD2, PKN1/PRK1, PLK4/SAK, PRKX, PYK2, RET, ROS/ROS1, RSK2, RSK3, RSK4, SIK1, SIK2, SLK/STK2, SNARK/NUAK2, STK16, STK22D/TSSK1, STK33, STK38/NDR1, STK38L/NDR2, STK39/STLK3, SYK, TBK1, TEC, TIE2/TEK, TLK2, TNK1, TRKA, TRKB, TRKC, TYK2, TYRO3/SKY, ULK1, ULK2, ULK3, WEE1, YES/YES1, YSK4/MAP3K19 and ZIPK/DAPK3.

17. The pharmaceutical composition according to claim 15, for use in prevention, alleviation, or treatment of cancer, wherein one or more of the protein kinases selected from the group consisting of CDK, NEK, Wee1, and GCN2.

18. The pharmaceutical composition according to claim 14, for use in prevention, alleviation, or treatment of cancer, wherein the diseases selected from the group consisting of stomach cancer, lung cancer, liver cancer, colon cancer, rectal cancer, pancreatic cancer, brain cancer, bone cancer, melanoma, breast cancer, sclerotic adenosis, uterine cancer, cervical cancer, oral cancer, esophageal cancer, thyroid cancer, parathyroid cancer, renal cancer, sarcoma, prostate cancer, ureter cancer, bladder cancer, leukemia, multiple myeloma, myelodysplastic syndrome, hematologic malignancies such as Hodgkin's disease and non-Hodgkin's lymphoma, and fibrosarcoma.
